# EUROPEAN PATENT SPECIFICATION

(11) **EP 2 195 432 B1**
(45) Date of publication and mention of the grant of the patent: **09.12.2015**
(21) Application number: 08834337.1
(22) Date of filing: 24.09.2008
(51) Int. Cl.: C12N 15/29, A01H 5/00, A01H 5/10, C12N 15/11, C12N 15/82

(54) **PLANTS HAVING INCREASED BIOMASS**
PFLANZEN MIT ERHÖHTER BIOMASSE
PLANTES AYANT UNE BIOMASSE ACCRUE

(30) Priority: 24.09.2007 US 974623 P
(43) Date of publication of application: 16.06.2010
(73) Proprietor: Performance Plants, Inc., Kingston, ON K7M 3X9 (CA)
(72) Inventor: WAN, Jiangxin, Bath Ontario K0H 1G0 (CA); HUANG, Yafan, Bath Ontario K0H 1G0 (CA); ZHANG, Zhiyong, Kingston Ontario K7L 2Z8 (CA)
(74) Representative: Cooley (UK) LLP
(86) International application number: PCT/IB2008/002931
(87) International publication number: WO 2009/040665

(56) References cited:
- US-A1- 2004 214 272
- US-A1- 2007 011 783
- DATABASE GENBANK [Online] 16 February 2005 TOBIAS, C.M. ET AL.: '5207_C01_F01 Switchgrass callus cDNA library Panicum virgatum cDNA clone 5207_C01_F01 5-, mRNA sequence', XP008147390 Database accession no. DN 151984
- AUSIN, I. ET AL.: 'Regulation of flowering time by FVE, a retinoblastoma-associated protein.' NAT. GENET. vol. 36, no. 2, February 2004, pages 162 - 166, XP008147392
- SALEHI, H. ET AL.: 'Delay in flowering and increase in biomass of transgenic tobacco expressing the Arabidopsis floral repressor gene FLOWERING LOCUS C.' J. PLANT PHYSIOL. vol. 162, no. 6, June 2005, pages 711 - 717, XP025312850
- SANDA, S.L. ET AL.: 'Interaction of FLC and late-flowering mutations in Arabidopsis thaliana.' MOL. GEN. GENET. vol. 251, no. 1, 24 April 1996, pages 69 - 74, XP002142417
- DATABASE GENBANK [Online] 08 June 2006 'Arabidopsis thaliana FVE (FVE) (FVE) mRNA, complete cds', XP008147396 Database accession no. AF375790NM-127510
- KO JIUNN-LIANG ET AL: "A new fungal immunomodulatory protein, FIP-fve isolated from the edible mushroom, Flammulina velutipes and its complete amino acid sequence", EUROPEAN JOURNAL OF BIOCHEMISTRY, vol. 228, no. 2, 1995, pages 244-249, ISSN: 0014-2956
- E. De Paoli ET AL: "Distinct extremely abundant siRNAs associated with cosuppression in petunia", RNA, vol. 15, no. 11, 1 November 2009 (2009-11-01), pages 1965-1970, XP55129213, ISSN: 1355-8382, DOI: 10.1261/rna.1706109
- SALEHI H ET AL: "Delay in flowering and increase in biomass of transgenic tobacco expressing the Arabidopsis floral repressor gene FLOWERING LOCUS C", JOURNAL OF PLANT PHYSIOLOGY, URBAN UND FISCHER VERLAG, DE, vol. 162, no. 6, 14 June 2005 (2005-06-14) , pages 711-717, XP025312850, ISSN: 0176-1617, DOI: 10.1016/J.JPLPH.2004.12.002 [retrieved on 2005-06-14]
- H. Vaucheret: "Post-transcriptional small RNA pathways in plants: mechanisms and regulations", Genes & Development, vol. 20, no. 7, 1 January 2006 (2006-01-01), pages 759-771, XP055129216, ISSN: 0890-9369, DOI: 10.1101/gad.1410506

### SEQUENCE LISTING

<110> Performance Plants Inc.
<120> Plants Having Increased Biomass
<130> 22542-015-001WO
<150> 60/974,623
   <151> 2007-09-24
<160> 379
<170> PatentIn version 3.5
<210> 1
   <211> 1581
   <212> DNA
   <213> ARABIDOPSIS THALIANA
<400> 1
   atggagagcg acgaagcagc agcagtgtct cctcaagcaa cgacaccgag cggaggaacc      60
   ggagcttctg ggccgaagaa gagaggtcgg aaacctaaaa ccaaggaaga ttctcagacg     120
   ccgtcgtctc agcaacagag cgatgttaaa atgaaagaaa gtgggaagaa aacgcagcag     180
   tcgccgagtg ttgacgagaa gtactctcag tggaaaggtc tcgtccccat tctctacgac     240
   tggctcgcta accataacct cgtctggcct tcactctctt gcagatgggg tccgcagctt     300
   gagcaagcaa cctacaagaa tcgccagcgt ctgtacctct cagagcaaac tgatggaagt     360
   gtgcccaata ctttggtcat agcaaattgt gaagttgtta agccaagggt tgctgcagca     420
   gagcacattt ctcagttcaa tgaagaagca cgttctccat ttgtgaagaa gtacaagacc     480
   atcattcacc ctggagaggt taacagaatc agggaactcc cacagaatag taagattgtt     540
   gctactcaca ccgacagtcc tgattcaaag agcatccttt gttcggacat atgtccagtt     600
   tcagagttat ctaaatacaa tatgtccagt ttggatgttg aaacccaacc aaaccgtcat     660
   gctgtgcttg gagctgcaaa ttcccgtcca gatttgatac taactgggca ccaagataat     720
   gctgaatttg ctcttgccat gtgcccaacg gaaccctttg tgctctccgg aggcaaggac     780
   aagtcagttg ttttgtggag tatccaagat cacatcacaa cgattgggac agattccaaa     840
   tcatctggat ctatcatcaa acagactggt gaaggtactg ataagaatga gagtcctact     900
   gttggcccac gaggtgtata tcatggccat gaagatacag ttgaagatgt ggcattcagc     960
   ccgacgagtg cacaagaatt ctgcagtgtt ggtgatgatt cttgccttat actatgggat    1020
   gcgagaactg gcacaaaccc tgtcacgaag gttgaaaaag cgcatgatgc tgatcttcat    1080
   tgtgttgatt ggaatcctca tgacgacaat ctgatcctga cagggtcagc agacaacact    1140
   gtccggttgt ttgatcgtag gaagcttacc gctaatggag ttggttcgcc tatttacaaa    1200
   tttgagggac acaaagctgc tgttctttgt gttcagtggt ctcctgataa gtcatccgtc    1260
   tttgggagct ctgcagaaga tggtctcttg aacatctggg attatgacag ggtcagtaag    1320
   aagtctgatc gtgcagctaa aagccccgct gggctcttct tccagcatgc tggtcacagg    1380
   gacaaagttg ttgatttcca ctggaatgct tcagaccctt ggactattgt cagtgtttct    1440
   gatgactgtg agactactgg tggaggtgga acattgcaga tatggcggat gagtgacttg    1500
   atttacagac cagaagaaga agtcgtggca gaattggaga agttcaagtc gcatgttatg    1560
   acttgtgcct ccaagcctta a                                              1581
<210> 2
   <211> 526
   <212> PRT
   <213> ARABIDOPSIS THALIANA
<400> 2
   Met Glu Ser Asp Glu Ala Ala Ala Val Ser Pro Gln Ala Thr Thr Pro
   1               5                   10                  15
   Ser Gly Gly Thr Gly Ala Ser Gly Pro Lys Lys Arg Gly Arg Lys Pro
               20                  25                  30
   Lys Thr Lys Glu Asp Ser Gln Thr Pro Ser Ser Gln Gln Gln Ser Asp
           35                  40                  45
   Val Lys Met Lys Glu Ser Gly Lys Lys Thr Gln Gln Ser Pro Ser Val
       50                  55                  60
   Asp Glu Lys Tyr Ser Gln Trp Lys Gly Leu Val Pro Ile Leu Tyr Asp
   65                  70                  75                  80
   Trp Leu Ala Asn His Asn Leu Val Trp Pro Ser Leu Ser Cys Arg Trp
                   85                  90                  95
   Gly Pro Gln Leu Glu Gln Ala Thr Tyr Lys Asn Arg Gln Arg Leu Tyr
               100                 105                 110
   Leu Ser Glu Gln Thr Asp Gly Ser Val Pro Asn Thr Leu Val Ile Ala
           115                 120                 125
   Asn Cys Glu Val Val Lys Pro Arg Val Ala Ala Ala Glu His Ile Ser
       130                 135                 140
   Gln Phe Asn Glu Glu Ala Arg Ser Pro Phe Val Lys Lys Tyr Lys Thr
   145                 150                 155                 160
   Ile Ile His Pro Gly Glu Val Asn Arg Ile Arg Glu Leu Pro Gln Asn
                   165                 170                 175
   Ser Lys Ile Val Ala Thr His Thr Asp Ser Pro Asp Ser Lys Ser Ile
               180                 185                 190
   Leu Cys Ser Asp Ile Cys Pro Val Ser Glu Leu Ser Lys Tyr Asn Met
           195                 200                 205
   Ser Ser Leu Asp Val Glu Thr Gln Pro Asn Arg His Ala Val Leu Gly
       210                 215                 220
   Ala Ala Asn Ser Arg Pro Asp Leu Ile Leu Thr Gly His Gln Asp Asn
   225                 230                 235                 240
   Ala Glu Phe Ala Leu Ala Met Cys Pro Thr Glu Pro Phe Val Leu Ser
                   245                 250                 255
   Gly Gly Lys Asp Lys Ser Val Val Leu Trp Ser Ile Gln Asp His Ile
               260                 265                 270
   Thr Thr Ile Gly Thr Asp Ser Lys Ser Ser Gly Ser Ile Ile Lys Gln
           275                 280                 285
   Thr Gly Glu Gly Thr Asp Lys Asn Glu Ser Pro Thr Val Gly Pro Arg
       290                 295                 300
   Gly Val Tyr His Gly His Glu Asp Thr Val Glu Asp Val Ala Phe Ser
   305                 310                 315                 320
   Pro Thr Ser Ala Gln Glu Phe Cys Ser Val Gly Asp Asp Ser Cys Leu
                   325                 330                 335
   Ile Leu Trp Asp Ala Arg Thr Gly Thr Asn Pro Val Thr Lys Val Glu
               340                 345                 350
   Lys Ala His Asp Ala Asp Leu His Cys Val Asp Trp Asn Pro His Asp
           355                 360                 365
   Asp Asn Leu Ile Leu Thr Gly Ser Ala Asp Asn Thr Val Arg Leu Phe
       370                 375                 380
   Asp Arg Arg Lys Leu Thr Ala Asn Gly Val Gly Ser Pro Ile Tyr Lys
   385 390 395 400
   Phe Glu Gly His Lys Ala Ala Val Leu Cys Val Gln Trp Ser Pro Asp
   405 410 415
   Lys Ser Ser Val Phe Gly Ser Ser Ala Glu Asp Gly Leu Leu Asn Ile
   420 425 430
   Trp Asp Tyr Asp Arg Val Ser Lys Lys Ser Asp Arg Ala Ala Lys Ser
   435 440 445
   Pro Ala Gly Leu Phe Phe Gln His Ala Gly His Arg Asp Lys Val Val
   450 455 460
   Asp Phe His Trp Asn Ala Ser Asp Pro Trp Thr Ile Val Ser Val Ser
   465 470 475 480
   Asp Asp Cys Glu Thr Thr Gly Gly Gly Gly Thr Leu Gln Ile Trp Arg
   485 490 495
   Met Ser Asp Leu Ile Tyr Arg Pro Glu Glu Glu Val Val Ala Glu Leu
   500 505 510
   Glu Lys Phe Lys Ser His Val Met Thr Cys Ala Ser Lys Pro
   515 520 525
<210> 3
   <211> 1524
   <212> DNA
   <213> ARABIDOPSIS THALIANA
<400> 3
   atggagagcg acgaagcagc agcagtgtct cctcaagcaa cgacaccgag cggaggaacc     60
   ggagcttctg ggccgaagaa gagaggtcgg aaacctaaaa ccaaggaaga ttctcagacg    120
   ccgtcgtctc agcaacagag cgatgttaaa atgaaagaaa gtgggaagaa aacgcagcag    180
   tcgccgagtg ttgacgagaa gtactctcag tggaaaggtc tcgtccccat tctctacgac    240
   tggctcgcta accataacct cgtctggcct tcactctctt gcagatgggg tccgcagctt    300
   gagcaagcaa cctacaagaa tcgccagcgt ctgtacctct cagagcaaac tgatggaagt    360
   gtgcccaata ctttggtcat agcaaattgt gaagttgtta agccaagggt tgctgcagca    420
   gagcacattt ctcagttcaa tgaagaagca cgttctccat ttgtgaagaa gtacaagacc    480
   atcattcacc ctggagaggt taacagaatc agggaactcc cacagaatag taagattgtt    540
   gctactcaca ccgacagtcc tgatgttctc atttgggatg ttgaaaccca accaaaccgt    600
   catgctgtgc ttggagctgc aaattcccgt ccagatttga tactaactgg gcaccaagat    660
   aatgctgaat ttgctcttgc catgtgccca acggaaccct ttgtgctctc cggaggcaag    720
   gacaagtcag ttgttttgtg gagtatccaa gatcacatca caacgattgg gacagattcc    780
   aaatcatctg gatctatcat caaacagact ggtgaaggta ctgataagaa tgagagtcct    840
   actgttggcc cacgaggtgt atatcatggc catgaagata cagttgaaga tgtggcattc    900
   agcccgacga gtgcacaaga attctgcagt gttggtgatg attcttgcct tatactatgg    960
   gatgcgagaa ctggcacaaa ccctgtcacg aaggttgaaa aagcgcatga tgctgatctt   1020
   cattgtgttg attggaatcc tcatgacgac aatctgatcc tgacagggtc agcagacaac   1080
   actgtccggt tgtttgatcg taggaagctt accgctaatg gagttggttc gcctatttac   1140
   aaatttgagg gacacaaagc tgctgttctt tgtgttcagt ggtctcctga taagtcatcc   1200
   gtctttggga gctctgcaga agatggtctc ttgaacatct gggattatga cagggtcagt   1260
   aagaagtctg atcgtgcagc taaaagcccc gctgggctct tcttccagca tgctggtcac   1320
   agggacaaag ttgttgattt ccactggaat gcttcagacc cttggactat tgtcagtgtt   1380
   tctgatgact gtgagactac tggtggaggt ggaacattgc agatatggcg gatgagtgac   1440
   ttgatttaca gaccagaaga agaagtcgtg gcagaattgg agaagttcaa gtcgcatgtt   1500
   atgacttgtg cctccaagcc ttaa                                          1524
<210> 4
   <211> 507
   <212> PRT
   <213> ARABIDOPSIS THALIANA
<400> 4
   Met Glu Ser Asp Glu Ala Ala Ala Val Ser Pro Gln Ala Thr Thr Pro
   1               5                   10                  15
   Ser Gly Gly Thr Gly Ala Ser Gly Pro Lys Lys Arg Gly Arg Lys Pro
               20                  25                  30
   Lys Thr Lys Glu Asp Ser Gln Thr Pro Ser Ser Gln Gln Gln Ser Asp
           35                  40                  45
   Val Lys Met Lys Glu Ser Gly Lys Lys Thr Gln Gln Ser Pro Ser Val
       50                  55                  60
   Asp Glu Lys Tyr Ser Gln Trp Lys Gly Leu Val Pro Ile Leu Tyr Asp
   65                  70                  75                  80
   Trp Leu Ala Asn His Asn Leu Val Trp Pro Ser Leu Ser Cys Arg Trp
                   85                  90                  95
   Gly Pro Gln Leu Glu Gln Ala Thr Tyr Lys Asn Arg Gln Arg Leu Tyr
               100                 105                 110
   Leu Ser Glu Gln Thr Asp Gly Ser Val Pro Asn Thr Leu Val Ile Ala
           115                 120                 125
   Asn Cys Glu Val Val Lys Pro Arg Val Ala Ala Ala Glu His Ile Ser
       130                 135                 140
   Gln Phe Asn Glu Glu Ala Arg Ser Pro Phe Val Lys Lys Tyr Lys Thr
   145                 150                 155                 160
   Ile Ile His Pro Gly Glu Val Asn Arg Ile Arg Glu Leu Pro Gln Asn
                   165                 170                 175
   Ser Lys Ile Val Ala Thr His Thr Asp Ser Pro Asp Val Leu Ile Trp
               180                 185                 190
   Asp Val Glu Thr Gln Pro Asn Arg His Ala Val Leu Gly Ala Ala Asn
           195                 200                 205
   Ser Arg Pro Asp Leu Ile Leu Thr Gly His Gln Asp Asn Ala Glu Phe
       210                 215                 220
   Ala Leu Ala Met Cys Pro Thr Glu Pro Phe Val Leu Ser Gly Gly Lys
   225                 230                 235                 240
   Asp Lys Ser Val Val Leu Trp Ser Ile Gln Asp His Ile Thr Thr Ile
                   245                 250                 255
   Gly Thr Asp Ser Lys Ser Ser Gly Ser Ile Ile Lys Gln Thr Gly Glu
               260                 265                 270
   Gly Thr Asp Lys Asn Glu Ser Pro Thr Val Gly Pro Arg Gly Val Tyr
           275                 280                 285
   His Gly His Glu Asp Thr Val Glu Asp Val Ala Phe Ser Pro Thr Ser
       290                 295                 300
   Ala Gln Glu Phe Cys Ser Val Gly Asp Asp Ser Cys Leu Ile Leu Trp
   305                 310                 315                 320
   Asp Ala Arg Thr Gly Thr Asn Pro Val Thr Lys Val Glu Lys Ala His
   325 330 335
   Asp Ala Asp Leu His Cys Val Asp Trp Asn Pro His Asp Asp Asn Leu
   340 345 350
   Ile Leu Thr Gly Ser Ala Asp Asn Thr Val Arg Leu Phe Asp Arg Arg
   355 360 365
   Lys Leu Thr Ala Asn Gly Val Gly Ser Pro Ile Tyr Lys Phe Glu Gly
   370 375 380
   His Lys Ala Ala Val Leu Cys Val Gln Trp Ser Pro Asp Lys Ser Ser
   385 390 395 400
   Val Phe Gly Ser Ser Ala Glu Asp Gly Leu Leu Asn Ile Trp Asp Tyr
   405 410 415
   Asp Arg Val Ser Lys Lys Ser Asp Arg Ala Ala Lys Ser Pro Ala Gly
   420 425 430
   Leu Phe Phe Gln His Ala Gly His Arg Asp Lys Val Val Asp Phe His
   435 440 445
   Trp Asn Ala Ser Asp Pro Trp Thr Ile Val Ser Val Ser Asp Asp Cys
   450 455 460
   Glu Thr Thr Gly Gly Gly Gly Thr Leu Gln Ile Trp Arg Met Ser Asp
   465 470 475 480
   Leu Ile Tyr Arg Pro Glu Glu Glu Val Val Ala Glu Leu Glu Lys Phe
   485 490 495
   Lys Ser His Val Met Thr Cys Ala Ser Lys Pro
   500 505
<210> 5
   <211> 3763
   <212> DNA
   <213> ARABIDOPSIS THALIANA
<400> 5
   ccgaaaaatc ataatattgt ctatatgtaa atataaatat aaatatttta gttatattta       60
   tatttaagat atgtttgggt accagttcgg gttcgggttt ttcaggtttt gaagtttaga      120
   ttcagtcgga tatttgaaaa tttcaggttc gaatttagat tgggattttt ggattgggat      180
   ttttggatcg ggttcagatc ggttttttcg gattcaggta ttatgtccaa agtaaaaatg      240
   gtaaacggtg gttgttttgt ttgtatttgt aaagcagaag agagagagat atagagacac      300
   tgaaagcaaa gaccaaaaaa gaaaaattaa aaagagagag aggaaaatgg agagcgacga      360
   agcagcagca gtgtctcctc aagcaacgac accgagcgga ggaaccggag cttctgggcc      420
   gaagaagaga ggtcggaaac ctaaaaccaa ggaagattct cagacgccgt cgtctcagca      480
   acagagcgat gttaaaatga aagaaagtgg gaagaaaacg cagcagtcgc cgagtgttga      540
   cgagaagtac tctcagtgga aaggtctcgt ccccattctc tacgactggc tcgctaacca      600
   taacctcgtc tggccttcac tctcttgcag gtctccccct ttctcctttc ctctccttct      660
   agggtttcgt ttcgtaatcg tttcttagct ttgaacattc tcatgtttgg aatgaattta      720
   gtaaaatctt acacatacat tttctcgatt tctgggttta agtgagattg ttgcgattgt      780
   tctagttagg gttttggatg tggctctgtc ttcatacctt gatatatctg atgttctatt      840
   catgaattgt tactattgat taccttgttg gttactaatg actaagagga attttcagtt      900
   tctctgagtg tttatatctg atgaagtctt tagttgttgt gctaagagtt tccatttggt      960
   gaattgttgt ttgatttttt tatagatggg gtccgcagct tgagcaagca acctacaaga     1020
   atcgccagcg tctgtacctc tcagagcaag taagttttta gctttctctt gtatcttgtt     1080
   gtctcatctt ctttatatac ttctcatcgt attatttgta tttttcttgg ttgtgtcacc     1140
   agactgatgg aagtgtgccc aatactttgg tcatagcaaa ttgtgaagtt gttaagccaa     1200
   gggttgctgc agcagagcac atttctcagg tattatgtgg tttaatacta agcttgtgtc     1260
   ctttccatat cctactccac actacaattg gtttcatgtt tgacacttat atactatctt     1320
   ctgaaaatgt gttctcagtt caatgaagaa gcacgttctc catttgtgaa gaagtacaag     1380
   accatcattc accctggaga ggtgtgaatt ctgcccactc ttgagatatt tctgtattga     1440
   cattgttctt tttagttcta tttggtttgt taattgtatc tgcatccctg tttcatctgt     1500
   ccatgaagtt attcgtttgg cacgttggtg aaagtaaatt ttgatgtgta ttcattacta     1560
   atttgcaatt gcaggttaac agaatcaggg aactcccaca gaatagtaag attgttgcta     1620
   ctcacaccga cagtcctgat gtgagtgctg cttctatttt gttatggtca tagcaacttg     1680
   aaatatgtcg gtttcatatt tctgtatttg gcagtcaaag agcatccttt gttcggacat     1740
   atgtccagtt tcagagttat ctaaatacaa tatgtccagt ttggatgttg aaacccaacc     1800
   aaaccgtcat gctgtgcttg gagctgcaaa ttcccgtcca gatttggtat gtccacttct     1860
   gagaatgttg ttttatgctt tattcttgtt tgtttctcat cattggaagt gataaatctc     1920
   tttgatatct tcttaaatag tgcttcttgt ttgcatcatc tgaatgaacc atttttcatg     1980
   cagatactaa ctgggcacca agataatgct gaatttgctc ttgccatgtg cccaacggaa 2040
   ccctttgtgc tctccggagg tttgtgtttc tgtaatttgt agagtccaat cctgtggttt 2100
   gccagtttct catacaaaag ttcttctctt aggcaaggac aagtcagttg ttttgtggag 2160
   tatccaagat cacatcacaa cgattgggac agattccaaa tcatctggat ctatcatcaa 2220
   acagactggt gaaggtactg ataagaatga gagtcctact gttggcccac gaggtgtata 2280
   tcatggccat gaagatacag ttgaagatgt ggcattcagc ccgacgaggt aacttcttag 2340
   aacagactcc ttctattgat atcgtgtttg tttatgcata ctgcagatat tttcatgatt 2400
   ttctaataat acttctggtg aacttttata ccgtgaagtg cacaagaatt ctgcagtgtt 2460
   ggtgatgatt cttgccttat actatgggat gcgagaactg gcacaaaccc tgtcacgaag 2520
   gtactctatc ttttgaatcc tatcaaaagt ttgaagattt acctcctttt gatattatat 2580
   cttacttttt tgttttccag gttgaaaaag cgcatgatgc tgatcttcat tgtgttgatt 2640
   ggaatcctca tgacgacaat ctgatcctga cagggtatgg agaaatacat acaaatagat 2700
   gattaataca tacttagtat ctaattaaga aattgatgaa tatttcaggt cagcagacaa 2760
   cactgtccgg ttgtttgatc gtaggaagct taccgctaat ggagttggtt cgcctattta 2820
   caaatttgag ggacacaaag ctgctgttct ttgtgttcag gtataatcaa cttttttttt 2880
   tttttccttt ctttgtatga agtatatctc ttaacccact gacactatct tgttattcaa 2940
   ttcagtggtc tcctgataag tcatccgtct ttgggagctc tgcagaagat ggtctcttga 3000
   acatctggga ttatgacagg gtgtgtacat agttcactca gatgtctaaa attaatcttt 3060
   cttcactatc atcactgaaa catattcatt gtactcatgt ttggtttgtt taattaacca 3120
   tcaggtcagt aagaagtctg atcgtgcagc taaaagcccc gctgggctct tcttccagca 3180
   tgctggtcac aggttcttaa agacttatct tgatttttct tgattgcttt ctcattttac 3240
   ttgcttctaa gttcccttgt ttataaacca tattagggac aaagttgttg atttccactg 3300
   gaatgcttca gacccttgga ctattgtcag tgtttctgat gactgtgaga ctactggtgg 3360
   aggtggaaca ttgcaggtaa ccttgaaatc tttcttggta ccttgataag caattttatt 3420
   gacataccgt taaatgttgt ttatactttc ttctatggca gatatggcgg atgagtgact 3480
   tgatttacag accagaagaa gaagtcgtgg cagaattgga gaagttcaag tcgcatgtta 3540
   tgacttgtgc ctccaagcct taagagtaaa gaaaacccat tgtctatcta tctatcgcct 3600
   atggtaaact aatgcgggtt ttagcgagga gtcttggttt ttgtaaggct ggtttgtctt 3660
   ttgagatatt ggtggtagct tttaggacct ttccatatca gttaggggta catggttctg 3720
   gttcatgatc ctgtttcatc agactcttag gtgctgtttt gtt 3763
<210> 6
   <211> 3776
   <212> DNA
   <213> ARABIDOPSIS THALIANA
<400> 6
   ccgaaaaatc ataatattgt ctatatgtaa atataaatat aaatatttta gttatattta      60
   tatttaagat atgtttgggt accagttcgg gttcgggttt ttcaggtttt gaagtttaga     120
   ttcagtcgga tatttgaaaa tttcaggttc gaatttagat tgggattttt ggattgggat     180
   ttttggatcg ggttcagatc ggttttttcg gattcaggta ttatgtccaa agtaaaaatg     240
   gtaaacggtg gttgttttgt ttgtatttgt aaagcagaag agagagagat atagagacac     300
   tgaaagcaaa gaccaaaaaa gaaaaattaa aaagagagag aggaaaatgg agagcgacga     360
   agcagcagca gtgtctcctc aagcaacgac accgagcgga ggaaccggag cttctgggcc     420
   gaagaagaga ggtcggaaac ctaaaaccaa ggaagattct cagacgccgt cgtctcagca     480
   acagagcgat gttaaaatga aagaaagtgg gaagaaaacg cagcagtcgc cgagtgttga     540
   cgagaagtac tctcagtgga aaggtctcgt ccccattctc tacgactggc tcgctaacca     600
   taacctcgtc tggccttcac tctcttgcag gtctccccct ttctcctttc ctctccttct     660
   agggtttcgt ttcgtaatcg tttcttagct ttgaacattc tcatgtttgg aatgaattta     720
   gtaaaatctt acacatacat tttctcgatt tctgggttta agtgagattg ttgcgattgt     780
   tctagttagg gttttggatg tggctctgtc ttcatacctt gatatatctg atgttctatt     840
   catgaattgt tactattgat taccttgttg gttactaatg actaagagga attttcagtt     900
   tctctgagtg tttatatctg atgaagtctt tagttgttgt gctaagagtt tccatttggt     960
   gaattgttgt ttgatttttt tatagatggg gtccgcagct tgagcaagca acctacaaga    1020
   atcgccagcg tctgtacctc tcagagcaag taagttttta gctttctctt gtatcttgtt   1080
   gtctcatctt ctttatatac ttctcatcgt attatttgta tttttcttgg ttgtgtcacc    1140
   agactgatgg aagtgtgccc aatactttgg tcatagcaaa ttgtgaagtt gttaagccaa    1200
   gggttgctgc agcagagcac atttctcagg tattatgtgg tttaatacta agcttgtgtc    1260
   ctttccatat cctactccac actacaattg gtttcatgtt tgacacttat atactatctt    1320
   ctgaaaatgt gttctcagtt caatgaagaa gcacgttctc catttgtgaa gaagtacaag    1380
   accatcattc accctggaga ggtgtgaatt ctgcccactc ttgagatatt tctgtattga    1440
   cattgttctt tttagttcta tttggtttgt taattgtatc tgcatccctg tttcatctgt    1500
   ccatgaagtt attcgtttgg cacgttggtg aaagtaaatt ttgatgtgta ttcattacta    1560
   atttgcaatt gcaggttaac agaatcaggg aactcccaca gaatagtaag attgttgcta 1620
   ctcacaccga cagtcctgat gtgagtgctg cttctatttt gttatggtca tagcaacttg 1680
   aaatatgtcg gtttcatatt tctgtatttg gcagtcaaag agcatccttt gttcggacat 1740
   atgtccagtt tcagagttat ctaaatacaa tatgttgatt tcaggttctc atttgggatg 1800
   ttgaaaccca accaaaccgt catgctgtgc ttggagctgc aaattcccgt ccagatttgg 1860
   tatgtccact tctgagaatg ttgttttatg ctttattctt gtttgtttct catcattgga 1920
   agtgataaat ctctttgata tcttcttaaa tagtgcttct tgtttgcatc atctgaatga 1980
   accatttttc atgcagatac taactgggca ccaagataat gctgaatttg ctcttgccat 2040
   gtgcccaacg gaaccctttg tgctctccgg aggtttgtgt ttctgtaatt tgtagagtcc 2100
   aatcctgtgg tttgccagtt tctcatacaa aagttcttct cttaggcaag gacaagtcag 2160
   ttgttttgtg gagtatccaa gatcacatca caacgattgg gacagattcc aaatcatctg 2220
   gatctatcat caaacagact ggtgaaggta ctgataagaa tgagagtcct actgttggcc 2280
   cacgaggtgt atatcatggc catgaagata cagttgaaga tgtggcattc agcccgacga 2340
   ggtaacttct tagaacagac tccttctatt gatatcgtgt ttgtttatgc atactgcaga 2400
   tattttcatg attttctaat aatacttctg gtgaactttt ataccgtgaa gtgcacaaga 2460
   attctgcagt gttggtgatg attcttgcct tatactatgg gatgcgagaa ctggcacaaa 2520
   ccctgtcacg aaggtactct atcttttgaa tcctatcaaa agtttgaaga tttacctcct 2580
   tttgatatta tatcttactt ttttgttttc caggttgaaa aagcgcatga tgctgatctt 2640
   cattgtgttg attggaatcc tcatgacgac aatctgatcc tgacagggta tggagaaata 2700
   catacaaata gatgattaat acatacttag tatctaatta agaaattgat gaatatttca 2760
   ggtcagcaga caacactgtc cggttgtttg atcgtaggaa gcttaccgct aatggagttg 2820
   gttcgcctat ttacaaattt gagggacaca aagctgctgt tctttgtgtt caggtataat 2880
   caactttttt tttttttttc cttctttgta tgaagtatat ctcttaaccc actgacacta 2940
   tcttgttatt caattcagtg gtctcctgat aagtcatccg tctttgggag ctctgcagaa 3000
   gatggtctct tgaacatctg ggattatgac agggtgtgta catagttcac tcagatgtct 3060
   aaaattaatc tttcttcact atcatcactg aaacatattc attgtactca tgtttggttt 3120
   gtttaattaa ccatcaggtc agtaagaagt ctgatcgtgc agctaaaagc cccgctgggc 3180
   tcttcttcca gcatgctggt cacaggttct taaagactta tcttgatttt tcttgattgc 3240
   tttctcattt tacttgcttc taagttccct tgtttataaa ccatattagg gacaaagttg 3300
   ttgatttcca ctggaatgct tcagaccctt ggactattgt cagtgtttct gatgactgtg 3360
   agactactgg tggaggtgga acattgcagg taaccttgaa atctttcttg gtaccttgat    3420
   aagcaatttt attgacatac cgttaaatgt tgtttatact ttcttctatg gcagatatgg    3480
   cggatgagtg acttgattta cagaccagaa gaagaagtcg tggcagaatt ggagaagttc    3540
   aagtcgcatg ttatgacttg tgcctccaag ccttaagagt aaagaaaacc cattgtctat    3600
   ctatctatcg cctatggtaa actaatgcgg gttttagcga ggagtcttgg tttttgtaag    3660
   gctggtttgt cttttgagat attggtggta gcttttagga cctttccata tcagttaggg    3720
   gtacatggtt ctggttcatg atcctgtttc atcagactct taggtgctgt tttgtt        3776
<210> 7
   <211> 1545
   <212> DNA
   <213> GOSSYPIUM HIRSUTUM
<400> 7
   atggagtctc ctcaacccca acaacagcaa caacaaggtc ctaagaagag aggtcgaaaa 60
   ccaaagccaa aggaagagaa agaacagcag caacaacaaa gtgctagtaa aatgaaagag 120
   ggaaagaaag ctcaccaacc tagcgttgat gagaagtaca ctcagtggaa gtctttggtt 180
   cctgttcttt atgactggct tgctaatcac aatctcgttt ggccttctct ttcttgccgc 240
   tggggaccac agcttgagca agctacttac aagaatcggc agcgtctcta cctctctgaa 300
   cagactgatg gtagtgttcc gaatactctt gtgattgcta attgtgaagt tgtcaaacct 360
   agggttgctg ctgctgagca catatctcag tttaatgaaa aaacacgctc accatttgtg 420
   aagaagtaca aaaccatcat acatcctgga gaagtcaaca gaatcaagga acttccacag 480
   agctctagga ttgtggcaac tcacactgat agtcctgatg ttcttatttg ggatgtcgag 540
   gctcaaccca accgccatgc ggttcttgga gcaacccatt ctcgcccaga tttgattttg 600
   actgggcatc aagataatgc tgaatttgct cttgcaatgt gtccaactga gccttatgtg 660
   ctctctggag ggaaggacaa atcagtggtt ttgtggagca tccaggacca cataacaaca 720
   atggctacag accctactaa atctcctgga tctggcggat caatcatcaa acaaaacaag 780
   cctggggaag gtaatgacaa agctgctgat gggccttctt taggtcctcg tggtgtcttc 840
   tgtgggcatg aggatacagt tgaagatgtt acattctgtc catcaagtgc acaagagttt 900
   tgtagtgtag gtgatgattc ctgcctcata ctatgggatg cacgagttgg cactggcccc 960
   actgtcaagg ttgaaaaggc acataatgct gatctccatt gtgttgattg gaacccccat 1020
   gatgacaatc ttatcctgac tgggtctgca gatcatactg tacgtatgtt tgatcgaaga 1080
   aatcttactt ctaatggaat tggatcaccc atttacaagt ttgagggtca taaagctgct 1140
   gttctatgcg tgcagtggtc tccagacaaa tcatctgtat ttggcagttc tgctgaggat 1200
   gggctcttga acatttggga ctatgacaag gttggcaaga aggtggaacg tgcttcaaga 1260
   tctcctagtg cttctgcggg actgtttttc cagcacgctg gacacaggga caaagttgtt 1320
   gacttccatt ggaatgcatt tgatccatgg actgttgtta gtgtgtttga tgactgtgaa 1380
   acaactggtg gaggagggac attgcaaata tggggcatga gtgatttgat ttataggccg 1440
   gaaaaggagg ttttagctta gctggagaaa ttcaagtccc atgtcatttt gtgggcgaca 1500
   aaggcattaa aaaggtttag tagccctaat ggtatgccta tttga 1545
<210> 8
   <211> 502
   <212> PRT
   <213> GOSSYPIUM HIRSUTUM
<220>
   <221> misc_feature
   <222> (487)..(487)
   <223> Xaa can be any naturally occurring amino acid
<400> 8
   Met Glu Ser Pro Gln Pro Gln Gln Gln Gln Gln Gln Gly Pro Lys Lys
   1               5                   10                  15
   Arg Gly Arg Lys Pro Lys Pro Lys Glu Glu Lys Glu Gln Gln Gln Gln
               20                  25                  30
   Gln Ser Ala Ser Lys Met Lys Glu Gly Lys Lys Ala His Gln Pro Ser
           35                  40                  45
   Val Asp Glu Lys Tyr Thr Gln Trp Lys Ser Leu Val Pro Val Leu Tyr
       50                  55                  60
   Asp Trp Leu Ala Asn His Asn Leu Val Trp Pro Ser Leu Ser Cys Arg
   65                  70                  75                  80
   Trp Gly Pro Gln Leu Glu Gln Ala Thr Tyr Lys Asn Arg Gln Arg Leu
                   85                  90                  95
   Tyr Leu Ser Glu Gln Thr Asp Gly Ser Val Pro Asn Thr Leu Val Ile
               100                 105                 110
   Ala Asn Cys Glu Val Val Lys Pro Arg Val Ala Ala Ala Glu His Ile
           115                 120                 125
   Ser Gln Phe Asn Glu Lys Thr Arg Ser Pro Phe Val Lys Lys Tyr Lys
       130                 135                 140
   Thr Ile Ile His Pro Gly Glu Val Asn Arg Ile Lys Glu Leu Pro Gln
   145                 150                 155                 160
   Ser Ser Arg Ile Val Ala Thr His Thr Asp Ser Pro Asp Val Leu Ile
                   165                 170                 175
   Trp Asp Val Glu Ala Gln Pro Asn Arg His Ala Val Leu Gly Ala Thr
               180                 185                 190
   His Ser Arg Pro Asp Leu Ile Leu Thr Gly His Gln Asp Asn Ala Glu
           195                 200                 205
   Phe Ala Leu Ala Met Cys Pro Thr Glu Pro Tyr Val Leu Ser Gly Gly
       210                 215                 220
   Lys Asp Lys Ser Val Val Leu Trp Ser Ile Gln Asp His Ile Thr Thr
   225                 230                 235                 240
   Met Ala Thr Asp Pro Thr Lys Ser Pro Gly Ser Gly Gly Ser Ile Ile
                   245                 250                 255
   Lys Gln Asn Lys Pro Gly Glu Gly Asn Asp Lys Ala Ala Asp Gly Pro
               260                 265                 270
   Ser Leu Gly Pro Arg Gly Val Phe Cys Gly His Glu Asp Thr Val Glu
           275                 280                 285
   Asp Val Thr Phe Cys Pro Ser Ser Ala Gln Glu Phe Cys Ser Val Gly
       290                 295                 300
   Asp Asp Ser Cys Leu Ile Leu Trp Asp Ala Arg Val Gly Thr Gly Pro
   305                 310                 315                 320
   Thr Val Lys Val Glu Lys Ala His Asn Ala Asp Leu His Cys Val Asp
                   325                 330                 335
   Trp Asn Pro His Asp Asp Asn Leu Ile Leu Thr Gly Ser Ala Asp His
               340                 345                 350
   Thr Val Arg Met Phe Asp Arg Arg Asn Leu Thr Ser Asn Gly Ile Gly
           355                 360                 365
   Ser Pro Ile Tyr Lys Phe Glu Gly His Lys Ala Ala Val Leu Cys Val
       370                 375                 380
   Gln Trp Ser Pro Asp Lys Ser Ser Val Phe Gly Ser Ser Ala Glu Asp
   385                 390                 395                 400
   Gly Leu Leu Asn Ile Trp Asp Tyr Asp Lys Val Gly Lys Lys Val Glu
                   405                 410                 415
   Arg Ala Ser Arg Ser Pro Ser Ala Ser Ala Gly Leu Phe Phe Gln His
               420                 425                 430
   Ala Gly His Arg Asp Lys Val Val Asp Phe His Trp Asn Ala Phe Asp
           435                 440                 445
   Pro Trp Thr Val Val Ser Val Phe Asp Asp Cys Glu Thr Thr Gly Gly
       450                 455                 460
   Gly Gly Thr Leu Gln Ile Trp Gly Met Ser Asp Leu Ile Tyr Arg Pro
   465                 470                 475                 480
   Glu Lys Glu Val Leu Ala Xaa Leu Glu Lys Phe Lys Ser His Val Ile
                   485                 490                 495
   Leu Trp Ala Thr Lys Ala
               500
<210> 9
   <211> 1542
   <212> DNA
   <213> GLYCINE MAX
<400> 9
   atggagactc ctcctcccca acaaggcgtc gtgaagaaga aggagacaag gggccgaaaa      60
   cccaaaccaa aggacgaaca cgggaagggt ttgaaggaag gtaggaaaac acaacaacaa     120
   caacaacaac aacaacaaca tcatcatcag cagcagcaac agcaacaaga tcaaccttcg     180
   gtggacgaga aatacacgca gtggaagtcc cttgttcctg tcctctacga ctggctcgcc     240
   aaccacaacc tcgtctggcc ctctctctct tgcaggtggg gcccccagct tgaacaagcc     300
   acttacaaga atcgccagag actctacctt tctgagcaga ctgatggtag tgtgccgaat     360
   actctggtga ttgcgaattg cgaggttgtg aagcctaggg ttgctgctgc tgagcacatt     420
   tcgcagttta atgaagaggc gcggtcccca tttgtgaaga agtacaagac catcatacat     480
   cctggtgagg taaacagaat tagggaattg ccacaaaatt ccaagatagt ggctacacat     540
   acagacagcc ctgatgtcct tgtttgggat gttgaaagtc aacctaatcg ccatgctgtc     600
   cttggagcta caaactctcg tcctgatttg atattgactg gacaccaaga taatgcggaa     660
   tttgctcttg caatgtgccc aactgaaccc tatgttcttt caggaggaaa ggacaaaaca     720
   gtggtgttgt ggagtattga agaccatata acatctgctg ccacagactc caaatctggt     780
   gggtcaatta tcaaacaaaa ctctaaatct ggagaaggca atgataaaac tgctgatggc     840
   cctactgttg gaccacgagg catttattgt gggcatgagg atacagttga agatgtgact     900
   ttctgcccat ctagtgcaca ggagttctgt agtgttggag atgattcttg tctcatctta     960
   tgggatgcac gtgttggctc tagccctgtg gttaaggttg agaaagctca taatgctgat    1020
   cttcactgtg tggactggaa tccccatgat gataatctga ttcttactgg gtcagcagat    1080
   aattctgttc gcatgtttga tcgccgcaat ctcaccacta atggagttgg gtcacccatc    1140
   cataaatttg agggtcacaa agctgctgtt ctttgtgttc agtggtctcc agacaaatca    1200
   tctgtatttg gaagttcagc tgaagatggt ctcttaaaca tttgggacta tgagaaggtt    1260
   ggtaaaaaga tagagcgatc tggaaaatca ataagttctc ctccagggtt gttttttcaa    1320
   catgcaggtc atagggataa agttgttgac ttccattgga atgcatatga tccatggacg    1380
   attgttagtg tgtctgatga ctgtgaaagt actggaggag ggggaacgtt gcagatatgg    1440
   cgcatgagtg atttgatcta cagaccagaa gatgaggttt tggccgagct ggagaaattc    1500
   aaatctcatg ttgtggcgtg tgcttcaaag actgaaaaat ga                       1542
<210> 10
   <211> 498
   <212> PRT
   <213> GLYCINE MAX
<400> 10
   Lys Glu Thr Arg Gly Arg Lys Pro Lys Pro Lys Asp Glu His Gly Lys
   1               5                   10                  15
   Gly Leu Lys Glu Gly Arg Lys Thr Gln Gln Gln Gln Gln Gln Gln Gln
               20                  25                  30
   Gln His His His Gln Gln Gln Gln Gln Gln Gln Asp Gln Pro Ser Val
           35                  40                  45
   Asp Glu Lys Tyr Thr Gln Trp Lys Ser Leu Val Pro Val Leu Tyr Asp
       50                  55                  60
   Trp Leu Ala Asn His Asn Leu Val Trp Pro Ser Leu Ser Cys Arg Trp
   65                  70                  75                  80
   Gly Pro Gln Leu Glu Gln Ala Thr Tyr Lys Asn Arg Gln Arg Leu Tyr
                   85                  90                  95
   Leu Ser Glu Gln Thr Asp Gly Ser Val Pro Asn Thr Leu Val Ile Ala
               100                 105                 110
   Asn Cys Glu Val Val Lys Pro Arg Val Ala Ala Ala Glu His Ile Ser
           115                 120                 125
   Gln Phe Asn Glu Glu Ala Arg Ser Pro Phe Val Lys Lys Tyr Lys Thr
       130                 135                 140
   Ile Ile His Pro Gly Glu Val Asn Arg Ile Arg Glu Leu Pro Gln Asn
   145                 150                 155                 160
   Ser Lys Ile Val Ala Thr His Thr Asp Ser Pro Asp Val Leu Val Trp
                   165                 170                 175
   Asp Val Glu Ser Gln Pro Asn Arg His Ala Val Leu Gly Ala Thr Asn
               180                 185                 190
   Ser Arg Pro Asp Leu Ile Leu Thr Gly His Gln Asp Asn Ala Glu Phe
           195                 200                 205
   Ala Leu Ala Met Cys Pro Thr Glu Pro Tyr Val Leu Ser Gly Gly Lys
       210                 215                 220
   Asp Lys Thr Val Val Leu Trp Ser Ile Glu Asp His Ile Thr Ser Ala
   225                 230                 235                 240
   Ala Thr Asp Ser Lys Ser Gly Gly Ser Ile Ile Lys Gln Asn Ser Lys
                   245                 250                 255
   Ser Gly Glu Gly Asn Asp Lys Thr Ala Asp Gly Pro Thr Val Gly Pro
               260                 265                 270
   Arg Gly Ile Tyr Cys Gly His Glu Asp Thr Val Glu Asp Val Thr Phe
           275                 280                 285
   Cys Pro Ser Ser Ala Gln Glu Phe Cys Ser Val Gly Asp Asp Ser Cys
       290                 295                 300
   Leu Ile Leu Trp Asp Ala Arg Val Gly Ser Ser Pro Val Val Lys Val
   305                 310                 315                 320
   Glu Lys Ala His Asn Ala Asp Leu His Cys Val Asp Trp Asn Pro His
   325 330 335
   Asp Asp Asn Leu Ile Leu Thr Gly Ser Ala Asp Asn Ser Val Arg Met
   340 345 350
   Phe Asp Arg Arg Asn Leu Thr Thr Asn Gly Val Gly Ser Pro Ile His
   355 360 365
   Lys Phe Glu Gly His Lys Ala Ala Val Leu Cys Val Gln Trp Ser Pro
   370 375 380
   Asp Lys Ser Ser Val Phe Gly Ser Ser Ala Glu Asp Gly Leu Leu Asn
   385 390 395 400
   Ile Trp Asp Tyr Glu Lys Val Gly Lys Lys Ile Glu Arg Ser Gly Lys
   405 410 415
   Ser Ile Ser Ser Pro Pro Gly Leu Phe Phe Gln His Ala Gly His Arg
   420 425 430
   Asp Lys Val Val Asp Phe His Trp Asn Ala Tyr Asp Pro Trp Thr Ile
   435 440 445
   Val Ser Val Ser Asp Asp Cys Glu Ser Thr Gly Gly Gly Gly Thr Leu
   450 455 460
   Gln Ile Trp Arg Met Ser Asp Leu Ile Tyr Arg Pro Glu Asp Glu Val
   465 470 475 480
   Leu Ala Glu Leu Glu Lys Phe Lys Ser His Val Val Ala Cys Ala Ser
   485 490 495
   Lys Thr
<210> 11
   <211> 1365
   <212> DNA
   <213> PETUNIA HYBRIDA
<400> 11
   atgaaggaga gaaccggaaa agggggtcag ccgtcggttg acgagaagta cacacagtgg        60
   aagtctctcg ttcccgttct ctatgactgg cttgctaacc ataacctcgt atggccatca       120
   ctttcctgca gatgggggcc tctggttgag caagcaactt acaagaatcg ccaacgactt       180
   tacctctcag agcagactga tggaagtgtg ccaaacactt tggttatagc aaattgtgaa       240
   gttgttaaac ctagggttgc agctgcagag cacatatcac agttcaatga agaatcacga       300
   tctccttttg tcaagaagta caaaaccatt atacacccag gggaggttaa cagaatcaga       360
   gagcttcctc aaaataaaaa catagtggca acccatactg atagtcctga agttctaatt       420
   tgggatgtgg aagcccaacc taatcgacat gctgttcttg gagctgttgc ttcacgccca       480
   gatttgacat tgattggaca tagtgagaat gcagaatttg cactggcaat gtgccccact       540
   gaaccctttg tgctctctgg aggaaaggac aaatctgtgg tactgtggag tattcaagat       600
   catatatcaa cattgagcac agatgcacaa aaacctgctg gtttcatcaa gccagccact       660
   acatctatta aggctggtga taatccctct attcaagctc gtggaatctt ccaaggccat       720
   gaggataccg ttgaagatgt tcagttctgc ccatcaagtt cacaggaatt ctgtagtgtt       780
   ggtgatgatt catgtctcat tctatgggat gctcgagttg gtactagtcc agttgtaaag       840
   gttgaaaaag ctcataatgc tgatctccac tgtgttgact ggaatcctca tgatggcaac       900
   tttattatca ctggatctgc agataattct gtccgcttgt ttgatcgacg tagtttaact       960
   tcgaatggag ttggctcacc agtccatatc tttgaaaatc acaaagcagc agttctttgt      1020
   gtgcagtggt gtccagacag gtcctctgta tttgggagta ctgcagagga tggtcgtttg      1080
   aatatttggg attacgataa ggttggtgag aaggacaatg agacacctgc tccaggttta      1140
   tttttccagc atgctgggca cagggataaa attgttgact tccactggaa tgtggctgat      1200
   ccatggacaa ttgtaagtgt atctgatgac tgtgactcca caggtggagg tggtacacta      1260
   cagatatggc ggatgttgga tttgctgtat cgtcctgaag aggaggctct ggccgagctt      1320
   caaaagttca aatcccatgt aagcaagtgc gctcgcaaaa tctga                      1365
<210> 12
   <211> 454
   <212> PRT
   <213> PETUNIA HYBRIDA
<400> 12
   Met Lys Glu Arg Thr Gly Lys Gly Gly Gln Pro Ser Val Asp Glu Lys
   1               5                   10                  15
   Tyr Thr Gln Trp Lys Ser Leu Val Pro Val Leu Tyr Asp Trp Leu Ala
               20                  25                  30
   Asn His Asn Leu Val Trp Pro Ser Leu Ser Cys Arg Trp Gly Pro Leu
           35                  40                  45
   Val Glu Gln Ala Thr Tyr Lys Asn Arg Gln Arg Leu Tyr Leu Ser Glu
       50                  55                  60
   Gln Thr Asp Gly Ser Val Pro Asn Thr Leu Val Ile Ala Asn Cys Glu
   65                  70                  75                  80
   Val Val Lys Pro Arg Val Ala Ala Ala Glu His Ile Ser Gln Phe Asn
                   85                  90                  95
   Glu Glu Ser Arg Ser Pro Phe Val Lys Lys Tyr Lys Thr Ile Ile His
               100                 105                 110
   Pro Gly Glu Val Asn Arg Ile Arg Glu Leu Pro Gln Asn Lys Asn Ile
           115                 120                 125
   Val Ala Thr His Thr Asp Ser Pro Glu Val Leu Ile Trp Asp Val Glu
       130                 135                 140
   Ala Gln Pro Asn Arg His Ala Val Leu Gly Ala Val Ala Ser Arg Pro
   145                 150                 155                 160
   Asp Leu Thr Leu Ile Gly His Ser Glu Asn Ala Glu Phe Ala Leu Ala
                   165                 170                 175
   Met Cys Pro Thr Glu Pro Phe Val Leu Ser Gly Gly Lys Asp Lys Ser
               180                 185                 190
   Val Val Leu Trp Ser Ile Gln Asp His Ile Ser Thr Leu Ser Thr Asp
           195                 200                 205
   Ala Gln Lys Pro Ala Gly Phe Ile Lys Pro Ala Thr Thr Ser Ile Lys
       210                 215                 220
   Ala Gly Asp Asn Pro Ser Ile Gln Ala Arg Gly Ile Phe Gln Gly His
   225                 230                 235                 240
   Glu Asp Thr Val Glu Asp Val Gln Phe Cys Pro Ser Ser Ser Gln Glu
                   245                 250                 255
   Phe Cys Ser Val Gly Asp Asp Ser Cys Leu Ile Leu Trp Asp Ala Arg
               260                 265                 270
   Val Gly Thr Ser Pro Val Val Lys Val Glu Lys Ala His Asn Ala Asp
           275                 280                 285
   Leu His Cys Val Asp Trp Asn Pro His Asp Gly Asn Phe Ile Ile Thr
       290                 295                 300
   Gly Ser Ala Asp Asn Ser Val Arg Leu Phe Asp Arg Arg Ser Leu Thr
   305                 310                 315                 320
   Ser Asn Gly Val Gly Ser Pro Val His Ile Phe Glu Asn His Lys Ala
                   325                 330                 335
   Ala Val Leu Cys Val Gln Trp Cys Pro Asp Arg Ser Ser Val Phe Gly
               340                 345                 350
   Ser Thr Ala Glu Asp Gly Arg Leu Asn Ile Trp Asp Tyr Asp Lys Val
           355                 360                 365
   Gly Glu Lys Asp Asn Glu Thr Pro Ala Pro Gly Leu Phe Phe Gln His
       370                 375                 380
   Ala Gly His Arg Asp Lys Ile Val Asp Phe His Trp Asn Val Ala Asp
   385                 390                 395                 400
   Pro Trp Thr Ile Val Ser Val Ser Asp Asp Cys Asp Ser Thr Gly Gly
                   405                 410                 415
   Gly Gly Thr Leu Gln Ile Trp Arg Met Leu Asp Leu Leu Tyr Arg Pro
               420                 425                 430
   Glu Glu Glu Ala Leu Ala Glu Leu Gln Lys Phe Lys Ser His Val Ser
           435                 440                 445
   Lys Cys Ala Arg Lys Ile
       450
<210> 13
   <211> 1548
   <212> DNA
   <213> PISUM SATIVUM
<400> 13
   atggagactc ctccgtcgca gcaaggcgta gtaaagaaga aggaaactcg aggtcgaaaa      60
   cctaaaccaa aagacgatca acaacaaaca cctgccaaaa cactgaaaga agcgaaaaag     120
   gcgcaactac aacagcagca acaacagcaa ctgcagcaac agcagcaaca acaacaacac     180
   caagcttcgg tagacgagaa atacactcaa tggaagtctc tcgttcccgt tctctacgat     240
   tggcttgcga atcataacct tgtttggcct tctctctctt gcaggtgggg tcctcagctt     300
   gaacaagcta cttataagaa tcggcagcgg ctttatcttt ctgagcagac tgatggtagt     360
   gtcccaaata ctttggtgat tgcgaattgt gaggttgtga aaactagggt tgcagctgca     420
   gagcatattt cacagtttaa tgaggaggct cgctctccat ttgttaagaa gtacaagacc     480
   atcatacatc ccggggaggt gaacagaatt agggaattgc cgcaaaattc taagatagtg     540
   gctactcaca cagacagccc tgatgttctc atttgggatg ttgaaagtca acctaaccgt     600
   cacgctgtcc ttggagccac aaactctcgt ccagatttga tattgaccgg acaccaagac     660
   aatgctgagt ttgctcttgc gatgtgccca actgagcctt atgtcctttc aggaggaaaa     720
   gataaaacag tggtgttgtg gagtattgaa gaccatgtaa catctgctgc tacggacaag     780
   tctggtggat ccattatcaa accgaactct aaatctgggg aaggcaatga caaaactgtt     840
   gatagccctt ctgtcgggcc aagaggtatc tactctgggc acgatgatac tgttgaagat     900
   gtggcctttt gcccttctag tgcgcaggaa ttctgtagtg ttggagatga ttcttgtctc     960
   atattatggg atgcacgtgt tggctctagt cctgttgtta aggttgaaaa agctcataat    1020
   gccgatcttc actgtgttga ctggaatccc catgatgata atctgattct tactgggtcg    1080
   gcagataatt ctgttcgctt gtttgatcgg cgcaatctca cctctaatgg ggttgggtct    1140
   cctattcata aatttgaggc tcataaagct gccgttcttt gtgttcagtg gtctccagac    1200
   aaatcatctg tatttggaag ttcagcagaa gacggtctct taaacatttg ggattatgag    1260
   aaggttggta aaaagattga gcgagctgga aaaacaataa actctcctcc agggttgttt    1320
   ttccaacatg ctggtcatag agacaaagtt gttgacttcc attggaatgc ttatgatcca    1380
   tggacaattg taagtgtgtc tgatgattgt gaaagtactg gtggaggagg aacattgcag    1440
   atatggcgca tgagtgattt gctctataga ccagaggatg aggttttggc agagttggag    1500
   aaattcaaat ctcacgtggt ggcttgtgct gcaaagaccg atacataa                 1548
<210> 14
   <211> 515
   <212> PRT
   <213> PISUM SATIVUM
<400> 14
   Met Glu Thr Pro Pro Ser Gln Gln Gly Val Val Lys Lys Lys Glu Thr
   1               5                   10                  15
   Arg Gly Arg Lys Pro Lys Pro Lys Asp Asp Gln Gln Gln Thr Pro Ala
               20                  25                  30
   Lys Thr Leu Lys Glu Ala Lys Lys Ala Gln Leu Gln Gln Gln Gln Gln
   35 40 45
   Gln Gln Leu Gln Gln Gln Gln Gln Gln Gln Gln His Gln Ala Ser Val
   50 55 60
   Asp Glu Lys Tyr Thr Gln Trp Lys Ser Leu Val Pro Val Leu Tyr Asp
   65 70 75 80
   Trp Leu Ala Asn His Asn Leu Val Trp Pro Ser Leu Ser Cys Arg Trp
   85 90 95
   Gly Pro Gln Leu Glu Gln Ala Thr Tyr Lys Asn Arg Gln Arg Leu Tyr
   100 105 110
   Leu Ser Glu Gln Thr Asp Gly Ser Val Pro Asn Thr Leu Val Ile Ala
   115 120 125
   Asn Cys Glu Val Val Lys Thr Arg Val Ala Ala Ala Glu His Ile Ser
   130 135 140
   Gln Phe Asn Glu Glu Ala Arg Ser Pro Phe Val Lys Lys Tyr Lys Thr
   145 150 155 160
   Ile Ile His Pro Gly Glu Val Asn Arg Ile Arg Glu Leu Pro Gln Asn
   165 170 175
   Ser Lys Ile Val Ala Thr His Thr Asp Ser Pro Asp Val Leu Ile Trp
   180 185 190
   Asp Val Glu Ser Gln Pro Asn Arg His Ala Val Leu Gly Ala Thr Asn
   195 200 205
   Ser Arg Pro Asp Leu Ile Leu Thr Gly His Gln Asp Asn Ala Glu Phe
   210 215 220
   Ala Leu Ala Met Cys Pro Thr Glu Pro Tyr Val Leu Ser Gly Gly Lys
   225 230 235 240
   Asp Lys Thr Val Val Leu Trp Ser Ile Glu Asp His Val Thr Ser Ala
   245 250 255
   Ala Thr Asp Lys Ser Gly Gly Ser Ile Ile Lys Pro Asn Ser Lys Ser
   260 265 270
   Gly Glu Gly Asn Asp Lys Thr Val Asp Ser Pro Ser Val Gly Pro Arg
   275 280 285
   Gly Ile Tyr Ser Gly His Asp Asp Thr Val Glu Asp Val Ala Phe Cys
   290 295 300
   Pro Ser Ser Ala Gln Glu Phe Cys Ser Val Gly Asp Asp Ser Cys Leu
   305 310 315 320
   Ile Leu Trp Asp Ala Arg Val Gly Ser Ser Pro Val Val Lys Val Glu
   325 330 335
   Lys Ala His Asn Ala Asp Leu His Cys Val Asp Trp Asn Pro His Asp
   340 345 350
   Asp Asn Leu Ile Leu Thr Gly Ser Ala Asp Asn Ser Val Arg Leu Phe
   355 360 365
   Asp Arg Arg Asn Leu Thr Ser Asn Gly Val Gly Ser Pro Ile His Lys
   370 375 380
   Phe Glu Ala His Lys Ala Ala Val Leu Cys Val Gln Trp Ser Pro Asp
   385 390 395 400
   Lys Ser Ser Val Phe Gly Ser Ser Ala Glu Asp Gly Leu Leu Asn Ile
   405 410 415
   Trp Asp Tyr Glu Lys Val Gly Lys Lys Ile Glu Arg Ala Gly Lys Thr
   420 425 430
   Ile Asn Ser Pro Pro Gly Leu Phe Phe Gln His Ala Gly His Arg Asp
   435 440 445
   Lys Val Val Asp Phe His Trp Asn Ala Tyr Asp Pro Trp Thr Ile Val
   450 455 460
   Ser Val Ser Asp Asp Cys Glu Ser Thr Gly Gly Gly Gly Thr Leu Gln
   465 470 475 480
   Ile Trp Arg Met Ser Asp Leu Leu Tyr Arg Pro Glu Asp Glu Val Leu
   485 490 495
   Ala Glu Leu Glu Lys Phe Lys Ser His Val Val Ala Cys Ala Ala Lys
   500 505 510
   Thr Asp Thr
   515
<210> 15
   <211> 1503
   <212> DNA
   <213> POPULUS
<400> 15
   atggaacctg ctgccactac tgctcaagtg ccaaagaaga gaggccgaaa accgaagccc      60
   aaagaagatc aacagcagca gcagcagcag caaagtgctg gtgcccgaat gaaagaggga     120
   aggaaggcac agcaaccctc tattgatgac aagtacactc agtggaagtc tttggttcct     180
   gttctttatg actggctagc caatcacaac ctcgtctggc catctctctc ttgccggtgg     240
   ggcccacagc ttgagcaagc tacttacaag aatcgccagc gcctctatct ctccgagcag     300
   actgatggta gtgttccaaa tactttggtc attgcaaatt gtgatgttgt caagtctaga     360
   gttgctgctg cggaacacat atctcagttt aatgaagaag cacgctctcc atttgttaag     420
   aagtacaaga ccatcataca tcctggagag gtaaacagaa tcagagaact cccccagaat     480
   agtaagatag tggctactca tactgacagc cctgatgttc ttatatggga tgttgaagca     540
   cagcctaacc gccatgctgt tcttggagct acaaattctc gtccagattt gattttgact     600
   ggacatcaag acaatgctga gtttgccctt gcaatgtgcc caactgatcc ctatgtgctt     660
   tctggaggga aggacaagtt cgtagttttg tggagtatcc aggaccatat aacatcatct     720
   gcctctgatc cagcaactaa gtctccagga tctggtggat caatcattaa aaagactggg     780
   gatggcagtg ataaagccac tgatggccct tctgttggac cgcgaggtat ctaccaaggg     840
   catgaggata cagttgaaga cgtggcattc tgtccatcca gtgcgcagga gttttgtagt     900
   gtaggagatg attcttgcct tatattatgg gatgctagag ctggcactag cccagctata     960
   aaggttgaaa gggcacataa tgctgatctt cactgtgttg attggaatcc tcaagatgat    1020
   aatctcattt taactgggtc tgcagatact tctgtttgca tgtttgatcg aaggaatctc    1080
   acttctaatg gagttggttt gccagtctat aaatttgagg gtcacaaagc tgctgttctc    1140
   tgtgtacagt ggtctccaga taaggcatct gtttttggga gttctgcgga ggatggtctc    1200
   ttgaatattt gggattatga gaaggttggc aaaaagacag agcgtccgac tagagctcca    1260
   agttctcctg caggattgtt tttccagcat gctgggcaca gggataaagt tgttgatttc    1320
   cattggaatg catctgatcc ttggacggtg gttagtgtct ctgatgactg tgataccact    1380
   ggcgggggag ggacactgca gatatggcgc atgagtgatt tgatctacag gccagaagat    1440
   gaggttttgg cagagcttga gaagttcaag tcacatgtgg tttcctgtgc ttcgaagcct    1500
   tga                                                                  1503
<210> 16
   <211> 500
   <212> PRT
   <213> POPULUS
<400> 16
   Met Glu Pro Ala Ala Thr Thr Ala Gln Val Pro Lys Lys Arg Gly Arg
   1               5                   10                  15
   Lys Pro Lys Pro Lys Glu Asp Gln Gln Gln Gln Gln Gln Gln Gln Ser
               20                  25                  30
   Ala Gly Ala Arg Met Lys Glu Gly Arg Lys Ala Gln Gln Pro Ser Ile
           35                  40                  45
   Asp Asp Lys Tyr Thr Gln Trp Lys Ser Leu Val Pro Val Leu Tyr Asp
       50                  55                  60
   Trp Leu Ala Asn His Asn Leu Val Trp Pro Ser Leu Ser Cys Arg Trp
   65                  70                  75                  80
   Gly Pro Gln Leu Glu Gln Ala Thr Tyr Lys Asn Arg Gln Arg Leu Tyr
                   85                  90                  95
   Leu Ser Glu Gln Thr Asp Gly Ser Val Pro Asn Thr Leu Val Ile Ala
               100                 105                 110
   Asn Cys Asp Val Val Lys Ser Arg Val Ala Ala Ala Glu His Ile Ser
           115                 120                 125
   Gln Phe Asn Glu Glu Ala Arg Ser Pro Phe Val Lys Lys Tyr Lys Thr
       130                 135                 140
   Ile Ile His Pro Gly Glu Val Asn Arg Ile Arg Glu Leu Pro Gln Asn
   145                 150                 155                 160
   Ser Lys Ile Val Ala Thr His Thr Asp Ser Pro Asp Val Leu Ile Trp
                   165                 170                 175
   Asp Val Glu Ala Gln Pro Asn Arg His Ala Val Leu Gly Ala Thr Asn
               180                 185                 190
   Ser Arg Pro Asp Leu Ile Leu Thr Gly His Gln Asp Asn Ala Glu Phe
           195                 200                 205
   Ala Leu Ala Met Cys Pro Thr Asp Pro Tyr Val Leu Ser Gly Gly Lys
   210 215 220
   Asp Lys Phe Val Val Leu Trp Ser Ile Gln Asp His Ile Thr Ser Ser
   225 230 235 240
   Ala Ser Asp Pro Ala Thr Lys Ser Pro Gly Ser Gly Gly Ser Ile Ile
   245 250 255
   Lys Lys Thr Gly Asp Gly Ser Asp Lys Ala Thr Asp Gly Pro Ser Val
   260 265 270
   Gly Pro Arg Gly Ile Tyr Gln Gly His Glu Asp Thr Val Glu Asp Val
   275 280 285
   Ala Phe Cys Pro Ser Ser Ala Gln Glu Phe Cys Ser Val Gly Asp Asp
   290 295 300
   Ser Cys Leu Ile Leu Trp Asp Ala Arg Ala Gly Thr Ser Pro Ala Ile
   305 310 315 320
   Lys Val Glu Arg Ala His Asn Ala Asp Leu His Cys Val Asp Trp Asn
   325 330 335
   Pro Gln Asp Asp Asn Leu Ile Leu Thr Gly Ser Ala Asp Thr Ser Val
   340 345 350
   Cys Met Phe Asp Arg Arg Asn Leu Thr Ser Asn Gly Val Gly Leu Pro
   355 360 365
   Val Tyr Lys Phe Glu Gly His Lys Ala Ala Val Leu Cys Val Gln Trp
   370 375 380
   Ser Pro Asp Lys Ala Ser Val Phe Gly Ser Ser Ala Glu Asp Gly Leu
   385 390 395 400
   Leu Asn Ile Trp Asp Tyr Glu Lys Val Gly Lys Lys Thr Glu Arg Pro
   405 410 415
   Thr Arg Ala Pro Ser Ser Pro Ala Gly Leu Phe Phe Gln His Ala Gly
   420 425 430
   His Arg Asp Lys Val Val Asp Phe His Trp Asn Ala Ser Asp Pro Trp
   435 440 445
   Thr Val Val Ser Val Ser Asp Asp Cys Asp Thr Thr Gly Gly Gly Gly
   450 455 460
   Thr Leu Gln Ile Trp Arg Met Ser Asp Leu Ile Tyr Arg Pro Glu Asp
   465 470 475 480
   Glu Val Leu Ala Glu Leu Glu Lys Phe Lys Ser His Val Val Ser Cys
   485 490 495
   Ala Ser Lys Pro
   500
<210> 17
   <211> 1389
   <212> DNA
   <213> VITIS VINIFERA
<400> 17
   atgaaggaga cgagcacagc cggcgagacg tcgatttcgg tggacgagag gtatacccag      60
   tggaagtctc tcgttccgac tgatggcagt gttccgaata ccttagttat agcaaactgt     120
   gaagttgtta aacctagggt agctgccgca gagcatattg ctcagttcaa cgaggaagcg     180
   cggtcaccct ttgtaaagaa gttcaaaaca attatacatc caggagaggt gaaccgaatc     240
   agggaactgc cacagaatag taagatagtg gccacacaca ctgacagtcc tgatgtcctc     300
   atttgggatg ttgaaactca acctaatcgt catgctgtat tgggaactcc agaatctcgt     360
   ccagatttgt tttggtttac attttcacta gctgtgaatt tacatgatgc atggagccca     420
   cccaacaaat cgggagaaag atttagtggc acctttgaac cccgtataac aggttctgct     480
   tatttagaga ctacagaggg gaaggttttg ttcctgatat taactgggca taaagataat     540
   gcagaatttg ctcttgccat gtgtccaact gaaccattgg tgctctctgg aggcaaggat     600
   aagtctgtgg tgttgtggag tattcaggat cacatctcga ctttggcagc agatccaggg     660
   tcagcaaaga gtacctctaa ggctggtggt ggtaatgata aacctgtaga aagcccttct     720
   ataggagcac gtggtatcta ccaagggcat gatgatactg ttgaagatgt gcaattctgc     780
   ccattaagcg cacaggagtt ctgtagcgta ggtgatgatt cttgccttat cttatgggat     840
   gcaagatctg gcaccactcc agccatcaag gtcgagaaag ctcataatgc tgatcttcac     900
   tgtgttgatt ggaatcctca tgacataaat cttattctaa ctggatcggc tgacaatact     960
   gttcgcatgt ttgatcgccg aaagcttaca tctggtggaa ttggatcacc aatccataca    1020
   tttgagggcc atactgctgc tgtcctttgt gtacagtggt ctccggacaa ggcttcgatc    1080
   tttgggagtt ctgcagaaga tggtatctta aacctctgga atcatgaaaa gattgataag    1140
   aagcaagctc ccaacgctcc tccaggttta ttcttcagac atgctggcca tagggataag    1200
   gtcgtggact tccattggaa tgcatcggat ccatggacaa tcgttagcgt atctgatgat    1260
   ggtgaaagta ctggtggagg cggtacactc cagatatggc ggatgatcga tctgatttac    1320
   aggaacgaag atgaggtgct ggctgagctc gataacttca aagctcattt ggccacatgc    1380
   tccccctga                                                            1389
<210> 18
   <211> 462
   <212> PRT
   <213> VITIS VINIFERA
<400> 18
   Met Lys Glu Thr Ser Thr Ala Gly Glu Thr Ser Ile Ser Val Asp Glu
   1               5                   10                  15
   Arg Tyr Thr Gln Trp Lys Ser Leu Val Pro Thr Asp Gly Ser Val Pro
               20                  25                  30
   Asn Thr Leu Val Ile Ala Asn Cys Glu Val Val Lys Pro Arg Val Ala
           35                  40                  45
   Ala Ala Glu His Ile Ala Gln Phe Asn Glu Glu Ala Arg Ser Pro Phe
       50                  55                  60
   Val Lys Lys Phe Lys Thr Ile Ile His Pro Gly Glu Val Asn Arg Ile
   65                  70                  75                  80
   Arg Glu Leu Pro Gln Asn Ser Lys Ile Val Ala Thr His Thr Asp Ser
                   85                  90                  95
   Pro Asp Val Leu Ile Trp Asp Val Glu Thr Gln Pro Asn Arg His Ala
               100                 105                 110
   Val Leu Gly Thr Pro Glu Ser Arg Pro Asp Leu Phe Trp Phe Thr Phe
           115                 120                 125
   Ser Leu Ala Val Asn Leu His Asp Ala Trp Ser Pro Pro Asn Lys Ser
       130                 135                 140
   Gly Glu Arg Phe Ser Gly Thr Phe Glu Pro Arg Ile Thr Gly Ser Ala
   145                 150                 155                 160
   Tyr Leu Glu Thr Thr Glu Gly Lys Val Leu Phe Leu Ile Leu Thr Gly
                   165                 170                 175
   His Lys Asp Asn Ala Glu Phe Ala Leu Ala Met Cys Pro Thr Glu Pro
               180                 185                 190
   Leu Val Leu Ser Gly Gly Lys Asp Lys Ser Val Val Leu Trp Ser Ile
           195                 200                 205
   Gln Asp His Ile Ser Thr Leu Ala Ala Asp Pro Gly Ser Ala Lys Ser
       210                 215                 220
   Thr Ser Lys Ala Gly Gly Gly Asn Asp Lys Pro Val Glu Ser Pro Ser
   225                 230                 235                 240
   Ile Gly Ala Arg Gly Ile Tyr Gln Gly His Asp Asp Thr Val Glu Asp
                   245                 250                 255
   Val Gln Phe Cys Pro Leu Ser Ala Gln Glu Phe Cys Ser Val Gly Asp
               260                 265                 270
   Asp Ser Cys Leu Ile Leu Trp Asp Ala Arg Ser Gly Thr Thr Pro Ala
           275                 280                 285
   Ile Lys Val Glu Lys Ala His Asn Ala Asp Leu His Cys Val Asp Trp
       290                 295                 300
   Asn Pro His Asp Ile Asn Leu Ile Leu Thr Gly Ser Ala Asp Asn Thr
   305                 310                 315                 320
   Val Arg Met Phe Asp Arg Arg Lys Leu Thr Ser Gly Gly Ile Gly Ser
                   325                 330                 335
   Pro Ile His Thr Phe Glu Gly His Thr Ala Ala Val Leu Cys Val Gln
               340                 345                 350
   Trp Ser Pro Asp Lys Ala Ser Ile Phe Gly Ser Ser Ala Glu Asp Gly
           355                 360                 365
   Ile Leu Asn Leu Trp Asn His Glu Lys Ile Asp Lys Lys Gln Ala Pro
       370                 375                 380
   Asn Ala Pro Pro Gly Leu Phe Phe Arg His Ala Gly His Arg Asp Lys
   385                 390                 395                 400
   Val Val Asp Phe His Trp Asn Ala Ser Asp Pro Trp Thr Ile Val Ser
   405 410 415
   Val Ser Asp Asp Gly Glu Ser Thr Gly Gly Gly Gly Thr Leu Gln Ile
   420 425 430
   Trp Arg Met Ile Asp Leu Ile Tyr Arg Asn Glu Asp Glu Val Leu Ala
   435 440 445
   Glu Leu Asp Asn Phe Lys Ala His Leu Ala Thr Cys Ser Pro
   450 455 460
<210> 19
   <211> 1272
   <212> DNA
   <213> SORGHUM BICOLOR
<220>
   <221> misc_feature
   <222> (723)..(723)
   <223> n is a, c, g, or t
<400> 19
   atgaaggaga gaagcggctc cagggcggcg gtggacgagc gctacgcgca gtggaagtcg        60
   ctcatccccg tcctctacga ctggtttgct aaccacaacc tcgtctggcc atccctctcc       120
   tgccggtggg ggccacagtt tgagaaagct acctacaaga atcgtcaacg cctttaccta       180
   tctgagcaga cggatgggag tgtgcctaat actctagtta tcgcaaactg tgaagttgtg       240
   aagccacggg ttgcagctgc tgaacatatc tcgcagttta acgaggaagc acgatcacct       300
   tttgttaaga agtacaagac catagttcat cctggtgaag ttaacagaat cagggagctt       360
   ccacagaaca gtaagatcat agccactcac actgacagtc cagatgtact tatttgggat       420
   gttgaagcac agccaaatag acatgccgtc ctaggagctt ctgaatctcg ccctgatctg       480
   atattaacgg gacataagga aaatgcggaa tttgcgcttg ctatgtgccc agcagaacca       540
   tatgtcctat caggaggaaa ggacaaatct gttgtcttgt ggagcatcca agaccacata       600
   tctgcccttg gggattcctc gtcttctcct ggagcatctg gcagcaagca gtctggtaaa       660
   tctgcaactg aaaaggagag ccctaaagtt gatcctagag gtatattctg tgggatgctc       720
   ganactggca ctggcccagc tgttaaggtt gagaaagctc acagtggaga tgttcattgt       780
   gttgattgga atccccttga cgttaactat atcttaactg gttctgccga taactctgtc       840
   cgaatgtggg atcgtcgtaa gctgggttcg ggaggagcta gttctccaat tcataaattt       900
   gagggccata aagctgctgt tctttgtgtt cagtggtcac ctgacagagc atctgttttt       960
   ggaagttctg cggaagatgg tttcttaaac gtgtgggatc atgagaaggt tgggactaag      1020
   aaaaatacta atgtcccagc tgggcttttc ttccagcatg ctggtcatag ggataagatt      1080
   gtagacttcc actggaattc gtcagatcct tggacaattg tcagtgtatc tgatgatggt      1140
   gagagcactg gtggaggcgg aacactgcag atatggcgca tgagtgattt gatctaccgc      1200
   cctgaggatg aagttcttac agaactggag aatttcaagg ctcacttggc cagctgtgct      1260
   ccaaggactt ga                                                           1272
<210> 20
   <211> 423
   <212> PRT
   <213> SORGHUM BICOLOR
<400> 20
   Met Lys Glu Arg Ser Gly Ser Arg Ala Ala Val Asp Glu Arg Tyr Ala
   1               5                   10                  15
   Gln Trp Lys Ser Leu Ile Pro Val Leu Tyr Asp Trp Phe Ala Asn His
               20                  25                  30
   Asn Leu Val Trp Pro Ser Leu Ser Cys Arg Trp Gly Pro Gln Phe Glu
           35                  40                  45
   Lys Ala Thr Tyr Lys Asn Arg Gln Arg Leu Tyr Leu Ser Glu Gln Thr
       50                  55                  60
   Asp Gly Ser Val Pro Asn Thr Leu Val Ile Ala Asn Cys Glu Val Val
   65                  70                  75                  80
   Lys Pro Arg Val Ala Ala Ala Glu His Ile Ser Gln Phe Asn Glu Glu
                   85                  90                  95
   Ala Arg Ser Pro Phe Val Lys Lys Tyr Lys Thr Ile Val His Pro Gly
               100                 105                 110
   Glu Val Asn Arg Ile Arg Glu Leu Pro Gln Asn Ser Lys Ile Ile Ala
           115                 120                 125
   Thr His Thr Asp Ser Pro Asp Val Leu Ile Trp Asp Val Glu Ala Gln
       130                 135                 140
   Pro Asn Arg His Ala Val Leu Gly Ala Ser Glu Ser Arg Pro Asp Leu
   145                 150                 155                 160
   Ile Leu Thr Gly His Lys Glu Asn Ala Glu Phe Ala Leu Ala Met Cys
   165 170 175
   Pro Ala Glu Pro Tyr Val Leu Ser Gly Gly Lys Asp Lys Ser Val Val
   180 185 190
   Leu Trp Ser Ile Gln Asp His Ile Ser Ala Leu Gly Asp Ser Ser Ser
   195 200 205
   Ser Pro Gly Ala Ser Gly Ser Lys Gln Ser Gly Lys Ser Ala Thr Glu
   210 215 220
   Lys Glu Ser Pro Lys Val Asp Pro Arg Gly Ile Phe Cys Gly Met Leu
   225 230 235 240
   Glu Leu Ala Leu Ala Gln Leu Leu Arg Leu Arg Lys Leu Thr Val Glu
   245 250 255
   Met Phe Ile Val Val Asp Trp Asn Pro Leu Asp Val Asn Tyr Ile Leu
   260 265 270
   Thr Gly Ser Ala Asp Asn Ser Val Arg Met Trp Asp Arg Arg Lys Leu
   275 280 285
   Gly Ser Gly Gly Ala Ser Ser Pro Ile His Lys Phe Glu Gly His Lys
   290 295 300
   Ala Ala Val Leu Cys Val Gln Trp Ser Pro Asp Arg Ala Ser Val Phe
   305 310 315 320
   Gly Ser Ser Ala Glu Asp Gly Phe Leu Asn Val Trp Asp His Glu Lys
   325 330 335
   Val Gly Thr Lys Lys Asn Thr Asn Val Pro Ala Gly Leu Phe Phe Gln
   340 345 350
   His Ala Gly His Arg Asp Lys Ile Val Asp Phe His Trp Asn Ser Ser
   355 360 365
   Asp Pro Trp Thr Ile Val Ser Val Ser Asp Asp Gly Glu Ser Thr Gly
   370 375 380
   Gly Gly Gly Thr Leu Gln Ile Trp Arg Met Ser Asp Leu Ile Tyr Arg
   385 390 395 400
   Pro Glu Asp Glu Val Leu Thr Glu Leu Glu Asn Phe Lys Ala His Leu
   405 410 415
   Ala Ser Cys Ala Pro Arg Thr
   420
<210> 21
   <211> 1362
   <212> DNA
   <213> ZEA MAYS
<400> 21
   atgaaagaga gaggcggctc cagcgcggcg gtggacgagc gctacgcgca gtggaagtcg      60
   ctcatccccg tcctctacga ctggtttgcc aaccacaacc tcgtctggcc atccctctcc     120
   tgccggtggg ggccacagtt tgagaaagct acctacaaga atcgtcagcg cctgtaccta     180
   tctgaacaga cggatgggag tgtgcctaat actctggtta tcgcaaattg tgaagttgtg     240
   aaacctaggg ttgcagctgc tgaacatatc tcacagttca atgaggaagc acggtcacct     300
   tttgtaaaga agtacaaaac tatagttcat cctggtgagg ttaacagaat cagggaactt     360
   ccacagaaca gtaagatcat agccactcac actgacagtc cagatgtact tatttgggat     420
   gttgaagcac aaccaaatag acatgctgtc ctaggagcaa gcgagtctcg ccctgatctg     480
   atattaacag gacataagga aaatgcggaa tttgcgcttg ccatgtgtcc agcagaacca     540
   tatgtcctat caggaggaaa ggacaaatct gttgtcttgt ggagcatcca agaccatata     600
   tctgcccttg gggattcctc gtcttctcct ggagcatctg gcagcaagca gtctattaaa     660
   actgcaaatg aaaaggagag ccctaaagtt gatcctagag gtatatttca tggacatgac     720
   agcacggttg aagatgttca gttctgccct tccagtgcgc aggagttttg tagtgtgggt     780
   gatgatgctt gtcttattct gtgggatgct cgaactggca ctgccccagc tgttaaggtt     840
   gagaaagctc acagtggaga tgttcattgt gttgattgga atccccttga tgttaactat     900
   atcttaactg ggtctgccga taactctgtc cgaatgtggg atcgtcggaa tctgggttca     960
   ggaggagctg gttctccaat tcacaaattt gaaggccata aagctgctgt tctttgtgtt    1020
   cagtggtcac ctgacagagc atctgttttt ggaagttctg cagaagatgg tttcttaaac    1080
   gtgtgggatc atgagaaggt ggggaagaag aaaaattcta atgtcccagc cgggcttttc    1140
   tttcagcacg ctggtcatag ggataagatt gtagacttcc actggaattc gtcagatcct    1200
   tggacaattg tcagtgtctc tgatgatggc gagagcactg gtggaggtgg aacactgcag    1260
   atttggcgca tgagtgattt gatctaccgc ccagaggatg aagttcttgc agagctggag    1320
   aatttcaagg ctcacttggc cagctgtgca ccgaagaatt ga                        1362
<210> 22
   <211> 453
   <212> PRT
   <213> ZEA MAYS
<400> 22
   Met Lys Glu Arg Gly Gly Ser Ser Ala Ala Val Asp Glu Arg Tyr Ala
   1               5                   10                  15
   Gln Trp Lys Ser Leu Ile Pro Val Leu Tyr Asp Trp Phe Ala Asn His
               20                  25                  30
   Asn Leu Val Trp Pro Ser Leu Ser Cys Arg Trp Gly Pro Gln Phe Glu
           35                  40                  45
   Lys Ala Thr Tyr Lys Asn Arg Gln Arg Leu Tyr Leu Ser Glu Gln Thr
       50                  55                  60
   Asp Gly Ser Val Pro Asn Thr Leu Val Ile Ala Asn Cys Glu Val Val
   65                  70                  75                  80
   Lys Pro Arg Val Ala Ala Ala Glu His Ile Ser Gln Phe Asn Glu Glu
                   85                  90                  95
   Ala Arg Ser Pro Phe Val Lys Lys Tyr Lys Thr Ile Val His Pro Gly
               100                 105                 110
   Glu Val Asn Arg Ile Arg Glu Leu Pro Gln Asn Ser Lys Ile Ile Ala
           115                 120                 125
   Thr His Thr Asp Ser Pro Asp Val Leu Ile Trp Asp Val Glu Ala Gln
       130                 135                 140
   Pro Asn Arg His Ala Val Leu Gly Ala Ser Glu Ser Arg Pro Asp Leu
   145                 150                 155                 160
   Ile Leu Thr Gly His Lys Glu Asn Ala Glu Phe Ala Leu Ala Met Cys
                   165                 170                 175
   Pro Ala Glu Pro Tyr Val Leu Ser Gly Gly Lys Asp Lys Ser Val Val
               180                 185                 190
   Leu Trp Ser Ile Gln Asp His Ile Ser Ala Leu Gly Asp Ser Ser Ser
           195                 200                 205
   Ser Pro Gly Ala Ser Gly Ser Lys Gln Ser Ile Lys Thr Ala Asn Glu
               210                 215                 220
   Lys Glu Ser Pro Lys Val Asp Pro Arg Gly Ile Phe His Gly His Asp
   225                 230                 235                 240
   Ser Thr Val Glu Asp Val Gln Phe Cys Pro Ser Ser Ala Gln Glu Phe
                   245                 250                 255
   Cys Ser Val Gly Asp Asp Ala Cys Leu Ile Leu Trp Asp Ala Arg Thr
               260                 265                 270
   Gly Thr Ala Pro Ala Val Lys Val Glu Lys Ala His Ser Gly Asp Val
           275                 280                 285
   His Cys Val Asp Trp Asn Pro Leu Asp Val Asn Tyr Ile Leu Thr Gly
       290                 295                 300
   Ser Ala Asp Asn Ser Val Arg Met Trp Asp Arg Arg Asn Leu Gly Ser
   305                 310                 315                 320
   Gly Gly Ala Gly Ser Pro Ile His Lys Phe Glu Gly His Lys Ala Ala
                   325                 330                 335
   Val Leu Cys Val Gln Trp Ser Pro Asp Arg Ala Ser Val Phe Gly Ser
               340                 345                 350
   Ser Ala Glu Asp Gly Phe Leu Asn Val Trp Asp His Glu Lys Val Gly
           355                 360                 365
   Lys Lys Lys Asn Ser Asn Val Pro Ala Gly Leu Phe Phe Gln His Ala
       370                 375                 380
   Gly His Arg Asp Lys Ile Val Asp Phe His Trp Asn Ser Ser Asp Pro
   385                 390                 395                 400
   Trp Thr Ile Val Ser Val Ser Asp Asp Gly Glu Ser Thr Gly Gly Gly
                   405                 410                 415
   Gly Thr Leu Gln Ile Trp Arg Met Ser Asp Leu Ile Tyr Arg Pro Glu
               420                 425                 430
   Asp Glu Val Leu Ala Glu Leu Glu Asn Phe Lys Ala His Leu Ala Ser
           435                 440                 445
           Cys Ala Pro Lys Asn
   450
<210> 23
   <211> 1362
   <212> DNA
   <213> ZEA MAYS
<400> 23
   atgaaggaga gaggcggctc cagggcggca gtggacgagc gctacgcgca gtggaagtcg      60
   ctcatccccg ttctctacga ctggtttgcc aaccacaacc tcgtttggcc atccctctcc     120
   tgccggtggg ggccacagtt tgagaaagct acctacaaga atcgtcaacg cctttaccta     180
   tctgaacaga ccgatgggag tgtgcctaat actctggtta tcgcaaactg tgaagttgtg     240
   aaaccaaggg ttgcagctgc tgaacatatc tcgcagttta acgaggaagc acgatcacct     300
   tttgtaaaga agtacaagac tatagttcat cctggtgaag ttaacagaat cagggagctt     360
   ccacagaaca gtaagatcat agccacacac actgacagtc cagatgtact tgtttgggat     420
   gttgaagcac aaccaaatag acatgctgtc ttaggagctt ctgaatctcg tcctgatctg     480
   atattaacgg gacaccagga aaatgcagaa tttgcgcttg ccatgtgtcc agcagaacca     540
   tatgtcctgt caggaggaaa ggacaaattt gttgtcttgt ggagcatcca agaccacata     600
   tctgcccttg gggattcctc gtcttctccc ggagcatctg gcagcaagca gtctggcaaa     660
   attgcaaatg aaaaggagag tcctaaagtt gatcctagag gtatattcca tggccatgac     720
   agtactgttg aagatgttca gttctgccct tccagtgcgc aggagttttg tagtgtgggt     780
   gatgatgctt gtcttattct gtgggatgct cgaactggca ctgacccagc tgttaaggtt     840
   gagaaagctc acagtggaga tgttcattgt gttgattgga atccccttga tgttaactat     900
   atcttaactg gttctgccga taactctgtc cgaatgtggg atcgtcgcaa tctgggttcg     960
   ggaggagctg gttctccaat tcacaaattt gagggccata aagctgctgt tctttgtgtt    1020
   cagtggtcac ctgacagagc atctgttttt ggaagttctg cagaagatgg tttcttaaat    1080
   gtttgggacc atgagaaggt tgggaagaag aaaaattcta atgtcccagc tgggcttttc    1140
   tttcagcatg ctggtcatag ggataagatc gtagacttcc actggaattc gtcagatcct    1200
   tggacaattg tcagtgtctc agatgatggt gagagcactg gtggaggtgg aacactgcag    1260
   atatggcgaa tgagtgattt gatctaccgc ccagaggatg aagttcttac agagctggag    1320
   aatttcaagg ctcacctggc cagttgcgct ccgaggaatt ga                        1362
<210> 24
   <211> 453
   <212> PRT
   <213> ZEA MAYS
<400> 24
   Met Lys Glu Arg Gly Gly Ser Arg Ala Ala Val Asp Glu Arg Tyr Ala
   1               5                   10                  15
   Gln Trp Lys Ser Leu Ile Pro Val Leu Tyr Asp Trp Phe Ala Asn His
               20                  25                  30
   Asn Leu Val Trp Pro Ser Leu Ser Cys Arg Trp Gly Pro Gln Phe Glu
           35                  40                  45
   Lys Ala Thr Tyr Lys Asn Arg Gln Arg Leu Tyr Leu Ser Glu Gln Thr
       50                  55                  60
   Asp Gly Ser Val Pro Asn Thr Leu Val Ile Ala Asn Cys Glu Val Val
   65                  70                  75                  80
   Lys Pro Arg Val Ala Ala Ala Glu His Ile Ser Gln Phe Asn Glu Glu
                   85                  90                  95
   Ala Arg Ser Pro Phe Val Lys Lys Tyr Lys Thr Ile Val His Pro Gly
               100                 105                 110
   Glu Val Asn Arg Ile Arg Glu Leu Pro Gln Asn Ser Lys Ile Ile Ala
           115                 120                 125
   Thr His Thr Asp Ser Pro Asp Val Leu Val Trp Asp Val Glu Ala Gln
       130                 135                 140
   Pro Asn Arg His Ala Val Leu Gly Ala Ser Glu Ser Arg Pro Asp Leu
   145                 150                 155                 160
   Ile Leu Thr Gly His Gln Glu Asn Ala Glu Phe Ala Leu Ala Met Cys
                   165                 170                 175
   Pro Ala Glu Pro Tyr Val Leu Ser Gly Gly Lys Asp Lys Phe Val Val
               180                 185                 190
   Leu Trp Ser Ile Gln Asp His Ile Ser Ala Leu Gly Asp Ser Ser Ser
           195                 200                 205
   Ser Pro Gly Ala Ser Gly Ser Lys Gln Ser Gly Lys Ile Ala Asn Glu
       210                 215                 220
   Lys Glu Ser Pro Lys Val Asp Pro Arg Gly Ile Phe His Gly His Asp
   225                 230                 235                 240
   Ser Thr Val Glu Asp Val Gln Phe Cys Pro Ser Ser Ala Gln Glu Phe
                   245                 250                 255
   Cys Ser Val Gly Asp Asp Ala Cys Leu Ile Leu Trp Asp Ala Arg Thr
               260                 265                 270
   Gly Thr Asp Pro Ala Val Lys Val Glu Lys Ala His Ser Gly Asp Val
           275                 280                 285
   His Cys Val Asp Trp Asn Pro Leu Asp Val Asn Tyr Ile Leu Thr Gly
       290                 295                 300
   Ser Ala Asp Asn Ser Val Arg Met Trp Asp Arg Arg Asn Leu Gly Ser
   305                 310                 315                 320
   Gly Gly Ala Gly Ser Pro Ile His Lys Phe Glu Gly His Lys Ala Ala
                   325                 330                 335
   Val Leu Cys Val Gln Trp Ser Pro Asp Arg Ala Ser Val Phe Gly Ser
               340                 345                 350
   Ser Ala Glu Asp Gly Phe Leu Asn Val Trp Asp His Glu Lys Val Gly
           355                 360                 365
   Lys Lys Lys Asn Ser Asn Val Pro Ala Gly Leu Phe Phe Gln His Ala
       370                 375                 380
   Gly His Arg Asp Lys Ile Val Asp Phe His Trp Asn Ser Ser Asp Pro
   385                 390                 395                 400
   Trp Thr Ile Val Ser Val Ser Asp Asp Gly Glu Ser Thr Gly Gly Gly
                   405                 410                 415
   Gly Thr Leu Gln Ile Trp Arg Met Ser Asp Leu Ile Tyr Arg Pro Glu
               420                 425                 430
   Asp Glu Val Leu Thr Glu Leu Glu Asn Phe Lys Ala His Leu Ala Ser
           435                 440                 445
   Cys Ala Pro Arg Asn
       450
<210> 25
   <211> 1362
   <212> DNA
   <213> ORYZA SATIVA
<400> 25
   atgaaggaga aggggtcgag ggcggcggtg gacgagcgct acgcgcagtg gaagtcgctc      60
   atcccggtgc tctacgactg gttcgccaac cacaacctcg tctggccgtc cctctcctgc     120
   cggtggggac cccagtttga gaaggctacc tacaagaatc gtcagcgtct ttacctttca     180
   gagcagacgg atgggactgt ccctaatact ctggttattg caaattgtga agttgtcaaa     240
   ccaagggttg cagctgctga acatatatct cagttcaatg aggaagcacg atcacctttt     300
   gtaaagaagt acaagactat aattcatcca ggcgaggtga acaggattag ggagcttccg     360
   cagaacagta agatcattgc cactcatacc gacagcccag atgttctcat ttgggatgtc     420
   gaggcccagc caaatagaca agctcaactg gcacagatgg agtctcgtcc tgatttgata     480
   ttaagaggac ataaggatat tgctgagttt gcgcttgcta tgtgcccagc tgagccatat     540
   gtgttatctg gaggaaaaga caaatctgtt gtatggtgga gcatccaaga ccacatatct     600
   gcactgggag attcctcgaa aactgaatct tctccagggg catcaggaag caagggaaaa     660
   actgcaaatg ataaggatag tcctaaagtt gatcctcgtg gtatctttct tggccacgac     720
   agtactgttg aagatgtcca gttctgccct tctagtgcac aggagttttg tagtgtaggc     780
   gatgattctt gtcttattct ttgggatgcc cggagtggta caggcccagc tgttaaggtt     840
   gagaaagctc atggtggtga tgttcattgt gttgactgga acctccatga tgttaactat     900
   atcttaactg gttctgcgga taattctgtc cgtatgtggg accggagaaa tctgggttct     960
   ggaggagctg gcattccagt tcacaaattt gagggtcata aagctgctgt tctttgtgtt    1020
   cagtggtcac ctgacaaggc atctgtattt ggaagctctg cggaagacgg cttcttaaat    1080
   gtgtgggatc atgagaaggt tggaaataag aaaaatccca acgcacctgc tgggcttttc    1140
   tttcaacatg ctggtcatag ggataagatt gtagacttcc actggaattc ttcggatcct    1200
   tggactattg tgagtgtgtc tgatgatggt gagagtactg gtggaggtgg aacattgcag    1260
   atatggcgca tgagtgacct gatataccgt ccagaagatg aagttcttgc agagctggag    1320
   aacttcaaga ctcacttggc cagctgtgct ccaaggagct ga                       1362
<210> 26
   <211> 453
   <212> PRT
   <213> ORYZA SATIVA
<400> 26
   Met Lys Glu Lys Gly Ser Arg Ala Ala Val Asp Glu Arg Tyr Ala Gln
   1               5                   10                  15
   Trp Lys Ser Leu Ile Pro Val Leu Tyr Asp Trp Phe Ala Asn His Asn
               20                  25                  30
   Leu Val Trp Pro Ser Leu Ser Cys Arg Trp Gly Pro Gln Phe Glu Lys
           35                  40                  45
   Ala Thr Tyr Lys Asn Arg Gln Arg Leu Tyr Leu Ser Glu Gln Thr Asp
       50                  55                  60
   Gly Thr Val Pro Asn Thr Leu Val Ile Ala Asn Cys Glu Val Val Lys
   65                  70                  75                  80
   Pro Arg Val Ala Ala Ala Glu His Ile Ser Gln Phe Asn Glu Glu Ala
                   85                  90                  95
   Arg Ser Pro Phe Val Lys Lys Tyr Lys Thr Ile Ile His Pro Gly Glu
               100                 105                 110
   Val Asn Arg Ile Arg Glu Leu Pro Gln Asn Ser Lys Ile Ile Ala Thr
           115                 120                 125
   His Thr Asp Ser Pro Asp Val Leu Ile Trp Asp Val Glu Ala Gln Pro
       130                 135                 140
   Asn Arg Gln Ala Gln Leu Ala Gln Met Glu Ser Arg Pro Asp Leu Ile
   145                 150                 155                 160
   Leu Arg Gly His Lys Asp Ile Ala Glu Phe Ala Leu Ala Met Cys Pro
                   165                 170                 175
   Ala Glu Pro Tyr Val Leu Ser Gly Gly Lys Asp Lys Ser Val Val Trp
               180                 185                 190
   Trp Ser Ile Gln Asp His Ile Ser Ala Leu Gly Asp Ser Ser Lys Thr
           195                 200                 205
   Glu Ser Ser Pro Gly Ala Ser Gly Ser Lys Gly Lys Thr Ala Asn Asp
       210                 215                 220
   Lys Asp Ser Pro Lys Val Asp Pro Arg Gly Ile Phe Leu Gly His Asp
   225                 230                 235                 240
   Ser Thr Val Glu Asp Val Gln Phe Cys Pro Ser Ser Ala Gln Glu Phe
                   245                 250                 255
   Cys Ser Val Gly Asp Asp Ser Cys Leu Ile Leu Trp Asp Ala Arg Ser
               260                 265                 270
   Gly Thr Gly Pro Ala Val Lys Val Glu Lys Ala His Gly Gly Asp Val
           275                 280                 285
   His Cys Val Asp Trp Asn Leu His Asp Val Asn Tyr Ile Leu Thr Gly
       290                 295                 300
   Ser Ala Asp Asn Ser Val Arg Met Trp Asp Arg Arg Asn Leu Gly Ser
   305                 310                 315                 320
   Gly Gly Ala Gly Ile Pro Val His Lys Phe Glu Gly His Lys Ala Ala
                   325                 330                 335
   Val Leu Cys Val Gln Trp Ser Pro Asp Lys Ala Ser Val Phe Gly Ser
               340                 345                 350
   Ser Ala Glu Asp Gly Phe Leu Asn Val Trp Asp His Glu Lys Val Gly
           355                 360                 365
   Asn Lys Lys Asn Pro Asn Ala Pro Ala Gly Leu Phe Phe Gln His Ala
       370                 375                 380
   Gly His Arg Asp Lys Ile Val Asp Phe His Trp Asn Ser Ser Asp Pro
   385                 390                 395                 400
   Trp Thr Ile Val Ser Val Ser Asp Asp Gly Glu Ser Thr Gly Gly Gly
                   405                 410                 415
   Gly Thr Leu Gln Ile Trp Arg Met Ser Asp Leu Ile Tyr Arg Pro Glu
               420                 425                 430
   Asp Glu Val Leu Ala Glu Leu Glu Asn Phe Lys Thr His Leu Ala Ser
           435                 440                 445
   Cys Ala Pro Arg Ser
       450
<210> 27
   <211> 1506
   <212> DNA
   <213> ORYZA SATIVA
<400> 27
   atgaaggaga aggggtcgag ggcggcggtg gacgagcgct acgcgcagtg gaagtcgctc      60
   atcccggtgc tctacgactg gttcgccaac cacaacctcg tctggccgtc cctctcctgc     120
   cggtggggac cccagtttga gaaggctacc tacaagaatc gtcagcgtct ttacctttca     180
   gagcaggcaa gtgctcccaa aataagttgt tctttttctt tcttttgctg tagggatgtt     240
   ttgaagctca aatttatgac ggatgggact gtccctaata ctctggttat tgcaaattgt     300
   gaagttgtca aaccaagggt tgcagctgct gaacatatat ctcagttcaa tgaggaagca     360
   cgatcacctt ttgtaaagaa gtacaagact ataattcatc caggcgaggt gaacaggatt     420
   agggagcttc cgcagaacag taagatcatt gccactcata ccgacagccc agatgttctc     480
   atttgggatg tcgaggccca gccaaataga caagctcaac tggcacagat ggagtctcgt     540
   cctgatttgg taccacctga ctctcgccct gatttgatat taagaggaca taaggatatt     600
   gctgagtttg cgcttgctat gtgcccagct gagccatatg tgttatctgg aggaaaagac     660
   aaatctgttg tatggtggag catccaagac cacatatctg cactgggaga ttcctcgaaa     720
   actgaatctt ctccaggggc atcaggaagc aagggaaaaa ctgcaaatga taaggatagt     780
   cctaaagttg atcctcgtgg tatctttctt ggccacgaca gtactgttga agatgtccag     840
   ttctgccctt ctagtgcaca ggagttttgt agtgtgggcg atgattcttg tcttattctt     900
   tgggatgccc ggagtggtac aggcccagct gttaaggttg agaaagctca tggtggtgat     960
   gttcattgtg ttgactggaa cctccatgat gttaactata tcttaactgg tgttcatttg    1020
   gtacaagaag ccgtggatac acttcttgat attctgcgga taattctgtc cgtatgtggg    1080
   accggagaaa tctgggttct ggaggagctg gcattccagt tcacaaattt gagggtcata    1140
   aagctgctgt tctttgtgtt caggcattgg tcacctgaca aggcatctgt atttggaagc    1200
   tctgcggaag acggtttctt aaatgtgtgg gatcatgaga aggttggaaa taagaaaaat    1260
   cccaacgcac ctgctgggct tttctttcaa catgctggtc atagggataa gattgtagac    1320
   ttccactgga attcttcgga tccttggact attgtgagtg tgtctgatga tggtgagagt    1380
   actggtggag gtggaacatt gcagatatgg cgcatgagtg acctgatata ccgtccagaa    1440
   gatgaagttc ttgcagagct ggagaacttc aagactcact tggccagctg tgctccaagg    1500
   agctga                                                               1506
<210> 28
   <211> 501
   <212> PRT
   <213> ORYZA SATIVA
<400> 28
   Met Lys Glu Lys Gly Ser Arg Ala Ala Val Asp Glu Arg Tyr Ala Gln
   1               5                   10                  15
   Trp Lys Ser Leu Ile Pro Val Leu Tyr Asp Trp Phe Ala Asn His Asn
               20                  25                  30
   Leu Val Trp Pro Ser Leu Ser Cys Arg Trp Gly Pro Gln Phe Glu Lys
           35                  40                  45
   Ala Thr Tyr Lys Asn Arg Gln Arg Leu Tyr Leu Ser Glu Gln Ala Ser
       50                  55                  60
   Ala Pro Lys Ile Ser Cys Ser Phe Ser Phe Phe Cys Cys Arg Asp Val
   65                  70                  75                  80
   Leu Lys Leu Lys Phe Met Thr Asp Gly Thr Val Pro Asn Thr Leu Val
                   85                  90                  95
   Ile Ala Asn Cys Glu Val Val Lys Pro Arg Val Ala Ala Ala Glu His
               100                 105                 110
   Ile Ser Gln Phe Asn Glu Glu Ala Arg Ser Pro Phe Val Lys Lys Tyr
           115                 120                 125
   Lys Thr Ile Ile His Pro Gly Glu Val Asn Arg Ile Arg Glu Leu Pro
       130                 135                 140
   Gln Asn Ser Lys Ile Ile Ala Thr His Thr Asp Ser Pro Asp Val Leu
   145                 150                 155                 160
   Ile Trp Asp Val Glu Ala Gln Pro Asn Arg Gln Ala Gln Leu Ala Gln
                   165                 170                 175
   Met Glu Ser Arg Pro Asp Leu Val Pro Pro Asp Ser Arg Pro Asp Leu
               180                 185                 190
   Ile Leu Arg Gly His Lys Asp Ile Ala Glu Phe Ala Leu Ala Met Cys
           195                 200                 205
   Pro Ala Glu Pro Tyr Val Leu Ser Gly Gly Lys Asp Lys Ser Val Val
       210                 215                 220
   Trp Trp Ser Ile Gln Asp His Ile Ser Ala Leu Gly Asp Ser Ser Lys
   225                 230                 235                 240
   Thr Glu Ser Ser Pro Gly Ala Ser Gly Ser Lys Gly Lys Thr Ala Asn
                   245                 250                 255
   Asp Lys Asp Ser Pro Lys Val Asp Pro Arg Gly Ile Phe Leu Gly His
               260                 265                 270
   Asp Ser Thr Val Glu Asp Val Gln Phe Cys Pro Ser Ser Ala Gln Glu
           275                 280                 285
   Phe Cys Ser Val Gly Asp Asp Ser Cys Leu Ile Leu Trp Asp Ala Arg
       290                 295                 300
   Ser Gly Thr Gly Pro Ala Val Lys Val Glu Lys Ala His Gly Gly Asp
   305                 310                 315                 320
   Val His Cys Val Asp Trp Asn Leu His Asp Val Asn Tyr Ile Leu Thr
                   325                 330                 335
   Gly Val His Leu Val Gln Glu Ala Val Asp Thr Leu Leu Asp Ile Leu
               340                 345                 350
   Arg Ile Ile Leu Ser Val Cys Gly Thr Gly Glu Ile Trp Val Leu Glu
           355                 360                 365
   Glu Leu Ala Phe Gln Phe Thr Asn Leu Arg Val Ile Lys Leu Leu Phe
       370                 375                 380
   Phe Val Phe Arg His Trp Ser Pro Asp Lys Ala Ser Val Phe Gly Ser
   385                 390                 395                 400
   Ser Ala Glu Asp Gly Phe Leu Asn Val Trp Asp His Glu Lys Val Gly
                   405                 410                 415
   Asn Lys Lys Asn Pro Asn Ala Pro Ala Gly Leu Phe Phe Gln His Ala
               420                 425                 430
   Gly His Arg Asp Lys Ile Val Asp Phe His Trp Asn Ser Ser Asp Pro
           435                 440                 445
   Trp Thr Ile Val Ser Val Ser Asp Asp Gly Glu Ser Thr Gly Gly Gly
       450                 455                 460
   Gly Thr Leu Gln Ile Trp Arg Met Ser Asp Leu Ile Tyr Arg Pro Glu
   465                 470                 475                 480
   Asp Glu Val Leu Ala Glu Leu Glu Asn Phe Lys Thr His Leu Ala Ser
   485 490 495
   Cys Ala Pro Arg Ser
   500
<210> 29
   <211> 1503
   <212> DNA
   <213> MEDICAGO TRUNCATULA
<400> 29
   atggagacac caccctcaac cggcgccgtg aagaagaagg agacaagagg acgcaaacct      60
   aaacccaagg aagacaagag agaagaacca tcacaagtga agtcaccgag agaatcgaag     120
   aaggaaaagc aacagcaaca gcttcatcag cagcagcaac aacaagcttc tgttgatgag     180
   aagtactctc aatggaaatc tcttgttcct gttctttatg attggcttgc taatcataac     240
   ctcgtttggc cttctctctc ttgccggtgg ggtcctcagc ttgaacaggc tacttacaag     300
   aatcgtcaga ggctttatct ttcggagcag actgatggaa gtgttccgaa tactttggtg     360
   attgcgaatt gtgaggttgt gaagactagg gttgcagctg ctgaacacat ttctcagttt     420
   aatgaagagg ctcgctctcc atttgtcaag aagtacaaga ctatcataca tccaggcgag     480
   gtgaacagaa ttagggaatt gccacaaaat tctaagattg tggccactca cacagacagc     540
   cctgatgttc tcatttggga tgttgaaagt caacctaacc gtcatgctgt ccttggagct     600
   acaaactctc gtccagatct gatattgact ggacaccaag ataatgcaga gtttgctctt     660
   gcaatgtgcc ctactcagcc ctatgtgctt tctggaggaa aagacaaaac agtcgtgttg     720
   tggagtattg aagaccatat aacatctgct gctacagact ccaacaagtc tggtggatcc     780
   attgccaaaa ctgctgatag ccctactgtc ggaccaagag gtatctactc tgggcatgag     840
   gatactgttg aagacgtggc tttttgccct tctagtgcgc aggagttctg tagtgttgga     900
   gatgattctt gtctcatatt atgggatgca cgtgttggct ctagtcctgt ggttaaggtt     960
   gaaaaagctc atgatgctga tcttcactgt gttgactgga atccccatga tgataatctg    1020
   attcttactg ggtcggcaga taattctatt cgcatgtttg atcgtcgcaa tctcacttct    1080
   aatggggttg ggtctcctat ccacaaattt gaggctcata aggctgctgt cctttgtgtt    1140
   cagtggtctc cagacaaatc atctgtattt ggaagttcag cagaagatgg tctcctgaac    1200
   atttgggatt atgagaaggt tggcaagaag atagagcgag ctggaaaaac aataaattct    1260
   cctccagggt tgtttttcca acatgctggt catagagaca aagttgttga ctttcactgg    1320
   aatgcacatg atccatggac acttgttagt gtgtctgatg attgcgaaag tactggtgga    1380
   gggggaacat tgcagatatg gcgcatgagt gatttgctct acagaccaga ggatgaggtt    1440
   ttggcagagc tggagaaatt caaatctcat gtggtggctt gtgcagcaaa gaccgacaaa    1500
   taa                                                                  1503
<210> 30
   <211> 500
   <212> PRT
   <213> MEDICAGO TRUNCATULA
<400> 30
   Met Glu Thr Pro Pro Ser Thr Gly Ala Val Lys Lys Lys Glu Thr Arg
   1               5                   10                  15
   Gly Arg Lys Pro Lys Pro Lys Glu Asp Lys Arg Glu Glu Pro Ser Gln
               20                  25                  30
   Val Lys Ser Pro Arg Glu Ser Lys Lys Glu Lys Gln Gln Gln Gln Leu
           35                  40                  45
   His Gln Gln Gln Gln Gln Gln Ala Ser Val Asp Glu Lys Tyr Ser Gln
       50                  55                  60
   Trp Lys Ser Leu Val Pro Val Leu Tyr Asp Trp Leu Ala Asn His Asn
   65                  70                  75                  80
   Leu Val Trp Pro Ser Leu Ser Cys Arg Trp Gly Pro Gln Leu Glu Gln
                   85                  90                  95
   Ala Thr Tyr Lys Asn Arg Gln Arg Leu Tyr Leu Ser Glu Gln Thr Asp
               100                 105                 110
   Gly Ser Val Pro Asn Thr Leu Val Ile Ala Asn Cys Glu Val Val Lys
           115                 120                 125
   Thr Arg Val Ala Ala Ala Glu His Ile Ser Gln Phe Asn Glu Glu Ala
       130                 135                 140
   Arg Ser Pro Phe Val Lys Lys Tyr Lys Thr Ile Ile His Pro Gly Glu
   145                 150                 155                 160
   Val Asn Arg Ile Arg Glu Leu Pro Gln Asn Ser Lys Ile Val Ala Thr
                   165                 170                 175
   His Thr Asp Ser Pro Asp Val Leu Ile Trp Asp Val Glu Ser Gln Pro
   180 185 190
   Asn Arg His Ala Val Leu Gly Ala Thr Asn Ser Arg Pro Asp Leu Ile
   195 200 205
   Leu Thr Gly His Gln Asp Asn Ala Glu Phe Ala Leu Ala Met Cys Pro
   210 215 220
   Thr Gln Pro Tyr Val Leu Ser Gly Gly Lys Asp Lys Thr Val Val Leu
   225 230 235 240
   Trp Ser Ile Glu Asp His Ile Thr Ser Ala Ala Thr Asp Ser Asn Lys
   245 250 255
   Ser Gly Gly Ser Ile Ala Lys Thr Ala Asp Ser Pro Thr Val Gly Pro
   260 265 270
   Arg Gly Ile Tyr Ser Gly His Glu Asp Thr Val Glu Asp Val Ala Phe
   275 280 285
   Cys Pro Ser Ser Ala Gln Glu Phe Cys Ser Val Gly Asp Asp Ser Cys
   290 295 300
   Leu Ile Leu Trp Asp Ala Arg Val Gly Ser Ser Pro Val Val Lys Val
   305 310 315 320
   Glu Lys Ala His Asp Ala Asp Leu His Cys Val Asp Trp Asn Pro His
   325 330 335
   Asp Asp Asn Leu Ile Leu Thr Gly Ser Ala Asp Asn Ser Ile Arg Met
   340 345 350
   Phe Asp Arg Arg Asn Leu Thr Ser Asn Gly Val Gly Ser Pro Ile His
   355 360 365
   Lys Phe Glu Ala His Lys Ala Ala Val Leu Cys Val Gln Trp Ser Pro
   370 375 380
   Asp Lys Ser Ser Val Phe Gly Ser Ser Ala Glu Asp Gly Leu Leu Asn
   385 390 395 400
   Ile Trp Asp Tyr Glu Lys Val Gly Lys Lys Ile Glu Arg Ala Gly Lys
   405 410 415
   Thr Ile Asn Ser Pro Pro Gly Leu Phe Phe Gln His Ala Gly His Arg
   420 425 430
   Asp Lys Val Val Asp Phe His Trp Asn Ala His Asp Pro Trp Thr Leu
   435 440 445
   Val Ser Val Ser Asp Asp Cys Glu Ser Thr Gly Gly Gly Gly Thr Leu
   450 455 460
   Gln Ile Trp Arg Met Ser Asp Leu Leu Tyr Arg Pro Glu Asp Glu Val
   465 470 475 480
   Leu Ala Glu Leu Glu Lys Phe Lys Ser His Val Val Ala Cys Ala Ala
   485 490 495
   Lys Thr Asp Lys
   500
<210> 31
   <211> 1440
   <212> DNA
   <213> MEDICAGO TRUNCATULA
<400> 31
   atgaatgaat cgaagaaaga gggaacaatt tcaacaaccc cactctcaat cgaagaaaga      60
   tacagtcaat ggaagtcact cgttccagtc ctctacgact ggctagcgaa tcacaatcta     120
   gtatggccat cacaatcatg ccgttggggt tcacttcttg atcatgctac ttacaagaat     180
   cgtcatcgtc tttatctttc tgaacaaact gatggaactg ctcccaatac gcttgttatt     240
   gctacttgtg aaattgttaa acctagggtt gctgctgctg aacatattgc tatgtttaat     300
   gaagaggcgc gttctccatt tgttaagaag gttaaaacta ttcttcatcc cggtgaggta     360
   aatagaatca gagaactccc gtcaaatact aatatagttg ccacacatac agatagtcca     420
   aatgttatga tttggaatgt tgaatctcaa cctaaccgga ataatgctgc tctggatgct     480
   cccacttcca tcccagactt ggtattaact ggacataagg ataatgctga atttgcacta     540
   gctatgtgtt caactgagcc ctttgttctt tccggaggga gagacaagct tgtggtgtta     600
   tggagtattc atgatcatat tgcaacttta gctacagaag aagaacccga tgttaatgag     660
   ggctctaatg ttgggggaaa tagcgagaaa gctgcacaaa gcccgtctgt tggagcaagg     720
   ggcgtctacc ggggtcataa agacactgtt gaagatgtgc agttttgccc ctcaagtgca     780
   caggagttct gtagtgtagg tgatgattct tgtctcatac tctgggatgc gcgtgttgga     840
   tcttttccag ctgtcaaggt tgagaaggca catgatggag atgtacattg cgttgattgg     900
   aatactcatg acatcaattt tattctgact ggctctgctg ataacacagt tcgcatgttt     960
   gatcgccgga aactaaacaa tcgtggagga attgggtctc ctgtttataa atttgaaggc    1020
   catgatgaac cagtcctctg tgtacagtgg aatcctgcta aatcatctgt atttggaagt    1080
   ggtgccgaag atggaattat aaacatctgg gaccatgaaa aggttggtaa aacatcaggt    1140
   tctgctgata ctacagtacc agagacttcc cctggtttat tctttcgtca tgcagggcat    1200
   agggataagg ttgttgactt tcattggaat gcatctgatc catggacaat tgttagtgta    1260
   tctgatgatt gtgcaagcac tggtggaggt ggcaccctgc agatctggcg gatgatggat    1320
   ctaatttatc gaccagagga cgaggtgatg gctgagctgg ataagttcaa atctcatatc    1380
   ttaggatgcg acactgtcac tgacagtgcc actgaccctg ccactccatc acatacgtga    1440
<210> 32
   <211> 479
   <212> PRT
   <213> MEDICAGO TRUNCATULA
<400> 32
   Met Asn Glu Ser Lys Lys Glu Gly Thr Ile Ser Thr Thr Pro Leu Ser
   1               5                   10                  15
   Ile Glu Glu Arg Tyr Ser Gln Trp Lys Ser Leu Val Pro Val Leu Tyr
               20                  25                  30
   Asp Trp Leu Ala Asn His Asn Leu Val Trp Pro Ser Gln Ser Cys Arg
           35                  40                  45
   Trp Gly Ser Leu Leu Asp His Ala Thr Tyr Lys Asn Arg His Arg Leu
       50                  55                  60
   Tyr Leu Ser Glu Gln Thr Asp Gly Thr Ala Pro Asn Thr Leu Val Ile
   65                  70                  75                  80
   Ala Thr Cys Glu Ile Val Lys Pro Arg Val Ala Ala Ala Glu His Ile
                   85                  90                  95
   Ala Met Phe Asn Glu Glu Ala Arg Ser Pro Phe Val Lys Lys Val Lys
               100                 105                 110
   Thr Ile Leu His Pro Gly Glu Val Asn Arg Ile Arg Glu Leu Pro Ser
           115                 120                 125
   Asn Thr Asn Ile Val Ala Thr His Thr Asp Ser Pro Asn Val Met Ile
       130                 135                 140
   Trp Asn Val Glu Ser Gln Pro Asn Arg Asn Asn Ala Ala Leu Asp Ala
   145 150 155 160
   Pro Thr Ser Ile Pro Asp Leu Val Leu Thr Gly His Lys Asp Asn Ala
   165 170 175
   Glu Phe Ala Leu Ala Met Cys Ser Thr Glu Pro Phe Val Leu Ser Gly
   180 185 190
   Gly Arg Asp Lys Leu Val Val Leu Trp Ser Ile His Asp His Ile Ala
   195 200 205
   Thr Leu Ala Thr Glu Glu Glu Pro Asp Val Asn Glu Gly Ser Asn Val
   210 215 220
   Gly Gly Asn Ser Glu Lys Ala Ala Gln Ser Pro Ser Val Gly Ala Arg
   225 230 235 240
   Gly Val Tyr Arg Gly His Lys Asp Thr Val Glu Asp Val Gln Phe Cys
   245 250 255
   Pro Ser Ser Ala Gln Glu Phe Cys Ser Val Gly Asp Asp Ser Cys Leu
   260 265 270
   Ile Leu Trp Asp Ala Arg Val Gly Ser Phe Pro Ala Val Lys Val Glu
   275 280 285
   Lys Ala His Asp Gly Asp Val His Cys Val Asp Trp Asn Thr His Asp
   290 295 300
   Ile Asn Phe Ile Leu Thr Gly Ser Ala Asp Asn Thr Val Arg Met Phe
   305 310 315 320
   Asp Arg Arg Lys Leu Asn Asn Arg Gly Gly Ile Gly Ser Pro Val Tyr
   325 330 335
   Lys Phe Glu Gly His Asp Glu Pro Val Leu Cys Val Gln Trp Asn Pro
   340 345 350
   Ala Lys Ser Ser Val Phe Gly Ser Gly Ala Glu Asp Gly Ile Ile Asn
   355 360 365
   Ile Trp Asp His Glu Lys Val Gly Lys Thr Ser Gly Ser Ala Asp Thr
   370 375 380
   Thr Val Pro Glu Thr Ser Pro Gly Leu Phe Phe Arg His Ala Gly His
   385 390 395 400
   Arg Asp Lys Val Val Asp Phe His Trp Asn Ala Ser Asp Pro Trp Thr
   405 410 415
   Ile Val Ser Val Ser Asp Asp Cys Ala Ser Thr Gly Gly Gly Gly Thr
   420 425 430
   Leu Gln Ile Trp Arg Met Met Asp Leu Ile Tyr Arg Pro Glu Asp Glu
   435 440 445
   Val Met Ala Glu Leu Asp Lys Phe Lys Ser His Ile Leu Gly Cys Asp
   450 455 460
   Thr Val Thr Asp Ser Ala Thr Asp Pro Ala Thr Pro Ser His Thr
   465 470 475
<210> 33
   <211> 1419
   <212> DNA
   <213> SILENE LATIFOLIA
<400> 33
   atgacggaga aagggaaagg aggcaagaaa gcattgagtg ttgatgagaa atacagtcaa 60
   tggaaatcac ttgttcctat tttatatgat tggcttgcca atcataacct cgtttggcct 120
   tctctctctt gcagatgggg cccgttgatt gaggaggcaa cttacaagaa ccgccaacgt 180
   ctttatctct cagaacagac tgatgggagt gttcctaata ccctcgtgat tgccaatgtt 240
   gaagtcgtga aacccagggt tgctgctgca gaacatatat cgaagttcaa tgaagaggct 300
   cgttctccct ttgtgaggaa gttcaaaacg ataatacatc ccggtgaggt aaacaggatt 360
   agggagctcc cccagaacag taatatagtt gcaactcata ctgacagtcc agatgtttat 420
   atttgggatc tggaaagcca acctaatcgt cctgctaatt tgggaacacc tgcctctcgt 480
   ccagatttga ctctgactgg tcatcaagat aatgctgaat tcgctcttgc aatgtgctct 540
   tctgaaccgc tggtgctttc tggaggaaag gataagtcag ttgttttgtg gagcatccat 600
   gatcatatct caacattggc aacagaacca gggtcagcaa aatctcccaa cagtggcagc 660
   aacataaaga aagctggtaa tggaaattca gataatcctt ctattggacc tcgaggcatc 720
   tatctgggac atgaggatac tgttgaagat gttcaatttt gcccctcaag tgcacagcag 780
   ttttgcagcg tgggtgatga ctcctgtctc atactatggg atgcaagagc tggattgacg 840
   ccagtgacta aggttgagaa agctcacaat gctgatcttc actgtgttga ctggaatcca 900
   catgatgaaa acctcattct aactgggtca gctgatagtt ctattaattt gtttgaccgc 960
   cgtaatctta ctgctagtgg agttggatca cctgttcata aatttcaggg tcatgatgct 1020
   cctgtccttt gtgttcagtg gtcaccgcat aatagatcga tttttggcag tgctgctgaa 1080
   gatggcttgc taaacatatg ggactatgaa aaggttagta agatggaaac agagagcgga 1140
   ggtaaaaagt caaaccatcc tgcaggcttg tttttcagac atgctggaca cagggacaaa 1200
   gtggtggact tccactggaa ttcgatagat ccatggactc tggttagtgt atcagatgac 1260
   tgttcaagct ctgctggagg tggaacattg cagatatggc gcataataga cttgctgtac 1320
   agaccagaag aggaagtttt ggctgagctg gacaagttca gatcccatgt agccgcttgc 1380
   tcgcctactc ctaccaaaga tgttaatcat tctgcctga 1419
<210> 34
   <211> 472
   <212> PRT
   <213> SILENE LATIFOLIA
<400> 34
   Met Thr Glu Lys Gly Lys Gly Gly Lys Lys Ala Leu Ser Val Asp Glu
   1               5                   10                  15
   Lys Tyr Ser Gln Trp Lys Ser Leu Val Pro Ile Leu Tyr Asp Trp Leu
               20                  25                  30
   Ala Asn His Asn Leu Val Trp Pro Ser Leu Ser Cys Arg Trp Gly Pro
           35                  40                  45
   Leu Ile Glu Glu Ala Thr Tyr Lys Asn Arg Gln Arg Leu Tyr Leu Ser
       50                  55                  60
   Glu Gln Thr Asp Gly Ser Val Pro Asn Thr Leu Val Ile Ala Asn Val
   65                  70                  75                  80
   Glu Val Val Lys Pro Arg Val Ala Ala Ala Glu His Ile Ser Lys Phe
                   85                  90                  95
   Asn Glu Glu Ala Arg Ser Pro Phe Val Arg Lys Phe Lys Thr Ile Ile
               100                 105                 110
   His Pro Gly Glu Val Asn Arg Ile Arg Glu Leu Pro Gln Asn Ser Asn
           115                 120                 125
   Ile Val Ala Thr His Thr Asp Ser Pro Asp Val Tyr Ile Trp Asp Leu
   130 135 140
   Glu Ser Gln Pro Asn Arg Pro Ala Asn Leu Gly Thr Pro Ala Ser Arg
   145 150 155 160
   Pro Asp Leu Thr Leu Thr Gly His Gln Asp Asn Ala Glu Phe Ala Leu
   165 170 175
   Ala Met Cys Ser Ser Glu Pro Leu Val Leu Ser Gly Gly Lys Asp Lys
   180 185 190
   Ser Val Val Leu Trp Ser Ile His Asp His Ile Ser Thr Leu Ala Thr
   195 200 205
   Glu Pro Gly Ser Ala Lys Ser Pro Asn Ser Gly Ser Asn Ile Lys Lys
   210 215 220
   Ala Gly Asn Gly Asn Ser Asp Asn Pro Ser Ile Gly Pro Arg Gly Ile
   225 230 235 240
   Tyr Leu Gly His Glu Asp Thr Val Glu Asp Val Gln Phe Cys Pro Ser
   245 250 255
   Ser Ala Gln Gln Phe Cys Ser Val Gly Asp Asp Ser Cys Leu Ile Leu
   260 265 270
   Trp Asp Ala Arg Ala Gly Leu Thr Pro Val Thr Lys Val Glu Lys Ala
   275 280 285
   His Asn Ala Asp Leu His Cys Val Asp Trp Asn Pro His Asp Glu Asn
   290 295 300
   Leu Ile Leu Thr Gly Ser Ala Asp Ser Ser Ile Asn Leu Phe Asp Arg
   305 310 315 320
   Arg Asn Leu Thr Ala Ser Gly Val Gly Ser Pro Val His Lys Phe Gln
   325 330 335
   Gly His Asp Ala Pro Val Leu Cys Val Gln Trp Ser Pro His Asn Arg
   340 345 350
   Ser Ile Phe Gly Ser Ala Ala Glu Asp Gly Leu Leu Asn Ile Trp Asp
   355 360 365
   Tyr Glu Lys Val Ser Lys Met Glu Thr Glu Ser Gly Gly Lys Lys Ser
   370 375 380
   Asn His Pro Ala Gly Leu Phe Phe Arg His Ala Gly His Arg Asp Lys
   385 390 395 400
   Val Val Asp Phe His Trp Asn Ser Ile Asp Pro Trp Thr Leu Val Ser
   405 410 415
   Val Ser Asp Asp Cys Ser Ser Ser Ala Gly Gly Gly Thr Leu Gln Ile
   420 425 430
   Trp Arg Ile Ile Asp Leu Leu Tyr Arg Pro Glu Glu Glu Val Leu Ala
   435 440 445
   Glu Leu Asp Lys Phe Arg Ser His Val Ala Ala Cys Ser Pro Thr Pro
   450 455 460
   Thr Lys Asp Val Asn His Ser Ala
   465 470
<210> 35
   <211> 1320
   <212> DNA
   <213> PHYSCOMITRELLA PATENS
<220>
   <221> misc_feature
   <222> (364)..(364)
   <223> n is a, c, g, or t
<220>
   <221> misc_feature
   <222> (938)..(938)
   <223> n is a, c, g, or t
<400> 35
   atgaagaagg ttccggggga tcggtacggg caatggaagt tgcttgtccc agctttgtat        60
   gactggctga ccaatcatca ccttatgggg ccctctcttt cttgccggtg ggggccgcag       120
   ttggaggcag ggtcctccaa gactcgtcaa cttctttttt actctgaaag gacggatgga       180
   gagtgtccaa acaccatcgt tgttgcccaa tgtgacatta tgaagccacg aactgctgca       240
   gcggaacaga tttcacagtt caaagaaggt ggaaaatcac ctcacctgaa gaagctcaaa       300
   actattattc atcctggtga ggttaacaga atacgagaaa ttcctcagaa tagcaatatt       360
   cttnccacgc atactgatag tcccaaggtt ctgatatgga ataccaaaac tcagccaaac       420
   cgagctacaa catcagcagc gtctgaatca aaacccgatc tggttttgat aggccacact       480
   gacaatgctg aattcgcttt gaacgtaagt cgaactgctc cttacgtcat atcaggaggt       540
   aagactccta aatcaatcac accgacggca gcagggtcca agcaatcagg aactgcaggg       600
   ggtgctgccg acactactaa tgtgtatacc agagggattt ttaagggcca tactgatact       660
   gtggaggatg tccaatttcg tccttcgagc atgaatgagt tttgcagtgt aggcgatgat       720
   tcttgcctcc tcttgtggga tgctcgcact ggataccaac caatatcgaa ggttgtgaag       780
   gctcacaatg cggacctcca ctgtgtcgat tggaatgctc atgacgagaa tcttatttta       840
   actggatcag cagataattc tgtaaggttg tttgatcacc gaaagatttt agctaggggt       900
   caagctattc ccgttgagca atttgaaggg cactccgntg ctgtgctctg tgtccagtgg       960
   tgcccagata gagcttctgt gtttgggagc tgcgccgagg atggattgct taatgtgtgg      1020
   gattatgaaa aggtcggtaa ggcattagat accaccaatt tgaaacaacc tgtaaaagtt      1080
   cctccaggcc ttttcttcca acacacaggg cacagggaca aagtggtgga ctttcattgg      1140
   gattcgcgcg atccgtggac aatagtcagt gtgtcggagg atgctaacac ccccgggggt      1200
   gggggcactt tgcagatttg gagaatgata gacttcattt acaggcctga agacgaagtt      1260
   ttggcagaac ttgacgaagt gcgacctcaa ttggtgtctc aaaacacccc tgccagttag      1320
<210> 36
   <211> 439
   <212> PRT
   <213> PHYSCOMITRELLA PATENS
<220>
   <221> misc_feature
   <222> (122)..(122)
   <223> Xaa can be any naturally occurring amino acid
<220>
   <221> misc_feature
   <222> (313)..(313)
   <223> Xaa can be any naturally occurring amino acid
<400> 36
   Met Lys Lys Val Pro Gly Asp Arg Tyr Gly Gln Trp Lys Leu Leu Val
   1               5                   10                  15
   Pro Ala Leu Tyr Asp Trp Leu Thr Asn His His Leu Met Gly Pro Ser
               20                  25                  30
   Leu Ser Cys Arg Trp Gly Pro Gln Leu Glu Ala Gly Ser Ser Lys Thr
           35                  40                  45
   Arg Gln Leu Leu Phe Tyr Ser Glu Arg Thr Asp Gly Glu Cys Pro Asn
           50                  55                  60
   Thr Ile Val Val Ala Gln Cys Asp Ile Met Lys Pro Arg Thr Ala Ala
   65                  70                  75                  80
   Ala Glu Gln Ile Ser Gln Phe Lys Glu Gly Gly Lys Ser Pro His Leu
                   85                  90                  95
   Lys Lys Leu Lys Thr Ile Ile His Pro Gly Glu Val Asn Arg Ile Arg
               100                 105                 110
   Glu Ile Pro Gln Asn Ser Asn Ile Leu Xaa Thr His Thr Asp Ser Pro
           115                 120                 125
   Lys Val Leu Ile Trp Asn Thr Lys Thr Gln Pro Asn Arg Ala Thr Thr
       130                 135                 140
   Ser Ala Ala Ser Glu Ser Lys Pro Asp Leu Val Leu Ile Gly His Thr
   145                 150                 155                 160
   Asp Asn Ala Glu Phe Ala Leu Asn Val Ser Arg Thr Ala Pro Tyr Val
                   165                 170                 175
   Ile Ser Gly Gly Lys Thr Pro Lys Ser Ile Thr Pro Thr Ala Ala Gly
               180                 185                 190
   Ser Lys Gln Ser Gly Thr Ala Gly Gly Ala Ala Asp Thr Thr Asn Val
           195                 200                 205
   Tyr Thr Arg Gly Ile Phe Lys Gly His Thr Asp Thr Val Glu Asp Val
       210                 215                 220
   Gln Phe Arg Pro Ser Ser Met Asn Glu Phe Cys Ser Val Gly Asp Asp
   225                 230                 235                 240
   Ser Cys Leu Leu Leu Trp Asp Ala Arg Thr Gly Tyr Gln Pro Ile Ser
                   245                 250                 255
   Lys Val Val Lys Ala His Asn Ala Asp Leu His Cys Val Asp Trp Asn
               260                 265                 270
   Ala His Asp Glu Asn Leu Ile Leu Thr Gly Ser Ala Asp Asn Ser Val
           275                 280                 285
   Arg Leu Phe Asp His Arg Lys Ile Leu Ala Arg Gly Gln Ala Ile Pro
   290 295 300
   Val Glu Gln Phe Glu Gly His Ser Xaa Ala Val Leu Cys Val Gln Trp
   305 310 315 320
   Cys Pro Asp Arg Ala Ser Val Phe Gly Ser Cys Ala Glu Asp Gly Leu
   325 330 335
   Leu Asn Val Trp Asp Tyr Glu Lys Val Gly Lys Ala Leu Asp Thr Thr
   340 345 350
   Asn Leu Lys Gln Pro Val Lys Val Pro Pro Gly Leu Phe Phe Gln His
   355 360 365
   Thr Gly His Arg Asp Lys Val Val Asp Phe His Trp Asp Ser Arg Asp
   370 375 380
   Pro Trp Thr Ile Val Ser Val Ser Glu Asp Ala Asn Thr Pro Gly Gly
   385 390 395 400
   Gly Gly Thr Leu Gln Ile Trp Arg Met Ile Asp Phe Ile Tyr Arg Pro
   405 410 415
   Glu Asp Glu Val Leu Ala Glu Leu Asp Glu Val Arg Pro Gln Leu Val
   420 425 430
   Ser Gln Asn Thr Pro Ala Ser
   435
<210> 37
   <211> 36
   <212> PRT
   <213> ZEA MAYS
<400> 37
   Asp Gly Ser Val Pro Asn Thr Leu Val Ile Ala Asn Cys Glu Val Val
   1               5                   10                  15
   Lys Pro Arg Val Ala Ala Ala Glu His Ile Ser Gln Phe Asn Glu Glu
               20                  25                  30
   Ala Arg Ser Pro
           35
<210> 38
   <211> 36
   <212> PRT
   <213> SORGHUM BICOLOR
<400> 38
   Asp Gly Ser Val Pro Asn Thr Leu Val Ile Ala Asn Cys Glu Val Val
   1               5                   10                  15
   Lys Pro Arg Val Ala Ala Ala Glu His Ile Ser Gln Phe Asn Glu Glu
               20                  25                  30
   Ala Arg Ser Pro
           35
<210> 39
   <211> 36
   <212> PRT
   <213> ZEA MAYS
<400> 39
   Asp Gly Ser Val Pro Asn Thr Leu Val Ile Ala Asn Cys Glu Val Val
   1               5                   10                  15
   Lys Pro Arg Val Ala Ala Ala Glu His Ile Ser Gln Phe Asn Glu Glu
               20                  25                  30
   Ala Arg Ser Pro
           35
<210> 40
   <211> 36
   <212> PRT
   <213> GLYCINE MAX
<400> 40
   Asp Gly Ser Val Pro Asn Thr Leu Val Ile Ala Asn Cys Glu Val Val
   1 5 10 15
   Lys Pro Arg Val Ala Ala Ala Glu His Ile Ser Gln Phe Asn Glu Glu
   20 25 30
   Ala Arg Ser Pro
   35
<210> 41
   <211> 36
   <212> PRT
   <213> ARABIDOPSIS THALIANA
<400> 41
   Asp Gly Ser Val Pro Asn Thr Leu Val Ile Ala Asn Cys Glu Val Val
   1 5 10 15
   Lys Pro Arg Val Ala Ala Ala Glu His Ile Ser Gln Phe Asn Glu Glu
   20 25 30
   Ala Arg Ser Pro
   35
<210> 42
   <211> 36
   <212> PRT
   <213> PISUM SATIVUM
<400> 42
   Asp Gly Ser Val Pro Asn Thr Leu Val Ile Ala Asn Cys Glu Val Val
   1 5 10 15
   Lys Thr Arg Val Ala Ala Ala Glu His Ile Ser Gln Phe Asn Glu Glu
   20 25 30
   Ala Arg Ser Pro
   35
<210> 43
   <211> 36
   <212> PRT
   <213> MEDICAGO TRUNCATULA
<400> 43
   Asp Gly Ser Val Pro Asn Thr Leu Val Ile Ala Asn Cys Glu Val Val
   1               5                   10                  15
   Lys Thr Arg Val Ala Ala Ala Glu His Ile Ser Gln Phe Asn Glu Glu
               20                  25                  30
   Ala Arg Ser Pro
           35
<210> 44
   <211> 36
   <212> PRT
   <213> POPULUS
<400> 44
   Asp Gly Ser Val Pro Asn Thr Leu Val Ile Ala Asn Cys Asp Val Val
   1               5                   10                  15
   Lys Ser Arg Val Ala Ala Ala Glu His Ile Ser Gln Phe Asn Glu Glu
               20                  25                  30
   Ala Arg Ser Pro
           35
<210> 45
   <211> 36
   <212> PRT
   <213> GOSSYPIUM HIRSUTUM
<400> 45
   Asp Gly Ser Val Pro Asn Thr Leu Val Ile Ala Asn Cys Glu Val Val
   1               5                   10                  15
   Lys Pro Arg Val Ala Ala Ala Glu His Ile Ser Gln Phe Asn Glu Lys
               20                  25                  30
   Thr Arg Ser Pro
           35
<210> 46
   <211> 36
   <212> PRT
   <213> PETUNIA HYBRIDA
<400> 46
   Asp Gly Ser Val Pro Asn Thr Leu Val Ile Ala Asn Cys Glu Val Val
   1               5                   10                  15
   Lys Pro Arg Val Ala Ala Ala Glu His Ile Ser Gln Phe Asn Glu Glu
               20                  25                  30
   Ser Arg Ser Pro
           35
<210> 47
   <211> 36
   <212> PRT
   <213> ORYZA SATIVA
<400> 47
   Asp Gly Thr Val Pro Asn Thr Leu Val Ile Ala Asn Cys Glu Val Val
   1               5                   10                  15
   Lys Pro Arg Val Ala Ala Ala Glu His Ile Ser Gln Phe Asn Glu Glu
               20                  25                  30
   Ala Arg Ser Pro
           35
<210> 48
   <211> 36
   <212> PRT
   <213> ORYZA SATIVA
<400> 48
   Asp Gly Thr Val Pro Asn Thr Leu Val Ile Ala Asn Cys Glu Val Val
   1               5                   10                  15
   Lys Pro Arg Val Ala Ala Ala Glu His Ile Ser Gln Phe Asn Glu Glu
               20                  25                  30
   Ala Arg Ser Pro
           35
<210> 49
   <211> 36
   <212> PRT
   <213> VITIS VINIFERA
<400> 49
   Asp Gly Ser Val Pro Asn Thr Leu Val Ile Ala Asn Cys Glu Val Val
   1 5 10 15
   Lys Pro Arg Val Ala Ala Ala Glu His Ile Ala Gln Phe Asn Glu Glu
   20 25 30
   Ala Arg Ser Pro
   35
<210> 50
   <211> 36
   <212> PRT
   <213> MEDICAGO TRUNCATULA
<400> 50
   Asp Gly Thr Ala Pro Asn Thr Leu Val Ile Ala Thr Cys Glu Ile Val
   1 5 10 15
   Lys Pro Arg Val Ala Ala Ala Glu His Ile Ala Met Phe Asn Glu Glu
   20 25 30
   Ala Arg Ser Pro
   35
<210> 51
   <211> 108
   <212> DNA
   <213> PISUM SATIVUM
<400> 51
   gatggtagtg tcccaaatac tttggtgatt gcgaattgtg aggttgtgaa aactagggtt 60
   gcagctgcag agcatatttc acagtttaat gaggaggctc gctctcca 108
<210> 52
   <211> 108
   <212> DNA
   <213> MEDICAGO TRUNCATULA
<400> 52
   gatggaagtg ttccgaatac tttggtgatt gcgaattgtg aggttgtgaa gactagggtt 60
   gcagctgctg aacacatttc tcagtttaat gaagaggctc gctctcca 108
<210> 53
   <211> 108
   <212> DNA
   <213> GLYCINE MAX
<400> 53
   gatggtagtg tgccgaatac tctggtgatt gcgaattgcg aggttgtgaa gcctagggtt 60
   gctgctgctg agcacatttc gcagtttaat gaagaggcgc ggtcccca 108
<210> 54
   <211> 108
   <212> DNA
   <213> GOSSYPIUM HIRSUTUM
<400> 54
   gatggtagtg ttccgaatac tcttgtgatt gctaattgtg aagttgtcaa acctagggtt 60
   gctgctgctg agcacatatc tcagtttaat gaaaaaacac gctcacca 108
<210> 55
   <211> 108
   <212> DNA
   <213> POPULUS
<400> 55
   gatggtagtg ttccaaatac tttggtcatt gcaaattgtg atgttgtcaa gtctagagtt 60
   gctgctgcgg aacacatatc tcagtttaat gaagaagcac gctctcca 108
<210> 56
   <211> 108
   <212> DNA
   <213> PETUNIA HYBRIDA
<400> 56
   gatggaagtg tgccaaacac tttggttata gcaaattgtg aagttgttaa acctagggtt 60
   gcagctgcag agcacatatc acagttcaat gaagaatcac gatctcct 108
<210> 57
   <211> 108
   <212> DNA
   <213> ARABIDOPSIS THALIANA
<400> 57
   gatggaagtg tgcccaatac tttggtcata gcaaattgtg aagttgttaa gccaagggtt 60
   gctgcagcag agcacatttc tcagttcaat gaagaagcac gttctcca 108
<210> 58
   <211> 108
   <212> DNA
   <213> VITIS VINIFERA
<400> 58
   gatggcagtg ttccgaatac cttagttata gcaaactgtg aagttgttaa acctagggta 60
   gctgccgcag agcatattgc tcagttcaac gaggaagcgc ggtcaccc 108
<210> 59
   <211> 108
   <212> DNA
   <213> ZEA MAYS
<400> 59
   gatgggagtg tgcctaatac tctggttatc gcaaactgtg aagttgtgaa accaagggtt 60
   gcagctgctg aacatatctc gcagtttaac gaggaagcac gatcacct 108
<210> 60
   <211> 108
   <212> DNA
   <213> SORGHUM BICOLOR
<400> 60
   gatgggagtg tgcctaatac tctagttatc gcaaactgtg aagttgtgaa gccacgggtt 60
   gcagctgctg aacatatctc gcagtttaac gaggaagcac gatcacct 108
<210> 61
   <211> 108
   <212> DNA
   <213> ZEA MAYS
<400> 61
   gatgggagtg tgcctaatac tctggttatc gcaaattgtg aagttgtgaa acctagggtt 60
   gcagctgctg aacatatctc acagttcaat gaggaagcac ggtcacct 108
<210> 62
   <211> 108
   <212> DNA
   <213> ORYZA SATIVA
<400> 62
   gatgggactg tccctaatac tctggttatt gcaaattgtg aagttgtcaa accaagggtt 60
   gcagctgctg aacatatatc tcagttcaat gaggaagcac gatcacct 108
<210> 63
   <211> 108
   <212> DNA
   <213> ORYZA SATIVA
<400> 63
   gatgggactg tccctaatac tctggttatt gcaaattgtg aagttgtcaa accaagggtt 60
   gcagctgctg aacatatatc tcagttcaat gaggaagcac gatcacct 108
<210> 64
   <211> 108
   <212> DNA
   <213> MEDICAGO TRUNCATULA
<400> 64
   gatggaactg ctcccaatac gcttgttatt gctacttgtg aaattgttaa acctagggtt 60
   gctgctgctg aacatattgc tatgtttaat gaagaggcgc gttctcca 108
<210> 65
   <211> 40
   <212> PRT
   <213> GOSSYPIUM HIRSUTUM
<400> 65
   Ile Gln Asp His Ile Thr Thr Met Ala Thr Asp Pro Thr Lys Ser Pro
   1               5                   10                  15
   Gly Ser Gly Gly Ser Ile Ile Lys Gln Asn Lys Pro Gly Glu Gly Asn
               20                  25                  30
   Asp Lys Ala Ala Asp Gly Pro Ser
           35                  40
<210> 66
   <211> 39
   <212> PRT
   <213> POPULUS
<400> 66
   Ile Gln Asp His Ile Thr Ser Ser Ala Ser Asp Pro Ala Thr Lys Ser
   1               5                   10                  15
   Pro Gly Ser Gly Gly Ser Ile Ile Lys Lys Thr Gly Asp Gly Ser Asp
               20                  25                  30
   Lys Ala Thr Asp Gly Pro Ser
           35
<210> 67
   <211> 37
   <212> PRT
   <213> GLYCINE MAX
<400> 67
   Ile Glu Asp His Ile Thr Ser Ala Ala Thr Asp Ser Lys Ser Gly Gly
   1               5                   10                  15
   Ser Ile Ile Lys Gln Asn Ser Lys Ser Gly Glu Gly Asn Asp Lys Thr
               20                  25                  30
   Ala Asp Gly Pro Thr
           35
<210> 68
   <211> 36
   <212> PRT
   <213> S: PISUM SATIVUM
<400> 68
   Ile Glu Asp His Val Thr Ser Ala Ala Thr Asp Lys Ser Gly Gly Ser
   1               5                   10                  15
   Ile Ile Lys Pro Asn Ser Lys Ser Gly Glu Gly Asn Asp Lys Thr Val
               20                  25                  30
   Asp Ser Pro Ser
           35
<210> 69
   <211> 27
   <212> PRT
   <213> MEDICAGO TRUNCATULA
<400> 69
   Ile Glu Asp His Ile Thr Ser Ala Ala Thr Asp Ser Asn Lys Ser Gly
   1               5                   10                  15
   Gly Ser Ile Ala Lys Thr Ala Asp Ser Pro Thr
               20                  25
<210> 70
   <211> 34
   <212> PRT
   <213> MEDICAGO TRUNCATULA
<400> 70
   Ile His Asp His Ile Ala Thr Leu Ala Thr Glu Glu Glu Pro Asp Val
   1               5                   10                  15
   Asn Glu Gly Ser Asn Val Gly Gly Asn Ser Glu Lys Ala Ala Gln Ser
               20                  25                  30
   Pro Ser
<210> 71
   <211> 33
   <212> PRT
   <213> VITIS VINIFERA
<400> 71
   Ile Gln Asp His Ile Ser Thr Leu Ala Ala Asp Pro Gly Ser Ala Lys
   1               5                   10                  15
   Ser Thr Ser Lys Ala Gly Gly Gly Asn Asp Lys Pro Val Glu Ser Pro
               20                  25                  30
   Ser
<210> 72
   <211> 33
   <212> PRT
   <213> PETUNIA HYBRIDA
<400> 72
   Ile Gln Asp His Ile Ser Thr Leu Ser Thr Asp Ala Gln Lys Pro Ala
   1               5                   10                  15
   Gly Phe Ile Lys Pro Ala Thr Thr Ser Ile Lys Ala Gly Asp Asn Pro
                       20                  25                  30
   Ser
<210> 73
   <211> 33
   <212> PRT
   <213> ARABIDOPSIS THALIANA
<400> 73
   Ile Gln Asp His Ile Thr Thr Ile Gly Thr Asp Ser Lys Ser Ser Gly
   1               5                   10                  15
   Ser Ile Ile Lys Gln Thr Gly Glu Gly Thr Asp Lys Asn Glu Ser Pro
               20                  25                  30
   Thr
<210> 74
   <211> 34
   <212> PRT
   <213> ZEA MAYS
<400> 74
   Ile Gln Asp His Ile Ser Ala Leu Gly Asp Ser Ser Ser Ser Pro Gly
   1               5                   10                  15
   Ala Ser Gly Ser Lys Gln Ser Ile Lys Thr Ala Asn Glu Lys Glu Ser
               20                  25                  30
   Pro Lys
<210> 75
   <211> 34
   <212> PRT
   <213> ZEA MAYS
<400> 75
   Ile Gln Asp His Ile Ser Ala Leu Gly Asp Ser Ser Ser Ser Pro Gly
   1 5 10 15
   Ala Ser Gly Ser Lys Gln Ser Gly Lys Ile Ala Asn Glu Lys Glu Ser
   20 25 30
   Pro Lys
<210> 76
   <211> 34
   <212> PRT
   <213> SORGHUM BICOLOR
<400> 76
   Ile Gln Asp His Ile Ser Ala Leu Gly Asp Ser Ser Ser Ser Pro Gly
   1 5 10 15
   Ala Ser Gly Ser Lys Gln Ser Gly Lys Ser Ala Thr Glu Lys Glu Ser
   20 25 30
   Pro Lys
<210> 77
   <211> 35
   <212> PRT
   <213> ORYZA SATIVA
<400> 77
   Ile Gln Asp His Ile Ser Ala Leu Gly Asp Ser Ser Lys Thr Glu Ser
   1 5 10 15
   Ser Pro Gly Ala Ser Gly Ser Lys Gly Lys Thr Ala Asn Asp Lys Asp
   20 25 30
   Ser Pro Lys
   35
<210> 78
   <211> 35
   <212> PRT
   <213> ORYZA SATIVA
<400> 78
   Ile Gln Asp His Ile Ser Ala Leu Gly Asp Ser Ser Lys Thr Glu Ser
   1               5                   10                  15
   Ser Pro Gly Ala Ser Gly Ser Lys Gly Lys Thr Ala Asn Asp Lys Asp
               20                  25                  30
   Ser Pro Lys
           35
<210> 79
   <211> 102
   <212> DNA
   <213> MEDICAGO TRUNCATULA
<400> 79
   attcatgatc atattgcaac tttagctaca gaagaagaac ccgatgttaa tgagggctct 60
   aatgttgggg gaaatagcga gaaagctgca caaagcccgt ct 102
<210> 80
   <211> 99
   <212> DNA
   <213> VITIS VINIFERA
<400> 80
   attcaggatc acatctcgac tttggcagca gatccagggt cagcaaagag tacctctaag 60
   gctggtggtg gtaatgataa acctgtagaa agcccttct 99
<210> 81
   <211> 108
   <212> DNA
   <213> PISUM SATIVUM
<400> 81
   attgaagacc atgtaacatc tgctgctacg gacaagtctg gtggatccat tatcaaaccg 60
   aactctaaat ctggggaagg caatgacaaa actgttgata gcccttct 108
<210> 82
   <211> 120
   <212> DNA
   <213> GOSSYPIUM HIRSUTUM
<400> 82
   atccaggacc acataacaac aatggctaca gaccctacta aatctcctgg atctggcgga 60
   tcaatcatca aacaaaacaa gcctggggaa ggtaatgaca aagctgctga tgggccttct 120
<210> 83
   <211> 117
   <212> DNA
   <213> POPULUS
<400> 83
   atccaggacc atataacatc atctgcctct gatccagcaa ctaagtctcc aggatctggt 60
   ggatcaatca ttaaaaagac tggggatggc agtgataaag ccactgatgg cccttct 117
<210> 84
   <211> 111
   <212> DNA
   <213> GLYCINE MAX
<400> 84
   attgaagacc atataacatc tgctgccaca gactccaaat ctggtgggtc aattatcaaa 60
   caaaactcta aatctggaga aggcaatgat aaaactgctg atggccctac t 111
<210> 85
   <211> 81
   <212> DNA
   <213> MEDICAGO TRUNCATULA
<400> 85
   attgaagacc atataacatc tgctgctaca gactccaaca agtctggtgg atccattgcc 60
   aaaactgctg atagccctac t 81
<210> 86
   <211> 99
   <212> DNA
   <213> PETUNIA HYBRIDA
<400> 86
   attcaagatc atatatcaac attgagcaca gatgcacaaa aacctgctgg tttcatcaag 60
   ccagccacta catctattaa ggctggtgat aatccctct 99
<210> 87
   <211> 99
   <212> DNA
   <213> ARABIDOPSIS THALIANA
<400> 87
   atccaagatc acatcacaac gattgggaca gattccaaat catctggatc tatcatcaaa 60
   cagactggtg aaggtactga taagaatgag agtcctact 99
<210> 88
   <211> 102
   <212> DNA
   <213> ZEA MAYS
<400> 88
   atccaagacc acatatctgc ccttggggat tcctcgtctt ctcccggagc atctggcagc 60
   aagcagtctg gcaaaattgc aaatgaaaag gagagtccta aa 102
<210> 89
   <211> 102
   <212> DNA
   <213> SORGHUM BICOLOR
<400> 89
   atccaagacc acatatctgc ccttggggat tcctcgtctt ctcctggagc atctggcagc 60
   aagcagtctg gtaaatctgc aactgaaaag gagagcccta aa 102
<210> 90
   <211> 102
   <212> DNA
   <213> ZEA MAYS
<400> 90
   atccaagacc atatatctgc ccttggggat tcctcgtctt ctcctggagc atctggcagc 60
   aagcagtcta ttaaaactgc aaatgaaaag gagagcccta aa 102
<210> 91
   <211> 105
   <212> DNA
   <213> ORYZA SATIVA
<400> 91
   atccaagacc acatatctgc actgggagat tcctcgaaaa ctgaatcttc tccaggggca 60
   tcaggaagca agggaaaaac tgcaaatgat aaggatagtc ctaaa 105
<210> 92
   <211> 105
   <212> DNA
   <213> ORYZA SATIVA
<400> 92
   atccaagacc acatatctgc actgggagat tcctcgaaaa ctgaatcttc tccaggggca      60
   tcaggaagca agggaaaaac tgcaaatgat aaggatagtc ctaaa                     105
<210> 93
   <211> 40
   <212> PRT
   <213> GLYCINE MAX
<400> 93
   Ser Arg Pro Asp Leu Ile Leu Thr Gly His Gln Asp Asn Ala Glu Phe
   1               5                   10                  15
   Ala Leu Ala Met Cys Pro Thr Glu Pro Tyr Val Leu Ser Gly Gly Lys
               20                  25                  30
   Asp Lys Thr Val Val Leu Trp Ser
           35                  40
<210> 94
   <211> 40
   <212> PRT
   <213> PISUM SATIVUM
<400> 94
   Ser Arg Pro Asp Leu Ile Leu Thr Gly His Gln Asp Asn Ala Glu Phe
   1               5                   10                  15
   Ala Leu Ala Met Cys Pro Thr Glu Pro Tyr Val Leu Ser Gly Gly Lys
               20                  25                  30
   Asp Lys Thr Val Val Leu Trp Ser
           35                  40
<210> 95
   <211> 40
   <212> PRT
   <213> MEDICAGO TRUNCATULA
<400> 95
   Ser Arg Pro Asp Leu Ile Leu Thr Gly His Gln Asp Asn Ala Glu Phe
   1 5 10 15
   Ala Leu Ala Met Cys Pro Thr Gln Pro Tyr Val Leu Ser Gly Gly Lys
   20 25 30
   Asp Lys Thr Val Val Leu Trp Ser
   35 40
<210> 96
   <211> 40
   <212> PRT
   <213> POPULUS
<400> 96
   Ser Arg Pro Asp Leu Ile Leu Thr Gly His Gln Asp Asn Ala Glu Phe
   1 5 10 15
   Ala Leu Ala Met Cys Pro Thr Asp Pro Tyr Val Leu Ser Gly Gly Lys
   20 25 30
   Asp Lys Phe Val Val Leu Trp Ser
   35 40
<210> 97
   <211> 40
   <212> PRT
   <213> GOSSYPIUM HIRSUTUM
<400> 97
   Ser Arg Pro Asp Leu Ile Leu Thr Gly His Gln Asp Asn Ala Glu Phe
   1 5 10 15
   Ala Leu Ala Met Cys Pro Thr Glu Pro Tyr Val Leu Ser Gly Gly Lys
   20 25 30
   Asp Lys Ser Val Val Leu Trp Ser
   35 40
<210> 98
   <211> 40
   <212> PRT
   <213> ZEA MAYS
<400> 98
   Ser Arg Pro Asp Leu Ile Leu Thr Gly His Lys Glu Asn Ala Glu Phe
   1 5 10 15
   Ala Leu Ala Met Cys Pro Ala Glu Pro Tyr Val Leu Ser Gly Gly Lys
   20 25 30
   Asp Lys Ser Val Val Leu Trp Ser
   35 40
<210> 99
   <211> 40
   <212> PRT
   <213> SORGHUM BICOLOR
<400> 99
   Ser Arg Pro Asp Leu Ile Leu Thr Gly His Lys Glu Asn Ala Glu Phe
   1 5 10 15
   Ala Leu Ala Met Cys Pro Ala Glu Pro Tyr Val Leu Ser Gly Gly Lys
   20 25 30
   Asp Lys Ser Val Val Leu Trp Ser
   35 40
<210> 100
   <211> 40
   <212> PRT
   <213> ORYZA SATIVA
<400> 100
   Ser Arg Pro Asp Leu Ile Leu Arg Gly His Lys Asp Ile Ala Glu Phe
   1 5 10 15
   Ala Leu Ala Met Cys Pro Ala Glu Pro Tyr Val Leu Ser Gly Gly Lys
   20 25 30
   Asp Lys Ser Val Val Trp Trp Ser
   35 40
<210> 101
   <211> 40
   <212> PRT
   <213> ORYZA SATIVA
<400> 101
   Ser Arg Pro Asp Leu Ile Leu Arg Gly His Lys Asp Ile Ala Glu Phe
   1 5 10 15
   Ala Leu Ala Met Cys Pro Ala Glu Pro Tyr Val Leu Ser Gly Gly Lys
   20 25 30
   Asp Lys Ser Val Val Trp Trp Ser
   35 40
<210> 102
   <211> 40
   <212> PRT
   <213> ZEA MAYS
<400> 102
   Ser Arg Pro Asp Leu Ile Leu Thr Gly His Gln Glu Asn Ala Glu Phe
   1 5 10 15
   Ala Leu Ala Met Cys Pro Ala Glu Pro Tyr Val Leu Ser Gly Gly Lys
   20 25 30
   Asp Lys Phe Val Val Leu Trp Ser
   35 40
<210> 103
   <211> 40
   <212> PRT
   <213> ARABIDOPSIS THALIANA
<400> 103
   Ser Arg Pro Asp Leu Ile Leu Thr Gly His Gln Asp Asn Ala Glu Phe
   1 5 10 15
   Ala Leu Ala Met Cys Pro Thr Glu Pro Phe Val Leu Ser Gly Gly Lys
   20 25 30
   Asp Lys Ser Val Val Leu Trp Ser
   35 40
<210> 104
   <211> 40
   <212> PRT
   <213> PETUNIA HYBRIDA
<400> 104
   Ser Arg Pro Asp Leu Thr Leu Ile Gly His Ser Glu Asn Ala Glu Phe
   1 5 10 15
   Ala Leu Ala Met Cys Pro Thr Glu Pro Phe Val Leu Ser Gly Gly Lys
   20 25 30
   Asp Lys Ser Val Val Leu Trp Ser
   35 40
<210> 105
   <211> 40
   <212> PRT
   <213> VITIS VINIFERA
<400> 105
   Lys Val Leu Phe Leu Ile Leu Thr Gly His Lys Asp Asn Ala Glu Phe
   1 5 10 15
   Ala Leu Ala Met Cys Pro Thr Glu Pro Leu Val Leu Ser Gly Gly Lys
   20 25 30
   Asp Lys Ser Val Val Leu Trp Ser
   35 40
<210> 106
   <211> 40
   <212> PRT
   <213> MEDICAGO TRUNCATULA
<400> 106
   Ser Ile Pro Asp Leu Val Leu Thr Gly His Lys Asp Asn Ala Glu Phe
   1 5 10 15
   Ala Leu Ala Met Cys Ser Thr Glu Pro Phe Val Leu Ser Gly Gly Arg
   20 25 30
   Asp Lys Leu Val Val Leu Trp Ser
   35 40
<210> 107
   <211> 120
   <212> DNA
   <213> PISUM SATIVUM
<400> 107
   tctcgtccag atttgatatt gaccggacac caagacaatg ctgagtttgc tcttgcgatg        60
   tgcccaactg agccttatgt cctttcagga ggaaaagata aaacagtggt gttgtggagt       120
<210> 108
   <211> 120
   <212> DNA
   <213> MEDICAGO TRUNCATULA
<400> 108
   tctcgtccag atctgatatt gactggacac caagataatg cagagtttgc tcttgcaatg        60
   tgccctactc agccctatgt gctttctgga ggaaaagaca aaacagtcgt gttgtggagt       120
<210> 109
   <211> 120
   <212> DNA
   <213> GLYCINE MAX
<400> 109
   tctcgtcctg atttgatatt gactggacac caagataatg cggaatttgc tcttgcaatg        60
   tgcccaactg aaccctatgt tctttcagga ggaaaggaca aaacagtggt gttgtggagt       120
<210> 110
   <211> 120
   <212> DNA
   <213> GOSSYPIUM HIRSUTUM
<400> 110
   tctcgcccag atttgatttt gactgggcat caagataatg ctgaatttgc tcttgcaatg        60
   tgtccaactg agccttatgt gctctctgga gggaaggaca aatcagtggt tttgtggagc       120
<210> 111
   <211> 120
   <212> DNA
   <213> POPULUS
<400> 111
   tctcgtccag atttgatttt gactggacat caagacaatg ctgagtttgc ccttgcaatg        60
   tgcccaactg atccctatgt gctttctgga gggaaggaca agttcgtagt tttgtggagt       120
<210> 112
   <211> 120
   <212> DNA
   <213> SORGHUM BICOLOR
<400> 112
   tctcgccctg atctgatatt aacgggacat aaggaaaatg cggaatttgc gcttgctatg 60
   tgcccagcag aaccatatgt cctatcagga ggaaaggaca aatctgttgt cttgtggagc 120
<210> 113
   <211> 120
   <212> DNA
   <213> ZEA MAYS
<400> 113
   tctcgccctg atctgatatt aacaggacat aaggaaaatg cggaatttgc gcttgccatg 60
   tgtccagcag aaccatatgt cctatcagga ggaaaggaca aatctgttgt cttgtggagc 120
<210> 114
   <211> 120
   <212> DNA
   <213> ZEA MAYS
<400> 114
   tctcgtcctg atctgatatt aacgggacac caggaaaatg cagaatttgc gcttgccatg 60
   tgtccagcag aaccatatgt cctgtcagga ggaaaggaca aatttgttgt cttgtggagc 120
<210> 115
   <211> 120
   <212> DNA
   <213> ORYZA SATIVA
<400> 115
   tctcgtcctg atttgatatt aagaggacat aaggatattg ctgagtttgc gcttgctatg 60
   tgcccagctg agccatatgt gttatctgga ggaaaagaca aatctgttgt atggtggagc 120
<210> 116
   <211> 120
   <212> DNA
   <213> ORYZA SATIVA
<400> 116
   tctcgccctg atttgatatt aagaggacat aaggatattg ctgagtttgc gcttgctatg 60
   tgcccagctg agccatatgt gttatctgga ggaaaagaca aatctgttgt atggtggagc 120
<210> 117
   <211> 120
   <212> DNA
   <213> PETUNIA HYBRIDA
<400> 117
   tcacgcccag atttgacatt gattggacat agtgagaatg cagaatttgc actggcaatg       60
   tgccccactg aaccctttgt gctctctgga ggaaaggaca aatctgtggt actgtggagt      120
<210> 118
   <211> 120
   <212> DNA
   <213> ARABIDOPSIS THALIANA
<400> 118
   tcccgtccag atttgatact aactgggcac caagataatg ctgaatttgc tcttgccatg       60
   tgcccaacgg aaccctttgt gctctccgga ggcaaggaca agtcagttgt tttgtggagt      120
<210> 119
   <211> 120
   <212> DNA
   <213> MEDICAGO TRUNCATULA
<400> 119
   tccatcccag acttggtatt aactggacat aaggataatg ctgaatttgc actagctatg       60
   tgttcaactg agccctttgt tctttccgga gggagagaca agcttgtggt gttatggagt      120
<210> 120
   <211> 120
   <212> DNA
   <213> VITIS VINIFERA
<400> 120
   aaggttttgt tcctgatatt aactgggcat aaagataatg cagaatttgc tcttgccatg       60
   tgtccaactg aaccattggt gctctctgga ggcaaggata agtctgtggt gttgtggagt      120
<210> 121
   <211> 40
   <212> PRT
   <213> POPULUS
<400> 121
   Phe Val Lys Lys Tyr Lys Thr Ile Ile His Pro Gly Glu Val Asn Arg
   1               5                   10                  15
   Ile Arg Glu Leu Pro Gln Asn Ser Lys Ile Val Ala Thr His Thr Asp
               20                  25                  30
   Ser Pro Asp Val Leu Ile Trp Asp
           35                  40
<210> 122
   <211> 40
   <212> PRT
   <213> ARABIDOPSIS THALIANA
<400> 122
   Phe Val Lys Lys Tyr Lys Thr Ile Ile His Pro Gly Glu Val Asn Arg
   1               5                   10                  15
   Ile Arg Glu Leu Pro Gln Asn Ser Lys Ile Val Ala Thr His Thr Asp
               20                  25                  30
   Ser Pro Asp Val Leu Ile Trp Asp
           35                  40
<210> 123
   <211> 40
   <212> PRT
   <213> PISUM SATIVUM
<400> 123
   Phe Val Lys Lys Tyr Lys Thr Ile Ile His Pro Gly Glu Val Asn Arg
   1               5                   10                  15
   Ile Arg Glu Leu Pro Gln Asn Ser Lys Ile Val Ala Thr His Thr Asp
               20                  25                  30
   Ser Pro Asp Val Leu Ile Trp Asp
           35                  40
<210> 124
   <211> 40
   <212> PRT
   <213> MEDICAGO TRUNCATULA
<400> 124
   Phe Val Lys Lys Tyr Lys Thr Ile Ile His Pro Gly Glu Val Asn Arg
   1               5                   10                  15
   Ile Arg Glu Leu Pro Gln Asn Ser Lys Ile Val Ala Thr His Thr Asp
               20                  25                  30
   Ser Pro Asp Val Leu Ile Trp Asp
           35                  40
<210> 125
   <211> 40
   <212> PRT
   <213> ZEA MAYS
<400> 125
   Phe Val Lys Lys Tyr Lys Thr Ile Val His Pro Gly Glu Val Asn Arg
   1               5                   10                  15
   Ile Arg Glu Leu Pro Gln Asn Ser Lys Ile Ile Ala Thr His Thr Asp
               20                  25                  30
   Ser Pro Asp Val Leu Ile Trp Asp
           35                  40
<210> 126
   <211> 40
   <212> PRT
   <213> SORGHUM BICOLOR
<400> 126
   Phe Val Lys Lys Tyr Lys Thr Ile Val His Pro Gly Glu Val Asn Arg
   1               5                   10                  15
   Ile Arg Glu Leu Pro Gln Asn Ser Lys Ile Ile Ala Thr His Thr Asp
               20                  25                  30
   Ser Pro Asp Val Leu Ile Trp Asp
           35                  40
<210> 127
   <211> 40
   <212> PRT
   <213> ZEA MAYS
<400> 127
   Phe Val Lys Lys Tyr Lys Thr Ile Val His Pro Gly Glu Val Asn Arg
   1               5                   10                  15
   Ile Arg Glu Leu Pro Gln Asn Ser Lys Ile Ile Ala Thr His Thr Asp
               20                  25                  30
   Ser Pro Asp Val Leu Val Trp Asp
           35                  40
<210> 128
   <211> 40
   <212> PRT
   <213> ORYZA SATIVA
<400> 128
   Phe Val Lys Lys Tyr Lys Thr Ile Ile His Pro Gly Glu Val Asn Arg
   1               5                   10                  15
   Ile Arg Glu Leu Pro Gln Asn Ser Lys Ile Ile Ala Thr His Thr Asp
               20                  25                  30
   Ser Pro Asp Val Leu Ile Trp Asp
           35                  40
<210> 129
   <211> 40
   <212> PRT
   <213> ORYZA SATIVA
<400> 129
   Phe Val Lys Lys Tyr Lys Thr Ile Ile His Pro Gly Glu Val Asn Arg
   1               5                   10                  15
   Ile Arg Glu Leu Pro Gln Asn Ser Lys Ile Ile Ala Thr His Thr Asp
               20                  25                  30
   Ser Pro Asp Val Leu Ile Trp Asp
           35                  40
<210> 130
   <211> 40
   <212> PRT
   <213> GLYCINE MAX
<400> 130
   Phe Val Lys Lys Tyr Lys Thr Ile Ile His Pro Gly Glu Val Asn Arg
   1 5 10 15
   Ile Arg Glu Leu Pro Gln Asn Ser Lys Ile Val Ala Thr His Thr Asp
   20 25 30
   Ser Pro Asp Val Leu Val Trp Asp
   35 40
<210> 131
   <211> 40
   <212> PRT
   <213> VITIS VINIFERA
<400> 131
   Phe Val Lys Lys Phe Lys Thr Ile Ile His Pro Gly Glu Val Asn Arg
   1 5 10 15
   Ile Arg Glu Leu Pro Gln Asn Ser Lys Ile Val Ala Thr His Thr Asp
   20 25 30
   Ser Pro Asp Val Leu Ile Trp Asp
   35 40
<210> 132
   <211> 40
   <212> PRT
   <213> GOSSYPIUM HIRSUTUM
<400> 132
   Phe Val Lys Lys Tyr Lys Thr Ile Ile His Pro Gly Glu Val Asn Arg
   1 5 10 15
   Ile Lys Glu Leu Pro Gln Ser Ser Arg Ile Val Ala Thr His Thr Asp
   20 25 30
   Ser Pro Asp Val Leu Ile Trp Asp
   35 40
<210> 133
   <211> 40
   <212> PRT
   <213> PETUNIA HYBRIDA
<400> 133
   Phe Val Lys Lys Tyr Lys Thr Ile Ile His Pro Gly Glu Val Asn Arg
   1 5 10 15
   Ile Arg Glu Leu Pro Gln Asn Lys Asn Ile Val Ala Thr His Thr Asp
   20 25 30
   Ser Pro Glu Val Leu Ile Trp Asp
   35 40
<210> 134
   <211> 40
   <212> PRT
   <213> MEDICAGO TRUNCATULA
<400> 134
   Phe Val Lys Lys Val Lys Thr Ile Leu His Pro Gly Glu Val Asn Arg
   1 5 10 15
   Ile Arg Glu Leu Pro Ser Asn Thr Asn Ile Val Ala Thr His Thr Asp
   20 25 30
   Ser Pro Asn Val Met Ile Trp Asn
   35 40
<210> 135
   <211> 120
   <212> DNA
   <213> PISUM SATIVUM
<400> 135
   tttgttaaga agtacaagac catcatacat cccggggagg tgaacagaat tagggaattg 60
   ccgcaaaatt ctaagatagt ggctactcac acagacagcc ctgatgttct catttgggat 120
<210> 136
   <211> 120
   <212> DNA
   <213> MEDICAGO TRUNCATULA
<400> 136
   tttgtcaaga agtacaagac tatcatacat ccaggcgagg tgaacagaat tagggaattg 60
   ccacaaaatt ctaagattgt ggccactcac acagacagcc ctgatgttct catttgggat 120
<210> 137
   <211> 120
   <212> DNA
   <213> GLYCINE MAX
<400> 137
   tttgtgaaga agtacaagac catcatacat cctggtgagg taaacagaat tagggaattg 60
   ccacaaaatt ccaagatagt ggctacacat acagacagcc ctgatgtcct tgtttgggat 120
<210> 138
   <211> 120
   <212> DNA
   <213> POPULUS
<400> 138
   tttgttaaga agtacaagac catcatacat cctggagagg taaacagaat cagagaactc 60
   ccccagaata gtaagatagt ggctactcat actgacagcc ctgatgttct tatatgggat 120
<210> 139
   <211> 120
   <212> DNA
   <213> GOSSYPIUM HIRSUTUM
<400> 139
   tttgtgaaga agtacaaaac catcatacat cctggagaag tcaacagaat caaggaactt 60
   ccacagagct ctaggattgt ggcaactcac actgatagtc ctgatgttct tatttgggat 120
<210> 140
   <211> 120
   <212> DNA
   <213> ARABIDOPSIS THALIANA
<400> 140
   tttgtgaaga agtacaagac catcattcac cctggagagg ttaacagaat cagggaactc 60
   ccacagaata gtaagattgt tgctactcac accgacagtc ctgatgttct catttgggat 120
<210> 141
   <211> 120
   <212> DNA
   <213> ZEA MAYS
<400> 141
   tttgtaaaga agtacaagac tatagttcat cctggtgaag ttaacagaat cagggagctt 60
   ccacagaaca gtaagatcat agccacacac actgacagtc cagatgtact tgtttgggat 120
<210> 142
   <211> 120
   <212> DNA
   <213> SORGHUM BICOLOR
<400> 142
   tttgttaaga agtacaagac catagttcat cctggtgaag ttaacagaat cagggagctt 60
   ccacagaaca gtaagatcat agccactcac actgacagtc cagatgtact tatttgggat 120
<210> 143
   <211> 120
   <212> DNA
   <213> ZEA MAYS
<400> 143
   tttgtaaaga agtacaaaac tatagttcat cctggtgagg ttaacagaat cagggaactt 60
   ccacagaaca gtaagatcat agccactcac actgacagtc cagatgtact tatttgggat 120
<210> 144
   <211> 120
   <212> DNA
   <213> ORYZA SATIVA
<400> 144
   tttgtaaaga agtacaagac tataattcat ccaggcgagg tgaacaggat tagggagctt 60
   ccgcagaaca gtaagatcat tgccactcat accgacagcc cagatgttct catttgggat 120
<210> 145
   <211> 120
   <212> DNA
   <213> ORYZA SATIVA
<400> 145
   tttgtaaaga agtacaagac tataattcat ccaggcgagg tgaacaggat tagggagctt 60
   ccgcagaaca gtaagatcat tgccactcat accgacagcc cagatgttct catttgggat 120
<210> 146
   <211> 120
   <212> DNA
   <213> VITIS VINIFERA
<400> 146
   tttgtaaaga agttcaaaac aattatacat ccaggagagg tgaaccgaat cagggaactg 60
   ccacagaata gtaagatagt ggccacacac actgacagtc ctgatgtcct catttgggat 120
<210> 147
   <211> 120
   <212> DNA
   <213> PETUNIA HYBRIDA
<400> 147
   tttgtcaaga agtacaaaac cattatacac ccaggggagg ttaacagaat cagagagctt 60
   cctcaaaata aaaacatagt ggcaacccat actgatagtc ctgaagttct aatttgggat 120
<210> 148
   <211> 120
   <212> DNA
   <213> MEDICAGO TRUNCATULA
<400> 148
   tttgttaaga aggttaaaac tattcttcat cccggtgagg taaatagaat cagagaactc 60
   ccgtcaaata ctaatatagt tgccacacat acagatagtc caaatgttat gatttggaat 120
<210> 149
   <211> 40
   <212> PRT
   <213> VITIS VINIFERA
<400> 149
   Ile Gly Ala Arg Gly Ile Tyr Gln Gly His Asp Asp Thr Val Glu Asp
   1 5 10 15
   Val Gln Phe Cys Pro Leu Ser Ala Gln Glu Phe Cys Ser Val Gly Asp
   20 25 30
   Asp Ser Cys Leu Ile Leu Trp Asp
   35 40
<210> 150
   <211> 40
   <212> PRT
   <213> PETUNIA HYBRIDA
<400> 150
   Ile Gln Ala Arg Gly Ile Phe Gln Gly His Glu Asp Thr Val Glu Asp
   1 5 10 15
   Val Gln Phe Cys Pro Ser Ser Ser Gln Glu Phe Cys Ser Val Gly Asp
   20 25 30
   Asp Ser Cys Leu Ile Leu Trp Asp
   35 40
<210> 151
   <211> 40
   <212> PRT
   <213> PISUM SATIVUM
<400> 151
   Val Gly Pro Arg Gly Ile Tyr Ser Gly His Asp Asp Thr Val Glu Asp
   1 5 10 15
   Val Ala Phe Cys Pro Ser Ser Ala Gln Glu Phe Cys Ser Val Gly Asp
   20 25 30
   Asp Ser Cys Leu Ile Leu Trp Asp
   35 40
<210> 152
   <211> 40
   <212> PRT
   <213> MEDICAGO TRUNCATULA
<400> 152
   Val Gly Pro Arg Gly Ile Tyr Ser Gly His Glu Asp Thr Val Glu Asp
   1 5 10 15
   Val Ala Phe Cys Pro Ser Ser Ala Gln Glu Phe Cys Ser Val Gly Asp
   20 25 30
   Asp Ser Cys Leu Ile Leu Trp Asp
   35 40
<210> 153
   <211> 40
   <212> PRT
   <213> POPULUS
<400> 153
   Val Gly Pro Arg Gly Ile Tyr Gln Gly His Glu Asp Thr Val Glu Asp
   1 5 10 15
   Val Ala Phe Cys Pro Ser Ser Ala Gln Glu Phe Cys Ser Val Gly Asp
   20 25 30
   Asp Ser Cys Leu Ile Leu Trp Asp
   35 40
<210> 154
   <211> 40
   <212> PRT
   <213> ARABIDOPSIS THALIANA
<400> 154
   Val Gly Pro Arg Gly Val Tyr His Gly His Glu Asp Thr Val Glu Asp
   1 5 10 15
   Val Ala Phe Ser Pro Thr Ser Ala Gln Glu Phe Cys Ser Val Gly Asp
   20 25 30
   Asp Ser Cys Leu Ile Leu Trp Asp
   35 40
<210> 155
   <211> 40
   <212> PRT
   <213> GOSSYPIUM HIRSUTUM
<400> 155
   Leu Gly Pro Arg Gly Val Phe Cys Gly His Glu Asp Thr Val Glu Asp
   1 5 10 15
   Val Thr Phe Cys Pro Ser Ser Ala Gln Glu Phe Cys Ser Val Gly Asp
   20 25 30
   Asp Ser Cys Leu Ile Leu Trp Asp
   35 40
<210> 156
   <211> 40
   <212> PRT
   <213> GLYCINE MAX
<400> 156
   Val Gly Pro Arg Gly Ile Tyr Cys Gly His Glu Asp Thr Val Glu Asp
   1 5 10 15
   Val Thr Phe Cys Pro Ser Ser Ala Gln Glu Phe Cys Ser Val Gly Asp
   20 25 30
   Asp Ser Cys Leu Ile Leu Trp Asp
   35 40
<210> 157
   <211> 40
   <212> PRT
   <213> MEDICAGO TRUNCATULA
<400> 157
   Val Gly Ala Arg Gly Val Tyr Arg Gly His Lys Asp Thr Val Glu Asp
   1 5 10 15
   Val Gln Phe Cys Pro Ser Ser Ala Gln Glu Phe Cys Ser Val Gly Asp
   20 25 30
   Asp Ser Cys Leu Ile Leu Trp Asp
   35 40
<210> 158
   <211> 40
   <212> PRT
   <213> ZEA MAYS
<400> 158
   Val Asp Pro Arg Gly Ile Phe His Gly His Asp Ser Thr Val Glu Asp
   1 5 10 15
   Val Gln Phe Cys Pro Ser Ser Ala Gln Glu Phe Cys Ser Val Gly Asp
   20 25 30
   Asp Ala Cys Leu Ile Leu Trp Asp
   35 40
<210> 159
   <211> 40
   <212> PRT
   <213> ZEA MAYS
<400> 159
   Val Asp Pro Arg Gly Ile Phe His Gly His Asp Ser Thr Val Glu Asp
   1               5                   10                  15
   Val Gln Phe Cys Pro Ser Ser Ala Gln Glu Phe Cys Ser Val Gly Asp
               20                  25                  30
   Asp Ala Cys Leu Ile Leu Trp Asp
           35                  40
<210> 160
   <211> 40
   <212> PRT
   <213> ORYZA SATIVA
<400> 160
   Val Asp Pro Arg Gly Ile Phe Leu Gly His Asp Ser Thr Val Glu Asp
   1               5                   10                  15
   Val Gln Phe Cys Pro Ser Ser Ala Gln Glu Phe Cys Ser Val Gly Asp
               20                  25                  30
   Asp Ser Cys Leu Ile Leu Trp Asp
           35                  40
<210> 161
   <211> 40
   <212> PRT
   <213> ORYZA SATIVA
<400> 161
   Val Asp Pro Arg Gly Ile Phe Leu Gly His Asp Ser Thr Val Glu Asp
   1               5                   10                  15
   Val Gln Phe Cys Pro Ser Ser Ala Gln Glu Phe Cys Ser Val Gly Asp
               20                  25                  30
   Asp Ser Cys Leu Ile Leu Trp Asp
           35                  40
<210> 162
   <211> 9
   <212> PRT
   <213> SORGHUM BICOLOR
<400> 162
   Val Asp Pro Arg Gly Ile Phe Cys Gly
   1               5
<210> 163
   <211> 120
   <212> DNA
   <213> PISUM SATIVUM
<400> 163
   gtcgggccaa gaggtatcta ctctgggcac gatgatactg ttgaagatgt ggccttttgc 60
   ccttctagtg cgcaggaatt ctgtagtgtt ggagatgatt cttgtctcat attatgggat 120
<210> 164
   <211> 120
   <212> DNA
   <213> MEDICAGO TRUNCATULA
<400> 164
   gtcggaccaa gaggtatcta ctctgggcat gaggatactg ttgaagacgt ggctttttgc 60
   ccttctagtg cgcaggagtt ctgtagtgtt ggagatgatt cttgtctcat attatgggat 120
<210> 165
   <211> 120
   <212> DNA
   <213> GLYCINE MAX
<400> 165
   gttggaccac gaggcattta ttgtgggcat gaggatacag ttgaagatgt gactttctgc 60
   ccatctagtg cacaggagtt ctgtagtgtt ggagatgatt cttgtctcat cttatgggat 120
<210> 166
   <211> 120
   <212> DNA
   <213> ZEA MAYS
<400> 166
   gttgatccta gaggtatatt ccatggccat gacagtactg ttgaagatgt tcagttctgc 60
   ccttccagtg cgcaggagtt ttgtagtgtg ggtgatgatg cttgtcttat tctgtgggat 120
<210> 167
   <211> 120
   <212> DNA
   <213> ZEA MAYS
<400> 167
   gttgatccta gaggtatatt tcatggacat gacagcacgg ttgaagatgt tcagttctgc 60
   ccttccagtg cgcaggagtt ttgtagtgtg ggtgatgatg cttgtcttat tctgtgggat 120
<210> 168
   <211> 120
   <212> DNA
   <213> ORYZA SATIVA
<400> 168
   gttgatcctc gtggtatctt tcttggccac gacagtactg ttgaagatgt ccagttctgc 60
   ccttctagtg cacaggagtt ttgtagtgta ggcgatgatt cttgtcttat tctttgggat 120
<210> 169
   <211> 120
   <212> DNA
   <213> ORYZA SATIVA
<400> 169
   gttgatcctc gtggtatctt tcttggccac gacagtactg ttgaagatgt ccagttctgc 60
   ccttctagtg cacaggagtt ttgtagtgtg ggcgatgatt cttgtcttat tctttgggat 120
<210> 170
   <211> 120
   <212> DNA
   <213> PETUNIA HYBRIDA
<400> 170
   attcaagctc gtggaatctt ccaaggccat gaggataccg ttgaagatgt tcagttctgc 60
   ccatcaagtt cacaggaatt ctgtagtgtt ggtgatgatt catgtctcat tctatgggat 120
<210> 171
   <211> 120
   <212> DNA
   <213> GOSSYPIUM HIRSUTUM
<400> 171
   ttaggtcctc gtggtgtctt ctgtgggcat gaggatacag ttgaagatgt tacattctgt 60
   ccatcaagtg cacaagagtt ttgtagtgta ggtgatgatt cctgcctcat actatgggat 120
<210> 172
   <211> 120
   <212> DNA
   <213> POPULUS
<400> 172
   gttggaccgc gaggtatcta ccaagggcat gaggatacag ttgaagacgt ggcattctgt 60
   ccatccagtg cgcaggagtt ttgtagtgta ggagatgatt cttgccttat attatgggat 120
<210> 173
   <211> 120
   <212> DNA
   <213> VITIS VINIFERA
<400> 173
   ataggagcac gtggtatcta ccaagggcat gatgatactg ttgaagatgt gcaattctgc 60
   ccattaagcg cacaggagtt ctgtagcgta ggtgatgatt cttgccttat cttatgggat 120
<210> 174
   <211> 120
   <212> DNA
   <213> ARABIDOPSIS THALIANA
<400> 174
   gttggcccac gaggtgtata tcatggccat gaagatacag ttgaagatgt ggcattcagc 60
   ccgacgagtg cacaagaatt ctgcagtgtt ggtgatgatt cttgccttat actatgggat 120
<210> 175
   <211> 120
   <212> DNA
   <213> MEDICAGO TRUNCATULA
<400> 175
   gttggagcaa ggggcgtcta ccggggtcat aaagacactg ttgaagatgt gcagttttgc 60
   ccctcaagtg cacaggagtt ctgtagtgta ggtgatgatt cttgtctcat actctgggat 120
<210> 176
   <211> 27
   <212> DNA
   <213> SORGHUM BICOLOR
<400> 176
   gttgatccta gaggtatatt ctgtggg 27
<210> 177
   <211> 30
   <212> DNA
   <213> ARTIFICIAL SEQUENCE
<220>
   <223> Synthetic Primer
<400> 177
   tttctagaga tggaagtgtg cccaatactt 30
<210> 178
   <211> 37
   <212> DNA
   <213> ARTIFICIAL SEQUENCE
<220>
   <223> Synthetic Primer
<400> 178
   ttggccaaga tggcctggag aacgtgcttc ttcattg 37
<210> 179
   <211> 30
   <212> DNA
   <213> ARTIFICIAL SEQUENCE
<220>
   <223> Synthetic Primer
<400> 179
   ttgagctcga tggaagtgtg cccaatactt 30
<210> 180
   <211> 37
   <212> DNA
   <213> ARTIFICIAL SEQUENCE
<220>
   <223> Synthetic Primer
<400> 180
   ttggccatct aggcctggag aacgtgcttc ttcattg 37
<210> 181
   <211> 30
   <212> DNA
   <213> ARTIFICIAL SEQUENCE
<220>
   <223> Synthetic Primer
<400> 181
   tttctagatt tgtgaagaag tacaagacca 30
<210> 182
   <211> 37
   <212> DNA
   <213> ARTIFICIAL SEQUENCE
<220>
   <223> Synthetic Primer
<400> 182
   ttggccaaga tggccatccc aaatgagaac atcagga 37
<210> 183
   <211> 30
   <212> DNA
   <213> ARTIFICIAL SEQUENCE
<220>
   <223> Synthetic Primer
<400> 183
   ttgagctctt tgtgaagaag tacaagacca 30
<210> 184
   <211> 37
   <212> DNA
   <213> ARTIFICIAL SEQUENCE
<220>
   <223> Synthetic Primer
<400> 184
   ttggccatct aggccatccc aaatgagaac atcagga 37
<210> 185
   <211> 30
   <212> DNA
   <213> ARTIFICIAL SEQUENCE
<220>
   <223> Synthetic Primer
<400> 185
   tttctagatc ccgtccagat ttgatactaa 30
<210> 186
   <211> 37
   <212> DNA
   <213> ARTIFICIAL SEQUENCE
<220>
   <223> Synthetic Primer
<400> 186
   ttggccaaga tggccactcc acaaaacaac tgacttg 37
<210> 187
   <211> 30
   <212> DNA
   <213> ARTIFICIAL SEQUENCE
<220>
   <223> Synthetic Primer
<400> 187
   ttgagctctc ccgtccagat ttgatactaa 30
<210> 188
   <211> 37
   <212> DNA
   <213> ARTIFICIAL SEQUENCE
<220>
   <223> Synthetic Primer
<400> 188
   ttggccatct aggccactcc acaaaacaac tgacttg 37
<210> 189
   <211> 30
   <212> DNA
   <213> ARTIFICIAL SEQUENCE
<220>
   <223> Synthetic Primer
<400> 189
   tttctagaat ccaagatcac atcacaacga 30
<210> 190
   <211> 37
   <212> DNA
   <213> ARTIFICIAL SEQUENCE
<220>
   <223> Synthetic Primer
<400> 190 37
   ttggccaaga tggccagtag gactctcatt cttatca 37
<210> 191
   <211> 30
   <212> DNA
   <213> ARTIFICIAL SEQUENCE
<220>
   <223> Synthetic Primer
<400> 191
   ttgagctcat ccaagatcac atcacaacga 30
<210> 192
   <211> 37
   <212> DNA
   <213> ARTIFICIAL SEQUENCE
<220>
   <223> Synthetic Primer
<400> 192
   ttggccatct aggccagtag gactctcatt cttatca 37
<210> 193
   <211> 30
   <212> DNA
   <213> ARTIFICIAL SEQUENCE
<220>
   <223> Synthetic Primer
<400> 193
   tttctagagt tggcccacga ggtgtatatc 30
<210> 194
   <211> 37
   <212> DNA
   <213> ARTIFICIAL SEQUENCE
<220>
   <223> Synthetic Primer
<400> 194
   ttggccaaga tggccatccc atagtataag gcaagaa 37
<210> 195
   <211> 30
   <212> DNA
   <213> ARTIFICIAL SEQUENCE
<220>
   <223> Synthetic Primer
<400> 195
   ttgagctcgt tggcccacga ggtgtatatc 30
<210> 196
   <211> 37
   <212> DNA
   <213> ARTIFICIAL SEQUENCE
<220>
   <223> Synthetic Primer
<400> 196
   ttggccatct aggccatccc atagtataag gcaagaa 37
<210> 197
   <211> 30
   <212> DNA
   <213> ARTIFICIAL SEQUENCE
<220>
   <223> Synthetic Primer
<400> 197
   tttctagaat ggagagcgac gaagcagcag 30
<210> 198
   <211> 30
   <212> DNA
   <213> ARTIFICIAL SEQUENCE
<220>
   <223> Synthetic Primer
<400> 198
   ttggatcctt aaggcttgga ggcacaagtc 30
<210> 199
   <211> 30
   <212> DNA
   <213> ARTIFICIAL SEQUENCE
<220>
   <223> Synthetic Primer
<400> 199
   ttggatccat ggagagcgac gaagcagcag 30
<210> 200
   <211> 30
   <212> DNA
   <213> ARTIFICIAL SEQUENCE
<220>
   <223> Synthetic Primer
<400> 200
   ttgtcgactt aaggcttgga ggcacaagtc 30
<210> 201
   <211> 23
   <212> DNA
   <213> ARTIFICIAL SEQUENCE
<220>
   <223> Synthetic Primer
<400> 201
   gaccctcaga gaaacggaaa atg 23
<210> 202
   <211> 22
   <212> DNA
   <213> ARTIFICIAL SEQUENCE
<220>
   <223> Synthetic Primer
<400> 202
   cattgaactg agaaatgtgc tc 22
<210> 203
   <211> 22
   <212> DNA
   <213> ARTIFICIAL SEQUENCE
<220>
   <223> Synthetic Primer
<400> 203
   agtgcacaag aattctgcag tg 22
<210> 204
   <211> 30
   <212> DNA
   <213> ARTIFICIAL SEQUENCE
<220>
   <223> Synthetic Primer
<400> 204
   tttctagaat ggagagcgac ggagcggcag 30
<210> 205
   <211> 30
   <212> DNA
   <213> ARTIFICIAL SEQUENCE
<220>
   <223> Synthetic Primer
<400> 205
   ttgagctctc aaggcttgga ggcacatgtc 30
<210> 206
   <211> 1524
   <212> DNA
   <213> BRASSICA NAPUS
<400> 206
   atggagagcg acggagcggc agcggcagga gcagcgtcgc cgagcggagg aaacgtgaca      60
   ccggcgccga agaagagagg acggaaacca aagagcaagg acgagtctca gacgcagcag     120
   gagcaggagg aaaggggtgg gaaaatgaag gaaagcggga agaaaacgaa gcagggggag     180
   cagagcgtgg acgagaagta cactcagtgg aaaggtctcg tccccatcct ctacgactgg     240
   ctcgccaatc acaacctcgt ctggccttcc ctctcttgca gatggggtcc acagatcgaa     300
   caagcaacct acaagaatcg acagcgtctt tacctctcag aacaaactga tggcagtgtg     360
   cccaacactc tagtcatagc gaattgcgaa gttgttaagc caagggtcgc tgcagcagag     420
   cacatatctc agttcaacga agaagcacgt tctccatttg tgaagaagtt caagaccatc     480
   attcaccctg gagaggttaa ccgaatcagg gaactcccac aaaacagtaa gattattgct     540
   actcacaccg acagtcctga tgttctcatt tgggatgttg aaacccaacc aaaccgtcat     600
   gctgtccttg gagctgcaca ttcccgtcct gatttggtat taactggaca tcaagacaat     660
   gctgaattcg ctcttgcaat gtgcccaacc gaaccctttg tcctctctgg aggcaaagac     720
   aagtcagttg ttttgtggag tatccaggac cacatcacaa ccgctggtag tacagactcc     780
   aaatcatctg gatccatcat caaacagact ggtgaaggtg gtgataagac tgagagtcct     840
   tctcttggcc cgcgaggtgt atatcatggc catgatgata ccgttgaaga tgtcgctttc     900
   agccccacta gtgcacaaga gttctgcagt gtcggtgacg attcttgcct tatactatgg     960
   gatgcaagaa caggcactag ccctgtcacg aaggttgaaa aggcgcacga tgctgatctt    1020
   cattgtgtcg attggaaccc tcatgatgac aatctgatcc tgacagggtc tgcagacaac    1080
   actgttcggt tgtatgatcg taggaaacta acctcaaatg gagtgggtac gcctatttac    1140
   aaatttgaag gccacaaagc tgctgttctt tgcgttcagt ggtctcctga taagtcatct    1200
   gtttttggga gttccgcgga agatggtctc ttgaacatct gggattatga cagggtcagt    1260
   aagaagtctg atcgtgcagc taaaagtccg gctggtctct tcttccagca tgctggtcac    1320
   agggacaaag ttgttgattt ccactggaat gcagaggacc cttggactat tgtcagtgtt    1380
   tctgatgact gcgagactac tggtggaggt ggaacactgc agatatggcg gatgagtgac    1440
   ttgatttaca gaccggagga ggaagttctg gcagagttgg agaagttcaa gtcgcatgtt    1500
   atgacatgtg cctccaagcc ttga                                           1524
<210> 207
   <211> 507
   <212> PRT
   <213> BRASSICA NAPUS
<400> 207
   Met Glu Ser Asp Gly Ala Ala Ala Ala Gly Ala Ala Ser Pro Ser Gly
   1               5                   10                  15
   Gly Asn Val Thr Pro Ala Pro Lys Lys Arg Gly Arg Lys Pro Lys Ser
               20                  25                  30
   Lys Asp Glu Ser Gln Thr Gln Gln Glu Gln Glu Glu Arg Gly Gly Lys
           35                  40                  45
   Met Lys Glu Ser Gly Lys Lys Thr Lys Gln Gly Glu Gln Ser Val Asp
       50                  55                  60
   Glu Lys Tyr Thr Gln Trp Lys Gly Leu Val Pro Ile Leu Tyr Asp Trp
   65                  70                  75                  80
   Leu Ala Asn His Asn Leu Val Trp Pro Ser Leu Ser Cys Arg Trp Gly
                   85                  90                  95
   Pro Gln Ile Glu Gln Ala Thr Tyr Lys Asn Arg Gln Arg Leu Tyr Leu
               100                 105                 110
   Ser Glu Gln Thr Asp Gly Ser Val Pro Asn Thr Leu Val Ile Ala Asn
           115                 120                 125
   Cys Glu Val Val Lys Pro Arg Val Ala Ala Ala Glu His Ile Ser Gln
       130                 135                 140
   Phe Asn Glu Glu Ala Arg Ser Pro Phe Val Lys Lys Phe Lys Thr Ile
   145                 150                 155                 160
   Ile His Pro Gly Glu Val Asn Arg Ile Arg Glu Leu Pro Gln Asn Ser
   165 170 175
   Lys Ile Ile Ala Thr His Thr Asp Ser Pro Asp Val Leu Ile Trp Asp
   180 185 190
   Val Glu Thr Gln Pro Asn Arg His Ala Val Leu Gly Ala Ala His Ser
   195 200 205
   Arg Pro Asp Leu Val Leu Thr Gly His Gln Asp Asn Ala Glu Phe Ala
   210 215 220
   Leu Ala Met Cys Pro Thr Glu Pro Phe Val Leu Ser Gly Gly Lys Asp
   225 230 235 240
   Lys Ser Val Val Leu Trp Ser Ile Gln Asp His Ile Thr Thr Ala Gly
   245 250 255
   Ser Thr Asp Ser Lys Ser Ser Gly Ser Ile Ile Lys Gln Thr Gly Glu
   260 265 270
   Gly Gly Asp Lys Thr Glu Ser Pro Ser Leu Gly Pro Arg Gly Val Tyr
   275 280 285
   His Gly His Asp Asp Thr Val Glu Asp Val Ala Phe Ser Pro Thr Ser
   290 295 300
   Ala Gln Glu Phe Cys Ser Val Gly Asp Asp Ser Cys Leu Ile Leu Trp
   305 310 315 320
   Asp Ala Arg Thr Gly Thr Ser Pro Val Thr Lys Val Glu Lys Ala His
   325 330 335
   Asp Ala Asp Leu His Cys Val Asp Trp Asn Pro His Asp Asp Asn Leu
   340 345 350
   Ile Leu Thr Gly Ser Ala Asp Asn Thr Val Arg Leu Tyr Asp Arg Arg
   355 360 365
   Lys Leu Thr Ser Asn Gly Val Gly Thr Pro Ile Tyr Lys Phe Glu Gly
   370 375 380
   His Lys Ala Ala Val Leu Cys Val Gln Trp Ser Pro Asp Lys Ser Ser
   385 390 395 400
   Val Phe Gly Ser Ser Ala Glu Asp Gly Leu Leu Asn Ile Trp Asp Tyr
   405 410 415
   Asp Arg Val Ser Lys Lys Ser Asp Arg Ala Ala Lys Ser Pro Ala Gly
   420 425 430
   Leu Phe Phe Gln His Ala Gly His Arg Asp Lys Val Val Asp Phe His
   435 440 445
   Trp Asn Ala Glu Asp Pro Trp Thr Ile Val Ser Val Ser Asp Asp Cys
   450 455 460
   Glu Thr Thr Gly Gly Gly Gly Thr Leu Gln Ile Trp Arg Met Ser Asp
   465 470 475 480
   Leu Ile Tyr Arg Pro Glu Glu Glu Val Leu Ala Glu Leu Glu Lys Phe
   485 490 495
   Lys Ser His Val Met Thr Cys Ala Ser Lys Pro
   500 505
<210> 208
   <211> 31
   <212> DNA
   <213> ARTIFICIAL SEQUENCE
<220>
   <223> Synthetic Primer
<400> 208
   tttctagaat gaaagagaga ggcggctcca g 31
<210> 209
   <211> 31
   <212> DNA
   <213> ARTIFICIAL SEQUENCE
<220>
   <223> Synthetic Primer
<400> 209
   ttgagctctc aattcttcgg tgcacagctg g 31
<210> 210
   <211> 31
   <212> DNA
   <213> ARTIFICIAL SEQUENCE
<220>
   <223> Synthetic Primer
<400> 210
   tttctagaat gaaggagaga ggcggctcca g 31
<210> 211
   <211> 31
   <212> DNA
   <213> ARTIFICIAL SEQUENCE
<220>
   <223> Synthetic Primer
<400> 211
   ttgagctctc aattcctcgg agcgcaactg g 31
<210> 212
   <211> 21
   <212> DNA
   <213> ARTIFICIAL SEQUENCE
<220>
   <223> Synthetic Primer
<400> 212
   atatgttcag cagctgcaac c 21
<210> 213
   <211> 21
   <212> DNA
   <213> ARTIFICIAL SEQUENCE
<220>
   <223> Synthetic Primer
<400> 213
   gctgctgttc tttgtgttca g 21
<210> 214
   <211> 31
   <212> DNA
   <213> ARTIFICIAL SEQUENCE
<220>
   <223> Synthetic Primer
<400> 214
   tttctagaat gaaggagagg agcggctcga g 31
<210> 215
   <211> 30
   <212> DNA
   <213> ARTIFICIAL SEQUENCE
<220>
   <223> Synthetic Primer
<400> 215
   ttgagctctc agttcctcgg agtgcagctg 30
<210> 216
   <211> 1362
   <212> DNA
   <213> PANICUM VIRGATUM
<400> 216
   atgaaggaga ggagcggctc gagggcggcg gtggatgagc gctacgcgca gtggaagtcg      60
   ctcattccgg tgctctacga ctggttcgcc aaccacaacc tcgtctggcc gtcgctgtcc     120
   tgccggtggg gcccgcaatt tgagaaagct acctacaaga atcgccagcg cctttaccta     180
   tctgagcaga cggatgggag tgtgcctaat actctggtta ttgcgaactg tgaagttgtc     240
   aaaccaaggg ttgcagctgc tgaacatatc tcacagttca atgaggaagc acgatcgcct     300
   tttgtgaaga agtataagac tatagttcat cctggtgagg ttaacagaat cagggagctt     360
   ccacagaaca gtaagatcat agccactcac accgacagtc cagatgtact tatttgggat     420
   gttgaagccc aaccaaatag acatgctgtc ctaggagcaa ctgaatctcg gcctgatctg     480
   atattaacag gacatcagga aaatgctgaa ttcgcgcttg ccatgtgtcc agcagaacca     540
   tatgtactgt caggaggaaa ggacaaatct gttgtcttgt ggagcatcca agaccacata     600
   tctgcccttg gggattcctc atcttctcct ggagcatctg gcagcaagca gtctggcaaa      660
   actgcgaatg aaaaggagag tcccaaagtt gatcctcggg gtatattcca tggacatgac     720
   agcactgttg aagatgttca gttctgccct tccagtgcac aggaattctg tagtgtgggt     780
   gatgatgctt gtcttattct ctgggatgcc cggactggta cgagcccggc tgttaaggtt     840
   gagaaagctc atagtgggga tgttcattgt gtggattgga atccgcttga tgttaactat     900
   atcttaactg gttctgctga taactctgtc cgtatgtggg atcgtcgcaa tctgggttct     960
   ggaggagctg gttctccaat tcacaaattt gagggccata aagctgctgt tctttgtgtc    1020
   cagtggtcac ctgacagagc atctgttttc ggaagttctg cggaagatgg tttcttaaat    1080
   gtgtgggatc atgagaaggt tgggaagaaa aaaaattcta acgtaccagc tgggcttttc    1140
   tttcaacatg ctggtcacag ggataagatt gtagacttcc actggaattc atcggatcct    1200
   tggacaatcg tcagtgtctc agatgatggt gagagcaccg gtggaggtgg aacactgcag    1260
   atatggcgga tgagtgattt gatctaccgc ccagaggagg aagttctcca agaactggag    1320
   aatttcaagg ctcacttggc cagctgcact ccgaggaact ga                        1362
<210> 217
   <211> 453
   <212> PRT
   <213> PANICUM VIRGATUM
<400> 217
   Met Lys Glu Arg Ser Gly Ser Arg Ala Ala Val Asp Glu Arg Tyr Ala
   1               5                   10                  15
   Gln Trp Lys Ser Leu Ile Pro Val Leu Tyr Asp Trp Phe Ala Asn His
               20                  25                  30
   Asn Leu Val Trp Pro Ser Leu Ser Cys Arg Trp Gly Pro Gln Phe Glu
           35                  40                  45
   Lys Ala Thr Tyr Lys Asn Arg Gln Arg Leu Tyr Leu Ser Glu Gln Thr
       50                  55                  60
   Asp Gly Ser Val Pro Asn Thr Leu Val Ile Ala Asn Cys Glu Val Val
   65                  70                  75                  80
   Lys Pro Arg Val Ala Ala Ala Glu His Ile Ser Gln Phe Asn Glu Glu
                   85                  90                  95
   Ala Arg Ser Pro Phe Val Lys Lys Tyr Lys Thr Ile Val His Pro Gly
               100                 105                 110
   Glu Val Asn Arg Ile Arg Glu Leu Pro Gln Asn Ser Lys Ile Ile Ala
           115                 120                 125
   Thr His Thr Asp Ser Pro Asp Val Leu Ile Trp Asp Val Glu Ala Gln
       130                 135                 140
   Pro Asn Arg His Ala Val Leu Gly Ala Thr Glu Ser Arg Pro Asp Leu
   145                 150                 155                 160
   Ile Leu Thr Gly His Gln Glu Asn Ala Glu Phe Ala Leu Ala Met Cys
                   165                 170                 175
   Pro Ala Glu Pro Tyr Val Leu Ser Gly Gly Lys Asp Lys Ser Val Val
               180                 185                 190
   Leu Trp Ser Ile Gln Asp His Ile Ser Ala Leu Gly Asp Ser Ser Ser
           195                 200                 205
   Ser Pro Gly Ala Ser Gly Ser Lys Gln Ser Gly Lys Thr Ala Asn Glu
       210                 215                 220
   Lys Glu Ser Pro Lys Val Asp Pro Arg Gly Ile Phe His Gly His Asp
   225 230 235 240
   Ser Thr Val Glu Asp Val Gln Phe Cys Pro Ser Ser Ala Gln Glu Phe
   245 250 255
   Cys Ser Val Gly Asp Asp Ala Cys Leu Ile Leu Trp Asp Ala Arg Thr
   260 265 270
   Gly Thr Ser Pro Ala Val Lys Val Glu Lys Ala His Ser Gly Asp Val
   275 280 285
   His Cys Val Asp Trp Asn Pro Leu Asp Val Asn Tyr Ile Leu Thr Gly
   290 295 300
   Ser Ala Asp Asn Ser Val Arg Met Trp Asp Arg Arg Asn Leu Gly Ser
   305 310 315 320
   Gly Gly Ala Gly Ser Pro Ile His Lys Phe Glu Gly His Lys Ala Ala
   325 330 335
   Val Leu Cys Val Gln Trp Ser Pro Asp Arg Ala Ser Val Phe Gly Ser
   340 345 350
   Ser Ala Glu Asp Gly Phe Leu Asn Val Trp Asp His Glu Lys Val Gly
   355 360 365
   Lys Lys Lys Asn Ser Asn Val Pro Ala Gly Leu Phe Phe Gln His Ala
   370 375 380
   Gly His Arg Asp Lys Ile Val Asp Phe His Trp Asn Ser Ser Asp Pro
   385 390 395 400
   Trp Thr Ile Val Ser Val Ser Asp Asp Gly Glu Ser Thr Gly Gly Gly
   405 410 415
   Gly Thr Leu Gln Ile Trp Arg Met Ser Asp Leu Ile Tyr Arg Pro Glu
   420 425 430
   Glu Glu Val Leu Gln Glu Leu Glu Asn Phe Lys Ala His Leu Ala Ser
   435 440 445
   Cys Thr Pro Arg Asn
   450
<210> 218
   <211> 30
   <212> DNA
   <213> ARTIFICIAL SEQUENCE
<220>
   <223> Synthetic Primer
<400> 218
   tttctagaat ggagactcct cctccccaac 30
<210> 219
   <211> 31
   <212> DNA
   <213> ARTIFICIAL SEQUENCE
<220>
   <223> Synthetic Primer
<400> 219
   ttgagctctc atttttcagt ctttgaagca c 31
<210> 220
   <211> 1362
   <212> DNA
   <213> ZEA MAYS
<400> 220
   atgaaagaga gaggcggctc cagggcggcg gtggacgagc gctacgcgca gtggaagtcg      60
   ctcatccccg tcctctacga ctggtttgcc aaccacaacc tcgtctggcc atccctctcc     120
   tgccggtggg ggccacagtt tgagaaagct acctacaaga atcgtcagcg cctgtaccta     180
   tctgaacaga cggatgggag tgtgcctaat actctggtta tcgcaaattg tgaagttgtg     240
   aaacctaggg ttgcagctgc tgaacatatc tcacagttca atgaggaagc acggtcacct     300
   tttgtaaaga agtacaaaac tatagttcat cctggtgagg ttaacagaat cagggaactt     360
   ccacagaaca gtaagatcat agccactcac actgacagtc cagatgtact tatttgggat     420
   gttgaagcac aaccaaatag acatgctgtc ctaggagcaa gcgagtctcg ccctgatctg     480
   atattaacag gacataagga aaatgcggaa tttgcgcttg ccatgtgtcc agcagaacca     540
   tatgtcctat caggaggaaa ggacaaatct gttgtcttgt ggagcatcca agaccatata     600
   tctgcccttg gggattcctc gtcttctcct ggagcatctg gcagcaagca gtctattaaa      660
   actgcaaatg aaaaggagag ccctaaagtt gatcctagag gtatatttca tggacatgac     720
   agcacggttg aagatgttca gttctgccct tccagtgcgc aggagttttg tagtgtgggt     780
   gatgatgctt gtcttattct gtgggatgct cgaactggca ctgccccagc tgttaaggtt     840
   gagaaagctc acagtggaga tgttcattgt gttgattgga atccccttga tgttaactat     900
   atcttaactg ggtctgccga taactctgtc cgaatgtggg atcgtcggaa tctgggttca     960
   ggaggagctg gttctccaat tcacaaattt gaaggccata aagctgctgt tctttgtgtt    1020
   cagtggtcac ctgacagagc atctgttttt ggaagttctg cagaagatgg tttcttaaac    1080
   gtgtgggatc atgagaaggt ggggaagaag aaaaattcta atgtcccagc cgggcttttc    1140
   tttcagcacg ctggtcatag ggataagatt gtagacttcc actggaattc gtcagatcct    1200
   tggacaattg tcagtgtctc tgatgatggc gagagcactg gtggaggtgg aacactgcag    1260
   atttggcgca tgagtgattt gatctaccgc ccagaggatg aagttcttgc agagctggag    1320
   aatttcaagg ctcatttggc cagctgtgca ccgaagaatt ga                       1362
<210> 221
   <211> 453
   <212> PRT
   <213> ZEA MAYS
<400> 221
   Met Lys Glu Arg Gly Gly Ser Arg Ala Ala Val Asp Glu Arg Tyr Ala
   1               5                   10                  15
   Gln Trp Lys Ser Leu Ile Pro Val Leu Tyr Asp Trp Phe Ala Asn His
               20                  25                  30
   Asn Leu Val Trp Pro Ser Leu Ser Cys Arg Trp Gly Pro Gln Phe Glu
           35                  40                  45
   Lys Ala Thr Tyr Lys Asn Arg Gln Arg Leu Tyr Leu Ser Glu Gln Thr
       50                  55                  60
   Asp Gly Ser Val Pro Asn Thr Leu Val Ile Ala Asn Cys Glu Val Val
   65                  70                  75                  80
   Lys Pro Arg Val Ala Ala Ala Glu His Ile Ser Gln Phe Asn Glu Glu
                   85                  90                  95
   Ala Arg Ser Pro Phe Val Lys Lys Tyr Lys Thr Ile Val His Pro Gly
               100                 105                 110
   Glu Val Asn Arg Ile Arg Glu Leu Pro Gln Asn Ser Lys Ile Ile Ala
           115                 120                 125
   Thr His Thr Asp Ser Pro Asp Val Leu Ile Trp Asp Val Glu Ala Gln
       130                 135                 140
   Pro Asn Arg His Ala Val Leu Gly Ala Ser Glu Ser Arg Pro Asp Leu
   145 150 155 160
   Ile Leu Thr Gly His Lys Glu Asn Ala Glu Phe Ala Leu Ala Met Cys
   165 170 175
   Pro Ala Glu Pro Tyr Val Leu Ser Gly Gly Lys Asp Lys Ser Val Val
   180 185 190
   Leu Trp Ser Ile Gln Asp His Ile Ser Ala Leu Gly Asp Ser Ser Ser
   195 200 205
   Ser Pro Gly Ala Ser Gly Ser Lys Gln Ser Ile Lys Thr Ala Asn Glu
   210 215 220
   Lys Glu Ser Pro Lys Val Asp Pro Arg Gly Ile Phe His Gly His Asp
   225 230 235 240
   Ser Thr Val Glu Asp Val Gln Phe Cys Pro Ser Ser Ala Gln Glu Phe
   245 250 255
   Cys Ser Val Gly Asp Asp Ala Cys Leu Ile Leu Trp Asp Ala Arg Thr
   260 265 270
   Gly Thr Ala Pro Ala Val Lys Val Glu Lys Ala His Ser Gly Asp Val
   275 280 285
   His Cys Val Asp Trp Asn Pro Leu Asp Val Asn Tyr Ile Leu Thr Gly
   290 295 300
   Ser Ala Asp Asn Ser Val Arg Met Trp Asp Arg Arg Asn Leu Gly Ser
   305 310 315 320
   Gly Gly Ala Gly Ser Pro Ile His Lys Phe Glu Gly His Lys Ala Ala
   325 330 335
   Val Leu Cys Val Gln Trp Ser Pro Asp Arg Ala Ser Val Phe Gly Ser
   340 345 350
   Ser Ala Glu Asp Gly Phe Leu Asn Val Trp Asp His Glu Lys Val Gly
   355 360 365
   Lys Lys Lys Asn Ser Asn Val Pro Ala Gly Leu Phe Phe Gln His Ala
   370 375 380
   Gly His Arg Asp Lys Ile Val Asp Phe His Trp Asn Ser Ser Asp Pro
   385 390 395 400
   Trp Thr Ile Val Ser Val Ser Asp Asp Gly Glu Ser Thr Gly Gly Gly
   405 410 415
   Gly Thr Leu Gln Ile Trp Arg Met Ser Asp Leu Ile Tyr Arg Pro Glu
   420 425 430
   Asp Glu Val Leu Ala Glu Leu Glu Asn Phe Lys Ala His Leu Ala Ser
   435 440 445
   Cys Ala Pro Lys Asn
   450
<210> 222
<400> 222
   000
<210> 223
   <211> 34
   <212> PRT
   <213> BRASSICA NAPUS
<400> 223
   Ile Gln Asp His Ile Thr Thr Ala Gly Ser Thr Asp Ser Lys Ser Ser
   1 5 10 15
   Gly Ser Ile Ile Lys Gln Thr Gly Glu Gly Gly Asp Lys Thr Glu Ser
   20 25 30
   Pro Ser
<210> 224
   <211> 30
   <212> DNA
   <213> ARTIFICIAL SEQUENCE
<220>
   <223> Synthetic Primer
<400> 224
   tttctagaat ccaggaccac atcacaaccg 30
<210> 225
   <211> 37
   <212> DNA
   <213> ARTIFICIAL SEQUENCE
<220>
   <223> Synthetic Primer
<400> 225
   ttggccaaga tggccagaag gactctcagt cttatca 37
<210> 226
   <211> 30
   <212> DNA
   <213> ARTIFICIAL SEQUENCE
<220>
   <223> Synthetic Primer
<400> 226
   ttgagctcat ccaggaccac atcacaaccg 30
<210> 227
   <211> 37
   <212> DNA
   <213> ARTIFICIAL SEQUENCE
<220>
   <223> Synthetic Primer
<400> 227
   ttggccatct aggccagaag gactctcagt cttatca 37
<210> 228
   <211> 40
   <212> PRT
   <213> BRASSICA NAPUS
<400> 228
   Ser Arg Pro Asp Leu Val Leu Thr Gly His Gln Asp Asn Ala Glu Phe
   1               5                   10                  15
   Ala Leu Ala Met Cys Pro Thr Glu Pro Phe Val Leu Ser Gly Gly Lys
               20                  25                  30
   Asp Lys Ser Val Val Leu Trp Ser
           35                  40
<210> 229
   <211> 30
   <212> DNA
   <213> ARTIFICIAL SEQUENCE
<220>
   <223> Synthetic Primer
<400> 229
   tttctagatc ccgtcctgat ttggtattaa 30
<210> 230
   <211> 37
   <212> DNA
   <213> ARTIFICIAL SEQUENCE
<220>
   <223> Synthetic Primer
<400> 230
   ttggccaaga tggccactcc acaaaacaac tgacttg 37
<210> 231
   <211> 30
   <212> DNA
   <213> ARTIFICIAL SEQUENCE
<220>
   <223> Synthetic Primer
<400> 231
   ttgagctctc ccgtcctgat ttggtattaa 30
<210> 232
   <211> 37
   <212> DNA
   <213> ARTIFICIAL SEQUENCE
<220>
   <223> Synthetic Primer
<400> 232
   ttggccatct aggccactcc acaaaacaac tgacttg 37
<210> 233
   <211> 102
   <212> DNA
   <213> BRASSICA NAPUS
<400> 233
   atccaggacc acatcacaac cgctggtagt acagactcca aatcatctgg atccatcatc 60
   aaacagactg gtgaaggtgg tgataagact gagagtcctt ct 102
<210> 234
   <211> 120
   <212> DNA
   <213> BRASSICA NAPUS
<400> 234
   tcccgtcctg atttggtatt aactggacat caagacaatg ctgaattcgc tcttgcaatg 60
   tgcccaaccg aaccctttgt cctctctgga ggcaaagaca agtcagttgt tttgtggagt 120
<210> 235
   <211> 120
   <212> DNA
   <213> BRASSICA NAPUS
<400> 235
   tttgtgaaga agttcaagac catcattcac cctggagagg ttaaccgaat cagggaactc 60
   ccacaaaaca gtaagattat tgctactcac accgacagtc ctgatgttct catttgggat 120
<210> 236
   <211> 40
   <212> PRT
   <213> BRASSICA NAPUS
<400> 236
   Phe Val Lys Lys Phe Lys Thr Ile Ile His Pro Gly Glu Val Asn Arg
   1 5 10 15
   Ile Arg Glu Leu Pro Gln Asn Ser Lys Ile Ile Ala Thr His Thr Asp
   20 25 30
   Ser Pro Asp Val Leu Ile Trp Asp
   35 40
<210> 237
   <211> 30
   <212> DNA
   <213> ARTIFICIAL SEQUENCE
<220>
   <223> Synthetic Primer
<400> 237
   tttctagatt tgtgaagaag ttcaagacca 30
<210> 238
   <211> 30
   <212> DNA
   <213> ARTIFICIAL SEQUENCE
<220>
   <223> Synthetic Primer
<400> 238
   ttgagctctt tgtgaagaag ttcaagacca 30
<210> 239
   <211> 1536
   <212> DNA
   <213> GLYCINE MAX
<400> 239
   atggagactc ctcctcccca acaaggcgtc gtgaagaaga aggagacaag gggccgaaaa      60
   cccaaaccaa aggacgaaca cgggaagggt ttgaaggaag gtaggaaaac acaacaacaa     120
   caacaacaac aacatcatca tcagcagcag caacagcaac aagatcaacc ttcggtggac     180
   gagaaataca cgcagtggaa gtcccttgtt cctgtcctct acgactggct cgccaaccac     240
   aacctcgtct ggccctctct ctcttgcagg tggggccccc agcttgaaca agccacttac     300
   aagaatcgcc agagactcta cctttctgag cagactgatg gtagtgtgcc gaatactctg     360
   gtgattgcga attgcgaggt tgtgaagcct agggttgctg ctgctgagca catttcgcag     420
   tttaatgaag aggcgcggtc cccatttgtg aagaagtaca agaccatcat acatcctggt     480
   gaggtaaaca gaattaggga attgccacaa aattccaaga tagtggctac acatacagac     540
   agccctgatg tccttgtttg ggatgttgaa agtcaaccta atcgccatgc tgtccttgga     600
   gctacaaact ctcgtcctga tttgatattg accggacacc aagataatgc ggaatttgct     660
   cttgctatgt gcccaactga accctatgtt ctttcaggag gaaaggacaa aacagtggtg     720
   ttgtggagta ttgaagacca tataacatct gctgctacag actccaaatc tggtgggtca     780
   attatcaaac aaaactctaa atctggagaa ggcaatgaca aaactgctga tggccctact     840
   gttggaccac gaggtatcta ttgtgggcat gaggatactg ttgaagacgt ggctttctgc     900
   ccatctagtg cacaggagtt ctgtagtgtt ggagatgatt cttgtctcat cttatgggat     960
   gcacgtgttg gctctagccc tgtggttaag gttgagaaag ctcataatgc tgatcttcac    1020
   tgtgtggact ggaatcccca tgatgataat ctgattctta ctgggtcagc agataattct    1080
   gttcgcatgt ttgatcgccg caatctcacc actaatggag ttgggtcacc catccataaa    1140
   tttgagggtc acaaagctgc tgttctttgt gttcagtggt ctccagacaa atcatctgta    1200
   tttggaagtt cagctgaaga tggtctctta aacatttggg actatgagaa ggttggtaaa    1260
   aagatagagc gatctggaaa atcaataagt tctcctccag ggttgttttt tcaacatgca    1320
   ggtcataggg ataaagttgt tgacttccat tggaatgcat atgatccatg gacgattgtt    1380
   agtgtgtctg atgactgtga aagtactgga ggagggggaa cgttgcagat atggcgcatg    1440
   agtgatttga tctacagacc agaagatgag gttttggccg agctggagaa attcaaatct    1500
   cacgttgtgg cgtgtgcttc aaagactgaa aaatga                               1536
<210> 240
   <211> 511
   <212> PRT
   <213> GLYCINE MAX
<400> 240
   Met Glu Thr Pro Pro Pro Gln Gln Gly Val Val Lys Lys Lys Glu Thr
   1               5                   10                  15
   Arg Gly Arg Lys Pro Lys Pro Lys Asp Glu His Gly Lys Gly Leu Lys
               20                  25                  30
   Glu Gly Arg Lys Thr Gln Gln Gln Gln Gln Gln Gln His His His Gln
           35                  40                  45
   Gln Gln Gln Gln Gln Gln Asp Gln Pro Ser Val Asp Glu Lys Tyr Thr
       50                  55                  60
   Gln Trp Lys Ser Leu Val Pro Val Leu Tyr Asp Trp Leu Ala Asn His
   65                  70                  75                  80
   Asn Leu Val Trp Pro Ser Leu Ser Cys Arg Trp Gly Pro Gln Leu Glu
                   85                  90                  95
   Gln Ala Thr Tyr Lys Asn Arg Gln Arg Leu Tyr Leu Ser Glu Gln Thr
               100                 105                 110
   Asp Gly Ser Val Pro Asn Thr Leu Val Ile Ala Asn Cys Glu Val Val
           115                 120                 125
   Lys Pro Arg Val Ala Ala Ala Glu His Ile Ser Gln Phe Asn Glu Glu
       130                 135                 140
   Ala Arg Ser Pro Phe Val Lys Lys Tyr Lys Thr Ile Ile His Pro Gly
   145                 150                 155                 160
   Glu Val Asn Arg Ile Arg Glu Leu Pro Gln Asn Ser Lys Ile Val Ala
                   165                 170                 175
   Thr His Thr Asp Ser Pro Asp Val Leu Val Trp Asp Val Glu Ser Gln
               180                 185                 190
   Pro Asn Arg His Ala Val Leu Gly Ala Thr Asn Ser Arg Pro Asp Leu
           195                 200                 205
   Ile Leu Thr Gly His Gln Asp Asn Ala Glu Phe Ala Leu Ala Met Cys
       210                 215                 220
   Pro Thr Glu Pro Tyr Val Leu Ser Gly Gly Lys Asp Lys Thr Val Val
   225                 230                 235                 240
   Leu Trp Ser Ile Glu Asp His Ile Thr Ser Ala Ala Thr Asp Ser Lys
                   245                 250                 255
   Ser Gly Gly Ser Ile Ile Lys Gln Asn Ser Lys Ser Gly Glu Gly Asn
               260                 265                 270
   Asp Lys Thr Ala Asp Gly Pro Thr Val Gly Pro Arg Gly Ile Tyr Cys
           275                 280                 285
   Gly His Glu Asp Thr Val Glu Asp Val Ala Phe Cys Pro Ser Ser Ala
       290                 295                 300
   Gln Glu Phe Cys Ser Val Gly Asp Asp Ser Cys Leu Ile Leu Trp Asp
   305                 310                 315                 320
   Ala Arg Val Gly Ser Ser Pro Val Val Lys Val Glu Lys Ala His Asn
                   325                 330                 335
   Ala Asp Leu His Cys Val Asp Trp Asn Pro His Asp Asp Asn Leu Ile
               340                 345                 350
   Leu Thr Gly Ser Ala Asp Asn Ser Val Arg Met Phe Asp Arg Arg Asn
           355                 360                 365
   Leu Thr Thr Asn Gly Val Gly Ser Pro Ile His Lys Phe Glu Gly His
       370                 375                 380
   Lys Ala Ala Val Leu Cys Val Gln Trp Ser Pro Asp Lys Ser Ser Val
   385                 390                 395                 400
   Phe Gly Ser Ser Ala Glu Asp Gly Leu Leu Asn Ile Trp Asp Tyr Glu
                   405                 410                 415
   Lys Val Gly Lys Lys Ile Glu Arg Ser Gly Lys Ser Ile Ser Ser Pro
               420                 425                 430
   Pro Gly Leu Phe Phe Gln His Ala Gly His Arg Asp Lys Val Val Asp
           435                 440                 445
   Phe His Trp Asn Ala Tyr Asp Pro Trp Thr Ile Val Ser Val Ser Asp
       450                 455                 460
   Asp Cys Glu Ser Thr Gly Gly Gly Gly Thr Leu Gln Ile Trp Arg Met
   465                 470                 475                 480
   Ser Asp Leu Ile Tyr Arg Pro Glu Asp Glu Val Leu Ala Glu Leu Glu
                   485                 490                 495
   Lys Phe Lys Ser His Val Val Ala Cys Ala Ser Lys Thr Glu Lys
               500                 505                 510
<210> 241
   <211> 223
   <212> DNA
   <213> BRACHYPODIUM DISTACHYON
<400> 241
   atgaaggaga ggagcggctc gagggcggcg gtggatgagc gctacgcgca gtggaagtcg     60
   ctcattccgg tgctctacga ctggttcgcc aaccacaacc tcgtctggcc gtcgctgtcc    120
   tgccggtggg gcccgcaatt tgagaaagct acctacaaga atcgccagcg cctttaccta    180
   tctgagcaga cggatgggag tgtgcctaat actctggtta tgc                      223
<210> 242
   <211> 74
   <212> PRT
   <213> BRACHYPODIUM DISTACHYON
<400> 242
   Met Lys Glu Arg Ser Gly Ser Arg Ala Ala Val Asp Glu Arg Tyr Ala
   1               5                   10                  15
   Gln Trp Lys Ser Leu Ile Pro Val Leu Tyr Asp Trp Phe Ala Asn His
               20                  25                  30
   Asn Leu Val Trp Pro Ser Leu Ser Cys Arg Trp Gly Pro Gln Phe Glu
           35                  40                  45
   Lys Ala Thr Tyr Lys Asn Arg Gln Arg Leu Tyr Leu Ser Glu Gln Thr
       50                  55                  60
   Asp Gly Ser Val Pro Asn Thr Leu Val Met
   65                  70
<210> 243
   <211> 36
   <212> PRT
   <213> BRASSICA NAPUS
<400> 243
   Asp Gly Ser Val Pro Asn Thr Leu Val Ile Ala Asn Cys Glu Val Val
   1 5 10 15
   Lys Pro Arg Val Ala Ala Ala Glu His Ile Ser Gln Phe Asn Glu Glu
   20 25 30
   Ala Arg Ser Pro
   35
<210> 244
   <211> 36
   <212> PRT
   <213> PANICUM VIRGATUM
<400> 244
   Asp Gly Ser Val Pro Asn Thr Leu Val Ile Ala Asn Cys Glu Val Val
   1 5 10 15
   Lys Pro Arg Val Ala Ala Ala Glu His Ile Ser Gln Phe Asn Glu Glu
   20 25 30
   Ala Arg Ser Pro
   35
<210> 245
   <211> 108
   <212> DNA
   <213> BRASSICA NAPUS
<400> 245
   gatggcagtg tgcccaacac tctagtcata gcgaattgcg aagttgttaa gccaagggtc 60
   gctgcagcag agcacatatc tcagttcaac gaagaagcac gttctcca 108
<210> 246
   <211> 108
   <212> DNA
   <213> PANICUM VIRGATUM
<400> 246
   gatgggagtg tgcctaatac tctggttatt gcgaactgtg aagttgtcaa accaagggtt 60
   gcagctgctg aacatatctc acagttcaat gaggaagcac gatcgcct 108
<210> 247
   <211> 34
   <212> PRT
   <213> PANICUM VIRGATUM
<400> 247
   Ile Gln Asp His Ile Ser Ala Leu Gly Asp Ser Ser Ser Ser Pro Gly
   1 5 10 15
   Ala Ser Gly Ser Lys Gln Ser Gly Lys Thr Ala Asn Glu Lys Glu Ser
   20 25 30
   Pro Lys
<210> 248
   <211> 102
   <212> DNA
   <213> PANICUM VIRGATUM
<400> 248
   atccaagacc acatatctgc ccttggggat tcctcatctt ctcctggagc atctggcagc 60
   aagcagtctg gcaaaactgc gaatgaaaag gagagtccca aa 102
<210> 249
   <211> 40
   <212> PRT
   <213> PANICUM VIRGATUM
<400> 249
   Phe Val Lys Lys Tyr Lys Thr Ile Val His Pro Gly Glu Val Asn Arg
   1 5 10 15
   Ile Arg Glu Leu Pro Gln Asn Ser Lys Ile Ile Ala Thr His Thr Asp
   20 25 30
   Ser Pro Asp Val Leu Ile Trp Asp
   35 40
<210> 250
   <211> 120
   <212> DNA
   <213> PANICUM VIRGATUM
<400> 250
   tttgtgaaga agtataagac tatagttcat cctggtgagg ttaacagaat cagggagctt 60
   ccacagaaca gtaagatcat agccactcac accgacagtc cagatgtact tatttgggat 120
<210> 251
   <211> 40
   <212> PRT
   <213> PANICUM VIRGATUM
<400> 251
   Ser Arg Pro Asp Leu Ile Leu Thr Gly His Gln Glu Asn Ala Glu Phe
   1 5 10 15
   Ala Leu Ala Met Cys Pro Ala Glu Pro Tyr Val Leu Ser Gly Gly Lys
   20 25 30
   Asp Lys Ser Val Val Leu Trp Ser
   35 40
<210> 252
   <211> 120
   <212> DNA
   <213> PANICUM VIRGATUM
<400> 252
   tctcggcctg atctgatatt aacaggacat caggaaaatg ctgaattcgc gcttgccatg 60
   tgtccagcag aaccatatgt actgtcagga ggaaaggaca aatctgttgt cttgtggagc 120
<210> 253
   <211> 40
   <212> PRT
   <213> PANICUM VIRGATUM
<400> 253
   Val Asp Pro Arg Gly Ile Phe His Gly His Asp Ser Thr Val Glu Asp
   1 5 10 15
   Val Gln Phe Cys Pro Ser Ser Ala Gln Glu Phe Cys Ser Val Gly Asp
   20 25 30
   Asp Ala Cys Leu Ile Leu Trp Asp
   35 40
<210> 254
   <211> 120
   <212> DNA
   <213> PANICUM VIRGATUM
<400> 254
   gttgatcctc ggggtatatt ccatggacat gacagcactg ttgaagatgt tcagttctgc      60
   ccttccagtg cacaggaatt ctgtagtgtg ggtgatgatg cttgtcttat tctctgggat     120
<210> 255
   <211> 40
   <212> PRT
   <213> BRASSICA NAPUS
<400> 255
   Leu Gly Pro Arg Gly Val Tyr His Gly His Asp Asp Thr Val Glu Asp
   1               5                   10                  15
   Val Ala Phe Ser Pro Thr Ser Ala Gln Glu Phe Cys Ser Val Gly Asp
               20                  25                  30
   Asp Ser Cys Leu Ile Leu Trp Asp
           35                  40
<210> 256
   <211> 120
   <212> DNA
   <213> BRASSICA NAPUS
<400> 256
   cttggcccgc gaggtgtata tcatggccat gatgataccg ttgaagatgt cgctttcagc      60
   cccactagtg cacaagagtt ctgcagtgtc ggtgacgatt cttgccttat actatgggat     120
<210> 257
   <211> 40
   <212> PRT
   <213> MEDICAGO TRUNCATULA
<400> 257
   Phe Pro Ala Val Lys Val Glu Lys Ala His Asp Gly Asp Val His Cys
   1               5                   10                  15
   Val Asp Trp Asn Thr His Asp Ile Asn Phe Ile Leu Thr Gly Ser Ala
               20                  25                  30
   Asp Asn Thr Val Arg Met Phe Asp
           35                  40
<210> 258
   <211> 40
   <212> PRT
   <213> VITIS VINIFERA
<400> 258
   Thr Pro Ala Ile Lys Val Glu Lys Ala His Asn Ala Asp Leu His Cys
   1               5                   10                  15
   Val Asp Trp Asn Pro His Asp Ile Asn Leu Ile Leu Thr Gly Ser Ala
               20                  25                  30
   Asp Asn Thr Val Arg Met Phe Asp
           35                  40
<210> 259
   <211> 40
   <212> PRT
   <213> GOSSYPIUM HIRSUTUM
<400> 259
   Gly Pro Thr Val Lys Val Glu Lys Ala His Asn Ala Asp Leu His Cys
   1               5                   10                  15
   Val Asp Trp Asn Pro His Asp Asp Asn Leu Ile Leu Thr Gly Ser Ala
               20                  25                  30
   Asp His Thr Val Arg Met Phe Asp
           35                  40
<210> 260
   <211> 40
   <212> PRT
   <213> POPULUS
<400> 260
   Ser Pro Ala Ile Lys Val Glu Arg Ala His Asn Ala Asp Leu His Cys
   1               5                   10                  15
   Val Asp Trp Asn Pro Gln Asp Asp Asn Leu Ile Leu Thr Gly Ser Ala
               20                  25                  30
   Asp Thr Ser Val Cys Met Phe Asp
           35                  40
<210> 261
   <211> 40
   <212> PRT
   <213> ARABIDOPSIS THALIANA
<400> 261
   Asn Pro Val Thr Lys Val Glu Lys Ala His Asp Ala Asp Leu His Cys
   1               5                   10                  15
   Val Asp Trp Asn Pro His Asp Asp Asn Leu Ile Leu Thr Gly Ser Ala
               20                  25                  30
   Asp Asn Thr Val Arg Leu Phe Asp
           35                  40
<210> 262
   <211> 40
   <212> PRT
   <213> GLYCINE MAX
<400> 262
   Ser Pro Val Val Lys Val Glu Lys Ala His Asn Ala Asp Leu His Cys
   1               5                   10                  15
   Val Asp Trp Asn Pro His Asp Asp Asn Leu Ile Leu Thr Gly Ser Ala
               20                  25                  30
   Asp Asn Ser Val Arg Met Phe Asp
           35                  40
<210> 263
   <211> 40
   <212> PRT
   <213> PISUM SATIVUM
<400> 263
   Ser Pro Val Val Lys Val Glu Lys Ala His Asn Ala Asp Leu His Cys
   1               5                   10                  15
   Val Asp Trp Asn Pro His Asp Asp Asn Leu Ile Leu Thr Gly Ser Ala
               20                  25                  30
   Asp Asn Ser Val Arg Leu Phe Asp
           35                  40
<210> 264
   <211> 40
   <212> PRT
   <213> MEDICAGO TRUNCATULA
<400> 264
   Ser Pro Val Val Lys Val Glu Lys Ala His Asp Ala Asp Leu His Cys
   1               5                   10                  15
   Val Asp Trp Asn Pro His Asp Asp Asn Leu Ile Leu Thr Gly Ser Ala
               20                  25                  30
   Asp Asn Ser Ile Arg Met Phe Asp
           35                  40
<210> 265
   <211> 40
   <212> PRT
   <213> PETUNIA HYBRIDA
<400> 265
   Ser Pro Val Val Lys Val Glu Lys Ala His Asn Ala Asp Leu His Cys
   1               5                   10                  15
   Val Asp Trp Asn Pro His Asp Gly Asn Phe Ile Ile Thr Gly Ser Ala
               20                  25                  30
   Asp Asn Ser Val Arg Leu Phe Asp
           35                  40
<210> 266
   <211> 40
   <212> PRT
   <213> ZEA MAYS
<400> 266
   Ala Pro Ala Val Lys Val Glu Lys Ala His Ser Gly Asp Val His Cys
   1               5                   10                  15
   Val Asp Trp Asn Pro Leu Asp Val Asn Tyr Ile Leu Thr Gly Ser Ala
               20                  25                  30
   Asp Asn Ser Val Arg Met Trp Asp
           35                  40
<210> 267
   <211> 40
   <212> PRT
   <213> ZEA MAYS
<400> 267
   Asp Pro Ala Val Lys Val Glu Lys Ala His Ser Gly Asp Val His Cys
   1               5                   10                  15
   Val Asp Trp Asn Pro Leu Asp Val Asn Tyr Ile Leu Thr Gly Ser Ala
               20                  25                  30
   Asp Asn Ser Val Arg Met Trp Asp
           35                  40
<210> 268
   <211> 24
   <212> PRT
   <213> SORGHUM BICOLOR
<400> 268
   Val Asp Trp Asn Pro Leu Asp Val Asn Tyr Ile Leu Thr Gly Ser Ala
   1               5                   10                  15
   Asp Asn Ser Val Arg Met Trp Asp
               20
<210> 269
   <211> 40
   <212> PRT
   <213> ORYZA SATIVA
<400> 269
   Gly Pro Ala Val Lys Val Glu Lys Ala His Gly Gly Asp Val His Cys
   1               5                   10                  15
   Val Asp Trp Asn Leu His Asp Val Asn Tyr Ile Leu Thr Gly Ser Ala
               20                  25                  30
   Asp Asn Ser Val Arg Met Trp Asp
           35                  40
<210> 270
   <211> 30
   <212> PRT
   <213> ORYZA SATIVA
<400> 270
   Gly Pro Ala Val Lys Val Glu Lys Ala His Gly Gly Asp Val His Cys
   1               5                   10                  15
   Val Asp Trp Asn Leu His Asp Val Asn Tyr Ile Leu Thr Gly
               20                  25                  30
<210> 271
   <211> 40
   <212> PRT
   <213> BRASSICA NAPUS
<400> 271
   Ser Pro Val Thr Lys Val Glu Lys Ala His Asp Ala Asp Leu His Cys
   1               5                   10                  15
   Val Asp Trp Asn Pro His Asp Asp Asn Leu Ile Leu Thr Gly Ser Ala
               20                  25                  30
   Asp Asn Thr Val Arg Leu Tyr Asp
           35                  40
<210> 272
   <211> 40
   <212> PRT
   <213> PANICUM VIRGATUM
<400> 272
   Ser Pro Ala Val Lys Val Glu Lys Ala His Ser Gly Asp Val His Cys
   1               5                   10                  15
   Val Asp Trp Asn Pro Leu Asp Val Asn Tyr Ile Leu Thr Gly Ser Ala
               20                  25                  30
   Asp Asn Ser Val Arg Met Trp Asp
           35                  40
<210> 273
   <211> 120
   <212> DNA
   <213> MEDICAGO TRUNCATULA
<400> 273
   tttccagctg tcaaggttga gaaggcacat gatggagatg tacattgcgt tgattggaat 60
   actcatgaca tcaattttat tctgactggc tctgctgata acacagttcg catgtttgat 120
<210> 274
   <211> 120
   <212> DNA
   <213> VITIS VINIFERA
<400> 274
   actccagcca tcaaggtcga gaaagctcat aatgctgatc ttcactgtgt tgattggaat 60
   cctcatgaca taaatcttat tctaactgga tcggctgaca atactgttcg catgtttgat 120
<210> 275
   <211> 120
   <212> DNA
   <213> GOSSYPIUM HIRSUTUM
<400> 275
   ggccccactg tcaaggttga aaaggcacat aatgctgatc tccattgtgt tgattggaac 60
   ccccatgatg acaatcttat cctgactggg tctgcagatc atactgtacg tatgtttgat 120
<210> 276
   <211> 120
   <212> DNA
   <213> POPULUS
<400> 276
   agcccagcta taaaggttga aagggcacat aatgctgatc ttcactgtgt tgattggaat 60
   cctcaagatg ataatctcat tttaactggg tctgcagata cttctgtttg catgtttgat 120
<210> 277
   <211> 120
   <212> DNA
   <213> ARABIDOPSIS THALIANA
<400> 277
   aaccctgtca cgaaggttga aaaagcgcat gatgctgatc ttcattgtgt tgattggaat 60
   cctcatgacg acaatctgat cctgacaggg tcagcagaca acactgtccg gttgtttgat 120
<210> 278
   <211> 120
   <212> DNA
   <213> GLYCINE MAX
<400> 278
   agccctgtgg ttaaggttga gaaagctcat aatgctgatc ttcactgtgt ggactggaat 60
   ccccatgatg ataatctgat tcttactggg tcagcagata attctgttcg catgtttgat 120
<210> 279
   <211> 120
   <212> DNA
   <213> PISUM SATIVUM
<400> 279
   agtcctgttg ttaaggttga aaaagctcat aatgccgatc ttcactgtgt tgactggaat 60
   ccccatgatg ataatctgat tcttactggg tcggcagata attctgttcg cttgtttgat 120
<210> 280
   <211> 120
   <212> DNA
   <213> MEDICAGO TRUNCATULA
<400> 280
   agtcctgtgg ttaaggttga aaaagctcat gatgctgatc ttcactgtgt tgactggaat 60
   ccccatgatg ataatctgat tcttactggg tcggcagata attctattcg catgtttgat 120
<210> 281
   <211> 120
   <212> DNA
   <213> PETUNIA HYBRIDA
<400> 281
   agtccagttg taaaggttga aaaagctcat aatgctgatc tccactgtgt tgactggaat 60
   cctcatgatg gcaactttat tatcactgga tctgcagata attctgtccg cttgtttgat 120
<210> 282
   <211> 120
   <212> DNA
   <213> ZEA MAYS
<400> 282
   gccccagctg ttaaggttga gaaagctcac agtggagatg ttcattgtgt tgattggaat 60
   ccccttgatg ttaactatat cttaactggg tctgccgata actctgtccg aatgtgggat 120
<210> 283
   <211> 120
   <212> DNA
   <213> ZEA MAYS
<400> 283
   gacccagctg ttaaggttga gaaagctcac agtggagatg ttcattgtgt tgattggaat 60
   ccccttgatg ttaactatat cttaactggt tctgccgata actctgtccg aatgtgggat 120
<210> 284
   <211> 72
   <212> DNA
   <213> SORGHUM BICOLOR
<400> 284
   gttgattgga atccccttga cgttaactat atcttaactg gttctgccga taactctgtc 60
   cgaatgtggg at 72
<210> 285
   <211> 120
   <212> DNA
   <213> ORYZA SATIVA
<400> 285
   ggcccagctg ttaaggttga gaaagctcat ggtggtgatg ttcattgtgt tgactggaac 60
   ctccatgatg ttaactatat cttaactggt tctgcggata attctgtccg tatgtgggac 120
<210> 286
   <211> 90
   <212> DNA
   <213> ORYZA SATIVA
<400> 286
   ggcccagctg ttaaggttga gaaagctcat ggtggtgatg ttcattgtgt tgactggaac 60
   ctccatgatg ttaactatat cttaactggt 90
<210> 287
   <211> 120
   <212> DNA
   <213> BRASSICA NAPUS
<400> 287
   agccctgtca cgaaggttga aaaggcgcac gatgctgatc ttcattgtgt cgattggaac 60
   cctcatgatg acaatctgat cctgacaggg tctgcagaca acactgttcg gttgtatgat 120
<210> 288
   <211> 120
   <212> DNA
   <213> PANICUM VIRGATUM
<400> 288
   agcccggctg ttaaggttga gaaagctcat agtggggatg ttcattgtgt ggattggaat 60
   ccgcttgatg ttaactatat cttaactggt tctgctgata actctgtccg tatgtgggat 120
<210> 289
   <211> 40
   <212> PRT
   <213> MEDICAGO TRUNCATULA
<400> 289
   Gly Ser Pro Val Tyr Lys Phe Glu Gly His Asp Glu Pro Val Leu Cys
   1               5                   10                  15
   Val Gln Trp Asn Pro Ala Lys Ser Ser Val Phe Gly Ser Gly Ala Glu
               20                  25                  30
   Asp Gly Ile Ile Asn Ile Trp Asp
           35                  40
<210> 290
   <211> 40
   <212> PRT
   <213> VITIS VINIFERA
<400> 290
   Gly Ser Pro Ile His Thr Phe Glu Gly His Thr Ala Ala Val Leu Cys
   1 5 10 15
   Val Gln Trp Ser Pro Asp Lys Ala Ser Ile Phe Gly Ser Ser Ala Glu
   20 25 30
   Asp Gly Ile Leu Asn Leu Trp Asn
   35 40
<210> 291
   <211> 40
   <212> PRT
   <213> GOSSYPIUM HIRSUTUM
<400> 291
   Gly Ser Pro Ile Tyr Lys Phe Glu Gly His Lys Ala Ala Val Leu Cys
   1 5 10 15
   Val Gln Trp Ser Pro Asp Lys Ser Ser Val Phe Gly Ser Ser Ala Glu
   20 25 30
   Asp Gly Leu Leu Asn Ile Trp Asp
   35 40
<210> 292
   <211> 40
   <212> PRT
   <213> POPULUS
<400> 292
   Gly Leu Pro Val Tyr Lys Phe Glu Gly His Lys Ala Ala Val Leu Cys
   1 5 10 15
   Val Gln Trp Ser Pro Asp Lys Ala Ser Val Phe Gly Ser Ser Ala Glu
   20 25 30
   Asp Gly Leu Leu Asn Ile Trp Asp
   35 40
<210> 293
   <211> 40
   <212> PRT
   <213> ARABIDOPSIS THALIANA
<400> 293
   Gly Ser Pro Ile Tyr Lys Phe Glu Gly His Lys Ala Ala Val Leu Cys
   1               5                   10                  15
   Val Gln Trp Ser Pro Asp Lys Ser Ser Val Phe Gly Ser Ser Ala Glu
               20                  25                  30
   Asp Gly Leu Leu Asn Ile Trp Asp
           35                  40
<210> 294
   <211> 40
   <212> PRT
   <213> GLYCINE MAX
<400> 294
   Gly Ser Pro Ile His Lys Phe Glu Gly His Lys Ala Ala Val Leu Cys
   1               5                   10                  15
   Val Gln Trp Ser Pro Asp Lys Ser Ser Val Phe Gly Ser Ser Ala Glu
               20                  25                  30
   Asp Gly Leu Leu Asn Ile Trp Asp
           35                  40
<210> 295
   <211> 40
   <212> PRT
   <213> PISUM SATIVUM
<400> 295
   Gly Ser Pro Ile His Lys Phe Glu Ala His Lys Ala Ala Val Leu Cys
   1               5                   10                  15
   Val Gln Trp Ser Pro Asp Lys Ser Ser Val Phe Gly Ser Ser Ala Glu
               20                  25                  30
   Asp Gly Leu Leu Asn Ile Trp Asp
           35                  40
<210> 296
   <211> 40
   <212> PRT
   <213> MEDICAGO TRUNCATULA
<400> 296
   Gly Ser Pro Ile His Lys Phe Glu Ala His Lys Ala Ala Val Leu Cys
   1 5 10 15
   Val Gln Trp Ser Pro Asp Lys Ser Ser Val Phe Gly Ser Ser Ala Glu
   20 25 30
   Asp Gly Leu Leu Asn Ile Trp Asp
   35 40
<210> 297
   <211> 40
   <212> PRT
   <213> PETUNIA HYBRIDA
<400> 297
   Gly Ser Pro Val His Ile Phe Glu Asn His Lys Ala Ala Val Leu Cys
   1 5 10 15
   Val Gln Trp Cys Pro Asp Arg Ser Ser Val Phe Gly Ser Thr Ala Glu
   20 25 30
   Asp Gly Arg Leu Asn Ile Trp Asp
   35 40
<210> 298
   <211> 40
   <212> PRT
   <213> ZEA MAYS
<400> 298
   Gly Ser Pro Ile His Lys Phe Glu Gly His Lys Ala Ala Val Leu Cys
   1 5 10 15
   Val Gln Trp Ser Pro Asp Arg Ala Ser Val Phe Gly Ser Ser Ala Glu
   20 25 30
   Asp Gly Phe Leu Asn Val Trp Asp
   35 40
<210> 299
   <211> 40
   <212> PRT
   <213> ZEA MAYS
<400> 299
   Gly Ser Pro Ile His Lys Phe Glu Gly His Lys Ala Ala Val Leu Cys
   1               5                   10                  15
   Val Gln Trp Ser Pro Asp Arg Ala Ser Val Phe Gly Ser Ser Ala Glu
               20                  25                  30
   Asp Gly Phe Leu Asn Val Trp Asp
           35                  40
<210> 300
   <211> 40
   <212> PRT
   <213> SORGHUM BICOLOR
<400> 300
   Ser Ser Pro Ile His Lys Phe Glu Gly His Lys Ala Ala Val Leu Cys
   1               5                   10                  15
   Val Gln Trp Ser Pro Asp Arg Ala Ser Val Phe Gly Ser Ser Ala Glu
               20                  25                  30
   Asp Gly Phe Leu Asn Val Trp Asp
           35                  40
<210> 301
   <211> 40
   <212> PRT
   <213> ORYZA SATIVA
<400> 301
   Gly Ile Pro Val His Lys Phe Glu Gly His Lys Ala Ala Val Leu Cys
   1               5                   10                  15
   Val Gln Trp Ser Pro Asp Lys Ala Ser Val Phe Gly Ser Ser Ala Glu
               20                  25                  30
   Asp Gly Phe Leu Asn Val Trp Asp
           35                  40
<210> 302
   <211> 22
   <212> PRT
   <213> ORYZA SATIVA
<400> 302
   Trp Ser Pro Asp Lys Ala Ser Val Phe Gly Ser Ser Ala Glu Asp Gly
   1               5                   10                  15
   Phe Leu Asn Val Trp Asp
               20
<210> 303
   <211> 40
   <212> PRT
   <213> BRASSICA NAPUS
<400> 303
   Gly Thr Pro Ile Tyr Lys Phe Glu Gly His Lys Ala Ala Val Leu Cys
   1               5                   10                  15
   Val Gln Trp Ser Pro Asp Lys Ser Ser Val Phe Gly Ser Ser Ala Glu
               20                  25                  30
   Asp Gly Leu Leu Asn Ile Trp Asp
           35                  40
<210> 304
   <211> 40
   <212> PRT
   <213> PANICUM VIRGATUM
<400> 304
   Gly Ser Pro Ile His Lys Phe Glu Gly His Lys Ala Ala Val Leu Cys
   1               5                   10                  15
   Val Gln Trp Ser Pro Asp Arg Ala Ser Val Phe Gly Ser Ser Ala Glu
               20                  25                  30
   Asp Gly Phe Leu Asn Val Trp Asp
           35                  40
<210> 305
   <211> 120
   <212> DNA
   <213> MEDICAGO TRUNCATULA
<400> 305
   gggtctcctg tttataaatt tgaaggccat gatgaaccag tcctctgtgt acagtggaat 60
   cctgctaaat catctgtatt tggaagtggt gccgaagatg gaattataaa catctgggac 120
<210> 306
   <211> 120
   <212> DNA
   <213> VITIS VINIFERA
<400> 306
   ggatcaccaa tccatacatt tgagggccat actgctgctg tcctttgtgt acagtggtct 60
   ccggacaagg cttcgatctt tgggagttct gcagaagatg gtatcttaaa cctctggaat 120
<210> 307
   <211> 120
   <212> DNA
   <213> GOSSYPIUM HIRSUTUM
<400> 307
   ggatcaccca tttacaagtt tgagggtcat aaagctgctg ttctatgcgt gcagtggtct 60
   ccagacaaat catctgtatt tggcagttct gctgaggatg ggctcttgaa catttgggac 120
<210> 308
   <211> 120
   <212> DNA
   <213> POPULUS
<400> 308
   ggtttgccag tctataaatt tgagggtcac aaagctgctg ttctctgtgt acagtggtct 60
   ccagataagg catctgtttt tgggagttct gcggaggatg gtctcttgaa tatttgggat 120
<210> 309
   <211> 120
   <212> DNA
   <213> ARABIDOPSIS THALIANA
<400> 309
   ggttcgccta tttacaaatt tgagggacac aaagctgctg ttctttgtgt tcagtggtct 60
   cctgataagt catccgtctt tgggagctct gcagaagatg gtctcttgaa catctgggat 120
<210> 310
   <211> 120
   <212> DNA
   <213> GLYCINE MAX
<400> 310
   gggtcaccca tccataaatt tgagggtcac aaagctgctg ttctttgtgt tcagtggtct 60
   ccagacaaat catctgtatt tggaagttca gctgaagatg gtctcttaaa catttgggac 120
<210> 311
   <211> 120
   <212> DNA
   <213> PISUM SATIVUM
<400> 311
   gggtctccta ttcataaatt tgaggctcat aaagctgccg ttctttgtgt tcagtggtct 60
   ccagacaaat catctgtatt tggaagttca gcagaagacg gtctcttaaa catttgggat 120
<210> 312
   <211> 120
   <212> DNA
   <213> MEDICAGO TRUNCATULA
<400> 312
   gggtctccta tccacaaatt tgaggctcat aaggctgctg tcctttgtgt tcagtggtct 60
   ccagacaaat catctgtatt tggaagttca gcagaagatg gtctcctgaa catttgggat 120
<210> 313
   <211> 120
   <212> DNA
   <213> PETUNIA HYBRIDA
<400> 313
   ggctcaccag tccatatctt tgaaaatcac aaagcagcag ttctttgtgt gcagtggtgt 60
   ccagacaggt cctctgtatt tgggagtact gcagaggatg gtcgtttgaa tatttgggat 120
<210> 314
   <211> 120
   <212> DNA
   <213> ZEA MAYS
<400> 314
   ggttctccaa ttcacaaatt tgaaggccat aaagctgctg ttctttgtgt tcagtggtca 60
   cctgacagag catctgtttt tggaagttct gcagaagatg gtttcttaaa cgtgtgggat 120
<210> 315
   <211> 120
   <212> DNA
   <213> ZEA MAYS
<400> 315
   ggttctccaa ttcacaaatt tgagggccat aaagctgctg ttctttgtgt tcagtggtca 60
   cctgacagag catctgtttt tggaagttct gcagaagatg gtttcttaaa tgtttgggac 120
<210> 316
   <211> 120
   <212> DNA
   <213> SORGHUM BICOLOR
<400> 316
   agttctccaa ttcataaatt tgagggccat aaagctgctg ttctttgtgt tcagtggtca 60
   cctgacagag catctgtttt tggaagttct gcggaagatg gtttcttaaa cgtgtgggat 120
<210> 317
   <211> 120
   <212> DNA
   <213> ORYZA SATIVA
<400> 317
   ggcattccag ttcacaaatt tgagggtcat aaagctgctg ttctttgtgt tcagtggtca 60
   cctgacaagg catctgtatt tggaagctct gcggaagacg gcttcttaaa tgtgtgggat 120
<210> 318
   <211> 66
   <212> DNA
   <213> ORYZA SATIVA
<400> 318
   tggtcacctg acaaggcatc tgtatttgga agctctgcgg aagacggttt cttaaatgtg        60
   tgggat                                                                   66
<210> 319
   <211> 120
   <212> DNA
   <213> BRASSICA NAPUS
<400> 319
   ggtacgccta tttacaaatt tgaaggccac aaagctgctg ttctttgcgt tcagtggtct        60
   cctgataagt catctgtttt tgggagttcc gcggaagatg gtctcttgaa catctgggat       120
<210> 320
   <211> 120
   <212> DNA
   <213> PANICUM VIRGATUM
<400> 320
   ggttctccaa ttcacaaatt tgagggccat aaagctgctg ttctttgtgt ccagtggtca        60
   cctgacagag catctgtttt cggaagttct gcggaagatg gtttcttaaa tgtgtgggat       120
<210> 321
   <211> 48
   <212> PRT
   <213> MEDICAGO TRUNCATULA
<400> 321
   Ser Pro Gly Leu Phe Phe Arg His Ala Gly His Arg Asp Lys Val Val
   1               5                   10                  15
   Asp Phe His Trp Asn Ala Ser Asp Pro Trp Thr Ile Val Ser Val Ser
               20                  25                  30
   Asp Asp Cys Ala Ser Thr Gly Gly Gly Gly Thr Leu Gln Ile Trp Arg
           35                  40                  45
<210> 322
   <211> 48
   <212> PRT
   <213> VITIS VINIFERA
<400> 322
   Pro Pro Gly Leu Phe Phe Arg His Ala Gly His Arg Asp Lys Val Val
   1 5 10 15
   Asp Phe His Trp Asn Ala Ser Asp Pro Trp Thr Ile Val Ser Val Ser
   20 25 30
   Asp Asp Gly Glu Ser Thr Gly Gly Gly Gly Thr Leu Gln Ile Trp Arg
   35 40 45
<210> 323
   <211> 48
   <212> PRT
   <213> GOSSYPIUM HIRSUTUM
<400> 323
   Ser Ala Gly Leu Phe Phe Gln His Ala Gly His Arg Asp Lys Val Val
   1 5 10 15
   Asp Phe His Trp Asn Ala Phe Asp Pro Trp Thr Val Val Ser Val Phe
   20 25 30
   Asp Asp Cys Glu Thr Thr Gly Gly Gly Gly Thr Leu Gln Ile Trp Gly
   35 40 45
<210> 324
   <211> 48
   <212> PRT
   <213> POPULUS
<400> 324
   Pro Ala Gly Leu Phe Phe Gln His Ala Gly His Arg Asp Lys Val Val
   1 5 10 15
   Asp Phe His Trp Asn Ala Ser Asp Pro Trp Thr Val Val Ser Val Ser
   20 25 30
   Asp Asp Cys Asp Thr Thr Gly Gly Gly Gly Thr Leu Gln Ile Trp Arg
   35 40 45
<210> 325
   <211> 48
   <212> PRT
   <213> ARABIDOPSIS THALIANA
<400> 325
   Pro Ala Gly Leu Phe Phe Gln His Ala Gly His Arg Asp Lys Val Val
   1               5                   10                  15
   Asp Phe His Trp Asn Ala Ser Asp Pro Trp Thr Ile Val Ser Val Ser
               20                  25                  30
   Asp Asp Cys Glu Thr Thr Gly Gly Gly Gly Thr Leu Gln Ile Trp Arg
           35                  40                  45
<210> 326
   <211> 48
   <212> PRT
   <213> GLYCINE MAX
<400> 326
   Pro Pro Gly Leu Phe Phe Gln His Ala Gly His Arg Asp Lys Val Val
   1               5                   10                  15
   Asp Phe His Trp Asn Ala Tyr Asp Pro Trp Thr Ile Val Ser Val Ser
               20                  25                  30
   Asp Asp Cys Glu Ser Thr Gly Gly Gly Gly Thr Leu Gln Ile Trp Arg
           35                  40                  45
<210> 327
   <211> 48
   <212> PRT
   <213> PISUM SATIVUM
<400> 327
   Pro Pro Gly Leu Phe Phe Gln His Ala Gly His Arg Asp Lys Val Val
   1               5                   10                  15
   Asp Phe His Trp Asn Ala Tyr Asp Pro Trp Thr Ile Val Ser Val Ser
               20                  25                  30
   Asp Asp Cys Glu Ser Thr Gly Gly Gly Gly Thr Leu Gln Ile Trp Arg
           35                  40                  45
<210> 328
   <211> 48
   <212> PRT
   <213> MEDICAGO TRUNCATULA
<400> 328
   Pro Pro Gly Leu Phe Phe Gln His Ala Gly His Arg Asp Lys Val Val
   1               5                   10                  15
   Asp Phe His Trp Asn Ala His Asp Pro Trp Thr Leu Val Ser Val Ser
               20                  25                  30
   Asp Asp Cys Glu Ser Thr Gly Gly Gly Gly Thr Leu Gln Ile Trp Arg
           35                  40                  45
<210> 329
   <211> 48
   <212> PRT
   <213> PETUNIA HYBRIDA
<400> 329
   Ala Pro Gly Leu Phe Phe Gln His Ala Gly His Arg Asp Lys Ile Val
   1               5                   10                  15
   Asp Phe His Trp Asn Val Ala Asp Pro Trp Thr Ile Val Ser Val Ser
               20                  25                  30
   Asp Asp Cys Asp Ser Thr Gly Gly Gly Gly Thr Leu Gln Ile Trp Arg
           35                  40                  45
<210> 330
   <211> 48
   <212> PRT
   <213> ZEA MAYS
<400> 330
   Pro Ala Gly Leu Phe Phe Gln His Ala Gly His Arg Asp Lys Ile Val
   1               5                   10                  15
   Asp Phe His Trp Asn Ser Ser Asp Pro Trp Thr Ile Val Ser Val Ser
               20                  25                  30
   Asp Asp Gly Glu Ser Thr Gly Gly Gly Gly Thr Leu Gln Ile Trp Arg
           35                  40                  45
<210> 331
   <211> 48
   <212> PRT
   <213> ZEA MAYS
<400> 331
   Pro Ala Gly Leu Phe Phe Gln His Ala Gly His Arg Asp Lys Ile Val
   1               5                   10                  15
   Asp Phe His Trp Asn Ser Ser Asp Pro Trp Thr Ile Val Ser Val Ser
               20                  25                  30
   Asp Asp Gly Glu Ser Thr Gly Gly Gly Gly Thr Leu Gln Ile Trp Arg
           35                  40                  45
<210> 332
   <211> 48
   <212> PRT
   <213> SORGHUM BICOLOR
<400> 332
   Pro Ala Gly Leu Phe Phe Gln His Ala Gly His Arg Asp Lys Ile Val
   1               5                   10                  15
   Asp Phe His Trp Asn Ser Ser Asp Pro Trp Thr Ile Val Ser Val Ser
               20                  25                  30
   Asp Asp Gly Glu Ser Thr Gly Gly Gly Gly Thr Leu Gln Ile Trp Arg
           35                  40                  45
<210> 333
   <211> 48
   <212> PRT
   <213> ORYZA SATIVA
<400> 333
   Pro Ala Gly Leu Phe Phe Gln His Ala Gly His Arg Asp Lys Ile Val
   1               5                   10                  15
   Asp Phe His Trp Asn Ser Ser Asp Pro Trp Thr Ile Val Ser Val Ser
               20                  25                  30
   Asp Asp Gly Glu Ser Thr Gly Gly Gly Gly Thr Leu Gln Ile Trp Arg
           35                  40                  45
<210> 334
   <211> 48
   <212> PRT
   <213> ORYZA SATIVA
<400> 334
   Pro Ala Gly Leu Phe Phe Gln His Ala Gly His Arg Asp Lys Ile Val
   1               5                   10                  15
   Asp Phe His Trp Asn Ser Ser Asp Pro Trp Thr Ile Val Ser Val Ser
               20                  25                  30
   Asp Asp Gly Glu Ser Thr Gly Gly Gly Gly Thr Leu Gln Ile Trp Arg
           35                  40                  45
<210> 335
   <211> 48
   <212> PRT
   <213> BRASSICA NAPUS
<400> 335
   Pro Ala Gly Leu Phe Phe Gln His Ala Gly His Arg Asp Lys Val Val
   1               5                   10                  15
   Asp Phe His Trp Asn Ala Glu Asp Pro Trp Thr Ile Val Ser Val Ser
               20                  25                  30
   Asp Asp Cys Glu Thr Thr Gly Gly Gly Gly Thr Leu Gln Ile Trp Arg
           35                  40                  45
<210> 336
   <211> 48
   <212> PRT
   <213> PANICUM VIRGATUM
<400> 336
   Pro Ala Gly Leu Phe Phe Gln His Ala Gly His Arg Asp Lys Ile Val
   1               5                   10                  15
   Asp Phe His Trp Asn Ser Ser Asp Pro Trp Thr Ile Val Ser Val Ser
               20                  25                  30
   Asp Asp Gly Glu Ser Thr Gly Gly Gly Gly Thr Leu Gln Ile Trp Arg
           35                  40                  45
<210> 337
   <211> 144
   <212> DNA
   <213> MEDICAGO TRUNCATULA
<400> 337
   tcccctggtt tattctttcg tcatgcaggg catagggata aggttgttga ctttcattgg 60
   aatgcatctg atccatggac aattgttagt gtatctgatg attgtgcaag cactggtgga 120
   ggtggcaccc tgcagatctg gcgg 144
<210> 338
   <211> 144
   <212> DNA
   <213> VITIS VINIFERA
<400> 338
   cctccaggtt tattcttcag acatgctggc catagggata aggtcgtgga cttccattgg 60
   aatgcatcgg atccatggac aatcgttagc gtatctgatg atggtgaaag tactggtgga 120
   ggcggtacac tccagatatg gcgg 144
<210> 339
   <211> 144
   <212> DNA
   <213> GOSSYPIUM HIRSUTUM
<400> 339
   tctgcgggac tgtttttcca gcacgctgga cacagggaca aagttgttga cttccattgg 60
   aatgcatttg atccatggac tgttgttagt gtgtttgatg actgtgaaac aactggtgga 120
   ggagggacat tgcaaatatg gggc 144
<210> 340
   <211> 144
   <212> DNA
   <213> POPULUS
<400> 340
   cctgcaggat tgtttttcca gcatgctggg cacagggata aagttgttga tttccattgg      60
   aatgcatctg atccttggac ggtggttagt gtctctgatg actgtgatac cactggcggg     120
   ggagggacac tgcagatatg gcgc                                            144
<210> 341
   <211> 144
   <212> DNA
   <213> ARABIDOPSIS THALIANA
<400> 341
   cccgctgggc tcttcttcca gcatgctggt cacagggaca aagttgttga tttccactgg      60
   aatgcttcag acccttggac tattgtcagt gtttctgatg actgtgagac tactggtgga     120
   ggtggaacat tgcagatatg gcgg                                            144
<210> 342
   <211> 144
   <212> DNA
   <213> GLYCINE MAX
<400> 342
   cctccagggt tgttttttca acatgcaggt catagggata aagttgttga cttccattgg      60
   aatgcatatg atccatggac gattgttagt gtgtctgatg actgtgaaag tactggagga     120
   gggggaacgt tgcagatatg gcgc                                            144
<210> 343
   <211> 144
   <212> DNA
   <213> PISUM SATIVUM
<400> 343
   cctccagggt tgtttttcca acatgctggt catagagaca aagttgttga cttccattgg      60
   aatgcttatg atccatggac aattgtaagt gtgtctgatg attgtgaaag tactggtgga     120
   ggaggaacat tgcagatatg gcgc                                            144
<210> 344
   <211> 144
   <212> DNA
   <213> MEDICAGO TRUNCATULA
<400> 344
   cctccagggt tgtttttcca acatgctggt catagagaca aagttgttga ctttcactgg      60
   aatgcacatg atccatggac acttgttagt gtgtctgatg attgcgaaag tactggtgga     120
   gggggaacat tgcagatatg gcgc                                            144
<210> 345
   <211> 144
   <212> DNA
   <213> PETUNIA HYBRIDA
<400> 345
   gctccaggtt tatttttcca gcatgctggg cacagggata aaattgttga cttccactgg      60
   aatgtggctg atccatggac aattgtaagt gtatctgatg actgtgactc cacaggtgga     120
   ggtggtacac tacagatatg gcgg                                            144
<210> 346
   <211> 144
   <212> DNA
   <213> ZEA MAYS
<400> 346
   ccagccgggc ttttctttca gcacgctggt catagggata agattgtaga cttccactgg      60
   aattcgtcag atccttggac aattgtcagt gtctctgatg atggcgagag cactggtgga     120
   ggtggaacac tgcagatttg gcgc                                            144
<210> 347
   <211> 144
   <212> DNA
   <213> ZEA MAYS
<400> 347
   ccagctgggc ttttctttca gcatgctggt catagggata agatcgtaga cttccactgg      60
   aattcgtcag atccttggac aattgtcagt gtctcagatg atggtgagag cactggtgga     120
   ggtggaacac tgcagatatg gcga                                            144
<210> 348
   <211> 144
   <212> DNA
   <213> SORGHUM BICOLOR
<400> 348
   ccagctgggc ttttcttcca gcatgctggt catagggata agattgtaga cttccactgg 60
   aattcgtcag atccttggac aattgtcagt gtatctgatg atggtgagag cactggtgga 120
   ggcggaacac tgcagatatg gcgc 144
<210> 349
   <211> 144
   <212> DNA
   <213> ORYZA SATIVA
<400> 349
   cctgctgggc ttttctttca acatgctggt catagggata agattgtaga cttccactgg 60
   aattcttcgg atccttggac tattgtgagt gtgtctgatg atggtgagag tactggtgga 120
   ggtggaacat tgcagatatg gcgc 144
<210> 350
   <211> 144
   <212> DNA
   <213> ORYZA SATIVA
<400> 350
   cctgctgggc ttttctttca acatgctggt catagggata agattgtaga cttccactgg 60
   aattcttcgg atccttggac tattgtgagt gtgtctgatg atggtgagag tactggtgga 120
   ggtggaacat tgcagatatg gcgc 144
<210> 351
   <211> 144
   <212> DNA
   <213> BRASSICA NAPUS
<400> 351
   ccggctggtc tcttcttcca gcatgctggt cacagggaca aagttgttga tttccactgg 60
   aatgcagagg acccttggac tattgtcagt gtttctgatg actgcgagac tactggtgga 120
   ggtggaacac tgcagatatg gcgg 144
<210> 352
   <211> 144
   <212> DNA
   <213> PANICUM VIRGATUM
<400> 352
   ccagctgggc ttttctttca acatgctggt cacagggata agattgtaga cttccactgg 60
   aattcatcgg atccttggac aatcgtcagt gtctcagatg atggtgagag caccggtgga 120
   ggtggaacac tgcagatatg gcgg 144
<210> 353
<400> 353
   000
<210> 354
<400> 354
   000
<210> 355
<400> 355
   000
<210> 356
<400> 356
   000
<210> 357
<400> 357
   000
<210> 358
<400> 358
   000
<210> 359
<400> 359
   000
<210> 360
<400> 360
   000
<210> 361
<400> 361
   000
<210> 362
<400> 362
   000
<210> 363
   <211> 227
   <212> DNA
   <213> ARABIDOPSIS THALIANA
<400> 363
   tgtttccttt gatatccgca ctttaagcat gagagagtcc ctttgatatt ggcctggttc      60
   actcagatct tacctgacca cacacgtaga tatacattat tctctctaga ttatctgatt     120
   gagccgcgcc aatatctcag tactctctcg tctctatttt ggactttgtg gtcttgtaga     180
   tcgatttgta tgtgtgtgtt gaaatggaga caagtacttg taacttc                   227
<210> 364
   <211> 21
   <212> DNA
   <213> ARTIFICIAL SEQUENCE
<220>
   <223> Synthetic Primer
<400> 364
   tgtttccttt gatatccgca c 21
<210> 365
   <211> 22
   <212> DNA
   <213> ARTIFICIAL SEQUENCE
<220>
   <223> Synthetic Primer
<400> 365
   gaagttacaa gtacttgtct cc 22
<210> 366
   <211> 66
   <212> DNA
   <213> ARTIFICIAL SEQUENCE
<220>
   <223> Synthetic Primer
<400> 366
   cgggatccat gagagagtcc ctttcacgtt ctcgatttgt gcagagatct tacctgacca      60
   cacacg                                                                 66
<210> 367
   <211> 65
   <212> DNA
   <213> ARTIFICIAL SEQUENCE
<220>
   <223> Synthetic Primer
<400> 367
   gcgagctcac gagagagtac tgacacgttc tccatttgtg aagagataat ctagagagaa    60
   taatg                                                                65
<210> 368
   <211> 135
   <212> DNA
   <213> ARTIFICIAL SEQUENCE
<220>
   <223> Amplification fragment
<400> 368
   ggatccatga gagagtccct ttcacgttct cgatttgtgc agagatctta cctgaccaca    60
   cacgtagata tacattattc tctctagatt atctcttcac aaatggagaa cgtgtcagta   120
   ctctctcgtg agctc                                                    135
<210> 369
   <211> 66
   <212> DNA
   <213> ARTIFICIAL SEQUENCE
<220>
   <223> Synthetic Primer
<400> 369
   cgggatccat gagagagtcc ctttacaagt cagatgtttt ggggagatct tacctgacca    60
   cacacg                                                               66
<210> 370
   <211> 65
   <212> DNA
   <213> ARTIFICIAL SEQUENCE
<220>
   <223> Synthetic Primer
<400> 370
   gcgagctcac gagagagtac tgaacaagtc agttgttttg tggagataat ctagagagaa    60
   taatg                                                                65
<210> 371
   <211> 135
   <212> DNA
   <213> ARTIFICIAL SEQUENCE
<220>
   <223> Amplification fragment
<400> 371
   ggatccatga gagagtccct ttacaagtca gatgttttgg ggagatctta cctgaccaca      60
   cacgtagata tacattattc tctctagatt atctccacaa aacaactgac ttgttcagta     120
   ctctctcgtg agctc                                                      135
<210> 372
   <211> 241
   <212> DNA
   <213> ARTIFICIAL SEQUENCE
<220>
   <223> Arabidopsis RNAi
<400> 372
   tctagagatg gaagtgtgcc caatactttg gtcatagcaa attgtgaagt tgttaagcca      60
   agggttgctg cagcagagca catttctcag ttcaatgaag aagcacgttc tccaggccat     120
   ctaggcctgg agaacgtgct tcttcattga actgagaaat gtgctctgct gcagcaaccc     180
   ttggcttaac aacttcacaa tttgctatga ccaaagtatt gggcacactt ccatcgagct     240
   c                                                                     241
<210> 373
   <211> 223
   <212> DNA
   <213> ARTIFICIAL SEQUENCE
<220>
   <223> Arabidopsis RNAi
<400> 373
   tctagaatcc aagatcacat cacaacgatt gggacagatt ccaaatcatc tggatctatc      60
   atcaaacaga ctggtgaagg tactgataag aatgagagtc ctactggcca tctaggccag     120
   taggactctc attcttatca gtaccttcac cagtctgttt gatgatagat ccagatgatt     180
   tggaatctgt cccaatcgtt gtgatgtgat cttggatgag ctc                       223
<210> 374
   <211> 229
   <212> DNA
   <213> ARTIFICIAL SEQUENCE
<220>
   <223> Canola RNAi
<400> 374
   tctagaatcc aggaccacat cacaaccgct ggtagtacag actccaaatc atctggatcc 60
   atcatcaaac agactggtga aggtggtgat aagactgaga gtccttctgg ccatctaggc 120
   cagaaggact ctcagtctta tcaccacctt caccagtctg tttgatgatg gatccagatg 180
   atttggagtc tgtactacca gcggttgtga tgtggtcctg gatgagctc 229
<210> 375
   <211> 265
   <212> DNA
   <213> ARTIFICIAL SEQUENCE
<220>
   <223> Arabidopsis RNAi
<400> 375
   tctagatccc gtccagattt gatactaact gggcaccaag ataatgctga atttgctctt 60
   gccatgtgcc caacggaacc ctttgtgctc tccggaggca aggacaagtc agttgttttg 120
   tggagtggcc atctaggcca ctccacaaaa caactgactt gtccttgcct ccggagagca 180
   caaagggttc cgttgggcac atggcaagag caaattcagc attatcttgg tgcccagtta 240
   gtatcaaatc tggacgggag agctc 265
<210> 376
   <211> 265
   <212> DNA
   <213> ARTIFICIAL SEQUENCE
<220>
   <223> Canola RNAi
<400> 376
   tctagatccc gtcctgattt ggtattaact ggacatcaag acaatgctga attcgctctt 60
   gcaatgtgcc caaccgaacc ctttgtcctc tctggaggca aagacaagtc agttgttttg 120
   tggagtggcc atctaggcca ctccacaaaa caactgactt gtctttgcct ccagagagga 180
   caaagggttc ggttgggcac attgcaagag cgaattcagc attgtcttga tgtccagtta 240
   ataccaaatc aggacgggag agctc 265
<210> 377
   <211> 265
   <212> DNA
   <213> ARTIFICIAL SEQUENCE
<220>
   <223> Arabidopsis RNAi
<400> 377
   tctagatttg tgaagaagta caagaccatc attcaccctg gagaggttaa cagaatcagg      60
   gaactcccac agaatagtaa gattgttgct actcacaccg acagtcctga tgttctcatt     120
   tgggatggcc atctaggcca tcccaaatga gaacatcagg actgtcggtg tgagtagcaa     180
   caatcttact attctgtggg agttccctga ttctgttaac ctctccaggg tgaatgatgg     240
   tcttgtactt cttcacaaag agctc                                           265
<210> 378
   <211> 265
   <212> DNA
   <213> ARTIFICIAL SEQUENCE
<220>
   <223> Canola RNAi
<400> 378
   tctagatttg tgaagaagtt caagaccatc attcaccctg gagaggttaa ccgaatcagg      60
   gaactcccac aaaacagtaa gattattgct actcacaccg acagtcctga tgttctcatt     120
   tgggatggcc atctaggcca tcccaaatga gaacatcagg actgtcggtg tgagtagcaa     180
   taatcttact gttttgtggg agttccctga ttcggttaac ctctccaggg tgaatgatgg     240
   tcttgaactt cttcacaaag agctc                                           265
<210> 379
   <211> 265
   <212> DNA
   <213> ARTIFICIAL SEQUENCE
<220>
   <223> Arabidopsis RNAi
<400> 379
   tctagagttg gcccacgagg tgtatatcat ggccatgaag atacagttga agatgtggca      60
   ttcagcccga cgagtgcaca agaattctgc agtgttggtg atgattcttg ccttatacta     120
   tgggatggcc atctaggcca tcccatagta taaggcaaga atcatcacca acactgcaga     180
   attcttgtgc actcgtcggg ctgaatgcca catcttcaac tgtatcttca tggccatgat     240
   atacacctcg tgggccaacg agctc                                           265

## Description

### FIELD OF THE INVENTION

The invention relates generally to the field of plant molecular biology and production of plants having an improved growth characteristic. More specifically, the invention relates to *FVE* polynucleotides and polypeptides, including portions of an FVE polynucleotide, useful in producing a plant having an improved agronomic trait. Further, the invention relates to a plant having an improved agronomic trait relative to untransformed plants. A plant produced according to the invention has decreased *FVE* expression or activity produced by a transgenic or non-transgenic method.

### BACKGROUND OF THE INVENTION

Agriculture has always sought means of increasing the yield obtained from a given unit of land. As arable land is limited there has always been development of crops having desirable characteristics. Conventionally, this has been through identification of valuable plants, breeding for desirable traits and production of hybrid plants. Genetic engineering has introduced a variety of methods to target genetic improvements to plants including the introduction of novel genes or gene constructs into plants that produce improved traits. In this way new cellular activities can be introduced or an existing gene activity may be modified. Additionally, molecular biology has provided means of introducing greater genetic variability into a population and selecting the plants that have desirable traits. More recently methods of introducing specific genetic manipulations into a plant have been developed. All of the methods have the desired result of producing and or identifying a plant having desirable traits, be that yield, environmental stress tolerance or novel product production for example. Yield is a variable term depending on the trait of interest. In some instances yield refers to production of seed or a component of a seed such as starch, oil or protein. Alternatively, yield may refer to production of total biomass or of specific plant parts, for example, tubers, leaf biomass, stem or plant architecture.

Improvement in biomass production has been a historical target for trait improvement. For example, in farm practice that has included production of improved forage crops or in silviculture production of trunk or branch material. A variety of plant material has been found useful in the production of alternative energy sources, be it as a petroleum additive or replacement such as ethanol or biodiesel, incineration technologies, gasification and pyrolysis processes. Means of increasing the biomass production of a plant will be beneficial to a variety of applications.

Herein, an *FVE* gene has been identified as a loss of function mutant that modifies the plant growth and development and finds utility in a method of increasing plant biomass and total plant production. The *FVE* gene encodes a protein having 6 copies of a WD40 domain motif. In *Arabidopsis*, WD40 motifs have been found in at least 237 proteins, in 143 distinct gene families (Nocker and Ludwig, 2003) and are believed to be involved generally as a component of transcription regulators controlling a variety of cellular processes.

*AtFVE* has also been reported as *AtMSI4*, a plant homolog of yeast MSI (multicopy suppressor of IRA1) and mammalian retinoblastoma-associated proteins RbAp46 and RbAp48 (Kenzior and Folk, 1998). A small gene family of 5 MSI-like genes was found in *Arabidopsis* genome (Ach *et al*., 1997). *AtMSI5* is the only MSI member sharing high homology (75% identity nucleic acid) to *AtFVE (AtMSI4)*, suggesting they may be functionally related. Although *AtMSI1* has been reported to be involved in regulation of flowering time, it doesn't share the same target gene as *AtFVE* (Hennig *et al*., 2003). Within the literature *FVE* has also been referred to as *ACG1, NFC4 and NFC04*.

The *FVE* gene has been found to be a component of the autonomous pathway of flowering time regulation. In studies to date *FVE* has been regarded as a repressor of *FLC*, which plays a central role in regulation of flowering time. Regulation of *FLC* is influenced by numerous pathways and the more numerous genes that make up these pathways, of which *FVE* is but one of these. The *Arabidopsis FLC* was expressed in tobacco and shown to delay flowering and increase biomass. However, as there are numerous inputs into *FLC* regulation it is not apparent what upstream component would permit the generation of desirable phenotypes. Furthermore a *FLC* orthologue has only been identified in *Brassica* and there is no known *FLC* orthologue identified outside of the Brassicaceae family, of which *Arabidopsis* is a member.

Genetic regulation of flowering time in response to environmental cues and developmental signals has been studied in *Arabidopsis*. Detection of these cues involves the light-dependent pathway (*FRI*, *FRL1*, *FL2*, and *FES1*); the vernalization pathway which initiates early flowering by cold treatment (*VIN3*, *VRN1*, *VRN2*, and *LHP1*); the autonomous pathway which mediates developmental signals (*FCA*, *FY, FLD, FPA, FYE*, *LD*, and *FLK*); and the gibberellin pathway promoting flowering by action of the hormone. The light-dependent pathway promotes the floral activator *CONSTANS* (*CO*), and in turn, activates the floral pathway integrators *FLOWERING TIME (FT)*, *SUPPRESSOR OF OVEREXPRESSION OF CONSTANS1 (SOCI)*, and *LEAFY (FLY)* that are also positively regulated by the gibberellin pathways. In a parallel manner, the vernalization and autonomous pathways repress *FLOWERING LOCUS C (FLC)*, a suppressor of the floral pathway integrators.

An *Arabidopsis fve* mutant is described by Ausin et al., Nature Genetics, Vol:36(2), 162-166. The mutant is described as having a late flowering phenotype. The wild-type *FVE* gene was over-expressed in a wild-type Columbia background with little observable phenotype to only a slight reduction in time to flowering. The authors conclude that *FVE* is not sufficient to significantly alter flowering time and that FVE polypeptide concentration in the cell seems not to be a limiting factor and FVE function may require other protein factors.

This invention is directed at the manipulation of *FVE* polynucleotides and polypeptides to decrease *FVE* expression or activity in a plant cell, tissue culture or plant. Inhibition of the *FVE* expression or activity provides a plant having an improved agronomic property, for example, increased biomass yield relative to a wild-type control plant.

### SUMMARY OF THE INVENTION

The present invention provides a method of increasing biomass accumulation in a plant relative to a wild-type plant, comprising the steps of:
a) providing a nucleic acid construct operably linked to a promoter, wherein said nucleic acid construct encodes an antisense oligonucleotide of FVE, an siRNA of FVE, an shRNA of FVE, an miRNA of FVE, an artificial miRNA of FVE, or a dominant negative mutant of FVE;
b) inserting said promoter-operably-linked nucleic acid construct into a vector;
c) transforming a plant, a tissue culture, or a plant cell with said vector to obtain a transformed plant, a transformed tissue culture, or a transformed plant cell with decreased FVE expression or activity; and
d) growing said transformed plant or regenerating a plant from said transformed tissue culture or transformed plant cell, wherein a plant having increased biomass accumulation relative to a wild-type plant is produced.

Biomass refers to an amount of plant material either in terms of dry weight or fresh weight as appropriate, and includes all plant parts, such as in reference to shoot biomass (all above ground plant parts), leaf biomass, and root biomass. As used herein, the term "increased biomass" refers to a plant biomass that is 2, 4, 5, 6, 8, 10, 20 or more fold greater as compared to the biomass of a corresponding wild-type plant. For example, a plant having increased biomass as compared to a wild-type plant may have 10%, 15%, 20%, 25%, 30%, 40%, 50%, 60 % 70%, 75% or greater biomass than the corresponding wild-type plant.

The methods of the invention involve inhibiting the expression or activity of an endogenous gene, such as *FVE,* wherein a plant is produced having increased biomass.

For example, a plant having increased biomass accumulation relative to a wild type plant is produced by a) providing a nucleic acid construct containing a promoter operably linked to a nucleic acid construct that inhibits FVE activity; b) inserting the nucleic construct into a vector; c) transforming a plant, tissue culture, or a plant cell with the vector to obtain a plant, tissue culture or a plant cell with decreased FVE activity; d) growing the plant or regenerating a plant from the tissue culture or plant cell, wherein a plant having increased biomass accumulation relative to a wild type plant is produced.

The term "nucleic acid construct" refers to a full length gene sequence or portion thereof, wherein a portion is preferably at least 19, 20, 21, 22, 23, 24, 25, 30, 40, 50, 60, 70, 75, 80, 90, 100, or 150 nucleotides in length, or the compliment thereof. Alternatively it may be an oligonucleotide, single or double stranded and made up of DNA or RNA or a DNA-RNA duplex. In a particular embodiment, the nucleic acid construct contains the full length *FVE* gene sequence, or a portion thereof, wherein the portion of the *FVE* sequence is at least 19, 20, 21, 22, 23, 24, 25, 30, 40, 50, 60, 70, 75, 80, 90, 100, or 150 nucleotides in length, or its compliment. In some embodiments, the portion of the *FVE* sequence includes an *FVE*-S1 motif, an *FVE*-S2 motif, or an *FVE*-WD40 motif.

Also provided by the invention is a stably transformed transgenic plant produced by the methods described herein. In one embodiment, the invention provides a plant having a non-naturally occurring mutation in an *FVE* gene, wherein the plant has decreased FVE expression or activity and the plant has increased biomass relative to a wild-type control. Decreased *FVE* expression or activity refers to a 2, 3, 4, 5, 6, 7, 8, 9, 10, 15, 20, 25, 30, 40, 50, 60, or 75-fold reduction or greater, at the DNA, RNA or protein level of an *FVE* gene as compared to wild-type *FVE*, or a 2, 3, 4, 5, 6, 7, 8, 9, 10, 15, 20, 25, 30, 40, 50, 60 or 75 fold reduction of FVE activity as compared to wild-type FVE activity. FVE activity includes but is not limited to regulation of flowering time, and response (acclimation) to cold.

The invention further provides a stably transformed transgenic seed produced by the transgenic plant(s) of the invention, wherein the seed produces a plant having increased biomass accumulation relative to a wild-type plant.

Disclosed herein are nucleic acids for expression of nucleic acids in a plant cell to produce a transgenic plant having an advantageous phenotype or improved property such as increased biomass. Such nucleic acids include SEQ ID NO's:1, 5, 206, 216, 220, 239, 241, 368, 371, 372, 373, 374, 375, 376, 377, 378, and 379. We also disclose compositions which contain the nucleic acids for expression in a plant cell to produce the transgenic plants described herein.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below.

In the case of conflict, the present specification, including definitions, will control. In addition, the materials, methods, and examples are illustrative only and not intended to be limiting.

### BRIEF DESCRIPTION OF THE FIGURES

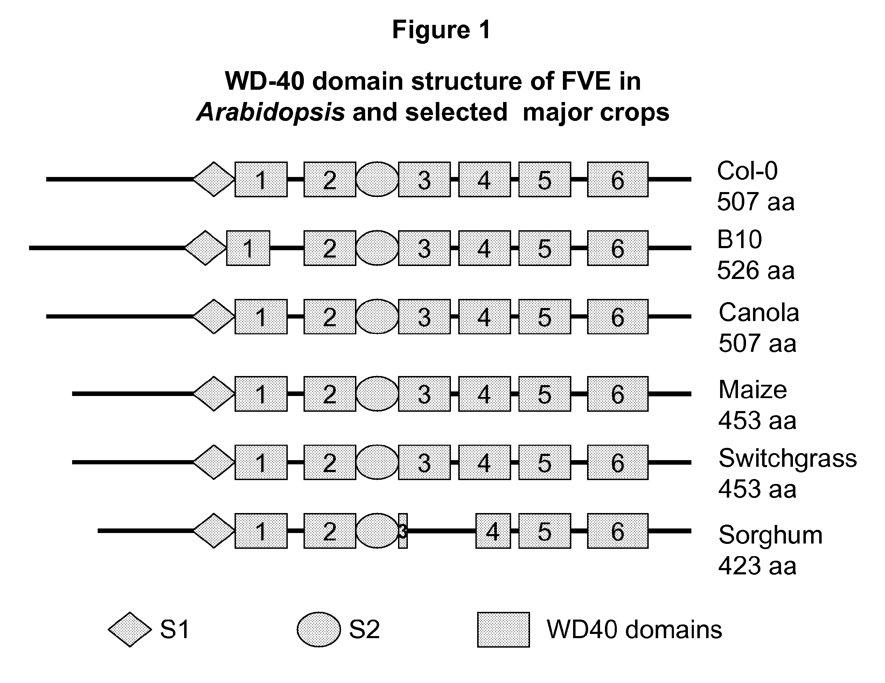

Figure 1 is an alignment of the WD-40 domain structure of *FVE* in *Arabidopsis* and selected major crops.

### DETAILED DESCRIPTION

The invention is based in part on the discovery of plants having an improved agronomic property, such as for example, increased biomass yield relative to a wild-type control. More specifically, the invention is based upon the discovery of a mutant *FVE* gene having reduced activity. Plants having this mutant *FVE* gene were observed to have increased biomass as compared to plants not having the mutant *FVE* gene.

Accordingly the invention provides methods of enhancing (e.g,, increasing) the biomass accumulation of plants by decreasing the expression or activity of a *FVE* gene or polypetide. A plant having increased biomass as compared to a wild type (e.g. control) plant is produced by introducing to a plant cell a nucleic acid construct that decreases the expression or activity of an *FVE* gene or polypeptide. The invention also includes stably transformed transgenic plants produced by the methods of the invention and the stably transformed transgenic seeds produced by the transgenic plants that produce a plant having increased biomass.

For convenience, before further description of the present invention, certain terms employed in the specification, examples and appended claims are defined herein. These definitions should be read in light of the remainder of the disclosure and as understood by a person of ordinary skill in the art.

A "promoter sequence", or "promoter", means a nucleic acid sequence capable of inducing transcription of an operably linked gene sequence in a plant cell

The term "expression cassette" means a construct, or portion thereof, comprising a gene sequence under the regulatory control of a 5' sequence, and optionally a 3' transcription termination sequence, wherein the gene sequence is transcribed. Additionally, the expressed mRNA may be translated into a polypeptide.

The terms "expression" or "over-expression" are used interchangeably and mean the expression of a gene such that the transgene is expressed. The total level of expression in a cell may be elevated relative to a wild-type cell.

The term "*FVE* nucleic acid" refers to at least a portion of a nucleic acid which encodes an *FVE* gene sequence. Similarly the term "FVE protein" or "FVE polypeptide" refers to at least a portion thereof. A portion of an *FVE* nucleic acid is at least 19, 20, 21, 22, 23, 24, 25, 30, 40, 50, 60, 70, 75, 80, 90, 100, or 150 nucleotides in length with respect to a nucleic acid which encodes an *FVE* gene, and a portion of a protein or polypeptide is at least 7, 8, 9, 10, 11, 12, 13, 14, 15, 20, 25, 30, 35, 40, 45 or 50 amino acids in length with respect to the amino acid sequence of an *FVE* gene.

The term "nucleic acid construct" means a full length gene sequence or portion thereof, wherein a portion is preferably at least 19, 20, 21, 22, 23, 24, 25, 30, 40, 50, 60, 70, 75, 80, 90, 100, or 150 nucleotides in length, or the compliment thereof. Alternatively it may be an oligonucleotide, single or double stranded and comprised of DNA or RNA or a DNA-RNA duplex. Optionally, a "nucleic acid construct" may further include an operably linked promoter and an operably linked terminator region so as to form an expression cassette.

The term "biomass" refers to an amount of plant material either in terms of dry weight or fresh weight as appropriate. Biomass includes all plant parts unless otherwise stipulated, such as in reference to shoot biomass (all above ground plant parts), leaf biomass, and root biomass.

The term "increased biomass" refers to a plant biomass that is 2, 4, 5, 6, 8, 10, 20 or more fold greater as compared to the biomass of a corresponding wild-type plant. For example, a plant having increased biomass as compared to a wild-type plant may have 10%, 15%, 20%, 25%, 30%, 40%, 50%, 60 % 70%, 75% or greater biomass than the corresponding wild-type plant.

The term "wild-type" refers to a plant or plant cell of the same species, wherein *FVE* expression has not been altered, mutated, or modulated by any natural or non-natural means.

The term "dry weight" means plant tissue that has been dried to remove the majority of the cellular water and is used synonymously and interchangeably with the term biomass.

The terms "decrease *FVE* expression or activity", "decreased *FVE* expression or activity", "inhibit *FVE* expression or activity", and "inhibition of FVE expression or activity" are used herein interchangeably, and refer to a 2, 3, 4, 5, 6, 7, 8, 9, 10, 15, 20, 25, 30, 40, 50, 60, or 75-fold reduction or greater, at the DNA, RNA or protein level of an *FVE* gene as compared to wild-type *FVE,* or a 2, 3, 4, 5, 6, 7, 8, 9, 10, 15, 20, 25, 30, 40, 50, 60 or 75 fold reduction of FVE activity as compared to wild-type FVE activity. FVE activity includes but is not limited to regulation of flowering time, and response (acclimation) to cold.

For clarity the use of gene names have followed the following format. Mutant genes (nucleic acids) in lower case italics; wild-type genes (nucleic acids) in upper case italics; proteins in regular font and lower case for mutant and regular case and upper case for wild-type. In some cases the contextual meaning may prevail over a format designation.

### Sequences

The following sequences and corresponding sequence identifiers are employed throughout the specification, examples and appended claims:

| | | |
|---|---|---|
| SEQ ID NO:1 | At*fve* b10 | nucleotide |
| SEQ ID NO:2 | Atfve b10 | polypeptide |
| SEQ ID NO:3 | At*FVE* WT | nucleotide |
| SEQ ID NO:4 | AtFVEWT | polypeptide |
| SEQ ID NO:5 | At*fve* b10 | Genomic |
| SEQ ID NO:6 | At*FVE* WT | Genomic |
| SEQ ID NO:7 | *FVE* cotton | nucleotide |
| SEQ ID NO:8 | FVE cotton | polypeptide |
| SEQ ID NO:9 | *FVE* soybean | nucleotide |
| SEQ ID NO:10 | FVE soybean | polypeptide |
| SEQ ID NO:11 | *FVE* petunia | nucleotide |
| SEQ ID NO:12 | FVE petunia | polypeptide |
| SEQ ID NO:13 | *FVE* P. sativum | nucleotide |
| SEQ ID NO:14 | FVE P. sativum | polypeptide |
| SEQ ID NO:15 | *FVE* poplar | nucleotide |
| SEQ ID NO:16 | FVE poplar | polypeptide |
| SEQ ID NO:17 | *FVE* grape | nucleotide |
| SEQ ID NO:18 | FVE grape | polypeptide |
| SEQ ID NO:19 | *FVE* sorghum | nucleotide |
| SEQ ID NO:20 | FVE sorgham | polypeptide |
| SEQ ID NO:21 | *FVE* maize-1 | nucleotide |
| SEQ ID NO:22 | FVE maize-1 | polypeptide |
| SEQ ID NO:23 | *FVE* maize-2 | nucleotide |
| SEQ ID NO:24 | FVE maize-2 | polypeptide |
| SEQ ID NO:25 | *FVE* rice-1 | nucleotide |
| SEQ ID NO:26 | FVE rice-1 | polypeptide |
| SEQ ID NO:27 | *FVE* rice-2 | nucleotide |
| SEQ ID NO:28 | FVE rice-2 | polypeptide |
| SEQ ID NO:29 | *FVE* medicago-1 | nucleotide |
| SEQ ID NO:30 | FVE medicago-1 | polypeptide |
| SEQ ID NO:31 | *FVE* medicago-2 | nucleotide |
| SEQ ID NO:32 | FVE medicago-2 | polypeptide |
| SEQ ID NO:33 | *FVE* silene latifoloa | nucleotide |
| SEQ ID NO:34 | FVE silene latifoloa | polypeptide |
| SEQ ID NO:35 | *FVE* physcomitrella | nucleotide |
| SEQ ID NO:36 | FVE physcomitrella | polypeptide |
| SEQ ID NO:206 | *FVE* Brassica | nucleotide |
| SEQ ID NO:207 | FVE Brassica | polypeptide |
| SEQ ID NO:216 | *FVE* Switchgrass | nucleotide |
| SEQ ID NO:217 | FVE switchgrass | polypeptide |
| SEQ ID NO:220 | *FVE* Maize-3 | nucleotide |
| SEQ ID NO:221 | FVE maize-3 | polypeptide |
| SEQ ID NO:239 | *FVE* soybean-2 | nucleotide |
| SEQ ID NO:240 | FVE soybean-2 | polypeptide |
| SEQ ID NO:241 | *FVE* Brachypodium | nucleotide |
| SEQ ID NO:242 | FVE Brachypodium | polypeptide |
| SEQ ID NO:363 | At miRNA171 pre | nucleotide |
| SEQ ID NO:368 | At amiR171-*FVE*-S1 | nucleotide |
| SEQ ID NO:371 | At amiR171-*FVE*-WD2 | nucleotide |
| SEQ ID NO:372-379 Hairpin-RNAi | expression cassettes | nucleotide |
| SEQ ID NO:37-50, 243, 244 | S1 motif | polypeptide |
| SEQ ID NO:51-64, 245, 246 | S1 motif | nucleotide |
| SEQ ID NO:65-78,223, 247 | S2 motif | polypeptide |
| SEQ ID NO:79-92, 233, 248 | S2 motif | nucleotide |
| SEQ ID NO:93-106, 228, 251 | WD40-2 motif | polypeptide |
| SEQ ID NO:107-120, 234, 252 | WD40-2 motif | nucleotide |
| SEQ ID NO:121-134, 236, 249 | WD40-1 motif | polypeptide |
| SEQ ID NO:135-148, 235, 250 | WD40-1 motif | nucleotide |
| SEQ ID NO:149-162, 255, 253 | WD40-3 motif | polypeptide |
| SEQ ID NO:163-176, 256, 254 | WD40-3 motif | nucleotide |
| SEQ ID NO:257-272 | WD40-4 motif | polypeptide |
| SEQ ID NO:273-288 | WD40-4 motif | nucleotide |
| SEQ ID NO:289-304 | WD40-5 motif | polypeptide |
| SEQ ID NO:305-320 | WD40-5 motif | nucleotide |
| SEQ ID NO:321-336 | WD40-6 motif | polypeptide |
| SEQ ID NO:337-352 | WD40-6 motif | nucleotide |

### Determining homology between two or more sequences

To determine the percent homology of two amino acid sequences or of two nucleic acids, the sequences are aligned for optimal comparison purposes (*e.g*., gaps can be introduced in either of the sequences being compared for optimal alignment between the sequences). The amino acid residues or nucleotides at corresponding amino acid positions or nucleotide positions are then compared. When a position in the first sequence is occupied by the same amino acid residue or nucleotide as the corresponding position in the second sequence, then the molecules are homologous at that position (*i*.*e*., as used herein amino acid or nucleic acid "homology" is equivalent to amino acid or nucleic acid "identity").

The nucleic acid sequence homology may be determined as the degree of identity between two sequences. The homology may be determined using computer programs known in the art, such as GAP software provided in the GCG program package. See, Needleman and Wunsch 1970 J Mol Biol 48: 443-453. Using GCG GAP software with the following settings for nucleic acid sequence comparison: GAP creation penalty of 5.0 and GAP extension penalty of 0.3, the coding region of the analogous nucleic acid sequences referred to above exhibits a degree of identity preferably of at least 70%, 75%, 80%, 85%, 90%, 95%, 98%, or 99%, with the coding sequence portion of the DNA sequence shown in SEQ ID NO:1.

The term "sequence identity" refers to the degree to which two polynucleotide or polypeptide sequences are identical on a residue-by-residue basis over a particular region of comparison. The term "percentage of sequence identity" is calculated by comparing two optimally aligned sequences over that region of comparison, determining the number of positions at which the identical nucleic acid base (*e.g*., A, T, C, G, U, or I, in the case of nucleic acids) occurs in both sequences to yield the number of matched positions, dividing the number of matched positions by the total number of positions in the region of comparison (*i*.*e*., the window size), and multiplying the result by 100 to yield the percentage of sequence identity. The term "substantial identity" as used herein denotes a characteristic of a polynucleotide sequence, wherein the polynucleotide comprises a sequence that has at least 80 percent sequence identity, preferably at least 85 percent identity and often 90 to 95 percent sequence identity, more usually at least 99 percent sequence identity as compared to a reference sequence over a comparison region. The term "percentage of positive residues" is calculated by comparing two optimally aligned sequences over that region of comparison, determining the number of positions at which the identical and conservative amino acid substitutions, as defined above, occur in both sequences to yield the number of matched positions, dividing the number of matched positions by the total number of positions in the region of comparison (*i.e*., the window size), and multiplying the result by 100 to yield the percentage of positive residues.

### AtFVE antibody preparation:

Polyclonal antibodies to FVE were produced using purified wild-type *Arabidopsis* FVE protein as an antigen. A full length cDNA of wild-type *AtFVE* was PCR amplified using the primer pair SEQ ID NO's:199 and 200 and cloned into the expression vector pMAL-c2h using BamH I and Sal I restriction sites.

The bacteria strain DH5α was used as a host to express AtFVE. A positive clone that produced fusion proteins of maltose binding protein (MBP) and AtFV*E* of the correct size (99KD) were selected for protein expression in large scale. After affinity purification through amylase resin, the fusion protein was separated in SDS-PAGE gel, then recovered and eluted through an Electro-Eluter and then condensed with Centriprep YM-30 and Centricom YM-30. Total of 500µg of MBP-AtFVE fusion protein were obtained and 400µg of MBP-AtFVE protein were used for the antibody preparation.

For antibody production, purified protein was diluted with PBS and mixed with an adjuvant of Titermax Gold. Pre-immune sera were collected as control and kept at -80°C. For the first injection, 150µg of MBP-AtFVE were injected into a rabbit; three weeks after the first injection, second injection of 150µg fusion protein was performed. Two weeks after the second injection, a third injection of 100µg was administered. The final anti-sera were collected 1 week after the third injection and the antibody titer was determined.

### Inhibition of endogenous FVE expression and activity

An aspect of the invention pertains to means and methods of inhibiting *FVE* expression and activity. There are numerous methods known to those skilled in the art of achieving such inhibition that effect a variety of steps in a gene expression pathway, for example transcriptional regulation, post transcriptional and translational regulation. Such methods include, but are not limited to the use of antisense oligonucleotides; RNA interference (RNAi), including short interfering RNAi (siRNA) and short hairpin constructs (shRNA); microRNA (miRNA), including artificial miRNA (amiRNA) (Schwab et al., The Plant Cell 18:1121-1133 (2006)) technologies; aptamers; ribozymes; mutagenesis and TILLING methods; *in vivo* site specific mutagenesis techniques; and dominant/negative inhibition approaches.

One method of inhibiting *FVE* expression and activity involves the use of an FVE antisense nucleic acid. Antisense nucleic acids do not act catalytically to degrade mRNA, but instead involve single-stranded RNA fragments which physically bind to mRNA and block protein translation (see *e.g.,* Weiss et al., Cell. Mol. Life Sci., 55:334-358 (1999)). As used herein, an FVE "antisense" nucleic acid refers to a nucleic acid capable of hybridizing by virtue of some sequence complementary to a portion of an RNA (preferably mRNA) encoding *FVE*. The antisense nucleic acid may be complementary to a coding and/or noncoding region of an mRNA encoding *FVE*. Such antisense nucleic acids have utility as compounds that inhibit *FVE* expression, and can be used to produce a plant having an improved agronomic trait. The antisense nucleic acids of the invention are double-stranded or single-stranded oligonucleotides, RNA or DNA or a modification or derivative thereof, and can be directly administered to a cell or produced intracellularly by transcription of exogenous, introduced sequences. The antisense portion needs not be a full length gene nor be 100% identical. Provided that the antisense is at least about 70% identical to the endogenous target gene and of about 50 nucleotides or greater in length the desired inhibition will be obtained. The expressed gene may be heterologous to the transformed species, for example an *Arabidopsis* gene may be introduced into a heterologous species such as *Brassica*, soybean, maize, wheat, or grasses.

The antisense oligonucleotides can be DNA or RNA or chimeric mixtures or derivatives or modified versions thereof and can be single-stranded or double-stranded. In addition, the antisense molecules may be polymers that are nucleic acid mimics, such as PNA, morpholino oligos, and LNA (see *e.g.,* Morcos, et al., Biochem Biophys Res Commun 358(2): 521-7 (2007).

Another method of FVE inhibition involves RNA interference (also referred to as "RNA-mediated interference" (RNAi) or post-transcriptional gene silencing (PTGS)) through the use of short interfering RNAs (siRNA) and/or or short hairpin RNA (shRNA) constructs. RNAi is a mechanism that inhibits gene expression at the stage of translation or by hindering the transcription of specific genes (see, *e.g.,* Fire et al., Nature 391(6669):806-11(1998)). siRNA are double-stranded RNA molecules that are involved in the RNAi pathway (see, *e.g.,* Hamilton et al., Science 286(5441):950-2 (1999)). A portion of the gene to be inhibited (*e.g*., *FVE*) is used and cloned in a sense and antisense direction having a spacer separating the sense and antisense portions. The size of the gene portions should be at least 20 nucleotides in length and the spacer may be a little as 13 nucleotides in length (see *e.g*., Kennerdell and Carthew, 2000), and preferably should be at least 50 nucleotides in length and the spacer may be a little as 4 nucleotides. A spacer sequence may be an intron sequence, a coding or non-coding sequence, or formed from the ligation at a splice site such as SfiI. The sequence selected for an RNAi construct should have specificity to the native target gene and should not alter the expression of non-target genes. For example, if a WD40 motif, or portion thereof, is selected it should be distinct from WD40 motifs of non-target genes, *i.e* non-*FVE* genes.

Specific RNAi pathway proteins are guided by the siRNA to the targeted messenger RNA (mRNA), where they "cleave" the target, breaking it down into smaller portions that can no longer be translated into protein. The RNAi pathway is initiated by the enzyme dicer, which cleaves long, dsRNA molecules into short fragments of 20-25 base pairs. One of the two strands of each fragment, known as the guide strand, is then incorporated into the RNA-induced silencing complex (RISC) and pairs with complementary sequences. SiRNAs can be exogenously (artificially) introduced into cells by various transfection methods known to those of skill in the art to bring about the specific knockdown of a gene of interest.

shRNA is a sequence of RNA that makes a tight hairpin turn that can be used to silence gene expression via RNA interference. The shRNA hairpin structure is cleaved by the cellular machinery into siRNA, which is then bound to the RNA-induced silencing complex (RISC). This complex binds to and cleaves mRNAs which match the siRNA that is bound to it. Accordingly, siRNAs/shRNAs capable of targeting and cleaving homologous FVE mRNA are useful for producing a plant having an improved agronomic trait.

Alternatively, artificial microRNA (amiRNA) inhibition can be used to inhibit gene expression and activity in a more specific manner. In contrast to small inhibitory RNA (siRNA) that requires a perfect match between the siRNA and the target mRNA, amiRNA allows up to 5 mismatches with no more than 2 consecutive mismatches. The construction of amiRNA needs to meet certain criteria described in Schawab *et al*., 2006. This provides a method to down-regulate a target gene expression and/or activity using a gene portion comprising of at least a 21 nucleotide sequence of the target gene, such as *FVE.* Artificial microRNA approaches can be applied more broadly for down-regulation of genes within a plant gene family. A single construct can be used to down regulate the homologs in closely related species. For example, if an amiRNA construct is based on a conserved region between *Arabidopsis* and canola, the same construct can be used to regulate the endogenous gene in both *Arabidopsis* and canola. Another example, a single common amiRNA construct can be used to down regulate homologous targets in several cereal species such as brachypodium, miscanthus, switchgrass, maize, sorghum and rice.

The first micro RNA (miRNA) *lin-4* was discovered in 1993 by Victor Ambros (Cell 75, 843-854, 1993). The miRNAs originate from capped & polyadenylated full length precursor-miRNA (pri-miRNA). Through a series of processing steps, a pri-miRNA produces a pre-miRNA then to a mature miRNA of about 21 nucleotides. miRNAs in plants were first identified and reported in Brenda J. Reinhart et al., Genes & Development 16:1616-1626, (2002). Endogenous miRNA sequences have been modified and used to produce artificial microRNA for gene silencing of desired target genes. For example, amiRNA171s have been reported to down-regulate target genes (Eneida Abreu Parizotto, et al., Genes & Development 18:2237-2242, (2004); Jing Qu et al., Journal of Virology, 81 (12): 6690-6699 (2007)).

Artificial miRNAi cassettes can be engineered using a PCR strategy that uses a first PCR reaction to amplify a precursor miRNA which serves as template for a second PCR reaction using primers that introduce the desired target gene sequence, and optionally a cloning site, into the product and amplifies the loop portion of the precursor amiRNA. This forms the amiRNA cassette that can then be cloned into a vector or transformation vector for introduction into a plant cell.

Another method of FVE inhibition involves the use of aptamers. Aptamers are capable of specifically binding to selected targets and modulating the target's activity, *e.g*., through binding aptamers may block their target's ability to function. A typical aptamer is 10-15 kDa in size (30-45 nucleotides), binds its target with sub-nanomolar affinity, and discriminates against closely related targets (*e.g*., aptamers will typically not bind other proteins from the same gene family). A series of structural studies have shown that aptamers are capable of using the same types of binding interactions (*e.g*., hydrogen bonding, electrostatic complementarities, hydrophobic contacts, steric exclusion) that drive affinity and specificity in antibody-antigen complexes. As such, aptamers have utility as compounds that inhibit *FVE* expression, and can be used to produce a plant having an improved agronomic trait (*e.g*., increased biomass). As used herein, an FVE aptamer refers to a nucleic acid capable of specifically binding and modulating (*e.g*., inhibiting) the function of *FVE.* The process known in the art as SELEX (Systematic Evolution of Ligands by Exponential Enrichment) can be used to generate FVE aptamers, as described in U.S. Pat. Nos. 5,475,096 and 5,270,163.

Yet another method of FVE inhibition involves the reduction of *FVE* expression and activity by using enzymatic nucleic acid molecules. The term enzymatic nucleic acid is used interchangeably with phrases such as ribozymes, catalytic RNA, enzymatic RNA, catalytic DNA, aptazyme or aptamer-binding ribozyme, regulatable ribozyme, catalytic oligonucleotides, nucleozyme, DNAzyme, RNA enzyme, endoribonuclease, endonuclease, minizyme, leadzyme, oligozyme or DNA enzyme. All of these terminologies describe nucleic acid molecules with enzymatic activity. Ribozymes, for example, can be designed to catalytically cleave gene mRNA transcripts encoding *FVE*, preventing translation of target gene mRNA and, therefore, expression of the gene product. The mechanism of ribozyme action involves sequence-specific hybridization of the ribozyme molecule to complementary target RNA, followed by an endonucleolytic cleavage event. The composition of ribozyme molecules must include one or more sequences complementary to the target gene mRNA, and must include the well known catalytic sequence responsible for mRNA cleavage (see, *e.g*., U.S. Pat. No. 5,093,246). While ribozymes that cleave mRNA at site-specific recognition sequences can be used to destroy mRNAs encoding *FVE*, the use of hammerhead ribozymes is preferred. Hammerhead ribozymes cleave mRNAs at locations dictated by flanking regions that form complementary base pairs with the target mRNA. The sole requirement is that the target mRNA has the following sequence of two bases: 5'-UG-3'. The construction and production of ribozymes (including modified ribozymes) is well known in the art (see *e.g*., U.S. Patent No. 6,448,009; U.S. Patent No. 6,890,908; Cammeron and Jennings (Proc.Natl.Acad.Sci. USA 86 (1986), 9139-9143); Cotten et al. (Mol.Cell.Biol. 9 (1989), 4479-4487; Perreault et al. (Nature 344 (1990), 565-567)).

The FVE antisense nucleic acids, siRNA/shRNA constructs, aptamers, and/or ribozymes may be modified at a base moiety, sugar moiety or phosphate backbone to improve, *e.g*., the stability, hybridization, or solubility of the molecule. For example, the deoxyribose phosphate backbone of the nucleic acid molecules can be modified to generate peptide nucleic acids (see *e.g*., Hyrup, B. and Nielsen, P. E. (1996) BIOORG. MED. CHEM. 4(1):5-23). Peptidyl nucleic acids have been shown to hybridize specifically to DNA and RNA under conditions of low ionic strength.

Dominant-negative inhibition is analogous to competitive inhibition of biochemical reactions. Expression of a modified or mutant polypeptide that lacks full functionality competes with the wild-type or endogenous polypeptide thereby reducing the total gene/protein activity. For example an expressed protein may bind to a protein complex or enzyme subunit to produce a non-functional complex or a complex having reduced levels of activity. Alternatively the expressed protein may bind substrate but not have full activity to perform the native function. Expression of sufficient levels of non active protein will reduce or inhibit the overall function. The strategies of dominant negative effects have been applied to study gene function by changing the activity of an endogenous gene through over-expression of inactive proteins, or over-expression of mutant transcription factors lacking DNA-binding domains, or over-expression of isolated DNA-binding domains. The over-expressed inactive protein will compete with the wild-type form of the protein resulting in dominant negative effects. The over-expression of a mutant transcription factor lacking a DNA-binding domain may form heterodimers with endogenous factors or may titrate interacting components of the transcription machinery, thereby preventing the endogenous factor from binding to DNA. An over-expressed isolated DNA-binding domain will occupy the endogenous DNA-binding sites such that the endogenous factor cannot bind to DNA and therefore can not make the necessary protein-protein contacts for transcription.

Expression of an *FVE* gene that produces an altered FVE protein that is lacks full functionality can be used for dominant-negative down-regulation of gene activity. An altered FVE polypeptide is produced that, for example, may associate with or bind to a target molecule but lacks endogenous activity. A target molecule may be an interacting protein or a nucleic acid sequence. In this manner the endogenous FVE protein is effectively diluted and downstream responses will be attenuated.

For example, expression of an *fve-b10* (SEQ ID NO:1) will produce a protein that has an altered amino acid sequence relative to a wild-type *FVE* gene and lacks full activity resulting in a plant having inhibited *FVE* expression and activity thereby producing a plant having an advantageous phenotype or improved property such as increased biomass. An altered *FVE*-like sequence such as *Atfve-b10* may have an additional polypeptide sequence that alters the conformation of the protein folding and alters the activity of the protein. Other FVE coding sequences can be produced that will insert or delete amino acids that result in a change in protein folding or conformation. The resulting proteins that do not have full activity are useful for expression in a dominant-negative type down-regulation strategy.

*In vivo* site specific mutagenesis is now available whereby one can introduce a mutation into a cells genome to create a specific mutation. The method as essentially described in Dong et al. Plant Cell Reports 25:457-465 (2006), or US patent application publication number 20060162024, which refer to the methods of oligonucleotide-directed gene repair. Alternatively one may use chimeric RNA/DNA oligonucleotides essentially as described Beetham, Pro. Natl. Acad. Sci. USA, Vol.96 pp. 8774-8778 (1999), or in Okuzaki and Toriyama, Plant Cell Reports, 22:509-512 (2004). Accordingly, a premature stop codon may be generated in the cells' endogenous gene thereby producing a specific null mutant. Alternatively, the mutation may interfere with splicing of the initial transcript thereby creating a non-translatable mRNA or an mRNA that produces an altered polypeptide which does not possess endogenous activity. Changes may also be introduced into the native promoter region to reduce gene transcription and thereby reduce or eliminate gene expression and activity.

TILLING is a method of isolating mutations in a known gene from an EMS-mutagenized population. The population is screened by methods essentially as described in (Greene *et al*., 2003). Provided with the disclosure concerning which gene is the best target gene, an *FVE* gene in this case, the TILLING methods are routine to isolate a plant having the desired mutation that inhibits gene activity.

Zinc fingers can be used to up-regulate or down-regulate any gene in a plant as described by Choo et al., in US Application 2008/0070306 and related patents and patent applications. By designing a zinc finger with a transactivating domain the induction of an endogenous gene can be accomplished specifically and bypass any endogenous regulation of the targeted gene. Previously, the only available method was to introduce a transgene in another location of the genome under the regulation of a separate promoter. The zinc fingers of the present invention can also be used to down-regulate any gene in any plant, which has previously only been possible using techniques such as, but not limited to, antisense, ribozymes, co-suppression and RNAi methods including hair-pinRNA, siRNA, microRNA and artificial-microRNA. The zinc finger approach to down-regulation is highly potent and allows the targeting of specific member of a gene family without affecting the other members.

A zinc finger chimera is a transcription factor that comprises a DNA binding domain (comprising a number of zinc fingers), designed to bind specifically to any DNA sequence and one or more further domains. Usually, a nuclear localization domain is attached to the zinc finger domain to direct the chimera to the nucleus. Generally, the chimera also includes an effector domain that can be a transactivation or repression domain to regulate the expression of the gene in question (see *e.g.,* Choo and Klug, Curr. Opin. Biotech. 6:431-436 (1995); Choo and Klug (1997); Rebar and Pabo, Science 263:671-673 (1994); and Jamieson et al. Biochem. 33:5689-5695 (1994)). The zinc finger chimera may also include other domains which may be advantageous. For example, DNA modifying domains (such as endonucleases and methylases) can be added to the zinc finger domain, conferring to the zinc finger chimera the ability to regulate expression of the gene of interest or modify any DNA specifically (*see e.g*., Wu et al., Proc. Natl. Acad. Sci. USA 92:344-348 (1995); Nahon and Raveh (1998); Smith *et al*. (1999); and Carroll *et al*. (1999)).

Transcription of the *FVE* gene may be modulated using zinc-finger derived transcription factors (ZFPs). These ZFPs comprise both a DNA recognition domain and a functional domain that causes activation or repression of a target nucleic acid such as an *FVE* nucleic acid. Therefore, activating and repressing ZFPs can be created that specifically recognize the *FVE* promoters described above and used to increase or decrease *FVE* expression in a plant, thereby modulating the phenotypes of the plant. Methods of altering a gene expression are described in US Application 2008/0070306 and related patents and patent applications.

Other strategies of gene inhibition will be apparent to the skilled worker including those not discussed here and those developed in the future.

### Identification of FVE homologues

*Arabidopsis FVE* homologs were identified using database sequence search tools, such as the Basic Local Alignment Search Tool (BLAST) (Altschul *et al.* I 215:403-410 (1990); and Altschul et al. Nucl. Acids Res. 25: 3389-3402 (1997)). The tblastn or blastn sequence analysis programs were employed using the BLOSUM-62 scoring matrix (Henikoff, S. and Henikoff, J. G. Proc. Natl. Acad. Sci. USA 89:10915-10919). The output of a BLAST report provides a score that takes into account the alignment of similar or identical residues and any gaps needed in order to align the sequences. The scoring matrix assigns a score for aligning any possible pair of sequences. The P values reflect how many times one expects to see a score occur by chance. Higher scores are preferred and a low threshold P value threshold is preferred. These are the sequence identity criteria. The tblastn sequence analysis program was used to query a polypeptide sequence against six-way translations of sequences in a nucleotide database. Hits with a P value less than -25, preferably less than -70, and more preferably less than -100, were identified as homologous sequences (exemplary selected sequence criteria). The blastn sequence analysis program was used to query a nucleotide sequence against a nucleotide sequence database. In this case too, higher scores were preferred and a preferred threshold P value was less than -13, preferably less than -50, and more preferably less than -100.

An *FVE* gene can be isolated via standard PCR amplification techniques. Use of primers to conserved regions of an *FVE* gene and PCR amplification produces a fragment or full length copy of the desired gene. The PCR template may be DNA, genomic or a cDNA library, or RNA for use with reverse transcriptase PCR (RT-PCR) techniques. Conserved regions can be identified using sequence comparison tools such as BLAST or CLUSTALW for example. Suitable primers have been used and described elsewhere in this application (See Tables 6-12).

Alternatively, a fragment of a sequence from an *FVE* gene, for example; SEQ ID NO:1, 3, 5, 6, 7, 9, 11,13, 15, 17, 19, 21, 23, 25, 27, 29, 31, 33, 35, 206, 216, 220, 239, and 241 is ³²P-radiolabeled by random priming (Sambrook et al., (1989) Molecular Cloning. A Laboratory Manual, 2nd Ed., Cold Spring Harbor Laboratory Press, New York) and used to screen a plant genomic library (the exemplary test polynucleotides). As an example, total plant DNA from *Arabidopsis thaliana*, *Nicotiana tabacum*, *Lycopersicon pimpinellifolium*, *Prunus avium, Prunus cerasus*, *Cucumis sativus*, or *Oryza sativa* are isolated according to Stockinger al (Stockinger, E. J., et al., J. Heredity, 87:214-218(1996)). Approximately 2 to 10 µg of each DNA sample are restriction digested, transferred to nylon membrane (Micron Separations, Westboro, Mass.) and hybridized. Hybridization conditions are: 42°C in 50% formamide, 5X SSC, 20 mM phosphate buffer 1X Denhardt's, 10% dextran sulfate, and 100 µg/ml herring sperm DNA. Four low stringency washes at RT in 2X SSC, 0.05% sodium sarcosyl and 0.02% sodium pyrophosphate are performed prior to high stringency washes at 55°C in 0.2X SSC, 0.05% sodium sarcosyl and 0.01% sodium pyrophosphate. High stringency washes are performed until no counts are detected in the washout according to Walling *et al.* (Walling, L. L., et al., Nucl. Acids Res. 16:10477-10492 (1988)). Positive isolates are identified, purified and sequenced. Other methods are available for hybridization, for example the ExpressHyb ^{™} hybridization solution available from Clontech.

### Identification of FVE sequence motifs for use in FVE gene inhibition

Another aspect of the invention is selection of sequence motifs to be used for specific down regulation of *FVE.* The *FVE* gene comprises six WD-40 motifs. The WD-40 motifs are reported to be commonly found in proteins involved in basic cell regulatory processes. It would therefore be expected that down regulation of a WD-40 motif will affect a large number of non-target proteins and likely result in a plethora of phenotypes if not lethality. In fact, WD40 domains are not absolutely identical and possess sequence flexibility while still meeting the requirements of the domain type. The conserved elements of a WD40 motif are widely spaced and therefore provide a surprising degree of variation in sequence. Use of a WD40 repeat can be used provided that the homology to the target gene motif is high.

A WD40 domain is found in a number of eukaryotic proteins that cover a wide variety of functions including adaptor/regulatory modules in signal transduction, pre-mRNA processing and cytoskeleton assembly. A WD40 domain typically contains a GH dipeptide 11-24 residues from its N-terminus, and the WD dipeptide at its C-terminus which is 40 residues long, hence the name WD40. Between GH and WD lies a conserved core that serves as a stable propeller-like platform to which proteins can bind either stably or reversibly. This conserved core forms a propeller-like structure with several blades where each blade is composed of a four-stranded anti-parallel b-sheet. Instances with few detectable copies are hypothesized to form larger structures by dimerization. Each WD40 sequence repeat forms the first three strands of one blade and the last strand in the next blade. The last C-terminal WD40 repeat completes the blade structure of the first WD40 repeat to create the closed ring propeller-structure. Residues on the top and bottom surface of the propeller are proposed to coordinate interactions with other proteins and/or small ligands. Six copies of the repeat are present in this alignment. Neither the GH dipeptide nor the WD dipeptide is absolutely conserved. When present in a protein, the WD motif is typically found as 4-10 tandem repeats (Smith *et al*., 1999; and Nocker and Ludwig, 2003).

Sequence analysis has identified all six WD40 motifs in At*FVE* and multiple alignments of each WD40 motif from various plant species were performed (see Figure 1). The first WD40 domain (WD40-1) of an *FVE* gene is found throughout the MSI gene family while the second WD40 domain (WD40-2) appears specifically in *FVE* (also reported as a MSI4) genes from the species analyzed to date. The remaining four WD40 domains are commonly found in a range of genes and as such require carful assessment for use as targets for specific suppression of *FVE* expression. Use of these motifs requires assessment as to the likelihood of non-target gene inhibition. For example, use of RNAi technology is highly specific and may be used to effect specific *FVE* inhibition using these motifs. Antisense and amiRNAi which has less specific inhibition effects necessitate special consideration as to the possibility of non-target effects.

Further sequences analysis shows two specific domains, S1 and S2. The S1 domain is conserved in all known *FVE* genes from all species, while the S2 domain appears to be a species specific, divergent domain. Each of the S1 and S2 domains and the WD40 domains is suitable for use in *FVE* inhibition, and useful in methods of producing a plant having increased biomass.

The S1 domain is found as the 36 amino acids preceding the WD40-1 repeat in At*FVE* and is identified as SEQ ID NO:41 encoded by SEQ ID NO:57 .Other S1-like regions of an *FVE* gene may be identified by those skilled in the art that have high homology specifically to an FVE polypeptide as described by SEQ ID NO:37-50, 243 and 244 encoded by the nucleotide sequences as described in SEQ ID NO:51-64, 245 and 246.

Additionally, one skilled in the art may identify additional S2-like regions in an FVE sequence from a species of interest that will be useful in the inhibition of that specific endogenous *FVE* gene expression and activity. Other S2-like regions of an *FVE* gene may be identified by those skilled in the art that have high homology specifically to an FVE polypeptide as described by SEQ ID NO:65-78, 223 and 247 encoded by the nucleotide sequences as described in SEQ ID NO:79-92, 233 and 248.

### FVE Recombinant Expression Vectors and Host Cells

Another aspect of the invention pertains to vectors, preferably expression vectors, containing a nucleic acid encoding an FVE protein, an *FVE* gene or genomic sequence or portions thereof and analogs or homologs thereof. As used herein the term expression vector includes vectors which are designed to provide transcription of the nucleic acid sequence. Transcribed sequences may be designed to inhibit the endogenous expression or activity of an endogenous gene activity correlating to the transcribed sequence. Optionally, the transcribed nucleic acid need not be translated but rather inhibits the endogenous gene expression as in antisense or hairpin down-regulation methodology. Alternatively, the transcribed nucleic acid may be translated into a polypeptide or protein product. The polypeptide may be a non-full length, mutant or modified variant of the endogenous protein. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (*e.g*., bacterial vectors having a bacterial origin of replication). Other vectors are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors or plant transformation vectors, binary or otherwise, which serve equivalent functions.

The recombinant expression vectors used in the invention comprise a nucleic acid in a form suitable for expression of the nucleic acid in a host cell, which means that the recombinant expression vectors include one or more regulatory sequences, selected on the basis of the host cells to be used for expression, that is operatively-linked to the nucleic acid sequence to be expressed. Within a recombinant expression vector, "operably-linked" is intended to mean that the nucleotide sequence of interest is linked to the regulatory sequence(s) in a manner that allows for expression of the nucleotide sequence (*e.g*., in an *in vitro* transcription/translation system or in a host cell when the vector is introduced into the host cell).

The term "regulatory sequence" is intended to include promoters, enhancers and other expression control elements (*e.g*., polyadenylation signals). Such regulatory sequences are described, for example, in Goeddel, GENE EXPRESSION TECHNOLOGY: METHODS IN ENZYMOLOGY 185, Academic Press, San Diego, Calif. (1990). Regulatory sequences include those that direct constitutive expression of a nucleotide sequence in many types of host cell and those that direct expression of the nucleotide sequence only in certain host cells (*e.g*., tissue-specific regulatory sequences) or inducible promoters (*e.g*., induced in response to abiotic factors such as environmental conditions, heat, drought, nutrient status or physiological status of the cell or biotic such as pathogen responsive). Examples of suitable promoters include for example constitutive promoters, ABA inducible promoters, tissue specific promoters and abiotic or biotic inducible promoters. It will be appreciated by those skilled in the art that the design of the expression vector can depend on such factors as the choice of the host cell to be transformed, the level of expression of protein desired as well as timing and location of expression, etc. The expression vectors can be introduced into host cells to thereby produce proteins or peptides, including fusion proteins or peptides, encoded by nucleic acids as described herein (*e.g*., FVE proteins, mutant forms of FVE proteins, fusion proteins, etc.).

The recombinant expression vectors can be designed for expression of *FVE* genes, FVE proteins, or portions thereof, in prokaryotic or eukaryotic cells. For example, *FVE* genes or FVE proteins can be expressed in bacterial cells such as *Escherichia coli*, insect cells (using baculovirus expression vectors), yeast cells, plant cells or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated *in vitro*, for example using T7 promoter regulatory sequences and T7 polymerase.

In one embodiment, a nucleic acid of the invention is expressed in plants cells using a plant expression vector, Examples of plant expression vectors systems include tumor inducing (Ti) plasmid or portion thereof found in *Agrobacterium*, cauliflower mosaic virus (CAMV) DNA and vectors such as pBI121.

For expression in plants, the recombinant expression cassette will contain in addition to the *FVE* nucleic acids, a promoter region that functions in a plant cell, a transcription initiation site (if the coding sequence to transcribed lacks one), and a transcription termination/polyadenylation sequence. The termination/polyadenylation region may be obtained from the same gene as the promoter sequence or may be obtained from different genes. Unique restriction enzyme sites at the 5' and 3' ends of the cassette are typically included to allow for easy insertion into a pre-existing vector.

Examples of suitable promoters include promoters from plant viruses such as the 35S promoter from cauliflower mosaic virus (CaMV) (Odell, et al., Nature, 313: 810-812 (1985)), promoters from genes such as rice actin (McElroy, et al., Plant Cell, 163-171 (1990)), ubiquitin (Christensen, et al., Plant Mol. Biol, 12: 619-632 (1992); and Christensen, et al., Plant Mol. Biol., 18: 675-689 (1992)), pEMU (Last, et al., Theor. Appl. Genet., 81: 581-588 (1991)), MAS (Velten, et al., EMBO J., 3: 2723-2730 (1984)), maize H3 histone (Lepetit, et al., Mol. Gen. Genet., 231: 276-285 (1992); and Atanassvoa, et al., Plant Journal, 2(3): 291-300 (1992)), the 5'- or 3'-promoter derived from T-DNA of *Agrobacterium tumefaciens*, the Smas promoter, the cinnamyl alcohol dehydrogenase promoter (U.S. Pat. No. 5,683,439), the Nos promoter, the rubisco promoter, the GRP1-8 promoter, ALS promoter, (WO 96/30530), a synthetic promoter, such as Rsyn7, SCP and UCP promoters, ribulose-1,3-diphosphate carboxylase, fruit-specific promoters, heat shock promoters, seed-specific promoters and other transcription initiation regions from various plant genes, for example, including the various opine initiation regions, such as for example, octopine, mannopine, and nopaline. Additional regulatory elements that may be connected to an FVE encoding nucleic acid sequence for expression in plant cells include terminators, polyadenylation sequences, and nucleic acid sequences encoding signal peptides that permit localization within a plant cell or secretion of the protein from the cell. Such regulatory elements and methods for adding or exchanging these elements with the regulatory elements of the *FVE* gene are known and include, but are not limited to, 3' termination and/or polyadenylation regions such as those of the *Agrobacterium tumefaciens* nopaline synthase (nos) gene (Bevan, et al., Nucl. Acids Res., 12: 369-385 (1983)); the potato proteinase inhibitor II (PINII) gene (Keil, et al., Nucl. Acids Res., 14: 5641-5650 (1986); and An, et al., Plant Cell, 1: 115-122 (1989)); and the CaMV 19S gene (Mogen, et al., Plant Cell, 2: 1261-1272 (1990)).

Plant signal sequences which are useful in the invention, include but are not limited to, signal-peptide encoding DNA/RNA sequences which target proteins to the extracellular matrix of the plant cell (Dratewka-Kos, et al., J. Biol. Chem., 264: 4896-4900 (1989)) and the *Nicotiana plumbaginifolia* extension gene (DeLoose, et al., Gene, 99: 95-100 (1991)), or signal peptides which target proteins to the vacuole like the sweet potato sporamin gene (Matsuka, et al., Proc. Nat'l Acad. Sci. (USA), 88: 834 (1991)) and the barley lectin gene (Wilkins, et al., Plant Cell, 2: 301-313 (1990)), or signals which cause proteins to be secreted such as that of PRIb (Lind, et al., Plant Mol. Biol., 18: 47-53 (1992)), or those which target proteins to the plastids such as that of rapeseed enoyl-ACP reductase (Verwaert, et al., Plant Mol. Biol., 26: 189-202 (1994)).

In another embodiment, the recombinant expression vector is capable of directing expression of the nucleic acid preferentially in a particular cell type (*e.g*., tissue-specific regulatory elements are used to express the nucleic acid). Tissue-specific regulatory elements are known in the art. Especially useful in connection with the nucleic acids of the present invention are expression systems which are operable in plants. These include systems which are under control of a tissue-specific promoter, as well as those which involve promoters that are operable in all plant tissues.

Organ-specific promoters are also well known. For example, the chalcone synthase-A gene (van der Meer et al., Plant Molecular Biology 15(1):95-109 (1990)) or the dihydroflavonol-4-reductase (dfr) promoter (Elomaa et al., The Plant Journal, 16(1) 93-99) direct expression in specific floral tissues. Also available are the patatin class I promoter which is transcriptionally activated only in the potato tuber and can be used to target gene expression in the tuber (Bevan, M., 1986, Nuc. Acids Res. 14:4625-4636). Another potato-specific promoter is the granule-bound starch synthase (GBSS) promoter (Visser, R.G.R, et al., Plant Mol. Bio. 17:691-699 (1991)).

Other organ-specific promoters appropriate for a desired target organ can be isolated using known procedures. These control sequences are generally associated with genes uniquely expressed in the desired organ. In a typical higher plant, each organ has thousands of mRNAs that are absent from other organ systems (reviewed in Goldberg, P., Trans. R. Soc. London B314:343 (1986)).

The resulting expression system or cassette is ligated into or otherwise constructed to be included in a recombinant vector which is appropriate for plant transformation. The vector may also contain a selectable marker gene by which transformed plant cells can be identified in culture. The marker gene may encode antibiotic resistance. These markers include resistance to G418, hygromycin, bleomycin, kanamycin, and gentamicin. Alternatively the marker gene may encode an herbicide tolerance gene that provides tolerance to glufosinate or glyphosate type herbicides. After transforming the plant cells, those cells having the vector will be identified by their ability to grow on a medium containing the particular antibiotic or herbicide. Replication sequences, of bacterial or viral origin, are generally also included to allow the vector to be cloned in a bacterial or phage host, preferably a broad host range prokaryotic origin of replication is included. A selectable marker for bacteria should also be included to allow selection of bacterial cells bearing the desired construct. Suitable prokaryotic selectable markers also include resistance to antibiotics such as kanamycin or tetracycline.

Other DNA sequences encoding additional functions may also be present in the vector, as is known in the art. For instance, in the case of *Agrobacterium* transformations, T-DNA sequences will also be included for subsequent transfer to plant chromosomes.

The method of the invention involves host cells into which a recombinant expression vector has been introduced. The terms "host cell" and "recombinant host cell" are used interchangeably herein. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (*e.g*., DNA) into a host cell.

A host cell, such as a prokaryotic or eukaryotic host cell in culture, can be used to produce (*i.e*., express) a polypeptide encoded in an open reading frame of a polynucleotide. Accordingly, we disclose methods for producing a polypeptide using the host cells of the invention. Optionally, the method comprises culturing the host cell of invention (into which a recombinant expression vector encoding a polypeptide of the invention has been introduced) in a suitable medium such that the polypeptide is produced. Optionally, the method further comprises isolating the polypeptide from the medium or the host cell.

A number of cell types may act as suitable host cell for expression of a polypeptide encoded by an open reading frame in a polynucleotide. Plant host cells include, for example, plant cells that could function as suitable hosts for the expression of a polynucleotide of the invention include epidermal cells, mesophyll and other ground tissues, and vascular tissues in leaves, stems, floral organs, and roots from a variety of plant species, such as *Arabidopsis thaliana, Nicotiana tabacum, Brassica napus*, *Zea mays*, *Oryza sativa*, *Gossypium hirsutum* and *Glycine max*.

Expression of an *FVE* nucleic acid encoding an altered or mutant FVE protein that is not fully functional can be useful in a dominant-negative inhibition method. An FVE variant polypeptide, or portion thereof, is expressed in a plant such that it has partial functionality. The variant polypeptide may for example have the ability to bind other molecules but does not permit proper activity of the complex, resulting in overall inhibition of FVE activity.

### Transformed Plants Cells and Transgenic Plants

The invention includes a protoplast, plants cell, plant tissue and plant (*e.g*., monocot or dicot) transformed with an *FVE* nucleic acid, a vector containing an *FVE* nucleic acid or an expression vector containing an *FVE* nucleic acid. As used herein, "plant" is meant to include not only a whole plant but also a portion thereof (*i.e*., cells, and tissues, including for example, leaves, stems, shoots, roots, flowers, fruits and seeds).

The plant can be any plant type including, but not limited to, for example, species from the genera *Arabidopsis, Brassica, Oryza Zea, Sorghum, Gossypium, Triticum, Glycine, Pisum, Phaseolus, Lycopersicon, Trifolium, Cannabis, Cucurbita, Rosa, Vitis, Juglans, Fragaria, Lotus, Medicago, Onobrychis, Trigonella, Vigna, Citrus, Linum, Geranium, Manihot, Daucus, Raphanus, Sinapis, Atropa, Capsicum, Datura, Hyoscyamus, Nicotiana, Solanum, Petunia, Digitalis, Majorana, Ciahorium, Helianthus, Lactuca, Bromus, Asparagus, Antirrhinum, Heterocallis, Nemesis, Pelargonium, Panieum, Pennisetum, Ranunculus, Senecio, Salpiglossis, Cucumis, Browaalia, Lolium, Avena, Hordeum, Secale, Picea, Caco, Solanaceae, Panicum, Brachypodium, Silene, Physcomitrella, Miscanthus, Jatropha, Salix, Andropogon, Saccharum, Camelina, Eucalyptus, Arundo* and *Populus.*

The invention also includes cells, tissues, including for example, leaves, stems, shoots, roots, flowers, fruits and seeds and the progeny derived from the transformed plant.

Numerous methods for introducing foreign genes into plants are known and can be used to insert a gene into a plant host, including biological and physical plant transformation protocols (See, for example, Miki et al., "Procedure for Introducing Foreign DNA into Plants", In: Methods in Plant Molecular Biology and Biotechnology, Glick and Thompson, eds., CRC Press, Inc., Boca Raton, pages 67-88 (1993); and Andrew Bent in, Clough SJ and Bent AF, "Floral dipping: a simplified method for Agrobacterium-mediated transformation of Arabidopsis thaliana " (1998)). The methods chosen vary with the host plant, and include chemical transfection methods such as calcium phosphate, polyethylene glycol (PEG) transformation, microorganism-mediated gene transfer such as *Agrobacterium* (Horsch, et al., Science, 227: 1229-31 (1985)), electroporation, protoplast transformation, micro-injection, flower dipping and biolistic bombardment.

### Agrobacterium-Mediated Transformation

The most widely utilized method for introducing an expression vector into plants is based on the natural transformation system of *Agrobacterium tumefaciens* and *A. rhizogenes* which are plant pathogenic bacteria which genetically transform plant cells. The Ti and Ri plasmids of *A. tumefaciens* and *A. rhizogenes*, respectfully, carry genes responsible for genetic transformation of plants (See, for example, Kado, Crit. Rev. Plant Sci., 10: 1-32 (1991)). Descriptions of the *Agrobacterium* vector systems and methods for *Agrobacterium*-mediated gene transfer are provided in Gruber *et al*., supra; and Moloney, et al, Plant Cell Reports, 8: 238-242 (1989).

Transgenic *Arabidopsis* plants can be produced easily by the method of dipping flowering plants into an *Agrobacterium* culture, based on the method of Andrew Bent in, Clough SJ and Bent AF, "Floral dipping: a simplified method for Agrobacterium-mediated transformation of Arabidopsis thaliana" (1998). Wild-type plants are grown until the plant has both developing flowers and open flowers. The plants are inverted for 1 minute into a solution of *Agrobacterium* culture carrying the appropriate gene construct. Plants are then left horizontal in a tray and kept covered for two days to maintain humidity and then righted and bagged to continue growth and seed development. Mature seed is bulk harvested.

### Direct Gene Transfer

A generally applicable method of plant transformation is microprojectile-mediated transformation, where DNA is carried on the surface of microprojectiles measuring about 1 to 4 µm. The expression vector is introduced into plant tissues with a biolistic device that accelerates the microprojectiles to speeds of 300 to 600 m/s which is sufficient to penetrate the plant cell walls and membranes. (Sanford, et al., Part. Sci. Technol., 5: 27-37 (1987); Sanford, Trends Biotech, 6: 299-302 (1988); Sanford, Physiol. Plant, 79: 206-209 (1990); Klein, et al., Biotechnology, 10: 286-291 (1992)).

Plant transformation can also be achieved by the Aerosol Beam Injector (ABI) method as described in U.S. Pat. 5,240,842, and U.S. Pat. 6,809,232. Aerosol beam technology is used to accelerate wet or dry particles to speeds enabling the particles to penetrate living cells. Aerosol beam technology employs the jet expansion of an inert gas as it passes from a region of higher gas pressure to a region of lower gas pressure through a small orifice. The expanding gas accelerates aerosol droplets, containing nucleic acid molecules to be introduced into a cell or tissue. The accelerated particles are positioned to impact a preferred target, for example a plant cell. The particles are constructed as droplets of a sufficiently small size so that the cell survives the penetration. The transformed cell or tissue is grown to produce a plant by standard techniques known to those in the applicable art.

### Regeneration of Transformants

The development or regeneration of plants from either single plant protoplasts or various explants is well known in the art (Weissbach and Weissbach, 1988). This regeneration and growth process typically includes the steps of selection of transformed cells, culturing those individualized cells through the usual stages of embryonic development through the rooted plantlet stage. Transgenic embryos and seeds are similarly regenerated. The resulting transgenic rooted shoots are thereafter planted in an appropriate plant growth medium such as soil.

The development or regeneration of plants containing the foreign, exogenous gene that encodes a polypeptide of interest introduced by *Agrobacterium* from leaf explants can be achieved by methods well known in the art such as described in Horsch *et al*., (1985). In this procedure, transformants are cultured in the presence of a selection agent and in a medium that induces the regeneration of shoots in the plant strain being transformed as described in Fraley *et al.* (1983). In particular, U.S. Pat. No. 5,349,124 details the creation of genetically transformed lettuce cells and plants resulting therefrom which express hybrid crystal proteins conferring insecticidal activity against Lepidopteran larvae to such plants.

This procedure typically produces shoots within two to four months and those shoots are then transferred to an appropriate root-inducing medium containing the selective agent and an antibiotic to prevent bacterial growth. Shoots that rooted in the presence of the selective agent to form plantlets are then transplanted to soil or other media to allow the production of roots. These procedures vary depending upon the particular plant strain employed, such variations being well known in the art.

Preferably, the regenerated plants are self-pollinated to provide homozygous transgenic plants, or pollen obtained from the regenerated plants is crossed to seed-grown plants of agronomically important, preferably inbred lines. Conversely, pollen from plants of those important lines is used to pollinate regenerated plants. A transgenic plant of the present invention containing a desired polypeptide is cultivated using methods well known to one skilled in the art.

A preferred transgenic plant is an independent segregate and can transmit the *FVE* gene construct to its progeny. A more preferred transgenic plant is homozygous for the gene construct, and transmits that gene construct to all offspring on sexual mating. Seed from a transgenic plant may be grown in the field or greenhouse, and resulting sexually mature transgenic plants are self-pollinated to generate true breeding plants. The progeny from these plants become true breeding lines that are evaluated for decreased expression of the *FVE* gene.

### Method of Producing Transgenic Plants

The invention provides methods of increasing biomass accumulation in a plant relative to a wild-type plant. The method includes introducing into one or more plant cells a compound that inhibits *FVE* expression or activity in the plant to generate a transgenic plant cell and regenerating a transgenic plant from the transgenic cell. The compound comprises a nucleic acid construct operably linked to a promoter, wherein said nucleic acid construct comprises an antisense nucleotide of *FVE,* an siRNA of *FVE*, an shRNA of *FVE*, an miRNA of FVE, an artificial miRNA of *FVE*, or a dominant negative mutant of *FVE*. A nucleic acid that decreases expression of an *FVE* nucleic acid may include promoters or enhancer elements. The *FVE* nucleic acid can be either endogenous or exogenous, for example an *Arabidopsis FVE* nucleic acid may be introduced into a *Brassica* or corn species. For example the compound comprises the nucleic acid sequence of SEQ ID NO: 1, 3, 5, 6, 7, 9, 11,13, 15, 17, 19, 21, 23, 25, 27, 29, 31, 33, 35, 206, 216, 220, 239, and 241 or a portion thereof, such as SEQ ID NO:51-64, 245, 246, 79-92, 233, 248, 107-120, 234, 252, 135-148, 235, 250, 163-176, 256, 254, 273-288, 305-320 and 337-352 or a expression cassette such as SEQ ID NO:372-379. Preferably, the compound is an *FVE* nucleic acid sequence endogenous to the species being transformed. Alternatively, the compound is an *FVE* nucleic acid sequence exogenous to the species being transformed and having at least 70% homology to the endogenous target sequence.

In various aspects the transgenic plant has an altered phenotype as compared to a wild-type plant (*i.e.,* untransformed). By altered phenotype is meant that the plant has a one or more characteristic that is different from the wild-type plant. W hen the transgenic plant has been contacted with a compound that decreases the expression or activity of an *FVE* nucleic acid, the plant has increased biomass as compared to a wild-type plant.

The plant can be any plant type including, but not limited to, for example, species from the genera *Arabidopsis*, *Brassica*, *Oryza*, *Zea*, *Sorghum, Gossypium, Triticum, Glycine, Pisum*, *Phaseolus, Lycopersicon*, *Trifolium, Cannabis, Cucurbita, Rosa, Vitis, Juglans, Fragaria, Lotus, Medicago, Onobrychis, Trigonella, Vigna, Citrus, Linum, Geranium, Manihot, Daucus, Raphanus, Sinapis, Atropa, Capsicum, Datura, Hyoscyamus, Nicotiana, Solanum, Petunia, Digitalis, Majorana, Ciahorium, Helianthus, Lactuca, Bromus, Asparagus, Antirrhinum, Heterocallis, Nemesis, Pelargonium, Panieum, Pennisetum, Ranunculus, Senecio, Salpiglossis, Cucumis, Browaalia, Lolium, Avena, Hordeum, Secale, Picea, Caco, Solanaceae, Panicum Brachypodium, Silene, Physcomitrella, Miscanthus, Jatropha, Salix, Andropogon, Saccharum, Camelina, Eucalyptus, Arundo* and *Populus.*

The invention will be further illustrated in the following non-limiting examples.

### EXAMPLES

### Example 1: Identification of a plant having increased biomass

A mutagenized population of *Arabidopsis thaliana* var. Columbia (Col-0) was screened and the *Arabidopsis fve-b10* mutant was identified as having beneficial phenotypes including increased biomass relative to a wild-type control, and a delay in the time to onset of flowering.

The *fve-b10* mutant was used to cross with another *Arabidopsis* wild-type accession Landsberg erecta (L*er*). The true Fls of L*er* x *fve-b10* were identified with simple-sequence length polymorphisms (SSLP) markers. An F2 population of 594 individuals were prepared for gene mapping. Rough mapping results based on Bulked Segregants Analysis (BSA) using 33 markers from Chromosome I, II, III, IV, and V, showed that the biomass enhancement trait (BET) was located between molecular markers of RGA and PLS3 on Chromosome II. The interval between RGA and PLS3 is about 8.75 mega base pairs (Mb).

Fine mapping was conducted using a total of 594 F2 individuals. Based on literature, a total of 61 genes involved in different pathways of flowering time were investigated for their phenotypes and genetic location. These genes are involved in flowering time of the long day pathway, autonomous pathway, vernalization pathway and gibberellin pathway, as well as floral pathway integrators and floral meristem identity.

Among the 61 flowering genes investigated, only two of them were located within the 8.75 Mb interval between RAG and PLS3. The At2g19520 (*FVE*) mutant displayed a delayed flowering phenotype, therefore At2g19520 (*FVE*) was selected as the gene candidate responsible for the *fve-b10* gene. Genomic fragments of *FVE* from both Col-0 and *fve-b10* were amplified and purified for sequencing.

A total of 3,763 bp of genomic DNA of the *FVE* locus from *fve-b10* was successfully sequenced, including 346bp upstream of the start codon of *FVE,* 3217bp between the start codon and stop codon, and 200bp downstream of the stop codon. The sequencing results revealed that compared with wild-type *FVE* in Col-0, *fve-b10* has a 13bp deletion at the junction point of the 5^{th} intron and 6^{th} exon, which changes the open reading frame (ORF) of the *FVE* gene in *fve-b10.* Further studies revealed that a longer FVE ORF *-fve-b10* (1581bp) encoding 526 amino acids was produced in *fve-b10*, while the wild-type FVE ORF has 1524bp encoding 507 amino acids. Protein tertiary structure analysis showed that *fve-b10* has two extra β-sheets as compared to Wt-FVE.

These results, combined with complementation test results therefore confirm that *FVE* is the gene responsive for the biomass enhancement trait (BET).

### Example 2: Physiological characterization of the fve-b10 mutant

### i) fve-b10 mutant has greatly enhanced biomass, chlorophyll content and seed yield

*Arabidopsis fve-b10* mutant plants and wild-type (WT) controls were grown in 3" pots (1 plant/pot) in soil-less mix (Sunshine) in a growth chamber under optimal conditions (22°C, 70%RH, 18hr light) either at an optimal light of 200 uE or low light of 80 uE. Plants were harvested at developmental stages including vegetative, bolting and mid-flowering stage, and total shoot biomass, as well as fresh and dry weights of stems and leaves, were determined. The enhanced shoot biomass of *fve-b10* mutant was the most pronounced during the mid- flowering stage. For *fve-b10* plants grown at optimal light (200uE), biomass enhancement over the WT plants was 2 fold for stem dry weight (DW), 6 fold for leaf DW (where twice as many leaves and larger leaves were seen), and stems were twice as thick in *fve-b10* as compared to WT. Although the leaf size was larger than wild-type the cell size was unchanged relative to WT. The flowering time of the *fve*-*b10* mutant was delayed by 8 to 13 days.

In plants grown at low light (80uE) the biomass enhancement of the *fve-b10* mutant was more apparent. Stem DW was 3 fold higher (more stems and thicker stems) and leaf DW was 26 fold higher (5 times more leaves and larger leaves) than in WT. A summary of the biomass enhancement for *fve-b10* mutant plants as compared to WT plants grown under optimal light and low light conditions is shown in Table 1 below.

Analysis of stem cross-sections was performed using a dissection microscope (Leica) to determine stem thickness measurements and structural observations of cell organization. Stem cross-sections were obtained from the main stem taken at 3 to 4 cm above the soil when plants were 3 days post-first flower.

Stems of *fve-b10* mutant plants were twice as thick as WT plants (2.4 mm vs 1.2 mm diameter) and had twice as many vascular bundles per cross section (14 vs 6) as WT. In addition, the inter-vascular layer of fiber was 2 fold thicker in *fve-b10* stems. The chlorophyll content of the leaves from plants grown both under optimal and low light was 40% higher in *fve-b10* mutant than WT plants, but there was no difference in chlorophyll a to b ratio between the mutant and the WT.

**Table 1. Biomass enhancement of fve-b10 mutant over WT at low or optimal light.**

| | **Low light (80uE)** | | **Optimal light (200 uE)** | |
|---|---|---|---|---|
| **entry** | **Leaf DW (mg)** | **Stem DW (mg)** | **Leaf DW (mg)** | **Stem DW (mg)** |
| *fve-b10* | 340±28 | 102±10 | 630±35 | 430±20 |
| WT | 13±2 | 33±3 | 95±6 | 210±18 |

Plants grown under optimal light and in 4 inch and 6 inch pots were grown to maturity for final biomass and seed yield harvests (n=8 to 14). The shoot biomass of plants grown to maturity in 6" pots under optimal conditions was 81% greater in the *fve-b10* mutant and the final seed yield was 15% greater than that of WT. When plants were grown in smaller pots (4") under optimal conditions the seed yield of the *fve-b10* mutant ranged from 20% to 46% greater than WT. The harvest index (ratio of seed yield to the total shoot biomass, including seeds) was lower in *fve-b10* than WT. Without intending to be bound by any theory, these results are indicative of more of the plant resources going towards shoot biomass (mostly stems) than seed production. A summary of the shoot biomass and seed yield for *fve-b10* mutant plants grown to maturity in 6" pots as compared to WT plants is shown in Table 2 below.

**Table 2. Total shoot biomass and seed yield of mature plants grown in 6"pots.**

| **entry** | **Total shoot DW (g)** | **Seed yield/plant (g)** | **Harvest index** |
|---|---|---|---|
| *fve-b10* | 15.8±0.3 | 5.2±0.2 | 0.33 |
| WT | 10.2±0.5 | 4.4±0.2 | 0.43 |

The *fve-b10* mutant and WT *Arabidopsis* plants were grown in magenta boxes containing 100 ml of solid agar media enriched with 0.5 MS nutrients for optimal growth. Plants were grown in a growth chamber at 22°C, 17hr light (200 uE) until four days into flowering. Plants were harvested at that time for shoot and root dry biomass determinations. The results showed that *fve-b10* mutant plants had both, shoot and root dry biomass enhanced as compared to WT controls. These were 2.1 and 2.4 fold greater in the *fve-b10* mutant than in WT, respectively. This data indicates that the biomass enhancement in the *fve-b10* mutant is present in the whole plant.

### ii) fve-b10 mutant has more efficient photosynthesis at lower lights and greater water use efficiency under optimal and water limited conditions.

Plants were grown one per 4" pot under optimal conditions in a growth chamber as described above. Gas exchange measurements were done on the two youngest fully developed leaves per plant 7 days after first open flower. Fourteen replicate plants per entry were measured. Gas exchange measurements were done in the growth chamber using the Li-6400 (LiCor) and a built in red light source set at 200 uE (light levels of the growth conditions). Ambient CO₂ concentration at the leaves was 400 ppm as set by Li-6400.

Specific average rate of photosynthesis under optimal conditions in µmol CO₂ per m² of a leaf per min was 15% lower in *fve-b10* than in WT (8.0±0.2 and 9.6±0.2, respectively). The rate of transpiration in mmol water per m² per second was 48% lower in *fve-b10* as compared to that of control WT (2.2±0.1 vs. 4.2±0.1, respectively). Water use efficiency (WUE) was calculated as the ratio of photosynthesis to transpiration and was significantly greater (63%) in the *fve-b10* mutant as compared to the control plants under optimal conditions.

The *fve-b10* mutant and WT control were used for measurements of maximum rates of photosynthesis at saturating light levels. From measurements of CO₂ exchange rates at very low light levels the light compensation point was determined (light level where the net CO₂ exchange rate is zero, or where rate of respiration equals rate of photosynthesis). These were done on youngest fully developed leaves using the Li-6400. A lower light compensation point is indicative of a plant having greater ability to utilize low light levels. The results of these measurements showed that the maximum rates of photosynthesis at saturating light were not significantly different between *fve-b10* and WT (12±1.1 and 14±0.2 umol CO₂/m²/s, respectively). The saturating light levels were also not different between *fve-b10* and WT (around 900 uE). However, the light compensation points was significantly lower (8.3±0.6 uE) in *fve-b10* than in WT (12.7±1.5 uE), indicating that *fve-b10* is able to utilize lower light intensities for photosynthesis better than the control (WT) but still have functional photosynthetic apparatus at the same high levels as controls. This data is in accordance with the results of biomass accumulation under low light (80 uE) growth conditions, where *fve-b10* mutant had greater enhancement of biomass over WT than at optimal light levels (around 200 uE).

Water use efficiency (WUE) was also measured gravimetrically on plants grown in 3" pots at a density of 5 plants per pot, under optimal conditions in a growth chamber as previously described. Plants were watered daily with a finite amount of water and the daily water loss from the pots was measured by weighing the pots. The shoot biomass was harvested on days 0, 2 and 4 of treatment, with day 0 being the first day of flower opening. A second group of plants were grown under drought stress conditions by watering with a finite amount of water on day 0 and cessation of subsequent watering. Drought stress conditions were achieved within 2 to 3 days. Pots were weighed daily to determine the daily water loss. These plants were harvested on day 4 of treatment. WUE was calculated from the ratio of biomass accumulated and the amount of water lost between days 0 and 4 for both optimal and drought treatments. The mutant *fve-b10* had 82% greater WUE than control under optimal conditions (2.49 vs 1.37) and 51% greater WUE under drought conditions (2.37 vs 1.57) than the control WT. This data supports the gas exchange data described above that the mutant *fve-b10* plants use water more efficiently to accumulate shoot biomass.

The growth rates in grams of shoot dry weight per day were calculated in plants maintained under water limited conditions for 7 days and the growth rate of *fve-b10* mutant plants was 6 fold higher (0.101g/day) than that of the control WT (0.018 g/day).

### iii) Upon vernalization of the seeds, biomass enhancement and water use efficiency is not dependent upon delay of flowering

Seeds of *fve-b10* mutant and WT were placed on agar plates containing ½ MS media at 4°C for 7 weeks to vernalize (cold exposure). The first two weeks were in darkness and once seeds germinated the young seedlings were placed in a chamber at 4°C at low light intensity (150 uE) with an 18 hr photoperiod for the remaining 5 weeks. After 7 weeks of vernalization plates were transferred to 22°C and allowed to grow for 5 days, after which young seedlings were transplanted into 3" pots containing soilless mix (sunshine mix #1). Five plants per pot were grown under optimal conditions in a growth chamber until first day of flowering. Optimal and drought treatments were started (day 0) by watering up all the plants with the same amount of water. In the optimal group of plants, water was replenished daily whereas the drought group received no further water. Daily water loss was measured from all plants and plants were harvested on days 0, 2 and 4 of treatment.

The results of this experiment showed that *fve-b10* plants following vernalization flowered at the same time as the WT plant (17 days after the end of vernalization). These mutant plants still showed enhanced leaf biomass as compared to WT especially under optimal conditions (25% to 51% greater). The water loss in the first two days of the treatment under optimal conditions was 14% lower in the *fve-b10* mutant and the water use efficiency in those two days of the treatment was 58% and 22% greater in the *fve-b10* mutant as compared to WT at optimal and drought conditions, respectively. The difference in WUE was associated with greater biomass accumulation in the *fve-b10* mutant.

### iv) fve-b10 plants are more resistant to oxidative stress, heat stress and show reduced nitrogen (N.) requirements

Plants were grown, one per 3" pot, under optimal conditions in a growth chamber. Three leaf disks were removed from the three youngest fully developed leaves of 17 day old plants. Leaf disks were placed on filter paper wet with 5 µM paraquat (PQ) (methylviologen) under a light intensity of 100 uE for 32 hours. Treatment resulted in oxidative damage to chlorophyll and bleaching of the leaves. After 32 hours the leaf disks were frozen in liquid nitrogen and extracted in methanol for assays of total chlorophyll. The extent of chlorophyll oxidation is associated with susceptibility to oxidative stress. The results shown in Table 3 indicate that chlorophyll content of *fve-b10* leaves under optimal conditions was 30% greater than that of WT. However, after paraquat treatment, which reduced the total chlorophyll by up to 80%, *fve-b10* leaf disks had 92% greater chlorophyll content than WT, indicating greater resistance to oxidative stress in *fve-b10* mutant.

**Table 3. Chlorophyll content per leaf area following oxidative stress**

| **entry** | **Optimal: Total chlorophyll (mg/m²)** | **PQ: Total chlorophyll (mg/m²)** | **PQ: Total chlorophyll (% optimal)** |
|---|---|---|---|
| *fve-b10* | 389 ± 11 | 121 ± 12 | 31% |
| WT | 299 ± 7 | 63 ± 11 | 21% |

Growth under heat stress conditions was evaluated as follows, *fve-b10* and WT plants were grown three per 3" pot for ten days under optimal conditions in short day light conditions of 10 hr to promote vegetative growth. Half of the plants were then exposed to a heat treatment of 4 hr per day at 40°C for 3 days and on day four plants were heated to 43°C for 4 hr. Three days following heat treatment the heated and optimal groups of plants were harvested for shoot biomass determinations (n=8 per entry and per treatment). The results (Table 4) showed that *fve-b10* mutant plants were 9% less inhibited in their growth as a result of the heat treatments, indicating resistance to heat stress at early vegetative stage of growth.

**Table 4. Shoot dry weight following 4 days of heat stress at vegetative growth stage.**

| **Entry** | **Optimal** | **Heat** | **Heat** |
|---|---|---|---|
| | **shoot DW (mg)** | **Shoot DW (mg)** | **shoot DW (% opt)** |
| *fve-b10* | 61 ± 1 | 43 ± 1 | 70% |
| WT | 58 ± 2 | 35 ± 1 | 61% |

Growth under low nitrogen conditions was evaluated as follows. Seedlings of *fve-b10* and WT were grown on agar plates (10 seedlings per plate) containing optimal growth media (1/2 MS with 20 mM N.) or media deficient in N. (0.5 mM N) for 14 days. These plates were kept in a vertical position under optimal conditions in a growth chamber (22°C, 18 hr light of 200 uE). After 14 days of growth, 8 seedlings were harvested for shoot biomass determinations and 2 seedlings were harvested for shoot chlorophyll content determinations. The results showed that growth on low N. resulted in 45% lower seedling shoot dry weight in the *fve-b10* mutant, and 48% lower seedling shoot dry weight in WT, as compared to plants grown under optimal conditions. This result suggests that the *fve-b10* mutant plants at early seedling growth do not require more N. for their growth than WT plants and in fact may be more efficient at nitrogen usage.

### Example 3: Identification of the fve-b10 and wild-type FVE genes

Map-based cloning was utilized to localize the mutation and identify the gene. Total RNA was isolated from *fve-b10* and Col-0 and used as template for RT-PCR using the primer pair SEQ ID NO:197 and 198 to produce full length cDNAs of *Atfve-b10* (SEQ ID NO:1) and *AtFVE* (SEQ ID NO:3). PCR products were isolated, cloned and sequenced.

Sequencing results show that the isolated full coding region of the wild-type Col-0 *AtFVE* is identical to the coding region of At2g19520 in the TAIR database. The genomic sequence of the mutant *Atfve-b10* has a 13 nucleotide deletion at the junction point of the 5^{th} intron and 6^{th} exon as compared with wild-type *AtFVE.* The altered open reading frame of the *Atfve-b10* results in an altered coding sequence having an additional 57 nucleotides encoding an additional 19 amino acids.

### Genomic Sequence Alignment: Intron sequence is underlined.

### cDNA sequence alignment

### Polypeptide sequence alignment

### Confirmation of FVE gene by complementation

Expression of a wild-type *FVE* gene in the mutant background should restore the wild-type growth phenotype and verify that the *FVE* gene is the desired target gene. A transgenic complementation test was done using a constitutive promoter to drive ectopic expression of At2g19520 (*FVE*) from *Arabidopsis* ecotype Columbia (Col-0) in the *Atfve*-*b10* background containing the mutant allele.

A full length coding sequence of wild-type (Col-0) *FVE* was PCR amplified using the primer pair according to SEQ ID NOS:197 and 198, and cloned into a binary vector pBI121ΔGUS-Basta using the restriction sites of Xba I and BamH I. The construct (*35S-AtFVE*) was transformed into the *Arabidopsis fve-b10* mutant by *Agrobacterium*-mediated transformation. The presence of the transgene was verified in each of the T1, T2 and T3 generations, each of which had a growth phenotype like a wild-type plant.

Western blotting was conducted to determine the association between expression levels of *FVE* in *fve-b10* and complementation grades of biomass enhancement. Inhibited expression of *FVE* is correlated with phenotypes such as increased biomass and confirms the role of *FVE.*

### Over-expression of the AtFVE gene in the Arabidopsis fve-b10 mutant background

Eleven transgenic lines and two nulls of 35S-*AtFVE* transformed into the *fve-b10* mutant plant background plus the control lines of the *fve-b10* mutant and a WT *Arabidopsis* (Col) were grown in a growth study. The results of this study showed that over-expression of a wild-type *FVE* gene in the *fve-b10* mutant background resulted in a gene rescue and reversion of the *fve-b10* mutant back towards wild-type-like phenotypes. The rescue of the mutant was not complete as indicated by some biomass accumulation and delay of flowering relative to the WT controls however the spectrum was reverted significantly from the *fve-b10* phenotype. These results are shown in Table 5 below.

**Table 5. Reversion of fve-b10 towards Wild-type by 35S-AtFVE construct**

| **entry** | **Days to flower** | **Stem DW (mg)** | **Rosette leaf #** | **Leaf DW (mg)** | **Shoot DW (mg)** | **Shoot DW (% of WT)** |
|---|---|---|---|---|---|---|
| *fve-b10* | 34 | 298±30 | 98 | 983±26 | 1280± | 377% |
| 60-1 | 31 | 310±21 | 38 | 465±19 | 775± | 228% |
| 65-4 | 27 | 279±19 | 46 | 423±25 | 702± | 207% |
| 51-3 | 25 | 291±15 | 43 | 335±21 | 626± | 184% |
| 37-4 | 26 | 281±15 | 43 | 333±30 | 614± | 181% |
| 76-11 | 25 | 295±23 | 41 | 295±18 | 590± | 174% |
| 95-1 | 25 | 242±16 | 47 | 332±15 | 574± | 169% |
| 75-2 | 24 | 278±20 | 39 | 260±15 | 538± | 158% |
| 103-5 | 25 | 266±16 | 37 | 270±9 | 536± | 158% |
| 99-6 | 25 | 234±13 | 39 | 300±27 | 534± | 157% |
| 105-7 | 25 | 258±16 | 37 | 265±14 | 523± | 154% |
| 100-9 | 24 | 216±26 | 34 | 204±12 | 420± | 124% |
| 105-5 (null) | 32 | 301±30 | 82 | 958±66 | 1259± | 370% |
| 37-2 (null) | 34 | 268±19 | 83 | 1000±73 | 1268± | 373% |
| WT | 22 | 155±15 | 33 | 185±13 | 340± | 100% |

### Example 4: Identification of FVE from plants

An *FVE* gene can be identified by selecting a gene that has high homology to an FVE sequence, for example SEQ ID NOs:1, 3, 5, 6, 7, 9, 11,13, 15, 17, 19, 21, 23, 25, 27, 29, 31, 33, 35, 206, 216, 220, 239, and 241. By high homology it is meant that a putative *FVE* nucleic acid sequence exhibits a degree of identity to a known *FVE* sequence of preferably at least 70%, 75%, 80%, 85%, 90%, 95%, 98%, or 99% identity. Further, an *FVE* gene is identified through analysis of candidate genes that share conformational protein structure. Such structural motifs, such as WD40, S1-like or S2-like motifs, may assist in identification of related proteins and their structure.

### Functional analysis of FVE homologs from major crops

### i) Brassica napus

A *Brassica napus, BnFVE,* sequence was isolated and ectopically expressed in the *Arabidopsis fve-b10* background thereby demonstrating the functional identity of the *BnFVE* sequence as being functionally equivalent to *AtFVE.* The 5'-end and the 3'-end of the cDNA were obtained using the primer pair SEQ ID NOs:201 and 202 for 5' RACE and by using the primer SEQ ID NOs:201 and 203 in 3'-RACE. The resulting 5'-RACE product and 3'-RACE product of *BnFVE* was purified and sequenced. Based on sequencing results, the primer pair SEQ ID NOs:204 and 205 were designed and synthesized to amplify a full length coding sequence for *FVE* from *Brassica* by RT-PCR. The resulting sequence is disclosed as SEQ ID NO:206. The *BnFVE* open reading frame is 1524 nucleotides in length and encodes for 507 amino acids.

The *BnFVE* gene was cloned into a binary vector such as pBI121-Basta using restriction sites of Xba I and Sac I. The resulting construct of *35S-BnFVE* was transformed into *fve-b10* mutant plant by *Agrobacterium*-mediated transformation.

The presence of the transgene was confirmed by PCR analysis and selection by Basta resistance. Transgenic T1 plants of *fve-b10*::35S*-BnFVE* display a wild-type phenotype thereby demonstrating that the *BnFVE* gene is functionally equivalent to the *AtFVE* gene.

### ii) Zea mays

Two *Zea mays, ZmFVE*, sequences were isolated and ectopically expressed in the *Ai°abidopsis fve-b10* background thereby demonstrating the functional identity of the *ZmFVE* sequences as being functionally equivalent to *AtFVE.* The *ZmFVE* genes were RT-PCR amplified using the primer pairs SEQ ID NOs:208 and 209 or SEQ ID NOs:210 and 211, then subsequently cloned and sequenced. Full length cDNAs were obtained and referred to as *ZmFVE*1 (SEQ ID NO: 220) and *ZmFVE*2 (SEQ ID NO:23). Each have an open reading frame of 1362 nucleotides, encode a 453 amino acid polypeptide, and are 98% identical. Compared to the *Arabidopsis* sequences the maize nucleotide sequences are 74% identical and the amino acid sequences are 77% identical.

The *ZmFVE* genes were cloned into a binary vector such as the pBI121-Basta using restriction sites of Xba I and Sac I. The resulting constructs of 35S-*ZmFVE and* 35S*-ZmFVE* were transformed into *fve-b10* by *Agrobacterium*-mediated transformation.

The presence of the transgene was confirmed by PCR analysis and selection by Basta resistance. Transgenic plants of *fve-b10*::35S*-ZmFVE1* and *fve-b10*::35S-*ZmFVE2* display a wild-type phenotype thereby demonstrating that the *ZmFVE1 and ZmFVE2* genes are functionally equivalent to the *AtFVE* gene.

### iii) Panicum virgatum (switchgrass)

A *Panicum virgatum* (switchgrass), *PvFVE,* sequence was isolated and ectopically expressed in the *Arabidopsis fve-b10* background thereby demonstrating the functional identity of the *PvFVE* sequence as being functionally equivalent to *AtFVE.* A partial *PvFVE* gene was amplified by RT-PCR using the primer pair SEQ ID NOs:212 and 213. The RT-PCR products were cloned and sequenced. A full length cDNA was subsequently obtained by using the primer pair SEQ ID NOs:214 and 215 for PCR and cloning the resulting product. The resulting sequence is disclosed as SEQ ID NO:216. The switchgrass *FVE* gene (*PvFVE*) has an open reading frame of 1362 nucleotides in length and encodes for 453 amino acids. The nucleotide sequence is 92% identical to maize *FVE*1 and the amino acid sequence 97% identical to maize *FVE*1.

The *PvFVE* gene was cloned into a binary vector such as pBI121-Basta using restriction sites of Xba I and Sac I. The resulting construct of 35S*-PvFVE* was transformed into *fve-b10* by *Agrobacterium*-mediated transformation. The presence of the transgene is confirmed by PCR analysis and selection by Basta resistance. Transgenic T1 plants of *fve-b10*::35S-*PvFVE* are shown to have a wild-type phenotype thereby demonstrating that the *PvFVE* gene is functionally equivalent to the *AtFVE* gene.

### iv) Glycine max (soybean)

A *Glycine max* (soybean), *GmFVE*, sequence was isolated and ectopically expressed in the *Arabidopsis fve-b10* background thereby demonstrating the functional identity of the *GmFVE* sequence as being functionally equivalent to *AtFVE.* A full length cDNA was obtained by using the primer pair SEQ ID NOs:218 and 219 for RT-PCR and cloning the resulting product. The resulting sequence is disclosed as SEQ ID NO:9. The soybean *FVE* gene (*GmFVE)* has an open reading frame of 1542 nucleotides in length and encodes for 513 amino acids (SEQ ID NO:10). The nucleotide sequence is 70% identical to *Arabidopsis FVE* and the amino acid sequence 80% identical to *Arabidopsis FVE.*

The *GmFVE* gene was cloned into a binary vector such as pBI121-Basta using restriction sites of Xba I and Sac I. The resulting construct of 35S*-GmFVE* was transformed into *fve-b10* by *Agrobacterium*-mediated transformation. The presence of the transgene was confirmed by PCR analysis and selection by Basta resistance. Transgenic plants of *fve-b10*::35S-*Gm*FVE were shown to have a wild-type phenotype thereby demonstrating that the *GmFVE* gene is functionally equivalent to the *AtFVE* gene.

### Example 5: Identification of FVE technical features, motifs

The conserved nature and distribution of the WD40 repeats in *FVE* genes provides that basis for identification of useful sequence motifs to effect *FVE* gene inhibition in a transgenic plant. In particular the S1, S2 and WD40 repeats have been identified as motifs useful in down-regulation of *FVE* gene expression and activity. One of skill in the art can analyze an FVE sequence and identify the WD40 repeat motifs using standard software, such as BLAST, CLUSTAL-W or similar programs. The S1 motif is found 5' to the first WD40 motif and has been found only in *FVE* genes. The S2 motif is found between the WD40-2 and WD40-3 motifs. The S2 motif is a species specific *FVE* gene motif and is not strongly conserved across plant species. It still, however, provides a useful target sequence based on the unique nature of this motif and can be used in the species from which it was derived. For example, use of a corn S2 *FVE* sequence motif to inhibit the native corn *FVE* gene. WD-40 repeats have only a few conserved amino acids at spaced intervals over the 40 amino acid motif. Although WD-40 repeats are common throughout numerous genes, the sequence encoding these motifs is not highly conserved, and as such are also useful targets to down-regulate FVE activity.

### Example 6: Constructs useful for FVE inhibition

According to the methods described herein, expression vector constructs can be produced using appropriate promoters and an FVE sequence of the invention. For example, any of the gene sequences of the invention may be operably linked to a promoter that is active in plant cells in such a fashion as to produce a double-strand RNA (dsRNA) molecule that results in a RNA interference (RNAi) response. Expression cassettes useful for inhibition can include the following hair pin constructs that inhibit an endogenous gene via an RNAi type mechanism. Other constructs may be generated using an antisense approach for example.

### Hairpin Constructs

For a hairpin construct, a DNA fragment is generated by producing an inverted repeat of target sequence separated by a spacer. The target sequence used can be at least 35, 50, 100, 150, 200, or 1000 nucleotides in length and the spacer should be at least 4 nucleotides in length. The sense and antisense fragments are amplified by PCR, the sense product having an XbaI site at the 5' end and a SfiI site (5'-GGCCATCTAGGCC-3') at the 3' end, while the antisense product has a SacI site at the 5' end and a SfiI site (5'-GGCCTAGATGGCC-3') at the 3' end. The choice of restriction site is one of convenience and other restriction sites may be used if desired. Purified sense and antisense products are both digested with SfiI, and ligated to generate fragments with a central, non-palindromic, ATCTA sequence that functions as a spacer. The resulting ligated sense-antisense fragment has a 5' XbaI cloning site and a 3' SacI site. The ligated fragment can be easily cloned into a transformation vector of choice. For example, a binary vector such as pBI121 using XbaI and SacI restriction sites. The constructs are confirmed by sequencing to contain the desired structure.

The following exemplary hairpin RNAi constructs were produced according the method described above:

The S1 motif (SEQ ID NO:57) was PCR amplified using the primer pair SEQ ID NOs:177 and 178 to produce a sense fragment, and the primer pair SEQ ID NOs:179 and 180 to produce an antisense fragment. Digestion with SfiI and ligation produced a sense-antisense hairpin cassette that was operably linked to a constitutive promoter such as a CaMV 35S promoter and included a 3' NOS terminator region (35S-HP-*AtFVE*-S1, SEQ ID NO:372). This construct specifically targets the *FVE* gene of *Arabidopsis* and is useful in a variety of other plant species. Similar constructs are generated using S1 sequences as described in SEQ ID NO: 51-64, 245 and 246, or identified according to the description herein.

The S2 motif (SEQ ID NO:87) was PCR amplified using the primer pair SEQ ID NOs:189 and 190 to produce a sense fragment, and the primer pair SEQ ID NOs:191 and 192 to produce an antisense fragment. Digestion with SfiI and ligation produced a sense-antisense hairpin cassette that was operably linked to a constitutive promoter such as a CaMV 35S promoter and included a 3' NOS terminator region (35S-HP-*AtFVE*-S2, SEQ ID NO:373). This construct specifically targets the *FVE* gene of *Arabidopsis.* Similar S2-like containing constructs, specific for a species of choice, are produced using a S2 sequence as described in SEQ ID NO: 79-92, 233 and 248, or identified according to the description herein.

The S2 motif from *Brassica* was identified as a fragment within SEQ ID NO:206 (*BnFVE*) and is shown as SEQ ID NO:233. To produce the 35S-HP-*BnFVE*-S2 construct, a *BnFVE-S2* motif was PCR amplified using the primer pair SEQ ID NOs:224 and 225 to produce a sense fragment, and the primer pair SEQ ID NOs:226 and 227 to produce an antisense fragment. Digestion with SfiI and ligation produced a sense-antisense hairpin cassette that was operably linked to a constitutive promoter such as a CaMV 35S promoter and included a 3' NOS terminator region (35S-HP-*BnFVE*-S2, SEQ ID NO:374).

The WD40-2 motif (SEQ ID NO:118) was PCR amplified using the primer pair SEQ ID NOs:185 and 186 to produce a sense fragment, and the primer pair SEQ ID NOs:187 and 188 to produce an antisense sense fragment. Digestion with SfiI and ligation produced a sense-antisense hairpin cassette that was operably linked to a constitutive promoter such as a CaMV 35S promoter and included a 3' NOS terminator region (35S-HP-*AtFVE*-WD40-2, SEQ ID NO:375). This construct specifically targets the *FVE* gene of *Arabidopsis* and will be useful in a variety of other plant species based on the high degree of sequence homology. Similar WD40-2-like containing constructs are produced using a WD40-2 sequence as described in SEQ ID NO: 107-120, 234 and 252, or identified according to the description herein.

The WD40-2 motif from *Brassica* was identified as a fragment within SEQ ID NO:206 and is shown as SEQ ID NO:234. To produce the 35S-HP-*BnFVE*-WD40-2 construct a *BnFVE-*WD40-2 motif was PCR amplified using the primer pair SEQ ID NO's:229 and 230 to produce a sense fragment and the primer pair SEQ ID NO's:231 and 232 to produce an antisense fragment. Digestion with SfiI and ligation produced a sense-antisense hairpin cassette that was operably linked to a constitutive promoter such as a CaMV 35S promoter and included a 3' NOS terminator region (35S-HP-*BnFVE*-WD40-2, SEQ ID NO:376).

The WD40-1 motif (SEQ ID NO:140) was operably linked to a constitutive promoter such as a CaMV 35S promoter and included a 3' NOS terminator region (35S-HP-*AtFVE-*WD40-1, SEQ ID NO:377).

The WD40-1 motif from *Brassica* was identified as a fragment within SEQ ID NO:206 and is shown as SEQ ID NO:235. To produce the 35S-HP-*BnFVE*-WD40-1 construct, a *BnFVE-*WD40-1 motif was PCR amplified using the primer pair SEQ ID NOs:237 and 182 to produce a sense fragment, and the primer pair SEQ ID NOs:238 and 184 to produce an antisense fragment. Digestion with SfiI and ligation produced a sense-antisense hairpin cassette that was operably linked to a constitutive promoter such as a CaMV 35S promoter and included a 3' NOS terminator region (35S-HP-B*nFVE*-WD40-1, SEQ ID NO:378).

The WD40-3 motif (SEQ ID NO:174) was operably linked to a constitutive promoter such as a CaMV 35S promoter and included a 3' NOS terminator region (35S*-HP-AtFVE-*WD40-3, SEQ ID NO:379). Constructs are generated having a WD40-3 motif as described in SEQ ID NO: 163-176, 256 and 254.

Similarly, constructs can be generated using any of the WD40-4, WD40-5, WD40-6, motifs as described in SEQ ID NOs:273-288, 305-320 or 337-352. Additionally, constructs need not be limited to an identified domain. Constructs may be generated that incorporate sequences across motifs, provided that the sequence selected will target the endogenous *FVE* gene activity.

To down-regulate a target gene by a dominant-negative methodology, a non-functional version of the target gene is expressed. The gene isolated from the *fve-b10* mutant described above contains a deletion that alters the encoded protein. The mutant allele is described as SEQ ID NO:1, its predicted protein product as SEQ ID NO:2, and the genomic sequence is described as SEQ ID NO:5. The mutant coding sequence was PCR amplified using primers such as SEQ ID NO:197 and SEQ ID NO:198 to produce a XbaI-BamHI fragment that was cloned into pBI121ΔGUS. The *fve-b10* coding sequence has an addition of 57 nucleotides of coding region and therefore makes an FVE mutant protein (fve) longer than wild-type FVE protein by 19 amino acids in the first WD40 domain, consequently destroying the first WD40 domain in fve. The *fve-b10* encodes a protein with 526 amino acids in contrast to a protein of 507 amino acids in wild-type FVE. Expression of the *fve-b10* gene produces an aberrant polypeptide, resulting in FVE activity inhibition. A plant having phenotypes such as increased biomass is produced. The construct (35S-*fve-b10*) was transformed into wild-type *Arabidopsis* Col-0 by *Agrobacterium-*mediated transformation approach. Transgenic plants of 35S-f*ve-b10* displayed the same phenotypes as the *fve-b10* mutant plants demonstrating that the over expression of a non-functional *FVE* gene is sufficient to produce the desired phenotypes.

### Constructs for amiRNA down-regulation

Artificial-miRNA down-regulation of an *FVE* gene is achieved by preparing a modified naturally occurring miRNA to target the desired gene sequence. A micro-RNA sequence will, when transcribed, form a stem and loop structure. The miRNA sequence comprises a first sequence that is target specific and will form one half of the stem structure with a second sequence that is essentially identical, or nearly identical, to the first but in an anti-parallel orientation relative to the first sequence, the first and second sequences separated by a non-target sequence that forms a loop structure.

Construction of amiRNA cassettes to down-regulate *FVE* involves a first step of PCR amplification and isolation of a miRNA171 precursor (miRNA171pre) sequence from *Arabidopsis.* A genomic fragment of 227 nucleotides that comprised the miRNA171pre of 123 bp was amplified from wild-type *Arabidopsis* accession Col-0 using primer pair of SEQ ID NOs:364 AND 365. The PCR amplified sequence is disclosed as SEQ ID NO:363.
**Genomic fragment harboring miRNA171pre in *Arabidopsis* (227bp)** (SEQ ID NO:363)

Using the miRNA171pre (SEQ ID NO: 363) as a template, *FVE*-S1 specific primers were designed that included a 5'restriction sites to facilitate cloning, a 21 nucleotide *FVE* gene targeting sequence, a portion of the miR171pre stem sequence and a partial loop sequence. In this case, the primer pair of SEQ ID NOs: 366 and 367 was used to amplify an *FVE*-S1 sequence. The resulting fragment (SEQ ID NO: 368) was cloned into a vector under the regulatory control of a 35S promoter using the BamHI and SacI sites designed into the primers. The construct is referred to as 35S-*miR-At-BnFVE*-S1 and is transformed into *Arabidopsis* and *Brassica* for down-regulation of *FVE* gene activity.

To generate a construct targeting a WD40-2 motif of an *FVE* gene the above methodology was followed. Using the miRNA171pre (SEQ ID NO: 368) as a template, *FVE-*WD40-2 specific primers were designed that included a 5'restriction sites to facilitate cloning, a 21 nucleotide *FVE* gene targeting sequence, a portion of the miR171pre stem sequence and a partial loop sequence. In this case, the primer pair of SEQ ID NOs: 369 and 370 was used to amplify an *FVE-* WD40-2 sequence. The resulting fragment (SEQ ID NO: 371) was cloned into a vector under the regulatory control of a 35S promoter using the BamHI and SacI sites designed into the primers. The construct is referred to as 35S-miR-*At*-*Bn*-WD2 -pre and is transformed into *Arabidopsis* and *Brassica* for down-regulation of *FVE* gene activity..

### Antisense Down regulation

Antisense constructs are generated using an FVE sequence or portion thereof. A portion is at least 50 nucleotides in length and can be 500, 1000, 1500 or more nucleotides in length. An antisense sequence is at least 70% homologous to the endogenous target sequence. More preferable the antisense sequence is from the target species and is therefore 99% or more homologous.

**Table 6: Primers for hair pin constructs for FVE inhibition**

| | **Motif** | **DNA sequences 5'-3'** | **SEQ ID NO:** |
|---|---|---|---|
| BS1SF | S1 | TTT CTA GAG ATG GAA GTG TGC CCA ATA CTT | 177 |
| BS1SR | S1 | TTG GCC AAG ATG GCC TGG AGA ACG TGC TTC TTC ATT G | 178 |
| BS1AF | S1 | TTG AGC TCG ATG GAA GTG TGC CCA ATA CTT | 179 |
| BS1AR | S1 | TTG GCC ATC TAG GCC TGG AGA ACG TGC TTC TTC ATT G | 180 |
| BD1SF | WD40-1 | TTT CTA GAT TTG TGA AGA AGT ACA AGA CCA | 181 |
| BD1SR | WD40-1 | TTG GCC AAG ATG GCC ATC CCA AAT GAG AAC ATC AGG A | 182 |
| BD1AF | WD40-1 | TTG AGC TCT TTG TGA AGA AGT ACA AGA CCA | 183 |
| BD1AR | WD40-1 | TTG GCC ATC TAG GCC ATC CCA AAT GAG AAC ATC AGG A | 184 |
| BD2SF | WD40-2 | TTT CTA GAT CCC GTC CAG ATT TGA TAC TAA | 185 |
| BD2SR | WD40-2 | TTG GCC AAG ATG GCC ACT CCA CAA AAC AAC TGA CTT G | 186 |
| BD2AF | WD40-2 | TTG AGC TCT CCC GTC CAG ATT TGA TAC TAA | 187 |
| BD2AR | WD40-2 | TTG GCC ATC TAG GCC ACT CCA CAA AAC AAC TGA CTT G | 188 |
| BS2SF | S2 | TTT CTA GAA TCC AAG ATC ACA TCA CAA CGA | 189 |
| BS2SR | S2 | TTG GCC AAG ATG GCC AGT AGG ACT CTC ATT CTT ATC A | 190 |
| BS2AF | S2 | TTG AGC TCA TCC AAG ATC ACA TCA CAA CGA | 191 |
| BS2AR | S2 | TTG GCC ATC TAG GCC AGT AGG ACT CTC ATT CTT ATC A | 192 |
| BD3SF | WD40-3 | TTT CTA GAG TTG GCC CAC GAG GTG TAT ATC | 193 |
| BD3SR | WD40-3 | TTG GCC AAG ATG GCC ATC CCA TAG TAT AAG GCA AGA A | 194 |
| BD3AF | WD40-3 | TTG AGC TCG TTG GCC CAC GAG GTG TAT ATC | 195 |
| BD3AR | WD40-3 | TTG GCC ATC TAG GCC ATC CCA TAG TAT AAG GCA AGA A For cloning into pBI121/XbaI / SacI | 196 |

**Table 7: Primers for expression fve-b10 dominant-negative inhibition**

| | **DNA sequences 5'**-**3'** | **SEQ ID NO:** |
|---|---|---|
| C1F/XbaI | TTTCTAGAATGGAGAGCG ACGAAGCAGC AG | 197 |
| C1R/BamHI | TTGGATCC TTAAGGCTTGGAGGCACAAGTC | 198 |
| | For cloning into pBI121AGUS | |

**Table 8: Primers for constructs for FVE inhibition in Canola**

| **Name** | **DNA sequences 5'-3'** | **SEQ ID NO:** |
|---|---|---|
| Bn-WD1-sense-Forward | TTTCTAGATTTGTGAAGAAGTTCAAGACCA | 237 |
| Bn-WD1-sense-Reverse | The same as BD1SR | 182 |
| Bn-WD1-antisense-Forward | TTGAGCTCTTTGTGAAGAAGTTCAAGACCA | 238 |
| Bn-WD1-antisense-Reverse | The same as BD1AR | 184 |
| Bn-WD2-sense-Forward | TTTCTAGATCCCGTCCTGATTTGGTATTAA | 229 |
| Bn-WD2-sense-Reverse | TTGGCCAAGATGGCCACTCCACAAAACAACTGACTTG | 230 |
| Bn-WD2-antisense-Forward | TTGAGCTCTCCCGTCCTGATTTGGTATTAA | 231 |
| Bn-WD2-antisense-Reverse | TTGGCCATCTAGGCCACTCCACAAAACAACTGACTTG | 232 |
| Bn-S2-sense-Forward | TTTCTAGAATCCAGGACCACATCACAACCG | 224 |
| Bn-S2-sense-Reverse | TTGGCCAAGATGGCCAGAAGGACTCTCAGTCTTATCA | 225 |
| Bn-S2-antisense-Forward | TTGAGCTCATCCAGGACCACATCACAACCG | 226 |
| Bn-S2-antisense-Reverse | TTGGCCATCTAGGCCAGAAGGACTCTCAGTCTTATCA | 227 |

**Table 9: Primers for constructs for complementation test and functional genomics**

| **Name** | **DNA sequences 5'-3'** | **SEQ ID NO:** |
|---|---|---|
| AtFVE-F | TTTCTAGAATGGAGAGCG ACGAAGCAGCAG | 197 |
| AtFVE-R | TTGGATCCTTAAGGCTTGGAGGCACAAGTC | 198 |
| Can-HF1 | TTTCTAGAATGGAGAGCGACGGAGCGGCAG | 204 |
| Can-HR2 | TTGAGCTCTCAAGGCTTGGAGGCACATGTC | 205 |
| Maize1-F | TTTCTAGAATGAAAGAGAGAGGCGGCTCCAG | 208 |
| Maize1-R | TTGAGCTCTCAATTCTTCGGTGCACAGCTGG | 209 |
| Maize2-F | TTTCTAGAATGAAGGAGAGAGGCGGCTCCAG | 210 |
| Maize2-R | TTGAGCTCTCAATTCCTCGGAGCGCAACTGG | 211 |
| PVF1 | TTTCTAGAATGAAGGAGAGGAGCGGCTCGAG | 214 |
| PVR1 | TTGAGCTCTCAGTTCCTCGGAGTGCAGCTG | 215 |
| GmFl | TTTCTAGAATGGAGACTCCTCCTCCCCAAC | 218 |
| GmR1 | TTGAGCTCTCATTTTTCAGTCTTTGAAGCAC | 219 |
| Bn-F1 | GACCCTCAGAGAAACGGAAAATG | 201 |
| At-S3R | CATTGAACTGAGAAATGTGCTC | 202 |
| At-S2F | AGTGCACAAGAATTCTGCAGTG | 203 |
| CRLR1 | ATATGTTCAGCAGCTGCAACC | 212 |
| CRLF4 | GCTGCTGTTCTTTGTGTTCAG | 213 |

**Table 10: Primers for construct of FVE protein heterologous expression**

| **Name** | **DNA sequences 5'-3'** | **SEQ ID NO:** |
|---|---|---|
| At-P1F | TTGGATCCATGGAGAGCGACGAAGCAGCAG | 199 |
| AT-P1R | TTGTCGACTTAAGGCTTGGAGGCACAAGTC | 200 |

**Table 11: Primers for constructs of amiRNA**

| **Name** | **DNA sequences 5'-3'** | **SEQ ID NO:** |
|---|---|---|
| MR171F1 | TGTTTCCTTTGATATCCGCAC | 364 |
| MR171R1 | GAAGTTACAAGTACTTGTCTCC | 365 |
| MI171B-S1F | CGGGATCCATGAGAGAGTCCCTTTCACGTTCTCGATTTGTGCAGAGATCTTACC TGACCACACACG | 366 |
| MI171B-S1R | GCGAGCTCACGAGAGAGTACTGACACGTTCTCCATTTGTGAAGAGATAATCTA GAGAGAATAATG | 367 |
| MI171B-D2F | CGGGATCCATGAGAGAGTCCCTTTACAAGTCAGATGTTTTGGGGAGATCTTAC TGACCACACACG | 369 |
| MI171B-D2R | GCGAGCTCACGAGAGAGTACTGAACAAGTCAGTTGTTTTGTGGAGATAATCTA GAGAGAATAATG | 370 |

**Table 12:**

| | **Primers used for FVE construct generation** | |
|---|---|---|
| **Construct** | **SEQ ID NO's:** | |
| 35S-HP-AtFVE-S1 | SEQ ID NO: 177, 178, 179, 180 | |
| 35S-HP-AtFVE-S2 | SEQ ID NO: 189, 190, 191, 192 | |
| 35S-HP-AtFVE-WD40-1 | SEQ ID NO: 181, 182, 183, 184 | |
| 35S-HP-AtFVE-WD40-2 | SEQ ID NO: 185, 186, 187, 188 | |
| 35S-HP-AtFVE-WD40-3 | SEQ ID NO: 193, 194, 195, 196 | |
| | | |
| 35S-HP-BnFVE-S2 | SEQ ID NO: 224, 225, 226, 227 | |
| 35S-HP-BnFVE-WD1 | SEQ ID NO: 237, 182, 238, 184 | |
| 35S-HP-BnFVE-WD2 | SEQ ID NO: 229, 230, 231, 232 | |
| | | |
| 35S-AtFVE | SEQ ID NO: 197, 198 | |
| 35S-*fve*-*b10* | SEQ ID NO: 197, 198 | |
| 35S-BnFVE | SEQ ID NO: 204, 205 | |
| 35S-ZmFVE1 | SEQ ID NO: 208, 209 | |
| 35S-ZmFVE2 | SEQ ID NO: 210, 211 | |
| 35S-PvFVE | SEQ ID NO: 214, 215 | |
| 35S-GmFVE | SEQ ID NO: 218, 219 | |
| Ptac-AtFVE | SEQ ID NO: 199,200 | |
| Ptac-*fve-b10* | SEQ ID NO: 199,200 | |
| | | |
| 35S-miR-At-Bn-S1 | SEQ ID NO: 366, 367 | |
| 35S-miR-At-Bn-WD2 | SEQ ID NO: 369, 370 | |

### Example 7: Plant Transformation

*Arabidopsis* transgenic plants were produced by the method of dipping flowering plants into an *Agrobacterium* culture, based on the method of Andrew Bent in, Clough SJ and Bent AF, "Floral dipping: a simplified method for *Agrobacterium*-mediated transformation of *Arabidopsis thaliana* " (1998). Wild-type plants were grown under standard conditions with 16, 8 hour light dark cycles, until the plant has both developing flowers and open flowers. The plants were inverted for 2 minutes into a solution of *Agrobacterium* culture carrying the appropriate gene construct. Plants were then left horizontal in a tray and kept covered for two days to maintain humidity and then righted and bagged to continue growth and seed development. Mature seed was bulk harvested.

T1 plants were germinated and grown on MS plates containing kanamycin (50 µg/ml), and kanamycin resistant T1 seedlings were selected and transferred to soil for further growth. Leaf samples were harvested, DNA isolated and PCR analysis done to select those individuals containing both the desired constructs. Plants were bagged to ensure self fertilization and the T2 seed of each plant harvested separately.

Transgenic *Brassica napus, Glycine max* and *Zea maize* plants can be produced using *Agrobacterium* mediated transformation of cotyledon petiole tissue. Seeds are sterilized as follows. Seeds are wetted with 95% ethanol for a short period of time such as 15 seconds. Approximately 30 ml of sterilizing solution I is added (70% Javex, 100µl Tween20) and left for approximately 15 minutes. Solution I is removed and replaced with 30 ml of solution II (0.25% mecuric chloride, 100µl Tween-20) and incubated for about 10 minutes. Seeds are rinsed with at least 500 ml double distilled sterile water and stored in a sterile dish. Seeds are germinated on plates of ¹/₂ MS medium, pH 5.8, supplemented with 1% sucrose and 0.7% agar. Fully expanded cotyledons are harvested and placed on Medium I (Murashige minimal organics (MMO), 3% sucrose, 4.5 mg/L benzyl adenine (BA), 0.7% phytoagar, pH5.8). An *Agrobacterium* culture containing the nucleic acid construct of interest is grown for 2 days in AB Minimal media. The cotyledon explants are dipped such that only the cut portion of the petiole is contacted by the *Agrobacterium* solution. The explants are then embedded in Medium I and maintained for 5 days at 24°C, with 16, 8 hr light dark cycles. Explants are transferred to Medium II (Medium I, 300 mg/L timentin,) for a further 7 days and then to Medium III (Medium II, 20 mg/L kanamycin). Any root or shoot tissue which has developed at this time is dissected away. Explants are transferred to fresh plates of Medium III after 14 -21 days. When regenerated shoot tissue develops the regenerated tissue is transferred to Medium IV (MMO, 3% sucrose, 1.0% phytoagar, 300 mg/L timentin, 20 mg/L 20 mg/L kanamycin). Once healthy shoot tissue develops, shoot tissue dissected from any callus tissue are dipped in 10X IBA and transferred to Medium V (Murashige and Skooge (MS), 3% sucrose, 0.2 mg/L indole butyric acid (IBA), 0.7% agar, 300 mg/L timentin, 20 mg/L 20 mg/L kanamycin) for rooting. Healthy plantlets are transferred to soil. The above method, with or without modifications, is suitable for the transformation of numerous plant species including *Brassica napus*, *Glycine max*, *Zea maize* and cotton.

Transgenic *Glycine max*, *Zea maize* and cotton can be produced using *Agrobacterium-*based methods which are known to one of skill in the art. Alternatively one can use a particle or non-particle biolistic bombardment transformation method. An example of non-particle biolistic transformation is given in U.S. Patent Application 20010026941, which is herein incorporated by reference in its entirety.

*Agrobacterium* can be used to transform variety of plant tissues according to published and established methods. Additionally, other means and methods are available to produce transgenic plants such as biolistics methods, aerosol beam injection methods, microinjection *methods, in vivo* site directed mutagenesis, using directed oligonucleotides for example.

### Example 8: Physiological Evaluation of Transgenic plants having down-regulated FVE expression

Plants transformed with expression vectors designed to down-regulate *FVE* expression were evaluated and their physiological characteristics assessed. The vectors used included a 35S-*fve-b10* construct that over expresses the mutant *fve* gene and affects down-regulation via a dominant-negative mechanism; a 35S-HP-*AtFVE*-S2 construct and a 35S-HP-*AtFVE*-WD40-3 construct which down-regulate *FVE* activity or expression via a hairpin-RNAi mechanism.

Evaluation of each set of transgenic plants comprised 7 to 10 transgenic lines plus 1 to 2 nulls, a WT control and *fve-b10* mutant plants (nulls being control plants that have been isolated as negative segregants during the process of isolating homozygous transgenic lines). Eight replicate plants were planted per entry and per harvest. Two destructive harvests were set up, first harvest at 5 days post first flower and second harvest at maturity. During plant growth and before the two destructive harvests non-destructive measurements were made at: the vegetative stage of 12 day old seedlings when the number of leaves were counted; and at the time of first open flower when the number of leaves and plant height were recorded. At the time of the two harvests, the number of leaves, plant height and number of branches were also counted in addition to total shoot biomass determinations.

The results of these growth studies showed that all three down-regulation constructs targeting the *FVE* gene successfully caused an enhancement in biomass production in the transgenic plants and delay in flowering. The differences between transgenic and control plants were first apparent shortly before flowering time.

### 35S-fve-b10 Dominant-Negative Construct

The results of ten 35S-*fve-b10* transgenic lines are shown in Table 13, and demonstrate delayed flowering of 2 to 5 days as compared to WT and null controls, whereas *fve-b10* mutant plants in this experiment flowered 15 days later than WT. Transgenic lines of this construct had up to 60% more leaves at 5 days from first open flower and their dry weight was increased up to 2.6 fold over the WT. The stem DW was enhanced up to 58% and the total shoot biomass enhancement was up to 2 fold over the WT. The *fve-b10* mutant showed almost 4 fold increase in shoot biomass.

Two of the transgenic lines (lines 57-6 and 73-8) that have shown a delay in flowering and enhanced shoot biomass plus the WT control and the *fve-b10* mutant were examined for main stem live cross sections 3 days post-first flower. These were examined using a dissection scope (Lica) and photos were taken at 8x magnification. From the photo measurement of the stem diameter, the thickness of the intervesicular fiber layer and counts of vascular bundles were determined. The two transgenic lines had 42 and 65% thicker stems (compared to 60% thicker in *fve-b10*) than those of WT. The transgenic lines had 48 and 56% more vascular bundles (compared to 87% in *fve-b10*), and 33% and 51% thicker fiber layer (compared to 47% in *fve-b10*). In addition, live epidermal peels were obtained from the stems of these plants and were examined using fluorescent microscope for chloroplast numbers in guard cells. The *fve-b10* plants had 20% more chloroplasts while the transgenic plants had 10 to 15% more chloroplasts than WT controls.

**Table 13. Growth enhancement in 35S-fve-b10 transgenic lines (5 days post-flower)**

| **entry** | **Days to flower** | **Stem DW (mg)** | **Rosette leaf #** | **Leaf DW (mg)** | **Shoot DW (mg)** | **Shoot DW (% of WT)** |
|---|---|---|---|---|---|---|
| *fve-b10* | 36 | 360±42 | 75 | 991±70 | 1351±104 | 384 |
| 73-8 | 26 | 289±22 | 46 | 437±47 | 727±63 | 207 |
| 95-4 | 25 | 287±28 | 50 | 400±30 | 686±51 | 195 |
| 98-2 | 26 | 275±18 | 50 | 365±21 | 641±28 | 182 |
| 62-2 | 26 | 207±12 | 44 | 415±52 | 622±59 | 177 |
| 57-6 | 25 | 258±18 | 42 | 360±31 | 619±46 | 176 |
| 71-3 | 25 | 232±19 | 43 | 347±24 | 579±38 | 165 |
| 70-6 | 24 | 215±10 | 42 | 351±18 | 567±26 | 161 |
| 81-7 | 25 | 223±13 | 42 | 340±27 | 563±36 | 160 |
| 68-2 | 23 | 225±15 | 40 | 259±12 | 484±24 | 138 |
| 71-4(null) | 20 | 153±13 | 31 | 153±6 | 310±1 | 87 |
| 98-4(null) | 21 | 160±13 | 33 | 179±14 | 340±3 | 97 |
| WT | 21 | 183±13 | 32 | 168±11 | 350±2 | 100 |

A group of plants (n=8) grown in 4" pots was grown to maturity and plants were harvested at the end of their life cycle for final shoot biomass and seed yield. The harvest index was also calculated. The results showed that the final total shoot biomass of the transgenic lines was also enhanced from 5% to 32% over that of controls. The seed yield varied from WT levels to 13% greater than that of controls. As a result the harvest index was reduced in all of the transgenic lines indicating, as shown before for the *fve-b10* mutant, that transgenic plants direct more of their resources towards the biomass than the seed production.

### 35S-fve-b10 Dominant-Negative Construct In Brassica

### Physiological characterization of transgenic canola plants.

*Brassica* plants were transformed with a 35S-*fve-b10* construct for dominant-negative inhibition of the *Brassica FVE* gene. Eleven transgenic events at a T1 stage were assessed for biomass enhancement and time to flowering. At a T1 stage the plants were confirmed as transgenic by PCR analysis, however the plants were heterozygous. These transgenic plants were grown in a greenhouse in 4" pots under optimal conditions (22°C, 18 hr light at 400uE). Days to first open flower were recorded and nine days from first flower all plants were harvested for leaf and stem dry biomass determinations. Plant height, number of leaves and number of branches were also recorded. The results indicate that three of the transgenic events had enhanced shoot biomass and delayed flowering (Table 14). The delay was up to seven days in the best event (165G-6) and the shoot biomass enhancement was increased as much as 40% relative to WT. There were no differences in plant height, number of branches and leaf number. The enhancement in the shoot biomass seemed to be a result of thicker stems, as indicated by greater stem DW, and either larger or thicker leaves as indicated by greater leaf DW. The transgenic events were heterozygous and this contributes to increased variability in the data. The plants were known to be transgenic however the degree of *FVE* inhibition was undetermined. Homozygous plants are selected and physiological analysis and best lines are selected.

The construct used to transform canola was an *Arabidopsis* construct (35S-*fve-b10*) further demonstrating the universality of the method and the cross species portability of the gene.

**Table 14: Growth enhancement in 35S-fve-b10 transgenic lines**

| **entry** | **Days to flower** | **Stem DW (g)** | **leaf #** | **Leaf DW (g)** | **Shoot DW (g)** | **Shoot DW (% of WT)** |
|---|---|---|---|---|---|---|
| 161G-1 | 41 | 8.9±0.5 | 30 | 5.3±0.9 | 14.2±1.3 | 114% |
| 165G-4 | 42 | 9.5±0.9 | 28 | 5.0±0.6 | 14.5±1.4 | 117% |
| 165G-6 | 45 | 10.2±1.0 | 30 | 7.2±1.6 | 17.4±2.5 | 140% |
| 159Y-5(null) | 39 | 6.7±0.4 | 28 | 4.1±0.2 | 10.8±0.5 | 87% |
| WT | 38 | 8.3±0.5 | 28 | 4.1±0.3 | 12.4±0.7 | 100% |

### 35S-HP-AtFVE-S2 Construct in Arabidopsis

The results of the experiment with eight 35S-HP-*AtFVE*-S2 transgenic lines as compared to WT and null controls is shown in Table 15. Transgenic plants showed a delay in flowering of up to 6 days as compared to WT, while *fve-b10* mutant in this study was delayed 13 days. Five days into flowering, transgenic lines showed up to twice as many rosette leaves, up to 2.4 fold greater stem biomass and up to 3.4 fold greater leaf biomass. The total shoot dry weight (DW) was up to 2.5 fold greater in the transgenic lines while the *fve-b10* mutant showed 5.4 fold enhancement of its shoot biomass.

**Table 15. Growth enhancement in 35S-HP-AtFVE-S2 transgenic lines**

| **entry** | **Days to flower** | **Stem DW (g)** | **Rosette leaf #** | **Leaf DW (g)** | **Shoot DW (g)** | **Shoot DW (% of WT)** |
|---|---|---|---|---|---|---|
| *fve-b10* | 35 | 255±24 | 79 | 974±36 | 1212±41 | 539 |
| 30-10 | 28 | 257±22 | 33 | 311±25 | 568±39 | 253 |
| 48-1 | 27 | 167±17 | 52 | 395±42 | 562±55 | 250 |
| 36-9 | 26 | 187±17 | 50 | 336±28 | 523±39 | 233 |
| 28-9 | 25 | 224±25 | 40 | 252±20 | 477±43 | 212 |
| 40-4 | 26 | 226±16 | 38 | 238±14 | 465±20 | 207 |
| 49-2 | 24 | 179±13 | 34 | 215±12 | 394±22 | 175 |
| 6-3 | 24 | 175±19 | 40 | 219±32 | 394±50 | 175 |
| 14-9 | 25 | 193±21 | 32 | 182±16 | 375±35 | 167 |
| 14-4(null) | 22 | 114±12 | 28 | 136±11 | 249±23 | 111 |
| WT | 22 | 108±14 | 26 | 117±10 | 225±22 | 100 |

A group of plants (n=8) was also grown to maturity and final total dry biomass and seed yield of the plants were determined at the end of their lifecycle. The results showed a range in biomass enhancement of up to 45% greater than the controls. The seed yield was increased in transgenic lines up to 30% over the controls and the harvest index was reduced accordingly.

### 35S-HP-AtFVE-WD40-3 Construct

The results of the experiment with five 35S-HP-*AtFVE*-WD40-3 transgenic lines (Table 16) showed a delay in flowering by as much as 10 days as compared to WT, while *fve-b10* mutant in this study was delayed by 13 days. During the mid-flower harvest (5days from first flower) transgenic lines showed up to 2.5 fold more rosette leaves, 1.6 fold greater stem dry weight and 4.4 fold greater leaf dry weight. The total shoot dry biomass was up to 3 fold greater in transgenics while the *fve-b10* mutant showed 3.9 fold enhancement.

**Table 16. Growth enhancement in 35S-HP-AtFVE-WD40-3 transgenic**

| **entry** | **Days to flower** | **Stem DW (g)** | **Rosette leaf #** | **Leaf DW (g)** | **Shoot DW (g)** | **Shoot DW (% of WT)** |
|---|---|---|---|---|---|---|
| *fve-b10* | 35 | 324±36 | 103 | 1266±69 | 1609±91 | 388 |
| 57-4 | 33 | 321±25 | 84 | 936±77 | 1256±99 | 303 |
| 10-7 | 27 | 241±25 | 55 | 543±48 | 785±66 | 189 |
| 55-3 | 27 | 297±32 | 51 | 467±45 | 765±73 | 184 |
| 30-8 | 26 | 255±16 | 45 | 403±31 | 658±38 | 159 |
| 5-10 | 24 | 233±22 | 34 | 228±10 | 462±28 | 111 |
| 10-1(null) | 22 | 154±12 | 29 | 166±7 | 320±19 | 77 |
| WT | 23 | 202±12 | 33 | 212±8 | 415±18 | 100 |

In conclusion, all three constructs aimed at down-regulation of the *FVE* gene resulted in transgenic lines which showed biomass enhancement and a delay in flowering phenotypes similar to those of the *fve-b10* mutant. The magnitude of the phenotypes observed spans a range between mutant and wild-type. This is expected as transgenic plants produce a range of gene inhibition in each line based on factors such as, insertion site, copy number and strength of transgene expression for example. Such a range of responses is typical and one uses such a varied response to select the lines having the most desirable phenotypes. In addition to enhanced shoot biomass down-regulation of *FVE* also results in increased chloroplast numbers and therefore higher chlorophyll content, thicker stems and enhanced vascular bundles. More vascular bundles and thicker intervesicular fiber layer results in higher fiber content of the stems.

### Example 9: Analysis of cellular structure in stems of Arabidopsis fve-b10 and transgenic plants having inhibited FVE activity

A series of microscopic studies using various tissue types during plant development were analyzed for structural differences between *FVE* inhibited plants including the mutant *fve-b10* and wild-type control plants. Leaf, root and stem tissues were collected at four developmental stages: young seedling (7 days after germination), bolting, first day of flowering, and post-flowering (fourth day of flowering). Tissues were fixed using paraformaldehyde, dehydrated using a graded ethanol series, and infiltrated using paraffin/TBA method and embedded. Blocks were sectioned, and sections were mounted on slides. Slides were stained either with Toluidine Blue O to visualize the cross-sections of the tissues, or with Calcofluor White to illustrate any qualitative change in cellulose content in these sections.

### Tolatidine Blare O Staining

Sections were deparaffinized in two changes of CitriSolv (Thermo Fisher Scientific), rinsed in absolute ethanol and allowed to dry. A filtered 1% stock solution of Toluidine Blue O (TBO) was prepared in 1% sodium borate. Stock solution was diluted 10-fold in sodium borate before use (final concentration - 0.1% TBO in 1% sodium borate). Sections were stained for 30-60 seconds and rinsed thoroughly in deionized water. Slides were allowed to dry, and cover slips were mounted using Permount (Thermo Fisher Scientific). Sections were observed and photographed using a Leica DMLB microscope with a Leica DFC300FX camera.

### Cellatlose Staining

Calcofluor White (Fluorescent Brightener 28, Sigma-Aldrich) was used as a 0.1% solution in half-normal saline pH 6.0. Sections were deparaffinized in 2 changes of CitriSolv, and re-hydrated using a graded ethanol series. Slides were washed under running tap water for 48 hours to remove all traces of organic solvents. After washing Calcoflour solution was applied to the sections and a cover slip placed over the sections. (Harrington and Raper, 1968) Sections were observed and photographed using a Leica DMLB microscope equipped with a BFP-A-Basic filter cube (Semrock - excitation filter BP340-380, dichromatic mirror 400 and suppression filter LP425) and Leica DFC300FX camera.

Data demonstrated that stems of *fve-b10* show approximately 100% increase in the number of vascular bundles, increased cellulose content and have more cells with unchanged cell size therefore the stems are physically larger in diameter compared to the control at each developmental stage investigated. The vascular tissue regions in leaves and roots of *fve-b10* are also enlarged while individual cell size remains unchanged resulting in the leaves and roots being thicker, and cellulose density is higher than controls.

### Example 10: Enhanced fiber content in the stems of fve-b10 and transgenic plants

Quantitative analysis of fiber content (both cellulose and lignin) in plants having reduced *FVE* activity was performed. Stem tissues were collected from the *Arabidopsis fve-b 10* mutant, transgenic plants expressing the mutant gene construct 35S-*fve-b10* and thereby inhibiting native *FVE* activity via a dominant negative mechanism and their respective controls (Col-0 and a segregated null). Plant tissues were collected on the seventh day of flowering for cellulose and lignin analysis. The total cellulose content including cellulose and hemi-cellulose in the stem tissues was measured by sugar monomers derived from complete hydrolysis of cellulose and hemi-cellulose using high performance liquid chromatography (HPLC). The sugar monomers quantified included fructose, arabinose, rhamnose, galactose, glucose, xylose and mannose. Glucose is the major breakdown product of cellulose, and xylose presents in the largest amount in hemi-cellulose (Rogers *et al.,* 2005). Values were expressed as a percent of its appropriate wild-type control or a segregated null. In parallel, the total lignin content including acid-insoluble and acid-soluble in these stem tissues was obtained by using Klason method (Chiang and Funaoka, 1990), and TAPPI Useful Method UM250 (TAPPI useful method UM250, 1991), respectively.

The mutant *fve-b10* and the dominant negative transgenic line demonstrated 21% and 15% increase in total sugar content respectively and in turn estimated 137% and 118% higher sugar content per plant compared to their appropriate controls. Similarly, the mutant *fve-b10* and the dominant negative transgenic line contained respective 17% and 8% higher total lignin, and in turn estimated 131% and 104% more total lignin per plant compared to their appropriate controls. Although acid-insoluble and acid-soluble lignin content/plant was approximately increased by 197% and 43% for *fve-b10,* and by 139% and 31% for the transgenic line, respectively, only acid-insoluble lignin content was increased by 51% and 27% for *fve-b10* and the transgenic line, respectively. In contrast, acid-soluble lignin content was reduced by 27% and 30% for *fve-b10* and the transgenic line, respectively (all figures shown are statically significant at 5%). These results are summarized in Tables 17 and 18.

In conclusion, inhibition of *FVE* activity in *Arabidopsis* increases total cellulose and lignin content in the stems, also alters the composition of cellulose and lignin, and even results in a slight change in proportion of cellulose and lignin in favor of cellulose content. Since the sequence of *FVE* is very conserved evolutionarily among various plant species, the effect of inhibition of *FVE* in *Arabidopsis* ought to be highly conserved in other plant species.

The *fve-b10* mutant and the 35S-*fve*-*b10* transgenic plants both had greater levels of each sugar, albeit to different degrees.

**Table 17: Fiber analysis of fve-b10 and transgenic plants relative to WT**

| **Entry** | **Total Sugars** | **Total Lignin** | **Acid Insoluble Lignin** | **Acid Soluble Lignin** |
|---|---|---|---|---|
| *fve-b10* | 121% | 117% | 151% | 73% |
| 35S-*fve-b10* | 115% | 108% | 127% | 70% |
| WT Columbia | 100 % | 100 % | 100 % | 100 % |

**Table 18: Sugar monomer analysis expressed as % of control**

| **Entry** | **fucose** | **arabinose** | **rhamnose** | **galactose** | **glucose** | **xylose** | **mannose** |
|---|---|---|---|---|---|---|---|
| *fve-b10* | 438 | 204 | 105 | 160 | 114 | 131 | 108 |
| *35S-fve-b10* | 114 | 149 | 109 | 116 | 111 | 127 | 111 |

### Example 11: Western blot analysis of plants

Western blot analysis was performed on transgenic *Arabidopsis* lines 35S-HP-*FVE*-S2, 35S-HP-*FVE*-WD40-3 and 35S-*FVE* in the *fve-b10* mutant. Over-expression of *FVE* in *Arabidopsis* mutant *fve-b10* restores wild-type protein levels and reverts an *fve-b10* phenotype to essentially that of a wild-type plant. Inhibition of *FVE* expression in *Arabidopsis* by either 35S-HP-*FVE*-S2 or 35S-HP-*FVE*-WD40-3 reduces or eliminates detectable FVE protein.

The antibody produced by against *Arabidopsis FVE* can recognize maize-FVE expressed in transformed *Arabidopsis Atfve-b10* plants with the 35S-*ZmFVE* construct. The maize-FVE expression levels are also correlated with biomass production in the same manner of *AtFVE,* which was verified with the maize *FVE* functional genomics analysis.

### Example 12: Amino acid and nucleic acid sequence analysis of FVE and motif identification

Below is a comparison of FVE motif amino acid sequences and nucleotide sequences from selected plant species. Sequence homology and multiple sequence alignments were performed using ClustalW at http://www.ebi.ac.uk/clustalw/.

### Exemplar S1 Motifs

| | |
|---|---|
| MAIZE-2 | DGSVPNTLVIANCEWKPRVAAAEHISQFNEEARSP 36 SEQ ID NO:37 |
| SORGHUM | DGSVPNTLVIANCEWKPRVAAAEHISQFNEEARSP 36 SEQ ID NO:38 |
| MAIZE-1 | DGSVPNTLVIANCEWKPRVAAAEHISQFNEEARSP 36 SEQ ID NO:39 |
| SOYBEAN-1 | DGSVPNTLVIANCEWKPRVAAAEHISQFNEEARSP 36 SEQ ID NO:40 |
| ARABIDOPSIS | DGSVPNTLVIANCEWKPRVAAAEHISQFNEEARSP 36 SEQ ID NO:41 |
| PISUM-SATIVUM | DGSVPNTLVIANCEWKTRVAAAEHISQFNEEARSP 36 SEQ ID NO:42 |
| MEDICAGO-1 | DGSVPNTLVIANCEWKTRVAAAEHISQFNEEARSP 36 SEQ ID NO:43 |
| POPLAR | DGSVPNTLVIANCDWKSRVAAAEHISQFNEEARSP 36 SEQ ID NO:44 |
| COTTON | DGSVPNTLVIANCEWKPRVAAAEHISQFNEKTRSP 36 SEQ ID NO:45 |
| PETUNIA | DGSVPNTLVIANCEWKPRVAAAEHISQFNEESRSP 36 SEQ ID NO:46 |
| RICE-1 | DGTVPNTLVIANCEWKPRVAAAEHISQFNEEARSP 36 SEQ ID NO:47 |
| RICE-2 | DGTVPNTLVIANCEWKPRVAAAEHISQFNEEARSP 36 SEQ ID NO:48 |
| GRAPE | DGSVPNTLVIANCEWKPRVAAAEHIAQFNEEARSP 36 SEQ ID NO:49 |
| MEDICAGO-2 | DGTAPNTLVIATCEIVKPRVAAAEHIAMFNEEARSP 36 SEQ ID NO:50 |
| CANOLA | DGSVPNTLVIANCEWKPRVAAAEHISQFNEEARSP 36 SEQ ID NO:243 |
| SWITCHGRASS | DGSVPNTLVIANCEWKPRVAAAEHISQFNEEARSP 36 SEQ ID NO:244 |

| | |
|---|---|
| PISUM-SATIVUM | GATGGTAGTGTCCCAAATACTTTGGTGATTGCGAATTGTGAGGTTGTGAAAACTAGGGTT |
| MEDICAGO-1 | GATGGAAGTGTTCCGAATACTTTGGTGATTGCGAATTGTGAGGTTGTGAAGACTAGGGTT |
| SOYBEAN-1 | GATGGTAGTGTGCCGAATACTCTGGTGATTGCGAATTGCGAGGTTGTGAAGCCTAGGGTT |
| COTTON | GATGGTAGTGTTCCGAATACTCTTGTGATTGCTAATTGTGAAGTTGTCAAACCTAGGGTT |
| POPLAR | GATGGTAGTGTTCCAAATACTTTGGTCATTGCAAATTGTGATGTTGTCAAGTCTAGAGTT |
| PETUNIA | GATGGAAGTGTGCCAAACACTTTGGTTATAGCAAATTGTGAAGTTGTTAAACCTAGGGTT |
| ARABIDOPSIS | GATGGAAGTGTGCCCAATACTTTGGTCATAGCAAATTGTGAAGTTGTTAAGCCAAGGGTT |
| GRAPE | GATGGCAGTGTTCCGAATACCTTAGTTATAGCAAACTGTGAAGTTGTTAAACCTAGGGTA |
| MAIZE-2 | GATGGGAGTGTGCCTAATACTCTGGTTATCGCAAACTGTGAAGTTGTGAAACCAAGGGTT |
| SORGHUM | GATGGGAGTGTGCCTAATACTCTAGTTATCGCAAACTGTGAAGTTGTGAAGCCACGGGTT |
| MAIZE-1 | GATGGGAGTGTGCCTAATACTCTGGTTATCGCAAATTGTGAAGTTGTGAAACCTAGGGTT |
| RICE-1 | GATGGGACTGTCCCTAATACTCTGGTTATTGCAAATTGTGAAGTTGTCAAACCAAGGGTT |
| RICE-2 | GATGGGACTGTCCCTAATACTCTGGTTATTGCAAATTGTGAAGTTGTCAAACCAAGGGTT |
| MEDICAGO-2 | GATGGAACTGCTCCCAATACGCTTGTTATTGCTACTTGTGAAATTGTTAAACCTAGGGTT |
| CANOLA | GATGGCAGTGTGCCCAACACTCTAGTCATAGCGAATTGCGAAGTTGTTAAGCCAAGGGTC |
| SWITCHGRASS | GATGGGAGTGTGCCTAATACTCTGGTTATTGCGAACTGTGAAGTTGTCAAACCAAGGGTT |

| | | | |
|---|---|---|---|
| PISUM-SATIVUM | GCAGCTGCAGAGCATATTTCACAGTTTAATGAGGAGGCTCGCTCTCCA | 108 | SEQ ID NO:51 |
| MEDICAGO-1 | GCAGCTGCTGAACACATTTCTCAGTTTAATGAAGAGGCTCGCTCTCCA | 108 | SEQ ID NO:52 |
| SOYBEAN-1 | GCTGCTGCTGAGCACATTTCGCAGTTTAATGAAGAGGCGCGGTCCCCA | 108 | SEQ ID NO:53 |
| COTTON | GCTGCTGCTGAGCACATATCTCAGTTTAATGAAAAAACACGCTCACCA | 108 | SEQ ID NO:54 |
| POPLAR | GCTGCTGCGGAACACATATCTCAGTTTAATGAAGAAGCACGCTCTCCA | 108 | SEQ ID NO:55 |
| PETUNIA | GCAGCTGCAGAGCACATATCACAGTTCAATGAAGAATCACGATCTCCT | 108 | SEQ ID NO:56 |
| ARABIDOPSIS | GCTGCAGCAGAGCACATTTCTCAGTTCAATGAAGAAGCACGTTCTCCA | 108 | SEQ ID NO:57 |
| GRAPE | GCTGCCGCAGAGCATATTGCTCAGTTCAACGAGGAAGCGCGGTCACCC | 108 | SEQ ID NO:58 |
| MAIZE-2 | GCAGCTGCTGAACATATCTCGCAGTTTAACGAGGAAGCACGATCACCT | 108 | SEQ ID NO:59 |
| SORGHUM | GCAGCTGCTGAACATATCTCGCAGTTTAACGAGGAAGCACGATCACCT | 108 | SEQ ID NO:60 |
| MAIZE-1 | GCAGCTGCTGAACATATCTCACAGTTCAATGAGGAAGCACGGTCACCT | 108 | SEQ ID NO:61 |
| RICE-1 | GCAGCTGCTGAACATATATCTCAGTTCAATGAGGAAGCACGATCACCT | 108 | SEQ ID NO:62 |
| RICE-2 | GCAGCTGCTGAACATATATCTCAGTTCAATGAGGAAGCACGATCACCT | 108 | SEQ ID NO:63 |
| MEDICAGO-2 | GCTGCTGCTGAACATATTGCTATGTTTAATGAAGAGGCGCGTTCTCCA | 108 | SEQ ID NO:64 |
| CANOLA | GCTGCAGCAGAGCACATATCTCAGTTCAACGAAGAAGCACGTTCTCCA | 108 | SEQ ID NO:245 |
| SWITCHGRASS | GCAGCTGCTGAACATATCTCACAGTTCAATGAGGAAGCACGATCGCCT | 108 | SEQ ID NO:246 |

### Exemplar S2 motifs

| | | | |
|---|---|---|---|
| COTTON | IQDHITTMATDP-TKSPGSGGSIIKQN-KPGEGNDKAADGPS | 40 | SEQ ID NO:65 |
| POPLAR | IQDHITSSASDPATKSPGSGGSIIK---KTGDGSDKATDGPS | 39 | SEQ ID NO:66 |
| SOYBEAN-1 | IEDHITSAATDS-----KSGGSIIKQNSKSGEGNDKTADGPT | 37 | SEQ ID NO:67 |
| PISUM-SATIVUM | IEDHVTSAATD------KSGGSIIKPNSKSGEGNDKTVDSPS | 36 | SEQ ID NO:68 |
| MEDICAGO-1 | IEDHITSAATDS----NKSGGSIAK-----------TADSPT | 27 | SEQ ID NO:69 |
| MEDICAGO-2 | IHDHIATLATE-------EEPDVNE-GSNVGGNSEKAAQSPS | 34 | SEQ ID NO:70 |
| GRAPE | IQDHISTLAADP-----GSAKST----SKAGGGNDKPVESPS | 33 | SEQ ID NO:71 |
| PETUNIA | IQDHISTLSTDA-----QKPAGFIK----PATTSIKAGDNPS | 33 | SEQ ID NO:72 |
| ARABIDOPSIS | IQDHITTIG-TD----SKSSGSIIK---QTGEGTD-KNESPT | 33 | SEQ ID NO:73 |
| MAIZE-1 | IQDHISALGDSS-----SSPGASGS---KQSIKTANEKESPK | 34 | SEQ ID NO:74 |
| MAIZE-2 | IQDHISALGDSS-----SSPGASGS---KQSGKIANEKESPK | 34 | SEQ ID NO:75 |
| SORGHUM | IQDHISALGDSS-----SSPGASGS---KQSGKSATEKESPK | 34 | SEQ ID NO:76 |
| RICE-1 | IQDHISALGDSSKT--ESSPGASGS---K--GKTANDKDSPK | 35 | SEQ ID NO:77 |
| RICE-2 | IQDHISALGDSSKT--ESSPGASGS---K--GKTANDKDSPK | 35 | SEQ ID NO:78 |
| CANOLA | IQDHITTAGSTD----SKSSGSIIK---QTGEGGD-KTESPS | 34 | SEQ ID NO:223 |
| SWITCHGRASS | IQDHISALGDSS-----SSPGASGS---KQSGKTANEKESPK | 34 | SEQ ID NO:247 |

| | | |
|---|---|---|
| MEDICAGO-2 | -ATTCATGATCATATTGCAACTTTAGCTA------CAGA------------AGAAGAACC | 41 |
| GRAPE | -ATTCAGGATCACATCTCGACTTTGGCAG------CAGA------------TCCAGGGTC | 41 |
| PISUM-SATIVUM | -ATTGAAGACCATGTAACATCTGCTGCTA------CGGAC------AAGTCTGGTGGATC | 47 |
| COTTON | -ATCCAGGACCACATAACAACAATGGCTA------CAGACCCTACTAAATCTCCTGGATC | 53 |
| POPLAR | -ATCCAGGACCATATAACATCATCTGCCTCTGATCCAG---CAACTAAGTCTCCAGGATC | 56 |
| SOYBEAN-1 | -ATTGAAGACCATATAACATCTGCTGCCA------CAGACTCC---AAATCTGGTGGGTC | 50 |
| MEDICAGO-1 | -ATTGAAGACCATATAACATCTGCTGCTA------CAGACTCCAACAAGTCTGGTGGATC | 53 |
| PETUNIA | -ATTCAAGATCATATATCAACATTGAGCA------CAGATGCACAAAAACCTGCTGGTTT | 53 |
| ARABIDOPSIS | -ATCCAAGATCACATCACAACGATTGGGA------CAGATTCCA---AATCATCTGGATC | 50 |
| MAIZE-2 | -ATCCAAGACCACATATCTGCCCTTGGGGATTCCTCG---------TCTTCTCCCGGAGC | 50 |
| SORGHUM | -ATCCAAGACCACATATCTGCCCTTGGGGATTCCTCG---------TCTTCTCCTGGAGC | 50 |
| MAIZE-1 | -ATCCAAGACCATATATCTGCCCTTGGGGATTCCTCG---------TCTTCTCCTGGAGC | 50 |
| RICE-1 | -ATCCAAGACCACATATCTGCACTGGGAGATTCCTCGAAAACTGAATCTTCTCCAGGGGC | 59 |
| RICE-2 | -ATCCAAGACCACATATCTGCACTGGGAGATTCCTCGAAAACTGAATCTTCTCCAGGGGC | 59 |
| CANOLA | -ATCCAGGACCACATCACAACCGCTGGTAGTA---CAGACTCCA---AATCATCTGGATC | 53 |
| SWITCHGRASS | -ATCCAAGACCACATATCTGCCCTTGGGGATTCCTCA---------TCTTCTCCTGGAGC | 50 |
| MEDICAGO-2 | C---------GATGTTAATGAGGGCTCTAATGTTGGGGGAAATAGCGAGAAAGCTGCACA | 92 |
| GRAPE | ------------AGCAAAGAGTACCTCTAAGGCTGGTGGTGGTAATGATAAACCTGTAGA | 89 |
| PISUM-SATIVUM | C---------ATTATCAAACCGAACTCTAAATCTGGGGAAGGCAATGACAAAACTGTTGA | 98 |
| COTTON | TGGCGGATCAATCATCAAACAAAAC---AAGCCTGGGGAAGGTAATGACAAAGCTGCTGA | 110 |
| POPLAR | TGGTGGATCAATCATTAAAAAGA---------CTGGGGATGGCAGTGATAAAGCCACTGA | 107 |
| SOYBEAN-1 | A---------ATTATCAAACAAAACTCTAAATCTGGAGAAGGCAATGATAAAACTGCTGA | 101 |
| MEDICAGO-1 | C---------ATTGCCAAAACTGCTGATAGCCCTACT----------------------- | 81 |
| PETUNIA | C------------ATCAAGCCAG---------CCACTACATCTATT---AAGGCTGGTGA | 89 |
| ARABIDOPSIS | T---------ATCATCAAACAGA---------CTGGTGAAGGTACTGATAAGAATG---A | 89 |
| MAIZE-2 | A------TCTGGCAGCAAGCAGTCTGGCAAAATTGCAAATGAAAAGGAGAGTCCTAAA-- | 102 |
| SORGHUM | A------TCTGGCAGCAAGCAGTCTGGTAAATCTGCAACTGAAAAGGAGAGCCCTAAA-- | 102 |
| MAIZE-1 | A------TCTGGCAGCAAGCAGTCTATTAAAACTGCAAATGAAAAGGAGAGCCCTAAA-- | 102 |
| RICE-1 | A------TCAGGAAGCAAG------GGAAAAACTGCAAATGATAAGGATAGTCCTAAA-- | 105 |
| RICE-2 | A------TCAGGAAGCAAG------GGAAAAACTGCAAATGATAAGGATAGTCCTAAA-- | 105 |
| CANOLA | C---------ATCATCAAACAGA---------CTGGTGAAGGTGGTGATAAGACTG---A | 92 |
| SWITCHGRASS | A------TCTGGCAGCAAGCAGTCTGGCAAAACTGCGAATGAAAAGGAGAGTCCCAAA-- | 102 |

| | | | |
|---|---|---|---|
| MEDICAGO-2 | AAGCCCGTCT | 102 | SEQ ID NO: 79 |
| GRAPE | AAGCCCTTCT | 99 | SEQ ID NO: 80 |
| PISUM-SATIVUM | TAGCCCTTCT | 108 | SEQ ID NO:81 |
| COTTON | TGGGCCTTCT | 120 | SEQ ID NO:82 |
| POPLAR | TGGCCCTTCT | 117 | SEQ ID NO:83 |
| SOYBEAN-1 | TGGCCCTACT | 111 | SEQ ID NO:84 |
| MEDICAGO-1 | ---------- | | SEQ ID NO:85 |
| PETUNIA | TAATCCCTCT | 99 | SEQ ID NO:86 |
| ARABIDOPSIS | GAGTCCTACT | 99 | SEQ ID NO:87 |
| MAIZE-2 | ---------- | | SEQ ID NO:88 |
| SORGHUM | ---------- | | SEQ ID NO:89 |
| MAIZE-1 | ---------- | | SEQ ID NO:90 |
| RICE-1 | ---------- | | SEQ ID NO:91 |
| RICE-2 | ---------- | | SEQ ID NO:92 |
| CANOLA | GAGTCCTTCT | 102 | SEQ ID NO:233 |
| SWITCHGRASS | ---------- | | SEQ ID NO:248 |

### Exemplar WD40-1 Motifs

| | | | |
|---|---|---|---|
| POPLAR | FVKKYKTIIHPGEVNRIRELPQNSKIVATHTDSPDVLIWD | 40 | SEQ ID NO:121 |
| ARABIDOPSIS | FVKKYKTIIHPGEVNRIRELPQNSKIVATHTDSPDVLIWD | 40 | SEQ ID NO:122 |
| PISUM-SATIVUM | FVKKYKTIIHPGEVNRIRELPQNSKIVATHTDSPDVLIWD | 40 | SEQ ID NO:123 |
| MEDICAGO-1 | FVKKYKTIIHPGEVNRIRELPQNSKIVATHTDSPDVLIWD | 40 | SEQ ID NO:124 |
| MAIZE-1 | FVKKYKTIVHPGEVNRIRELPQNSKIIATHTDSPDVLIWD | 40 | SEQ ID NO:125 |
| SORGHUM | FVKKYKTIVHPGEVNRIRELPQNSKIIATHTDSPDVLIWD | 40 | SEQ ID NO:126 |
| MAIZE-2 | FVKKYKTIVHPGEVNRIRELPQNSKIIATHTDSPDVLVWD | 40 | SEQ ID NO:127 |
| RICE-1 | FVKKYKTIIHPGEVNRIRELPQNSKIIATHTDSPDVLIWD | 40 | SEQ ID NO:128 |
| RICE-2 | FVKKYKTIIHPGEVNRIRELPQNSKIIATHTDSPDVLIWD | 40 | SEQ ID NO:129 |
| SOYBEAN-1 | FVKKYKTIIHPGEVNRIRELPQNSKIVATHTDSPDVLVWD | 40 | SEQ ID NO:130 |
| GRAPE | FVKKFKTIIHPGEVNRIRELPQNSKIVATHTDSPDVLIWD | 40 | SEQ ID NO:131 |
| COTTON | FVKKYKTIIHPGEVNRIKELPQSSRIVATHTDSPDVLIWD | 40 | SEQ ID NO:132 |
| PETUNIA | FVKKYKTIIHPGEVNRIRELPQNKNIVATHTDSPEVLIWD | 40 | SEQ ID NO:133 |
| MEDICAGO-2 | FVKKVKTILHPGEVNRIRELPSNTNIVATHTDSPNVMIWN | 40 | SEQ ID NO:134 |
| CANOLA | FVKKFKTIIHPGEVNRIRELPQNSKIIATHTDSPDVLIWD | 40 | SEQ ID NO:236 |
| SWITCHGRASS | FVKKYKTIVHPGEVNRIRELPQNSKIIATHTDSPDVLIWD | 40 | SEQ ID NO:249 |

| | | |
|---|---|---|
| PISUM-SATIVUM | TTTGTTAAGAAGTACAAGACCATCATACATCCCGGGGAGGTGAACAGAATTAGGGAATTG | 60 |
| MEDICAGO-1 | TTTGTCAAGAAGTACAAGACTATCATACATCCAGGCGAGGTGAACAGAATTAGGGAATTG | 60 |
| SOYBEAN-1 | TTTGTGAAGAAGTACAAGACCATCATACATCCTGGTGAGGTAAACAGAATTAGGGAATTG | 60 |
| POPLAR | TTTGTTAAGAAGTACAAGACCATCATACATCCTGGAGAGGTAAACAGAATCAGAGAACTC | 60 |
| COTTON | TTTGTGAAGAAGTACAAAACCATCATACATCCTGGAGAAGTCAACAGAATCAAGGAACTT | 60 |
| ARABIDOPSIS | TTTGTGAAGAAGTACAAGACCATCATTCACCCTGGAGAGGTTAACAGAATCAGGGAACTC | 60 |
| MAIZE-2 | TTTGTAAAGAAGTACAAGACTATAGTTCATCCTGGTGAAGTTAACAGAATCAGGGAGCTT | 60 |
| SORGHUM | TTTGTTAAGAAGTACAAGACCATAGTTCATCCTGGTGAAGTTAACAGAATCAGGGAGCTT | 60 |
| MAIZE-1 | TTTGTAAAGAAGTACAAAACTATAGTTCATCCTGGTGAGGTTAACAGAATCAGGGAACTT | 60 |
| RICE-1 | TTTGTAAAGAAGTACAAGACTATAATTCATCCAGGCGAGGTGAACAGGATTAGGGAGCTT | 60 |
| RICE-2 | TTTGTAAAGAAGTACAAGACTATAATTCATCCAGGCGAGGTGAACAGGATTAGGGAGCTT | 60 |
| GRAPE | TTTGTAAAGAAGTTCAAAACAATTATACATCCAGGAGAGGTGAACCGAATCAGGGAACTG | 60 |
| PETUNIA | TTTGTCAAGAAGTACAAAACCATTATACACCCAGGGGAGGTTAACAGAATCAGAGAGCTT | 60 |
| MEDICAGO-2 | TTTGTTAAGAAGGTTAAAACTATTCTTCATCCCGGTGAGGTAAATAGAATCAGAGAACTC | 60 |
| CANOLA | TTTGTGAAGAAGTTCAAGACCATCATTCACCCTGGAGAGGTTAACCGAATCAGGGAACTC | 60 |
| SWITCHGRASS | TTTGTGAAGAAGTATAAGACTATAGTTCATCCTGGTGAGGTTAACAGAATCAGGGAGCTT | 60 |

| | | | |
|---|---|---|---|
| PISUM-SATIVUM | CCGCAAAATTCTAAGATAGTGGCTACTCACACAGACAGCCCTGATGTTCTCATTTGGGAT | 120 | SEQ ID NO:135 |
| MEDICAGO-1 | CCACAAAATTCTAAGATTGTGGCCACTCACACAGACAGCCCTGATGTTCTCATTTGGGAT | 120 | SEQ ID NO:136 |
| SOYBEAN-1 | CCACAAAATTCCAAGATAGTGGCTACACATACAGACAGCCCTGATGTCCTTGTTTGGGAT | 120 | SEQ ID NO:137 |
| POPLAR | CCCCAGAATAGTAAGATAGTGGCTACTCATACTGACAGCCCTGATGTTCTTATATGGGAT | 120 | SEQ ID NO:138 |
| COTTON | CCACAGAGCTCTAGGATTGTGGCAACTCACACTGATAGTCCTGATGTTCTTATTTGGGAT | 120 | SEQ ID NO:139 |
| ARABIDOPSIS | CCACAGAATAGTAAGATTGTTGCTACTCACACCGACAGTCCTGATGTTCTCATTTGGGAT | 120 | SEQ ID NO:140 |
| MAIZE-2 | CCACAGAACAGTAAGATCATAGCCACACACACTGACAGTCCAGATGTACTTGTTTGGGAT | 120 | SEQ ID NO:141 |
| SORGHUM | CCACAGAACAGTAAGATCATAGCCACTCACACTGACAGTCCAGATGTACTTATTTGGGAT | 120 | SEQ ID NO:142 |
| MAIZE-1 | CCACAGAACAGTAAGATCATAGCCACTCACACTGACAGTCCAGATGTACTTATTTGGGAT | 120 | SEQ ID NO:143 |
| RICE-1 | CCGCAGAACAGTAAGATCATTGCCACTCATACCGACAGCCCAGATGTTCTCATTTGGGAT | 120 | SEQ ID NO:144 |
| RICE-2 | CCGCAGAACAGTAAGATCATTGCCACTCATACCGACAGCCCAGATGTTCTCATTTGGGAT | 120 | SEQ ID NO:145 |
| GRAPE | CCACAGAATAGTAAGATAGTGGCCACACACACTGACAGTCCTGATGTCCTCATTTGGGAT | 120 | SEQ ID NO:146 |
| PETUNIA | CCTCAAAATAAAAACATAGTGGCAACCCATACTGATAGTCCTGAAGTTCTAATTTGGGAT | 120 | SEQ ID NO:147 |
| MEDICAGO-2 | CCGTCAAATACTAATATAGTTGCCACACATACAGATAGTCCAAATGTTATGATTTGGAAT | 120 | SEQ ID NO:148 |
| CANOLA | CCACAAAACAGTAAGATTATTGCTACTCACACCGACAGTCCTGATGTTCTCATTTGGGAT | 120 | SEQ ID NO:235 |
| SWITCHGRASS | CCACAGAACAGTAAGATCATAGCCACTCACACCGACAGTCCAGATGTACTTATTTGGGAT | 120 | SEQ ID NO:250 |

### Exemplar WD40-2 motifs

| | | | |
|---|---|---|---|
| SOYBEAN-1 | SRPDLILTGHQDNAEFALAMCPTEPYVLSGGKDKTVVLWS | 40 | SEQ ID NO:93 |
| PISUM-SATIVUM | SRPDLILTGHQDNAEFALAMCPTEPYVLSGGKDKTVVLWS | 40 | SEQ ID NO:94 |
| MEDICAGO-1 | SRPDLILTGHQDNAEFALAMCPTQPYVLSGGKDKTVVLWS | 40 | SEQ ID NO:95 |
| POPLAR | SRPDLILTGHQDNAEFALAMCPTDPYVLSGGKDKFVVLWS | 40 | SEQ ID NO:96 |
| COTTON | SRPDLILTGHQDNAEFALAMCPTEPYVLSGGKDKSVVLWS | 40 | SEQ ID NO:97 |
| MAIZE-1 | SRPDLILTGHKENAEFALAMCPAEPYVLSGGKDKSVVLWS | 40 | SEQ ID NO:98 |
| SORGHUM | SRPDLILTGHKENAEFALAMCPAEPYVLSGGKDKSVVLWS | 40 | SEQ ID NO:99 |
| RICE-1 | SRPDLILRGHKDIAEFALAMCPAEPYVLSGGKDKSVVWWS | 40 | SEQ ID NO:100 |
| RICE-2 | SRPDLILRGHKDIAEFALAMCPAEPYVLSGGKDKSVVWWS | 40 | SEQ ID NO:101 |
| MAIZE-2 | SRPDLILTGHQENAEFALAMCPAEPYVLSGGKDKFVVLWS | 40 | SEQ ID NO:102 |
| ARABIDOPSIS | SRPDLILTGHQDNAEFALAMCPTEPFVLSGGKDKSVVLWS | 40 | SEQ ID NO:103 |
| PETUNIA | SRPDLTLIGHSENAEFALAMCPTEPFVLSGGKDKSVVLWS | 40 | SEQ ID NO:104 |
| GRAPE | KVLFLILTGHKDNAEFALAMCPTEPLVLSGGKDKSVVLWS | 40 | SEQ ID NO:105 |
| MEDICAGO-2 | SIPDLVLTGHKDNAEFALAMCSTEPFVLSGGRDKLVVLWS | 40 | SEQ ID NO:106 |
| CANOLA | SRPDLVLTGHQDNAEFXLAMCPTEPFVLSGGKDKSVVLWS | 40 | SEQ ID NO:228 |
| SWITCHGRASS | SRPDLILTGHQENAEFALAMCPAEPYVLSGGKDKSVVLWS | 40 | SEQ ID NO:251 |

| | | |
|---|---|---|
| PISUM-SATIVUM | TCTCGTCCAGATT-TGATATTGACCGGACACCAAGACAATGCTGAGTTTGCTCTTGCGAT | 59 |
| MEDICAGO-1 | TCTCGTCCAGATC-TGATATTGACTGGACACCAAGATAATGCAGAGTTTGCTCTTGCAAT | 59 |
| SOYBEAN-1 | TCTCGTCCTGATT-TGATATTGACTGGACACCAAGATAATGCGGAATTTGCTCTTGCAAT | 59 |
| COTTON | TCTCGCCCAGATT-TGATTTTGACTGGGCATCAAGATAATGCTGAATTTGCTCTTGCAAT | 59 |
| POPLAR | TCTCGTCCAGATT-TGATTTTGACTGGACATCAAGACAATGCTGAGTTTGCCCTTGCAAT | 59 |
| SORGHUM | TCTCGCCCTGATC-TGATATTAACGGGACATAAGGAAAATGCGGAATTTGCGCTTGCTAT | 59 |
| MAIZE-1 | TCTCGCCCTGATC-TGATATTAACAGGACATAAGGAAAATGCGGAATTTGCGCTTGCCAT | 59 |
| MAIZE-2 | TCTCGTCCTGATC-TGATATTAACGGGACACCAGGAAAATGCAGAATTTGCGCTTGCCAT | 59 |
| RICE-1 | TCTCGTCCTGATT-TGATATTAAGAGGACATAAGGATATTGCTGAGTTTGCGCTTGCTAT | 59 |
| RICE-2 | TCTCGCCCTGATT-TGATATTAAGAGGACATAAGGATATTGCTGAGTTTGCGCTTGCTAT | 59 |
| PETUNIA | TCACGCCCAGATT-TGACATTGATTGGACATAGTGAGAATGCAGAATTTGCACTGGCAAT | 59 |
| ARABIDOPSIS | TCCCGTCCAGATT-TGATACTAACTGGGCACCAAGATAATGCTGAATTTGCTCTTGCCAT | 59 |
| MEDICAGO-2 | TCCATCCCAGACT-TGGTATTAACTGGACATAAGGATAATGCTGAATTTGCACTAGCTAT | 59 |
| GRAPE | -AAGGTTTTGTTCCTGATATTAACTGGGCATAAAGATAATGCAGAATTTGCTCTTGCCAT | 59 |
| CANOLA | TCCCGTCCTGATT-TGGTATTAACTGGACATCAAGACAATGCTGAATTCSCTCTTGCAAT | 59 |
| SWITCHGRASS | TCTCGGCCTGATC-TGATATTAACAGGACATCAGGAAAATGCTGAATTCGCGCTTGCCAT | 59 |

| | | |
|---|---|---|
| PISUM-SATIVUM | GTGCCCAACTGAGCCTTATGTCCTTTCAGGAGGAAAAGATAAAACAGTGGTGTTGTGGAG | 119 |
| MEDICAGO-1 | GTGCCCTACTCAGCCCTATGTGCTTTCTGGAGGAAAAGACAAAACAGTCGTGTTGTGGAG | 119 |
| SOYBEAN-1 | GTGCCCAACTGAACCCTATGTTCTTTCAGGAGGAAAGGACAAAACAGTGGTGTTGTGGAG | 119 |
| COTTON | GTGTCCAACTGAGCCTTATGTGCTCTCTGGAGGGAAGGACAAATCAGTGGTTTTGTGGAG | 119 |
| POPLAR | GTGCCCAACTGATCCCTATGTGCTTTCTGGAGGGAAGGACAAGTTCGTAGTTTTGTGGAG | 119 |
| SORGHUM | GTGCCCAGCAGAACCATATGTCCTATCAGGAGGAAAGGACAAATCTGTTGTCTTGTGGAG | 119 |
| MAIZE-1 | GTGTCCAGCAGAACCATATGTCCTATCAGGAGGAAAGGACAAATCTGTTGTCTTGTGGAG | 119 |
| MAIZE-2 | GTGTCCAGCAGAACCATATGTCCTGTCAGGAGGAAAGGACAAATTTGTTGTCTTGTGGAG | 119 |
| RICE-1 | GTGCCCAGCTGAGCCATATGTGTTATCTGGAGGAAAAGACAAATCTGTTGTATGGTGGAG | 119 |
| RICE-2 | GTGCCCAGCTGAGCCATATGTGTTATCTGGAGGAAAAGACAAATCTGTTGTATGGTGGAG | 119 |
| PETUNIA | GTGCCCCACTGAACCCTTTGTGCTCTCTGGAGGAAAGGACAAATCTGTGGTACTGTGGAG | 119 |
| ARABIDOPSIS | GTGCCCAACGGAACCCTTTGTGCTCTCCGGAGGCAAGGACAAGTCAGTTGTTTTGTGGAG | 119 |
| MEDICAGO-2 | GTGTTCAACTGAGCCCTTTGTTCTTTCCGGAGGGAGAGACAAGCTTGTGGTGTTATGGAG | 119 |
| GRAPE | GTGTCCAACTGAACCATTGGTGCTCTCTGGAGGCAAGGATAAGTCTGTGGTGTTGTGGAG | 119 |
| CANOLA | GTGCCCAACCGAACCCTTTGTCCTCTCTGGAGGCAAAGACAAGTCAGTTGTTTTGTGGAG | 119 |
| SWITCHGRASS | GTGTCCAGCAGAACCATATGTACTGTCAGGAGGAAAGGACAAATCTGTTGTCTTGTGGAG | 119 |

| | | | |
|---|---|---|---|
| PISUM-SATIVUM | T | 120 | SEQ ID NO:107 |
| MEDICAGO-1 | T | 120 | SEQ ID NO:108 |
| SOYBEAN-1 | T | 120 | SEQ ID NO:109 |
| COTTON | C | 120 | SEQ ID NO:110 |
| POPLAR | T | 120 | SEQ ID NO:111 |
| SORGHUM | C | 120 | SEQ ID NO:112 |
| MAIZE-1 | C | 120 | SEQ ID NO:113 |
| MAIZE-2 | C | 120 | SEQ ID NO:114 |
| RICE-1 | C | 120 | SEQ ID NO:115 |
| RICE-2 | C | 120 | SEQ ID NO:116 |
| PETUNIA | T | 120 | SEQ ID NO:117 |
| ARABIDOPSIS | T | 120 | SEQ ID NO:118 |
| MEDICAGO-2 | T | 120 | SEQ ID NO:119 |
| GRAPE | T | 120 | SEQ ID NO:120 |
| CANOLA | T | 120 | SEQ ID NO:234 |
| SWITCHGRASS | C | 120 | SEQ ID NO:252 |

### Exemplar WD40-3 D Motifs

| | | | |
|---|---|---|---|
| GRAPE | IGARGIYQGHDDTVEDVQFCPLSAQEFCSVGDDSCLILWD | 40 | SEQ ID NO:149 |
| PETUNIA | IQARGIFQGHEDTVEDVQFCPSSSQEFCSVGDDSCLILWD | 40 | SEQ ID NO:150 |
| PISUM-SATIVUM | VGPRGIYSGHDDTVEDVAFCPSSAQEFCSVGDDSCLILWD | 40 | SEQ ID NO:151 |
| MEDICAGO-1 | VGPRGIYSGHEDTVEDVAFCPSSAQEFCSVGDDSCLILWD | 40 | SEQ ID NO:152 |
| POPLAR | VGPRGIYQGHEDTVEDVAFCPSSAQEFCSVGDDSCLILWD | 40 | SEQ ID NO:153 |
| ARABIDOPSIS | VGPRGVYHGHEDTVEDVAFSPTSAQEFCSVGDDSCLILWD | 40 | SEQ ID NO:154 |
| COTTON | LGPRGVFCGHEDTVEDVTFCPSSAQEFCSVGDDSCLILWD | 40 | SEQ ID NO:155 |
| SOYBEAN-1 | VGPRGIYCGHEDTVEDVTFCPSSAQEFCSVGDDSCLILWD | 40 | SEQ ID NO:156 |
| MEDICAGO-2 | VGARGVYRGHKDTVEDVQFCPSSAQEFCSVGDDSCLILWD | 40 | SEQ ID NO:157 |
| MAIZE-1 | VDPRGIFHGHDSTVEDVQFCPSSAQEFCSVGDDACLILWD | 40 | SEQ ID NO:158 |
| MAIZE-2 | VDPRGIFHGHDSTVEDVQFCPSSAQEFCSVGDDACLILWD | 40 | SEQ ID NO:159 |
| RICE-1 | VDPRGIFLGHDSTVEDVQFCPSSAQEFCSVGDDSCLILWD | 40 | SEQ ID NO:160 |
| RICE-2 | VDPRGIFLGHDSTVEDVQFCPSSAQEFCSVGDDSCLILWD | 40 | SEQ ID NO:161 |
| SORGHUM | VDPRGIFCG------------------------------- | 9 | SEQ ID NO:162 |
| CANOLA | LGPRGVYHGHDDTVEDVAFSPTSAQEFCSVGDDSCLILWD | 40 | SEQ ID NO:255 |
| SWITCHGRASS | VDPRGIFHGHDSTVEDVQFCPSSAQEFCSVGDDACLILWD | 40 | SEQ ID NO:253 |

| | | |
|---|---|---|
| PISUM-SATIVUM | GTCGGGCCAAGAGGTATCTACTCTGGGCACGATGATACTGTTGAAGATGTGGCCTTTTGC | 60 |
| MEDICAGO-1 | GTCGGACCAAGAGGTATCTACTCTGGGCATGAGGATACTGTTGAAGACGTGGCTTTTTGC | 60 |
| SOYBEAN-1 | GTTGGACCACGAGGCATTTATTGTGGGCATGAGGATACAGTTGAAGATGTGACTTTCTGC | 60 |
| MAIZE-2 | GTTGATCCTAGAGGTATATTCCATGGCCATGACAGTACTGTTGAAGATGTTCAGTTCTGC | 60 |
| MAIZE-1 | GTTGATCCTAGAGGTATATTTCATGGACATGACAGCACGGTTGAAGATGTTCAGTTCTGC | 60 |
| RICE-1 | GTTGATCCTCGTGGTATCTTTCTTGGCCACGACAGTACTGTTGAAGATGTCCAGTTCTGC | 60 |
| RICE-2 | GTTGATCCTCGTGGTATCTTTCTTGGCCACGACAGTACTGTTGAAGATGTCCAGTTCTGC | 60 |
| PETUNIA | ATTCAAGCTCGTGGAATCTTCCAAGGCCATGAGGATACCGTTGAAGATGTTCAGTTCTGC | 60 |
| COTTON | TTAGGTCCTCGTGGTGTCTTCTGTGGGCATGAGGATACAGTTGAAGATGTTACATTCTGT | 60 |
| POPLAR | GTTGGACCGCGAGGTATCTACCAAGGGCATGAGGATACAGTTGAAGACGTGGCATTCTGT | 60 |
| GRAPE | ATAGGAGCACGTGGTATCTACCAAGGGCATGATGATACTGTTGAAGATGTGCAATTCTGC | 60 |
| ARABIDOPSIS | GTTGGCCCACGAGGTGTATATCATGGCCATGAAGATACAGTTGAAGATGTGGCATTCAGC | 60 |
| MEDICAGO-2 | GTTGGAGCAAGGGGCGTCTACCGGGGTCATAAAGACACTGTTGAAGATGTGCAGTTTTGC | 60 |
| SORGHUM | GTTGATCCTAGAGGTATATTCTGTGGG--------------------------------- | 27 |
| CANOLA | CTTGGCCCGCGAGGYGTATATCATGGCCATGATBATACCGTTGAAGATGTCGCTTTCAGC | 60 |
| SWITCHGRASS | GTTGATCCTCGGGGTATATTCCATGGACATGACAGCACTGTTGAAGATGTTCAGTTCTGC | 60 |

| | | | |
|---|---|---|---|
| PISUM-SATIVUM | CCTTCTAGTGCGCAGGAATTCTGTAGTGTTGGAGATGATTCTTGTCTCATATTATGGGAT | 120 | SEQ ID NO:163 |
| MEDICAGO-1 | CCTTCTAGTGCGCAGGAGTTCTGTAGTGTTGGAGATGATTCTTGTCTCATATTATGGGAT | 120 | SEQ ID NO:164 |
| SOYBEAN-1 | CCATCTAGTGCACAGGAGTTCTGTAGTGTTGGAGATGATTCTTGTCTCATCTTATGGGAT | 120 | SEQ ID NO:165 |
| MAIZE-2 | CCTTCCAGTGCGCAGGAGTTTTGTAGTGTGGGTGATGATGCTTGTCTTATTCTGTGGGAT | 120 | SEQ ID NO:166 |
| MAIZE-1 | CCTTCCAGTGCGCAGGAGTTTTGTAGTGTGGGTGATGATGCTTGTCTTATTCTGTGGGAT | 120 | SEQ ID NO:167 |
| RICE-1 | CCTTCTAGTGCACAGGAGTTTTGTAGTGTAGGCGATGATTCTTGTCTTATTCTTTGGGAT | 120 | SEQ ID NO:168 |
| RICE-2 | CCTTCTAGTGCACAGGAGTTTTGTAGTGTGGGCGATGATTCTTGTCTTATTCTTTGGGAT | 120 | SEQ ID NO:169 |
| PETUNIA | CCATCAAGTTCACAGGAATTCTGTAGTGTTGGTGATGATTCATGTCTCATTCTATGGGAT | 120 | SEQ ID NO:170 |
| COTTON | CCATCAAGTGCACAAGAGTTTTGTAGTGTAGGTGATGATTCCTGCCTCATACTATGGGAT | 120 | SEQ ID NO:171 |
| POPLAR | CCATCCAGTGCGCAGGAGTTTTGTAGTGTAGGAGATGATTCTTGCCTTATATTATGGGAT | 120 | SEQ ID NO:172 |
| GRAPE | CCATTAAGCGCACAGGAGTTCTGTAGCGTAGGTGATGATTCTTGCCTTATCTTATGGGAT | 120 | SEQ ID NO:173 |
| ARABIDOPSIS | CCGACGAGTGCACAAGAATTCTGCAGTGTTGGTGATGATTCTTGCCTTATACTATGGGAT | 120 | SEQ ID NO:174 |
| MEDICAGO-2 | CCCTCAAGTGCACAGGAGTTCTGTAGTGTAGGTGATGATTCTTGTCTCATACTCTGGGAT | 120 | SEQ ID NO:175 |
| SORGHUM | ------------------------------------------------------------ | | SEQ ID NO:176 |
| CANOLA | CCCACTAGTGCACAAGAGTTCTGCAGTGTCGGTGACGATTCTTGCCTTATACTATGGGAT | 120 | SEQ ID NO:256 |
| SWITCHGRASS | CCTTCCAGTGCACAGGAATTCTGTAGTGTGGGTGATGATGCTTGTCTTATTCTCTGGGAT | 120 | SEQ ID NO:254 |

### Exemplar WD40-4 D Motifs

| | | | |
|---|---|---|---|
| ARABIDOPSIS | NPVTKVEKAHDADLHCVDWNPHDDNLILTGSADNTVRLFD | 40 | SEQ ID NO:257 |
| PISUM-SATIVUM | SPWKVEKAHNADLHCVDWNPHDDNLILTGSADNSVRLFD | 40 | SEQ ID NO:258 |
| PETUNIA | SPWKVEKAHNADLHCVDWNPHDGNFIITGSADNSVRLFD | 40 | SEQ ID NO:259 |
| SOYBEAN-1 | SPWKVEKAHNADLHCVDWNPHDDNLILTGSADNSVRMFD | 40 | SEQ ID NO:260 |
| MEDICAGO-1 | SPWKVEKAHDADLHCVDWNPHDDNLILTGSADNSIRMFD | 40 | SEQ ID NO:261 |
| POPLAR | SPAIKVERAHNADLHCVDWNPQDDNLILTGSADTSVCMFD | 40 | SEQ ID NO:262 |
| COTTON | GPTVKVEKAHNADLHCVDWNPHDDNLILTGSADHTVRMFD | 40 | SEQ ID NO:263 |
| GRAPE | TPAIKVEKAHNADLHCVDWNPHDINLILTGSADNTVRMFD | 40 | SEQ ID NO:264 |
| MAIZE-1 | APAVKVEKAHSGDVHCVDWNPLDVNYILTGSADNSVRMWD | 40 | SEQ ID NO:265 |
| SORGHUM | ----------------VDWNPLDVNYILTGSADNSVRMWD | 24 | SEQ ID NO:266 |
| MAIZE-2 | DPAVKVEKAHSGDVHCVDWNPLDVNYILTGSADNSVRMWD | 40 | SEQ ID NO:267 |
| SWITCHGRASS | SPAVKVEKAHSGDVHCVDWNPLDVNYILTGSADNSVRMWD | 40 | SEQ ID NO:268 |
| RICE-1 | GPAVKVEKAHGGDVHCVDWNLHDVNYILTGSADNSVRMWD | 40 | SEQ ID NO:269 |
| RICE-2 | GPAVKVEKAHGGDVHCVDWNLHDVNYILTG---------- | 30 | SEQ ID NO:270 |
| MEDICAGO-2 | FPAVKVEKAHDGDVHCVDWNTHDINFILTGSADNTVRMFD | 40 | SEQ ID NO:271 |
| CANOLA | SPVTKVEKAHDADLHCVDWNPHDDNLILTGSADNTVRLYD | 40 | SEQ ID NO:272 |

| | | |
|---|---|---|
| PISUM-SATIVUM | AGTCCTGTTGTTAAGGTTGAAAAAGCTCATAATGCCGATCTTCACTGTGTTGACTGGAAT | 60 |
| MEDICAGO-1 | AGTCCTGTGGTTAAGGTTGAAAAAGCTCATGATGCTGATCTTCACTGTGTTGACTGGAAT | 60 |
| SOYBEAN-1 | AGCCCTGTGGTTAAGGTTGAGAAAGCTCATAATGCTGATCTTCACTGTGTGGACTGGAAT | 60 |
| ARABIDOPSIS | AACCCTGTCACGAAGGTTGAAAAAGCGCATGATGCTGATCTTCATTGTGTTGATTGGAAT | 60 |
| CANOLA | AGCCCTGTCACGAAGGTTGAAAAGGCGCACGATGCTGATCTTCATTGTGTCGATTGGAAC | 60 |
| COTTON | GGCCCCACTGTCAAGGTTGAAAAGGCACATAATGCTGATCTCCATTGTGTTGATTGGAAC | 60 |
| POPLAR | AGCCCAGCTATAAAGGTTGAAAGGGCACATAATGCTGATCTTCACTGTGTTGATTGGAAT | 60 |
| PETUNIA | AGTCCAGTTGTAAAGGTTGAAAAAGCTCATAATGCTGATCTCCACTGTGTTGACTGGAAT | 60 |
| MEDICAGO-2 | TTTCCAGCTGTCAAGGTTGAGAAGGCACATGATGGAGATGTACATTGCGTTGATTGGAAT | 60 |
| GRAPE | ACTCCAGCCATCAAGGTCGAGAAAGCTCATAATGCTGATCTTCACTGTGTTGATTGGAAT | 60 |
| MAIZE-2 | GACCCAGCTGTTAAGGTTGAGAAAGCTCACAGTGGAGATGTTCATTGTGTTGATTGGAAT | 60 |
| SORGHUM | ------------------------------------------------GTTGATTGGAAT | 12 |
| MAIZE-1 | GCCCCAGCTGTTAAGGTTGAGAAAGCTCACAGTGGAGATGTTCATTGTGTTGATTGGAAT | 60 |
| SWITCHGRASS | AGCCCGGCTGTTAAGGTTGAGAAAGCTCATAGTGGGGATGTTCATTGTGTGGATTGGAAT | 60 |
| RICE-1 | GGCCCAGCTGTTAAGGTTGAGAAAGCTCATGGTGGTGATGTTCATTGTGTTGACTGGAAC | 60 |
| RICE-2 | GGCCCAGCTGTTAAGGTTGAGAAAGCTCATGGTGGTGATGTTCATTGTGTTGACTGGAAC | 60 |

| | | | |
|---|---|---|---|
| PISUM-SATIVUM | CCCCATGATGATAATCTGATTCTTACTGGGTCGGCAGATAATTCTGTTCGCTTGTTTGAT | 120 | SEQ ID NO:273 |
| MEDICAGO-1 | CCCCATGATGATAATCTGATTCTTACTGGGTCGGCAGATAATTCTATTCGCATGTTTGAT | 120 | SEQ ID NO:274 |
| SOYBEAN-1 | CCCCATGATGATAATCTGATTCTTACTGGGTCAGCAGATAATTCTGTTCGCATGTTTGAT | 120 | SEQ ID NO:275 |
| ARABIDOPSIS | CCTCATGACGACAATCTGATCCTGACAGGGTCAGCAGACAACACTGTCCGGTTGTTTGAT | 120 | SEQ ID NO:276 |
| CANOLA | CCTCATGATGACAATCTGATCCTGACAGGGTCTGCAGACAACACTGTTCGGTTGTATGAT | 120 | SEQ ID NO:277 |
| COTTON | CCCCATGATGACAATCTTATCCTGACTGGGTCTGCAGATCATACTGTACGTATGTTTGAT | 120 | SEQ ID NO:278 |
| POPLAR | CCTCAAGATGATAATCTCATTTTAACTGGGTCTGCAGATACTTCTGTTTGCATGTTTGAT | 120 | SEQ ID NO:279 |
| PETUNIA | CCTCATGATGGCAACTTTATTATCACTGGATCTGCAGATAATTCTGTCCGCTTGTTTGAT | 120 | SEQ ID NO:280 |
| MEDICAGO-2 | ACTCATGACATCAATTTTATTCTGACTGGCTCTGCTGATAACACAGTTCGCATGTTTGAT | 120 | SEQ ID NO:281 |
| GRAPE | CCTCATGACATAAATCTTATTCTAACTGGATCGGCTGACAATACTGTTCGCATGTTTGAT | 120 | SEQ ID NO:282 |
| MAIZE-2 | CCCCTTGATGTTAACTATATCTTAACTGGTTCTGCCGATAACTCTGTCCGAATGTGGGAT | 120 | SEQ ID NO:283 |
| SORGHUM | CCCCTTGACGTTAACTATATCTTAACTGGTTCTGCCGATAACTCTGTCCGAATGTGGGAT | 72 | SEQ ID NO:284 |
| MAIZE-1 | CCCCTTGATGTTAACTATATCTTAACTGGGTCTGCCGATAACTCTGTCCGAATGTGGGAT | 120 | SEQ ID NO:285 |
| SWITCHGRASS | CCGCTTGATGTTAACTATATCTTAACTGGTTCTGCTGATAACTCTGTCCGTATGTGGGAT | 120 | SEQ ID NO:286 |
| RICE-1 | CTCCATGATGTTAACTATATCTTAACTGGTTCTGCGGATAATTCTGTCCGTATGTGGGAC | 120 | SEQ ID NO:287 |
| RICE-2 | CTCCATGATGTTAACTATATCTTAACTGGT------------------------------ | 90 | SEQ ID NO:288 |

### Exemplar WD40-5 D Motifs

| | | | |
|---|---|---|---|
| SWITCHGRASS | GSPIHKFEGHKAAVLCVQWSPDRASVFGSSAEDGFLNVWD | 40 | SEQ ID NO:289 |
| MAIZE-2 | GSPIHKFEGHKAAVLCVQWSPDRASVFGSSAEDGFLNVWD | 40 | SEQ ID NO:290 |
| MAIZE-1 | GSPIHKFEGHKAAVLCVQWSPDRASVFGSSAEDGFLNVWD | 40 | SEQ ID NO:291 |
| SORGHUM | SSPIHKFEGHKAAVLCVQWSPDRASVFGSSAEDGFLNVWD | 40 | SEQ ID NO:292 |
| RICE-1 | GIPVHKFEGHKAAVLCVQWSPDKASVFGSSAEDGFLNVWD | 40 | SEQ ID NO:293 |
| RICE-2 | ------------------WSPDKASVFGSSAEDGFLNVWD | 22 | SEQ ID NO:294 |
| GRAPE | GSPIHTFEGHTAAVLCVQWSPDKASIFGSSAEDGILNLWN | 40 | SEQ ID NO:295 |
| COTTON | GSPIYKFEGHKAAVLCVQWSPDKSSVFGSSAEDGLLNIWD | 40 | SEQ ID NO:296 |
| ARABIDOPSIS | GSPIYKFEGHKAAVLCVQWSPDKSSVFGSSAEDGLLNIWD | 40 | SEQ ID NO:297 |
| CANOLA | GTPIYKFEGHKAAVLCVQWSPDKSSVFGSSAEDGLLNIWD | 40 | SEQ ID NO:298 |
| SOYBEAN-1 | GSPIHKFEGHKAAVLCVQWSPDKSSVFGSSAEDGLLNIWD | 40 | SEQ ID NO:299 |
| PISUM-SATIVUM | GSPIHKFEAHKAAVLCVQWSPDKSSVFGSSAEDGLLNIWD | 40 | SEQ ID NO:300 |
| MEDICAGO-1 | GSPIHKFEAHKAAVLCVQWSPDKSSVFGSSAEDGLLNIWD | 40 | SEQ ID NO:301 |
| POPLAR | GLPVYKFEGHKAAVLCVQWSPDKASVFGSSAEDGLLNIWD | 40 | SEQ ID NO:302 |
| PETUNIA | GSPVHIFENHKAAVLCVQWCPDRSSVFGSTAEDGRLNIWD | 40 | SEQ ID NO:303 |
| MEDICAGO-2 | GSPVYKFEGHDEPVLCVQWNPAKSSVFGSGAEDGIINIWD | 40 | SEQ ID NO:304 |

| | | |
|---|---|---|
| RICE-1 | GGCATTCCAGTTCACAAATTTGAGGGTCATAAAGCTGCTGTTCTTTGTGTTCAGTGGTCA | 60 |
| RICE-2 | ------------------------------------------------------------------------TGGTCA | 6 |
| SORGHUM | AGTTCTCCAATTCATAAATTTGAGGGCCATAAAGCTGCTGTTCTTTGTGTTCAGTGGTCA | 60 |
| SWITCHGRASS | GGTTCTCCAATTCACAAATTTGAGGGCCATAAAGCTGCTGTTCTTTGTGTCCAGTGGTCA | 60 |
| MAIZE-1 | GGTTCTCCAATTCACAAATTTGAAGGCCATAAAGCTGCTGTTCTTTGTGTTCAGTGGTCA | 60 |
| MAIZE-2 | GGTTCTCCAATTCACAAATTTGAGGGCCATAAAGCTGCTGTTCTTTGTGTTCAGTGGTCA | 60 |
| PISUM-SATIVUM | GGGTCTCCTATTCATAAATTTGAGGCTCATAAAGCTGCCGTTCTTTGTGTTCAGTGGTCT | 60 |
| MEDICAGO-1 | GGGTCTCCTATCCACAAATTTGAGGCTCATAAGGCTGCTGTCCTTTGTGTTCAGTGGTCT | 60 |
| SOYBEAN-1 | GGGTCACCCATCCATAAATTTGAGGGTCACAAAGCTGCTGTTCTTTGTGTTCAGTGGTCT | 60 |
| COTTON | GGATCACCCATTTACAAGTTTGAGGGTCATAAAGCTGCTGTTCTATGCGTGCAGTGGTCT | 60 |
| POPLAR | GGTTTGCCAGTCTATAAATTTGAGGGTCACAAAGCTGCTGTTCTCTGTGTACAGTGGTCT | 60 |
| PETUNIA | GGCTCACCAGTCCATATCTTTGAAAATCACAAAGCAGCAGTTCTTTGTGTGCAGTGGTGT | 60 |
| ARABIDOPSIS | GGTTCGCCTATTTACAAATTTGAGGGACACAAAGCTGCTGTTCTTTGTGTTCAGTGGTCT | 60 |
| CANOLA | GGTACGCCTATTTACAAATTTGAAGGCCACAAAGCTGCTGTTCTTTGCGTTCAGTGGTCT | 60 |
| MEDICAGO-2 | GGGTCTCCTGTTTATAAATTTGAAGGCCATGATGAACCAGTCCTCTGTGTACAGTGGAAT | 60 |
| GRAPE | GGATCACCAATCCATACATTTGAGGGCCATACTGCTGCTGTCCTTTGTGTACAGTGGTCT | 60 |

| | | | |
|---|---|---|---|
| RICE-1 | CCTGACAAGGCATCTGTATTTGGAAGCTCTGCGGAAGACGGCTTCTTAAATGTGTGGGAT | 120 | SEQ ID NO:305 |
| RICE-2 | CCTGACAAGGCATCTGTATTTGGAAGCTCTGCGGAAGACGGTTTCTTAAATGTGTGGGAT | 66 | SEQ ID NO:306 |
| SORGHUM | CCTGACAGAGCATCTGTTTTTGGAAGTTCTGCGGAAGATGGTTTCTTAAACGTGTGGGAT | 120 | SEQ ID NO:307 |
| SWITCHGRASS | CCTGACAGAGCATCTGTTTTCGGAAGTTCTGCGGAAGATGGTTTCTTAAATGTGTGGGAT | 120 | SEQ ID NO:308 |
| MAIZE-1 | CCTGACAGAGCATCTGTTTTTGGAAGTTCTGCAGAAGATGGTTTCTTAAACGTGTGGGAT | 120 | SEQ ID NO:309 |
| MAIZE-2 | CCTGACAGAGCATCTGTTTTTGGAAGTTCTGCAGAAGATGGTTTCTTAAATGTTTGGGAC | 120 | SEQ ID NO:310 |
| PISUM-SATIVUM | CCAGACAAATCATCTGTATTTGGAAGTTCAGCAGAAGACGGTCTCTTAAACATTTGGGAT | 120 | SEQ ID NO:311 |
| MEDICAGO-1 | CCAGACAAATCATCTGTATTTGGAAGTTCAGCAGAAGATGGTCTCCTGAACATTTGGGAT | 120 | SEQ ID NO:312 |
| SOYBEAN-1 | CCAGACAAATCATCTGTATTTGGAAGTTCAGCTGAAGATGGTCTCTTAAACATTTGGGAC | 120 | SEQ ID NO:313 |
| COTTON | CCAGACAAATCATCTGTATTTGGCAGTTCTGCTGAGGATGGGCTCTTGAACATTTGGGAC | 120 | SEQ ID NO:314 |
| POPLAR | CCAGATAAGGCATCTGTTTTTGGGAGTTCTGCGGAGGATGGTCTCTTGAATATTTGGGAT | 120 | SEQ ID NO:315 |
| PETUNIA | CCAGACAGGTCCTCTGTATTTGGGAGTACTGCAGAGGATGGTCGTTTGAATATTTGGGAT | 120 | SEQ ID NO:316 |
| ARABIDOPSIS | CCTGATAAGTCATCCGTCTTTGGGAGCTCTGCAGAAGATGGTCTCTTGAACATCTGGGAT | 120 | SEQ ID NO:317 |
| CANOLA | CCTGATAAGTCATCTGTTTTTGGGAGTTCCGCGGAAGATGGTCTCTTGAACATCTGGGAT | 120 | SEQ ID NO:318 |
| MEDICAGO-2 | CCTGCTAAATCATCTGTATTTGGAAGTGGTGCCGAAGATGGAATTATAAACATCTGGGAC | 120 | SEQ ID NO:319 |
| GRAPE | CCGGACAAGGCTTCGATCTTTGGGAGTTCTGCAGAAGATGGTATCTTAAACCTCTGGAAT | 120 | SEQ ID NO:320 |

### Exemplar WD40-6 D Motifs

| | | | |
|---|---|---|---|
| MEDICAGO-2 | SPGLFFRHAGHRDKWDFHWNASDPWTIVSVSDDCASTGGGGTLQIWR | 48 | SEQ ID NO:321 |
| GRAPE | PPGLFFRHAGHRDKWDFHWNASDPWTIVSVSDDGESTGGGGTLQIWR | 48 | SEQ ID NO:322 |
| SOYBEAN-1 | PPGLFFQHAGHRDKWDFHWNAYDPWTIVSVSDDCESTGGGGTLQIWR | 48 | SEQ ID NO:323 |
| PISUM-SATIVUM | PPGLFFQHAGHRDKWDFHWNAYDPWTIVSVSDDCESTGGGGTLQIWR | 48 | SEQ ID NO:324 |
| MEDICAGO-1 | PPGLFFQHAGHRDKWDFHWNAHDPWTLVSVSDDCESTGGGGTLQIWR | 48 | SEQ ID NO:325 |
| COTTON | SAGLFFQHAGHRDKWDFHWNAFDPWTVVSVFDDCETTGGGGTLQIWG | 48 | SEQ ID NO:326 |
| POPLAR | PAGLFFQHAGHRDKWDFHWNASDPWTVVSVSDDCDTTGGGGTLQIWR | 48 | SEQ ID NO:327 |
| ARABIDOPSIS | PAGLFFQHAGHRDKWDFHWNASDPWTIVSVSDDCETTGGGGTLQIWR | 48 | SEQ ID NO:328 |
| CANOLA | PAGLFFQHAGHRDKWDFHWNAEDPWTIVSVSDDCETTGGGGTLQIWR | 48 | SEQ ID NO:329 |
| SWITCHGRASS | PAGLFFQHAGHRDKIVDFHWNSSDPWTIVSVSDDGESTGGGGTLQIWR | 48 | SEQ ID NO:330 |
| RICE-2 | PAGLFFQHAGHRDKIVDFHWNSSDPWTIVSVSDDGESTGGGGTLQIWR | 48 | SEQ ID NO:331 |
| RICE-1 | PAGLFFQHAGHRDKIVDFHWNSSDPWTIVSVSDDGESTGGGGTLQIWR | 48 | SEQ ID NO:332 |
| SORGHUM | PAGLFFQHAGHRDKIVDFHWNSSDPWTIVSVSDDGESTGGGGTLQIWR | 48 | SEQ ID NO:333 |
| MAIZE-2 | PAGLFFQHAGHRDKIVDFHWNSSDPWTIVSVSDDGESTGGGGTLQIWR | 48 | SEQ ID NO:334 |
| MAIZE-1 | PAGLFFQHAGHRDKIVDFHWNSSDPWTIVSVSDDGESTGGGGTLQIWR | 48 | SEQ ID NO:335 |
| PETUNIA | APGLFFQHAGHRDKIVDFHWNVADPWTIVSVSDDCDSTGGGGTLQIWR | 48 | SEQ ID NO:336 |

| | | |
|---|---|---|
| PISUM-SATIVUM | CCTCCAGGGTTGTTTTTCCAACATGCTGGTCATAGAGACAAAGTTGTTGACTTCCATTGG | 60 |
| MEDICAGO-1 | CCTCCAGGGTTGTTTTTCCAACATGCTGGTCATAGAGACAAAGTTGTTGACTTTCACTGG | 60 |
| SOYBEAN-1 | CCTCCAGGGTTGTTTTTTCAACATGCAGGTCATAGGGATAAAGTTGTTGACTTCCATTGG | 60 |
| COTTON | TCTGCGGGACTGTTTTTCCAGCACGCTGGACACAGGGACAAAGTTGTTGACTTCCATTGG | 60 |
| POPLAR | CCTGCAGGATTGTTTTTCCAGCATGCTGGGCACAGGGATAAAGTTGTTGATTTCCATTGG | 60 |
| MEDICAGO-2 | TCCCCTGGTTTATTCTTTCGTCATGCAGGGCATAGGGATAAGGTTGTTGACTTTCATTGG | 60 |
| GRAPE | CCTCCAGGTTTATTCTTCAGACATGCTGGCCATAGGGATAAGGTCGTGGACTTCCATTGG | 60 |
| PETUNIA | GCTCCAGGTTTATTTTTCCAGCATGCTGGGCACAGGGATAAAATTGTTGACTTCCACTGG | 60 |
| MAIZE-1 | CCAGCCGGGCTTTTCTTTCAGCACGCTGGTCATAGGGATAAGATTGTAGACTTCCACTGG | 60 |
| MAIZE-2 | CCAGCTGGGCTTTTCTTTCAGCATGCTGGTCATAGGGATAAGATCGTAGACTTCCACTGG | 60 |
| SORGHUM | CCAGCTGGGCTTTTCTTCCAGCATGCTGGTCATAGGGATAAGATTGTAGACTTCCACTGG | 60 |
| SWITCHGRASS | CCAGCTGGGCTTTTCTTTCAACATGCTGGTCACAGGGATAAGATTGTAGACTTCCACTGG | 60 |
| RICE-1 | CCTGCTGGGCTTTTCTTTCAACATGCTGGTCATAGGGATAAGATTGTAGACTTCCACTGG | 60 |
| RICE-2 | CCTGCTGGGCTTTTCTTTCAACATGCTGGTCATAGGGATAAGATTGTAGACTTCCACTGG | 60 |
| ARABIDOPSIS | CCCGCTGGGCTCTTCTTCCAGCATGCTGGTCACAGGGACAAAGTTGTTGATTTCCACTGG | 60 |
| CANOLA | CCGGCTGGTCTCTTCTTCCAGCATGCTGGTCACAGGGACAAAGTTGTTGATTTCCACTGG | 60 |

| | | | |
|---|---|---|---|
| PISUM-SATIVUM | AATGCTTATGATCCATGGACAATTGTAAGTGTGTCTGATGATTGTGAAAGTACTGGTGGA | 120 | SEQ ID NO:337 |
| MEDICAGO-1 | AATGCACATGATCCATGGACACTTGTTAGTGTGTCTGATGATTGCGAAAGTACTGGTGGA | 120 | SEQ ID NO:338 |
| SOYBEAN-1 | AATGCATATGATCCATGGACGATTGTTAGTGTGTCTGATGACTGTGAAAGTACTGGAGGA | 120 | SEQ ID NO:339 |
| COTTON | AATGCATTTGATCCATGGACTGTTGTTAGTGTGTTTGATGACTGTGAAACAACTGGTGGA | 120 | SEQ ID NO:340 |
| POPLAR | AATGCATCTGATCCTTGGACGGTGGTTAGTGTCTCTGATGACTGTGATACCACTGGCGGG | 120 | SEQ ID NO:341 |
| MEDICAGO-2 | AATGCATCTGATCCATGGACAATTGTTAGTGTATCTGATGATTGTGCAAGCACTGGTGGA | 120 | SEQ ID NO:342 |
| GRAPE | AATGCATCGGATCCATGGACAATCGTTAGCGTATCTGATGATGGTGAAAGTACTGGTGGA | 120 | SEQ ID NO:343 |
| PETUNIA | AATGTGGCTGATCCATGGACAATTGTAAGTGTATCTGATGACTGTGACTCCACAGGTGGA | 120 | SEQ ID NO:344 |
| MAIZE-1 | AATTCGTCAGATCCTTGGACAATTGTCAGTGTCTCTGATGATGGCGAGAGCACTGGTGGA | 120 | SEQ ID NO:345 |
| MAIZE-2 | AATTCGTCAGATCCTTGGACAATTGTCAGTGTCTCAGATGATGGTGAGAGCACTGGTGGA | 120 | SEQ ID NO:346 |
| SORGHUM | AATTCGTCAGATCCTTGGACAATTGTCAGTGTATCTGATGATGGTGAGAGCACTGGTGGA | 120 | SEQ ID NO:347 |
| SWITCHGRASS | AATTCATCGGATCCTTGGACAATCGTCAGTGTCTCAGATGATGGTGAGAGCACCGGTGGA | 120 | SEQ ID NO:348 |
| RICE-1 | AATTCTTCGGATCCTTGGACTATTGTGAGTGTGTCTGATGATGGTGAGAGTACTGGTGGA | 120 | SEQ ID NO:349 |
| RICE-2 | AATTCTTCGGATCCTTGGACTATTGTGAGTGTGTCTGATGATGGTGAGAGTACTGGTGGA | 120 | SEQ ID NO:350 |
| ARABIDOPSIS | AATGCTTCAGACCCTTGGACTATTGTCAGTGTTTCTGATGACTGTGAGACTACTGGTGGA | 120 | SEQ ID NO:351 |
| CANOLA | AATGCAGAGGACCCTTGGACTATTGTCAGTGTTTCTGATGACTGCGAGACTACTGGTGGA | 120 | SEQ ID NO:352 |

### Example 13: Functional confirmation of FVE homologues and useful motifs

Expression vectors comprising a candidate sequence are introduced into *Arabidopsis* and assessed for biomass production, stem diameter, delayed flowering and other physiological phenotypes associated with the *FVE* mutant. Sequences disclosed as SEQ ID NO:1, 3, 5, 6, 7, 9, 11,13, 15, 17, 19, 21, 23, 25, 27, 29, 31, 33, 35, 206, 216, 220, 239, and 241or a portion thereof, such as SEQ ID NO:51-64, 245, 246, 79-92, 233, 248, 107-120, 234, 252, 135-148, 235, 250, 163-176, 256, 254, 273-288, 305-320 and 337-352 or an expression cassette such as SEQ ID NO:372-379, are expressed in *Arabidopsis* plants, and biomass productivity assessed as described herein. Optionally, the expression of *FVE* genes can be evaluated in any transformable species, for example, *Brassica*, *maize,* cotton, soybean or rice.

## Claims

1. A method of increasing biomass accumulation in a plant relative to a wild-type plant, comprising the steps of:
a) providing a nucleic acid construct operably linked to a promoter, wherein said nucleic acid construct encodes an antisense oligonucleotide of FVE, an siRNA of FVE, an shRNA of FVE, an miRNA of FVE, an artificial miRNA of FVE, or a dominant negative mutant of FVE;
b) inserting said promoter-operably-linked nucleic acid construct into a vector;
c) transforming a plant, a tissue culture, or a plant cell with said vector to obtain a transformed plant, a transformed tissue culture, or a transformed plant cell with decreased FVE expression or activity; and
d) growing said transformed plant or regenerating a plant from said transformed tissue culture or transformed plant cell, wherein a plant having increased biomass accumulation relative to a wild-type plant is produced.

2. The method of claim 1, wherein said nucleic acid construct comprises an *FVE*-S1 motif.

3. The method of claim 2, wherein said nucleic acid construct comprises an *FVE*-S1 motif nucleic acid sequence selected from SEQ ID NOs: 51-64, 245, and 246.

4. The method of claim 1, wherein said nucleic acid construct comprises an *FVE*-S2 motif.

5. The method of claim 4, wherein said nucleic acid construct comprises an *FVE*-S2 motif nucleic acid sequence selected from SEQ ID NOs: 79-92, 233, and 248.

6. The method of claim 1, wherein said nucleic acid construct comprises an *FVE-*WD40 motif.

7. The method of claim 6, wherein said nucleic acid construct comprises an *FVE*-WD40-1 motif nucleic acid sequence selected from SEQ ID NOs: 135-148, 235, and 250.

8. The method of claim 6, wherein said nucleic acid construct comprises an *FVE*-WD40-2 motif nucleic acid sequence selected from SEQ ID NOs: 107-120, 234, and 252.

9. The method of claim 6, wherein said nucleic acid construct comprises an *FVE*-WD40-3 motif nucleic acid sequence selected from SEQ ID NOs: 163-176, 256, and 254.

10. The method of claim 6, wherein said nucleic acid construct comprises an *FVE*-WD40-4 motif nucleic acid sequence selected from SEQ ID NOs: 273-288.

11. The method of claim 6, wherein said nucleic acid construct comprises an *FVE*-WD40-5 motif nucleic acid sequence selected from SEQ ID NOs: 305-320.

12. The method of claim 6, wherein said nucleic acid construct comprises an *FVE*-WD40-6 motif nucleic acid sequence selected from SEQ ID NOs: 337-352.

13. The method of claim 1, wherein said nucleic acid construct comprises a sequence of at least 21 nucleotides in length.

14. A stably transformed transgenic plant having increased biomass accumulation relative to a wild-type plant, produced by a method comprising the steps of:
a) providing a nucleic acid construct operably linked to a promoter, wherein said nucleic acid construct encodes an antisense oligonucleotide of FVE, an shRNA of FVE, an miRNA of FVE, an artificial miRNA of FVE, or a dominant negative mutant of FVE;
b) inserting said promoter-operably-linked nucleic acid construct into a vector;
c) transforming a plant, a tissue culture or a plant cell with said vector to obtain a transformed plant, a transformed tissue culture or a transformed plant cell with decreased FVE expression or activity; and
d) growing a transformed plant or regenerating a plant from said transformed tissue culture or transformed plant cell,
wherein a stably transformed transgenic plant having increased biomass accumulation relative to a wild-type plant is produced.

## Patentansprüche

1. Verfahren zum Erhöhen der Biomasseakkumulation in einer Pflanze im Verhältnis zu einer Wildtyp-Pflanze, umfassend die Schritte:
a) das Bereitstellen eines mit einem Promotor funktionell verbundenen Nukleinsäurekonstrukts, wobei das Nukleinsäurekonstrukt ein Antisense-Oligonukleotid von FVE, eine siRNA von FVE, eine shRNA von FVE, eine miRNA von FVE, eine künstliche miRNA von FVE oder eine dominant-negative Mutante von FVE codiert;
b) das Einfügen des mit dem Promotor funktionell verbundenen Nukleinsäurekonstrukts in einen Vektor;
c) das Transformieren einer Pflanze, einer Gewebekultur oder einer Pflanzenzelle mit dem Vektor, um eine transformierte Pflanze, eine transformierte Gewebekultur oder eine transformierte Pflanzenzelle mit herabgesetzter FVE-Expression oder -Aktivität zu erhalten; und
d) das Wachsenlassen der transformierten Pflanze oder das Regenerieren einer Pflanze aus der transformierten Gewebekultur oder transformierten Pflanzenzelle, wobei eine Pflanze hergestellt wird, die eine erhöhte Biomasseakkumulation im Verhältnis zu einer Wildtyp-Pflanze aufweist.

2. Verfahren nach Anspruch 1, wobei das Nukleinsäurekonstrukt ein *FVE*-S1-Motiv umfasst.

3. Verfahren nach Anspruch 2, wobei das Nukleinsäurekonstrukt eine *FVE*-S1-Motiv-Nukleinsäuresequenz, ausgewählt aus SEQ ID NO: 51-64, 245 und 246, umfasst.

4. Verfahren nach Anspruch 1, wobei das Nukleinsäurekonstrukt ein *FVE*-S2-Motiv umfasst.

5. Verfahren nach Anspruch 4, wobei das Nukleinsäurekonstrukt eine *FVE*-S2-Motiv-Nukleinsäuresequenz, ausgewählt aus SEQ ID NO: 79-92, 233 und 248, umfasst.

6. Verfahren nach Anspruch 1, wobei das Nukleinsäurekonstrukt ein *FVE*-WD40-Motiv umfasst.

7. Verfahren nach Anspruch 6, wobei das Nukleinsäurekonstrukt eine *FVE*-WD40-1-Motiv-Nukleinsäuresequenz, ausgewählt aus SEQ ID NO: 135-148, 235 und 250, umfasst.

8. Verfahren nach Anspruch 6, wobei das Nukleinsäurekonstrukt eine *FVE*-WD40-2-Motiv-Nukleinsäuresequenz, ausgewählt aus SEQ ID NO: 107-120, 234 und 252, umfasst.

9. Verfahren nach Anspruch 6, wobei das Nukleinsäurekonstrukt eine *FVE*-WD40-3-Motiv-Nukleinsäuresequenz, ausgewählt aus SEQ ID NO: 163-176, 256 und 254, umfasst.

10. Verfahren nach Anspruch 6, wobei das Nukleinsäurekonstrukt eine *FVE*-WD40-4-Motiv-Nukleinsäuresequenz, ausgewählt aus SEQ ID NO: 273-288, umfasst.

11. Verfahren nach Anspruch 6, wobei das Nukleinsäurekonstrukt eine *FVE*-WD40-5-Motiv-Nukleinsäuresequenz, ausgewählt aus SEQ ID NO: 305-320, umfasst.

12. Verfahren nach Anspruch 6, wobei das Nukleinsäurekonstrukt eine *FVE*-WD40-6-Motiv-Nukleinsäuresequenz, ausgewählt aus SEQ ID NO: 337-352, umfasst.

13. Verfahren nach Anspruch 1, wobei das Nukleinsäurekonstrukt eine Sequenz mit einer Länge von wenigstens 21 Nukleotiden umfasst.

14. Stabil transformierte transgene Pflanze, die eine erhöhte Biomasseakkumulation im Verhältnis zu einer Wildtyp-Pflanze aufweist, hergestellt durch ein Verfahren, umfassend die Schritte:
a) das Bereitstellen eines mit einem Promotor funktionell verbundenen Nukleinsäurekonstrukts, wobei das Nukleinsäurekonstrukt ein Antisense-Oligonukleotid von FVE, eine shRNA von FVE, eine miRNA von FVE, eine künstliche miRNA von FVE oder eine dominant-negative Mutante von FVE codiert;
b) das Einfügen des mit dem Promotor funktionell verbundenen Nukleinsäurekonstrukts in einen Vektor;
c) das Transformieren einer Pflanze, einer Gewebekultur oder einer Pflanzenzelle mit dem Vektor, um eine transformierte Pflanze, eine transformierte Gewebekultur oder eine transformierte Pflanzenzelle mit herabgesetzter FVE-Expression oder -Aktivität zu erhalten; und
d) das Wachsenlassen einer transformierten Pflanze oder das Regenerieren einer Pflanze aus der transformierten Gewebekultur oder transformierten Pflanzenzelle,
wobei eine stabil transformierte transgene Pflanze hergestellt wird, die eine erhöhte Biomasseakkumulation im Verhältnis zu einer Wildtyp-Pflanze aufweist.

## Revendications

1. Procédé pour augmenter l'accumulation de biomasse dans une plante par rapport à une plante de type sauvage, comprenant les étapes consistant à :
a) mettre à disposition un produit de recombinaison d'acide nucléique en liaison fonctionnelle avec un promoteur, où ledit produit de recombinaison d'acide nucléique code pour un oligonucléotide antisens de FVE, un ARNsi de FVE, un ARNsh de FVE, un ARNmi de FVE, un ARNmi artificiel de FVE, ou un mutant dominant négatif de FVE ;
b) insérer ledit produit de recombinaison d'acide nucléique en liaison fonctionnelle avec un promoteur dans un vecteur ;
c) transformer une plante, une culture tissulaire, ou une cellule végétale avec ledit vecteur afin d'obtenir une plante transformée, une culture tissulaire transformée, ou une cellule végétale transformée présentant une expression ou une activité FVE réduite ; et
d) cultiver ladite plante transformée ou régénérer une plante à partir de ladite culture tissulaire transformée ou cellule végétale transformée, où une plante présentant une accumulation de biomasse accrue par rapport à une plante de type sauvage est produite.

2. Procédé selon la revendication 1, dans lequel ledit produit de recombinaison d'acide nucléique comprend un motif *FVE*-S1.

3. Procédé selon la revendication 2, dans lequel ledit produit de recombinaison d'acide nucléique comprend une séquence d'acide nucléique à motif *FVE*-S1 sélectionnée parmi SEQ ID NO: 51-64, 245, et 246.

4. Procédé selon la revendication 1, dans lequel ledit produit de recombinaison d'acide nucléique comprend un motif *FVE*-S2.

5. Procédé selon la revendication 4, dans lequel ledit produit de recombinaison d'acide nucléique comprend une séquence d'acide nucléique à motif *FVE*-S2 sélectionnée parmi SEQ ID NO: 79-92, 233, et 248.

6. Procédé selon la revendication 1, dans lequel ledit produit de recombinaison d'acide nucléique comprend un motif *FVE*-WD40.

7. Procédé selon la revendication 6, dans lequel ledit produit de recombinaison d'acide nucléique comprend une séquence d'acide nucléique à motif *FVE*-WD40-1 sélectionnée parmi SEQ ID NO: 135-148, 235, et 250.

8. Procédé selon la revendication 6, dans lequel ledit produit de recombinaison d'acide nucléique comprend une séquence d'acide nucléique à motif *FVE*-WD40-2 sélectionnée parmi SEQ ID NO: 107-120, 234, et 252.

9. Procédé selon la revendication 6, dans lequel ledit produit de recombinaison d'acide nucléique comprend une séquence d'acide nucléique à motif *FVE*-WD40-3 sélectionnée parmi SEQ ID NO: 163-176, 256, et 254.

10. Procédé selon la revendication 6, dans lequel ledit produit de recombinaison d'acide nucléique comprend une séquence d'acide nucléique à motif *FVE*-WD40-4 sélectionnée parmi SEQ ID NO: 273-288.

11. Procédé selon la revendication 6, dans lequel ledit produit de recombinaison d'acide nucléique comprend une séquence d'acide nucléique à motif *FVE*-WD40-5 sélectionnée parmi SEQ ID NO: 305-320.

12. Procédé selon la revendication 6, dans lequel ledit produit de recombinaison d'acide nucléique comprend une séquence d'acide nucléique à motif *FVE*-WD40-6 sélectionnée parmi SEQ ID NO: 337-352.

13. Procédé selon la revendication 1, dans lequel ledit produit de recombinaison d'acide nucléique comprend une séquence d'une longueur d'au moins 21 nucléotides.

14. Plante transgénique stablement transformée présentant une accumulation de biomasse accrue par rapport à une plante de type sauvage, produite par un procédé comprenant les étapes consistant à :
a) mettre à disposition un produit de recombinaison d'acide nucléique en liaison fonctionnelle avec un promoteur, où ledit produit de recombinaison d'acide nucléique code pour un oligonucléotide antisens de FVE, un ARNsh de FVE, un ARNmi de FVE, un ARNmi artificiel de FVE, ou un mutant dominant négatif de FVE ;
b) insérer ledit produit de recombinaison d'acide nucléique en liaison fonctionnelle avec un promoteur dans un vecteur ;
c) transformer une plante, une culture tissulaire ou une cellule végétale avec ledit vecteur afin d'obtenir une plante transformée, une culture tissulaire transformée ou une cellule végétale transformée présentant une expression ou une activité FVE réduite ; et
d) cultiver une plante transformée ou régénérer une plante à partir de ladite culture tissulaire transformée ou cellule végétale transformée,
où une plante transgénique stablement transformée présentant une accumulation de biomasse accrue par rapport à une plante de type sauvage est produite.
